# EUROPEAN PATENT APPLICATION

(11) **EP 2 078 753 A2**
(43) Date of publication of application: **15.07.2009**
(21) Application number: 09153378.6
(22) Date of filing: 23.12.2003
(51) Int. Cl.: C12N 15/29, C12N 15/82, C07K 14/415, A01H 5/00

(54) **Cell proliferation-related polypeptides and uses therefor**

(30) Priority: 26.12.2002 US 436565 P
(62) Divisional of application: 03808558.5
(71) Applicant: Syngenta Participations AG, 4058 Basel (CH)
(72) Inventor: Cooper, Bret, Laurel, MD 20708 (US)
(74) Representative: Adams, Harvey Vaughan John

(57) **Abstract**

Disclosed are proteins, and nucleic acids encoding such proteins, involved in or associated with cell proliferation, senesence, differentiation, development, and stress response in plants. Also disclosed are uses for such proteins.

## Description

### Cross Reference To Related Applications

This application is based on and claims priority to United States Provisional Application Serial Number 60/436,565, filed December 26, 2002, which is herein incorporated by reference in its entirety.

### Technical Field

The presently disclosed subject matter relates, in general, to transgenic plants. More particularly, the presently disclosed subject matter relates to cell proliferation-related polypeptides, nucleic acid molecues encoding the polypeptides, and uses thereof.

### Sequence Listing Provided on CD-R

The Sequence Listing associated with the instant disclosure has been submitted as a 1.5 MB file on CD-R (in triplicate) instead of on paper. Each CD-R is marked in indelible ink to identify the Applicants, Title, File Name (1392-10-19 PCT.ST25.txt)), Creation Date (December 23, 2003), Computer System (IBM-PC/MS-DOS/MS-Windows), and Docket No. (1392-10-19 PCT). The Sequence Listing submitted on CD-R is hereby incorporated by reference into the instant disclosure.

| | Table of Abbreviations |
|---|---|
| 2,4-D | - 2,4-dichlorophenoxyacetic acid |
| 53BP1 | - p53-binding protein |
| ABA | - abscisic acid |
| ABC | - ATP-binding cassettes |
| ADPGlc | - ADP-glucose |
| AMV | - Alfalfa Mosaic Virus |
| AOBP | - ascorbate oxidase promoter binding protein |
| AOS | - active oxygen species |
| APC | - Adenomatous Polyposis Coli |
| APP | - amyloid precursor protein |
| BAP | - benzylamino purine |
| bp | - basepair(s) |
| BiP | - human immunoglobulin heavy-chain binding protein |
| BR | - brassinosteroid |
| BRCT | - BRCA1 C-terminus |
| BRI1 | - brassinosteroid-insensitive 1 |
| bZIP | - basic leucine zipper domain |
| CalS | - callose synthase |
| CaM | - calmodulin |
| CaMV | - cauliflower mosaic virus |
| cDNA | - complementary DNA |
| CDK | - cyclin dependent kinase |
| CNS | - central nervous system |
| CPO | - coproporphyrinogen III oxidase |
| CRT | - calreticulin |
| DHFR | - dihydrofolate reductase |
| EDTA | - ethylenediamine tetraacetic acid |
| eIF3 | - eukaryotic initiation factor 3 |
| eIF4E | - eukaryotic initiation factor 4E |
| ELISAs | - enzyme-linked immunosorbent assays |
| EMCV | - encephalomyocarditis virus |
| EPSP | - 5-enolpyruvylshikimate-3-phosphate |
| EPSPS | - 5-enolpyruvylshikimate-3-phosphate synthase |
| ER | - endoplasmic reticulum |
| ESTs | - Expressed Sequence Tags |
| FPD | - Functional Protein Domain |
| FTZ-F1 | - fushitarazu factor 1 |
| GA | - gibberellin |
| GUS | - β-glucuronidase |
| HD | - histone deacetylase |
| HLH | - helix-loop-helix |
| HR | - hypersensitive response |
| HSPs | - heat shock proteins |
| IAA | - indole acetic acid |
| INCENP | - inner centromere protein |
| JA | - jasmonic acid |
| kb | - kilobase(s) |
| KCBP | - kinesin-like calmodulin-binding protein |
| KNOX | - knotted-like homeobox |
| LR | - local resistance |
| MCMV | - Maize Chlorotic Mottle Virus |
| MDMV | - Maize Dwarf Mosaic Virus |
| MIP | - Major Intrinsic Protein |
| MRP | - multidrug resistance-associated protein |
| MT | - microtubule |
| NPTII | - neomycin phosphotransferase II |
| OsDAD1 | - *O*. *sativa* Defender Against Apoptotic Death 1 |
| Pcps | - pyrrolidone carboxyl peptidase |
| PGA | - 3-phosphoglyceric acid |
| P-gp | - P-glycoprotein |
| PH | - pleckstrin homology |
| PMI | - phosphomannose isomerase |
| PI4P5K | - phosphatidylinositol-4-phosphate 5-kinase |
| PP2A | - type 2A serine/threonine protein phosphatase |
| PPDK | - pyruvate orthophosphate dikinase |
| PR | - pathogenesis-related |
| pRB | - retinoblastoma protein |
| PTGS | - post-transcriptional gene silencing |
| Ro | - a parental transformant |
| RAB | - responsive to abscisic acid |
| RB | - retinoblastoma |
| RNAi | - RNA interference |
| RUBISCO | - ribulose-1,5-bisphosphate carboxylase/oxygenase |
| RuBP | - ribulose 1,5-bisphosphate |
| SA | - salicylic acid |
| SAR | - systemic acquired resistance |
| SDS | - sodium dodecyl sulfate |
| SITIP | - salt stress induced tonoplast intrinsic protein |
| SSC | - standard saline citrate (1 X SSC is 0.15 M NaCI, 0.015 M sodium citrate, pH 7.0) |
| PCR | - polymerase chain reaction |
| SSS | - soluble starch synthase |
| TDP | - transcription factor E2F/dimerization partner |
| TEV | - Tobacco Etch Virus |
| Tₘ | - thermal melting point |
| TMRI | - Torrey Mesa Research Institute |
| TMV | - Tobacco Mosaic Virus |
| UBPs | - ubiquitin-specific proteases |

### Amino Acid Abbreviations and Corresponding mRNA Codons

| Amino Acid | 3-Letter | 1-Letter | mRNA Codons |
|---|---|---|---|
| Alanine | Ala | A | GCA GCC GCG GCU |
| Arginine | Arg | R | AGA AGG CGA CGC CGG CGU |
| Asparagine | Asn | N | AAC AAU |
| Aspartic Acid | Asp | D | GAC GAU |
| Cysteine | Cys | C | UGC UGU |
| Glutamic Acid | Glu | E | GAA GAG |
| Glutamine | Gln | Q | CAA CAG |
| Glycine | Gly | G | GGA GGC GGG GGU |
| Histidine | His | H | CAC CAU |
| Isoleucine | lie | I | AUA AUC AUU |
| Leucine | Leu | L | UUA UUG CUA CUC CUG CUU |
| Lysine | Lys | K | AAA AAG |
| Methionine | Met | M | AUG |
| Proline | Pro | P | CCA CCC CCG CCU |
| Phenylalanine | Phe | F | UUC UUU |
| Serine | Ser | S | ACG AGU UCA UCC UCG UCU |
| Threonine | Thr | T | ACA ACC ACG ACU |
| Tryptophan | Trp | W | UGG |
| Tyrosine | Tyr | Y | UAC UAU |
| Valine | Val | V | GUA GUC GUG GUU |

### Background Art

As some of the major human staples, monocot plants such as rice, corn, and wheat have been a target of genetic engineering for higher yields and resistance to diseases, pests, and environmental stresses of various kinds. The timing of the transition from vegetative growth to flowering, for example, is an important step in plant development that determines the quality and quantity of most crop species by affecting the balance between vegetative and reproductive growth. Therefore, control of flowering time in genetically engineered cereal crops is important in agriculture. Knowledge of the proteins and molecular interactions associated with cell cycle processes, development, and stress response in monocot plants, such as rice, could lead to important applications in agriculture. Modulation of these interactions can be exploited to effect changes in plant development or growth that can result in increased crop yield and, in addition, can be used to increase tolerance to environmental stress conditions.

Similarly, the development of plant organs (e.g., root and stem), and the ability of a plant to respond to stress and to defend itself from insects and pathogens are likewise important targets for genetic engineering. Genes encoding proteins involved in the plant response to pathogens are important to agriculture, as their discovery can allow genetic manipulation of crops to obtain plants with enhanced or reduced disease resistance.

Thus, there is a need to identify proteins that are involved in plant growth (including cell cycle and senescence), plant development, and plant responses to stress. Knowledge of the interactions of such proteins will allow opportunities to produce enhanced food crops.

### Summary

This Summary lists several embodiments of the presently disclosed subject matter, and in many cases lists variations and permutations of these embodiments. This Summary is merely exemplary of the numerous and varied embodiments. Mention of one or more representative features of a given embodiment is likewise exemplary. Such an embodiment can typically exist with or without the feature(s) mentioned; likewise, those features can be applied to other embodiments of the presently disclosed subject matter, whether listed in this Summary or not. To avoid excessive repetition, this Summary does not list or suggest all possible combinations of such features.

The presently disclosed subject matter provides proteins and nucleic acid molecules encoding such proteins that are involved in the control and regulation of plant maturation and development, including proliferation, senescence, disease-resistance, stress-resistance, and differentiation. The presently disclosed subject matter provides compositions comprising at least one of the proteins described herein, as well as methods for using the proteins disclosed herein to affect plant maturation, development, and responses to stress.

The presently disclosed subject matter provides an isolated nucleic acid molecule encoding a cell proliferation-related polypeptide, wherein the polypeptide binds in a yeast two hybrid assay to a fragment of a protein selected from the group consisting of OsE2F1 (SEQ ID NO: 194), Os018989-4003 (SEQ ID NO: 2), OsE2F2 (SEQ ID NO: 10), OsS49462 (SEQ ID NO: 206), OsCYCOS2 (SEQ ID NO: 210), OsMADS45 (SEQ ID NO: 202), OsRAP1B (SEQ ID NO: 244), OsMADS6 (SEQ ID NO: 236), OsFDRMADS8 (SEQ ID NO: 228), OsMADS3 (SEQ ID NO: 232), OsMADS5 (SEQ ID NO: 234), OsMADS15 (SEQ ID NO: 240), OsHOS59 (SEQ ID NO: 258), OsGF14-c (SEQ ID NO: 278), OsDAD1 (SEQ ID NO: 292), Os006819-2510 (SEQ ID NO: 296), OsCRTC (SEQ ID NO: 300), OsSGT1 (SEQ ID NO: 310), OsERP (SEQ ID NO: 312), OsCHIB1 (SEQ ID NO: 318), OsCS (SEQ ID NO: 322), OsPP2A-2 (SEQ ID NO: 330), and OsCAA90866 (SEQ ID NO: 336). In one embodiment, the isolated nucleic acid molecule is derived from rice (Oryza sativa). In another embodiment, the isolated nucleic acid molecule comprises a nucleic acid sequence selected from the group consisting of odd numbered SEQ ID NOs: 1-191.

The presently disclosed subject matter also provides a description of interactions between cell proliferation-related proteins and polypeptides encoded by the isolated nucleic acid molecules disclosed herein. In one embodiment, the isolated nucleic acid molecule comprises a nucleic acid sequence of one of odd numbered SEQ ID NOs: 1-7 and the protein comprises an amino acid sequence of SEQ ID NO: 194. In another embodiment, the isolated nucleic acid molecule comprises a nucleic acid sequence of one of SEQ ID NOs: 9 and 11 and the protein comprises an amino acid sequence of SEQ ID NO: 2. In another embodiment, the isolated nucleic acid molecule comprises a nucleic acid sequence of one of SEQ ID NOs: 1 and 13 and the protein comprises an amino acid sequence of SEQ ID NO: 10. In another embodiment, the isolated nucleic acid molecule comprises a nucleic acid sequence of one of odd numbered SEQ ID NOs: 15-21 and the protein comprises an amino acid sequence of SEQ ID NO: 206. In another embodiment, the isolated nucleic acid molecule comprises a nucleic acid sequence of one of odd numbered SEQ ID NOs: 15, 17, 23-53 and the protein comprises an amino acid sequence of SEQ ID NO: 210. In another embodiment, the isolated nucleic acid molecule comprises a nucleic acid sequence of SEQ ID NO: 55 and the protein comprises an amino acid sequence of SEQ ID NO: 202. In another embodiment, the isolated nucleic acid molecule comprises a nucleic acid sequence of SEQ ID NO: 57 and the protein comprises an amino acid sequence of SEQ ID NO: 244. In another embodiment, the isolated nucleic acid molecule comprises a nucleic acid sequence of SEQ ID NO: 59 and the protein comprises an amino acid sequence of SEQ ID NO: 236. In another embodiment, the isolated nucleic acid molecule comprises a nucleic acid sequence of SEQ ID NO: 61 and the protein comprises an amino acid sequence of SEQ ID NO: 232. In another embodiment, the isolated nucleic acid molecule comprises a nucleic acid sequence of SEQ ID NO: 63 and the protein comprises an amino acid sequence of SEQ ID NO: 234. In another embodiment, the isolated nucleic acid molecule comprises a nucleic acid sequence of SEQ ID NO: 65 and the protein comprises an amino acid sequence of SEQ ID NO: 240. In another embodiment, the isolated nucleic acid molecule comprises a nucleic acid sequence of one of odd numbered SEQ ID NOs: 67-79 and the protein comprises an amino acid sequence of SEQ ID NO: 258. In another embodiment, the isolated nucleic acid molecule comprises a nucleic acid sequence of SEQ ID NO: 81 and the protein comprises an amino acid sequence of SEQ ID NO: 260. In another embodiment, the isolated nucleic acid molecule comprises a nucleic acid sequence of one of odd numbered SEQ ID NOs: 83-97 and the protein comprises an amino acid sequence of SEQ ID NO: 278. In another embodiment, the isolated nucleic acid molecule comprises a nucleic acid sequence of one of SEQ ID NOs: 89 and 99 and the protein comprises an amino acid sequence of SEQ ID NO: 286. In another embodiment, the isolated nucleic acid molecule comprises a nucleic acid sequence of one of odd numbered SEQ ID NOs: 101-105 and the protein comprises an amino acid sequence of SEQ ID NO: 296. In another embodiment, the isolated nucleic acid molecule comprises a nucleic acid sequence of SEQ ID NO: 107 and the protein comprises an amino acid sequence of SEQ ID NO: 300. In another embodiment, the isolated nucleic acid molecule comprises a nucleic acid sequence of SEQ ID NO: 109 and the protein comprises an amino acid sequence of SEQ ID NO: 304. In another embodiment, the isolated nucleic acid molecule comprises a nucleic acid sequence of one of odd numbered SEQ ID NOs: 111-123 and the protein comprises an amino acid sequence of SEQ ID NO: 310. In another embodiment, the isolated nucleic acid molecule comprises a nucleic acid sequence of one of odd numbered SEQ ID NOs: 125-147 and the protein comprises an amino acid sequence of SEQ ID NO: 312. In another embodiment, the isolated nucleic acid molecule comprises a nucleic acid sequence of one of odd numbered SEQ ID NOs: 151-157 and the protein comprises an amino acid sequence of SEQ ID NO: 318. In another embodiment, the isolated nucleic acid molecule comprises a nucleic acid sequence of one of odd numbered SEQ ID NOs: 159-175 and the protein comprises an amino acid sequence of SEQ ID NO: 322. In another embodiment, the isolated nucleic acid molecule comprises a nucleic acid sequence of one of odd numbered SEQ ID NOs: 177-175 and the protein comprises an amino acid sequence of SEQ ID NO: 330. And in still another embodiment, the isolated nucleic acid molecule comprises a nucleic acid sequence of one of odd numbered SEQ ID NOs: 177, 187-191 and the protein comprises an amino acid sequence of SEQ ID NO: 336.

The presently disclosed subject matter also provides an isolated nucleic acid molecule encoding a cell proliferation-related polypeptide, wherein the nucleic acid molecule is selected from the group consisting of:
(a) a nucleic acid molecule encoding a polypeptide comprising an amino acid sequence of one of even numbered SEQ ID NOs: 2-192;
(b) a nucleic acid molecule comprising a nucleic acid sequence of one of odd numbered SEQ ID NOs: 1-191;
(c) a nucleic acid molecule that has a nucleic acid sequence at least 90% identical to the nucleic acid sequence of the nucleic acid molecule of (a) or (b);
(d) a nucleic acid molecule that hybridizes to (a) or (b) under conditions of hybridization selected from the group consisting of:
   (i) 7% sodium dodecyl sulfate (SDS), 0.5 M NaPO4, 1 mM ethylenediamine tetraacetic acid (EDTA) at 50°C with a final wash in 2X standard saline citrate (SSC), 0.1% SDS at 50°C;
   (ii) 7% SDS, 0.5 M NaPO4, 1 mM EDTA at 50°C with a final wash in 1X SSC, 0.1% SDS at 50°C;
   (iii) 7% SDS, 0.5 M NaPO4, 1 mM EDTA at 50°C with a final wash in 0.5X SSC, 0.1% SDS at 50°C;
   (iv) 7% sodium dodecyl sulfate (SDS), 0.5 M NaPO4, 1 mM EDTA at 50°C with a final wash in 0.1 X SSC, 0.1 % SDS at 50°C; and
   (v) 7% sodium dodecyl sulfate (SDS), 0.5 M NaPO4, 1 mM EDTA at 50°C with a final wash in 0.1X SSC, 0.1% SDS at 65°C;
(e) a nucleic acid molecule comprising a nucleic acid sequence fully complementary to (a); and
(f) a nucleic acid molecule comprising a nucleic acid sequence that is the full reverse complement of (a).

The presently disclosed subject matter also provides an isolated cell proliferation-related polypeptide encoded by the disclosed isolated nucleic acid molecules, or a functional fragment, domain, or feature thereof.

The presently disclosed subject matter also provides a method for producing a polypeptide disclosed herein, the method comprising the steps of:
(a) growing cells comprising an expression cassette under suitable growth conditions, the expression cassette comprising a nucleic acid molecule as disclosed herein; and
(b) isolating the polypeptide from the cells.

The presently disclosed subject matter also provides a transgenic plant cell comprising an isolated nucleic acid molecule disclosed herein. In one embodiment, the plant is selected from the group consisting of corn (Zea mays), Brassica sp., alfalfa (Medicago sativa), rice (Oryza sativa ssp.), rye (Secale cereale), sorghum (Sorghum bicolor, Sorghum vulgare), pearl millet (Pennisetum glaucum), proso millet (Panicum miliaceum), foxtail millet (Setaria italica), finger millet (Eleusine coracana), sunflower (Helianthus annuus), safflower (Carthamus tinctorius), wheat (Triticum aestivum), soybean (Glycine max), tobacco (Nicotiana tabacum), potato (Solanum tuberosum), peanut (Arachis hypogaea), cotton, sweet potato (Ipomoea batatus), cassava (Manihot esculenta), coffee (Cofea spp.), coconut (Cocos nucifera), pineapple (Ananas comosus), citrus trees (Citrus spp.), cocoa (Theobroma cacao), tea (Camellia sinensis), banana (Musa spp.), avocado (Persea ultilane), fig (Ficus casica), guava (Psidium guajava), mango (Mangifera indica), olive (Olea europaea), papaya (Carica papaya), cashew (Anacardium occidentale), macadamia (Macadamia integrifolia), almond (Prunus amygdalus), sugar beets (Beta vulgaris), sugarcane (Saccharum spp.), oats, duckweed (Lemna), barley, a vegetable, an ornamental, and a conifer. In another embodiment, the plant is rice (Oryza sativa ssp.). In one embodiment, the duckweed is selected from the group consisting of genus Lemna, genus Spirodela, genus Woffia, and genus Wofiella. In one embodiment, the vegetable is selected from the group consisting of tomatoes, lettuce, guar, locust bean, fenugreek, soybean, garden beans, cowpea, mungbean, lima bean, fava bean, lentils, chickpea, green bean, lima bean, pea, and members of the genus Cucumis. In one embodiment, the ornamental is selected from the group consisting of impatiens, Begonia, Pelargonium, Viola, Cyclamen, Verbena, Vinca, Tagetes, Primula, Saint Paulia, Agertum, Amaranthus, Antihirrhinum, Aquilegia, Cineraria, Clover, Cosmo, Cowpea, Dahlia, Datura, Delphinium, Gerbera, Gladiolus, Gloxinia, Hippeastrum, Mesembryanthemum, Salpiglossos, and Zinnia, azalea, hydrangea, hibiscus, rose, tulip, daffodil, petunia, carnation, poinsettia, and chrysanthemum. In one embodiment, the conifer is selected from the group consisting of loblolly pine, slash pine, ponderosa pine, lodgepole pine, Monterey pine, Douglas-fir, Western hemlock, Sitka spruce, redwood, silver fir, balsam fir, Western red cedar, and Alaska yellow-cedar.

In another embodiment, the transgenic plant is a plant selected from the group consisting of Acacia, aneth, artichoke, arugula, blackberry, canola, cilantro, clementines, escarole, eucalyptus, fennel, grapefruit, honey dew, jicama, kiwifruit, lemon, lime, mushroom, nut, okra, orange, parsley, persimmon, plantain, pomegranate, poplar, radiata pine, radicchio, Southern pine, sweetgum, tangerine, triticale, vine, yams, apple, pear, quince, cherry, apricot, melon, hemp, buckwheat, grape, raspberry, chenopodium, blueberry, nectarine, peach, plum, strawberry, watermelon, eggplant, pepper, cauliflower, Brassica, broccoli, cabbage, ultilan sprouts, onion, carrot, leek, beet, broad bean, celery, radish, pumpkin, endive, gourd, garlic, snapbean, spinach, squash, turnip, ultilane, and zucchini.

The presently disclosed subject matter also provides an isolated cell proliferation-related polypeptide, wherein the polypeptide binds in a yeast two hybrid assay to a fragment of a protein selected from the group consisting of OsE2F1 (SEQ ID NO: 194), Os018989-4003 (SEQ ID NO: 2), OsE2F2 (SEQ ID NO: 10), OsS49462 (SEQ ID NO: 206), OsCYCOS2 (SEQ ID NO: 210), OsMADS45 (SEQ ID NO: 202), OsRAP1B (SEQ ID NO: 244), OsMADS6 (SEQ ID NO: 236), OsFDRMADS8 (SEQ ID NO: 228), OsMADS3 (SEQ ID NO: 232), OsMADS5 (SEQ ID NO: 234), OsMADS15 (SEQ ID NO: 240), OsHOS59 (SEQ ID NO: 258), OsGF14-c (SEQ ID NO: 278), OsDAD1 (SEQ ID NO: 292), Os006819-2510 (SEQ ID NO: 296), OsCRTC (SEQ ID NO: 300), OsSGT1 (SEQ ID NO: 310), OsERP (SEQ ID NO: 312), OsCHIB1 (SEQ ID NO: 318), OsCS (SEQ ID NO: 322), OsPP2A-2 (SEQ ID NO: 330), and OsCAA90866 (SEQ ID NO: 336). In one embodiment, the isolated proliferation-related polypeptide is selected from the group consisting of (a) a polypeptide comprising an amino acid sequence of even numbered SEQ ID NOs: 2-192; and (b) a polypeptide comprising an amino acid sequence at least 80% similar to the polypeptide of (a) using the GCG Wisconsin Package SEQWEB® application of GAP with the default GAP analysis parameters. In another embodiment, the polypeptide comprises an amino acid sequence of one of even numbered SEQ ID NOs: 2-192.

The presently disclosed subject matter also provides an expression cassette comprising a nucleic acid molecule encoding a cell proliferation-related polypeptide disclosed herein. In one embodiment, the nucleic acid molecule encoding a cell proliferation-related polypeptide comprises a nucleic acid sequence selected from odd numbered SEQ ID NOs: 1-191. In one embodiment, the expression cassette further comprises a regulatory element operatively linked to the nucleic acid molecule. In one embodiment, the regulatory element comprises a promoter. In one embodiment, the promoter is a plant promoter. In another embodiment, the promoter is a constitutive promoter. In another embodiment, the promoter is a tissue-specific or a cell type-specific promoter. In one embodiment, the tissue-specific or cell type-specific promoter directs expression of the expression cassette in a location selected from the group consisting of epidermis, root, vascular tissue, meristem, cambium, cortex, pith, leaf, flower, seed, and combinations thereof.

The presently disclosed subject matter also provides a transgenic plant cell comprising a disclosed expression cassette. In one embodiment, the expression cassette comprises an isolated nucleic acid molecule comprising a nucleic acid sequence of one of odd numbered SEQ ID NOs: 1-191.

The presently disclosed subject matter also provides transgenic plants comprising a disclosed expression cassette, as well as transgenic seeds and progeny of the trangenic plants disclosed herein.

The presently disclosed subject matter also provides a method for modulating proliferation of a plant cell comprising introducing into the plant cell an expression cassette comprising an isolated nucleic acid molecule encoding a cell proliferation-related polypeptide, wherein the polypeptide binds in a yeast two hybrid assay to a fragment of a protein selected from the group consisting of OsE2F1 (SEQ ID NO: 194), Os018989-4003 (SEQ ID NO: 2), OsE2F2 (SEQ ID NO: 10), OsS49462 (SEQ ID NO: 206), OsCYCOS2 (SEQ ID NO: 210), OsMADS45 (SEQ ID NO: 202), OsRAP1B (SEQ ID NO: 244), OsMADS6 (SEQ ID NO: 236), OsFDRMADS8 (SEQ ID NO: 228), OsMADS3 (SEQ ID NO: 232), OsMADS5 (SEQ ID NO: 234), OsMADS15 (SEQ ID NO: 240), OsHOS59 (SEQ ID NO: 258), OsGF14-c (SEQ ID NO: 278), OsDAD1 (SEQ ID NO: 292), Os006819-2510 (SEQ ID NO: 296), OsCRTC (SEQ ID NO: 300), OsSGT1 (SEQ ID NO: 310), OsERP (SEQ ID NO: 312), OsCHIB1 (SEQ ID NO: 318), OsCS (SEQ ID NO: 322), OsPP2A-2 (SEQ ID NO: 330), and OsCAA90866 (SEQ ID NO: 336). In one embodiment of the disclosed method, the expression of the polypeptide in the cell results in an enhancement of a rate or extent of proliferation of the cell. In another embodiment, the expression of the polypeptide in the cell results in a decrease in a rate or extent of proliferation of the cell.

In another embodiment of the instant method, the isolated nucleic acid molecule comprises a nucleic acid sequence selected from one of odd numbered SEQ ID NOs: 1-339. In another embodiment, the isolated nucleic acid molecule comprises a nucleic acid sequence selected from one of odd numbered SEQ ID NOs: 1-191.

Accordingly, it is an object of the presently disclosed subject matter to provide methods and compositions that can be used to enhance agriculturally important plants. This object is achieved in whole or in part by the presently disclosed subject matter.

An object of the presently disclosed subject matter having been stated above, other objects and advantages will become apparent to those of ordinary skill in the art after a study of the following description of the presently claimed subject matter and non-limiting Examples.

### Brief Description of the Drawings

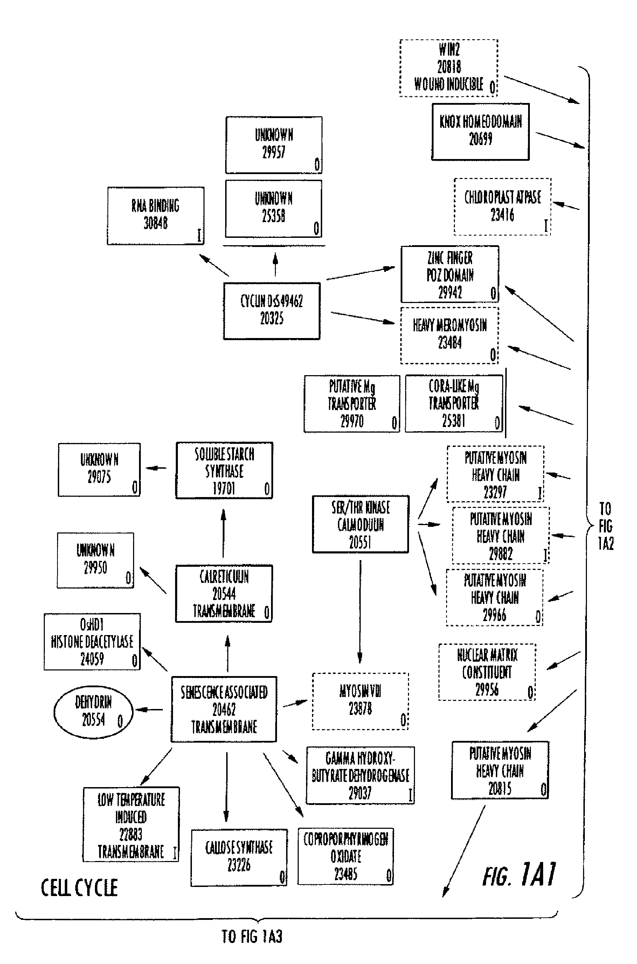

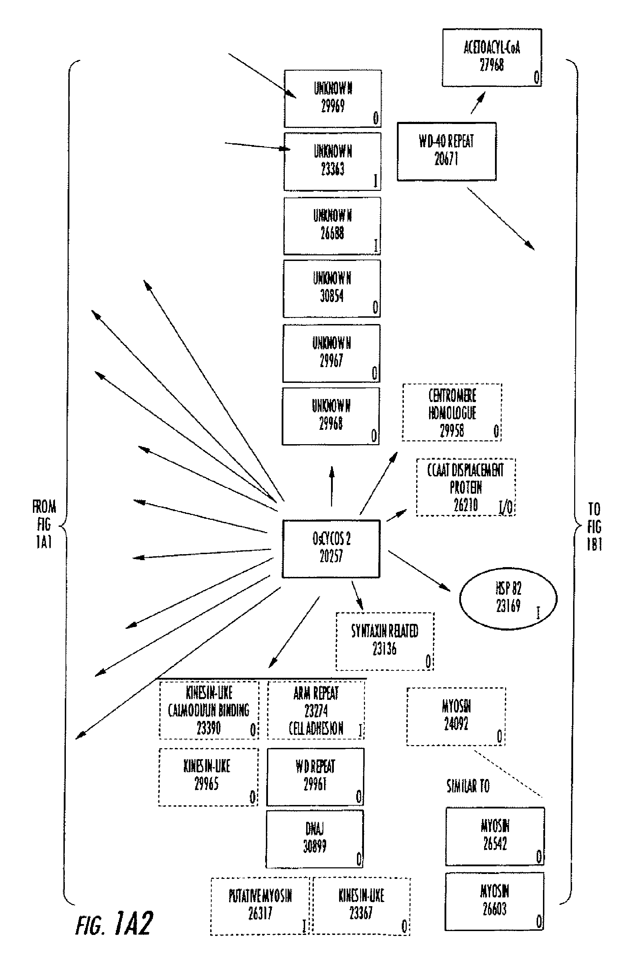

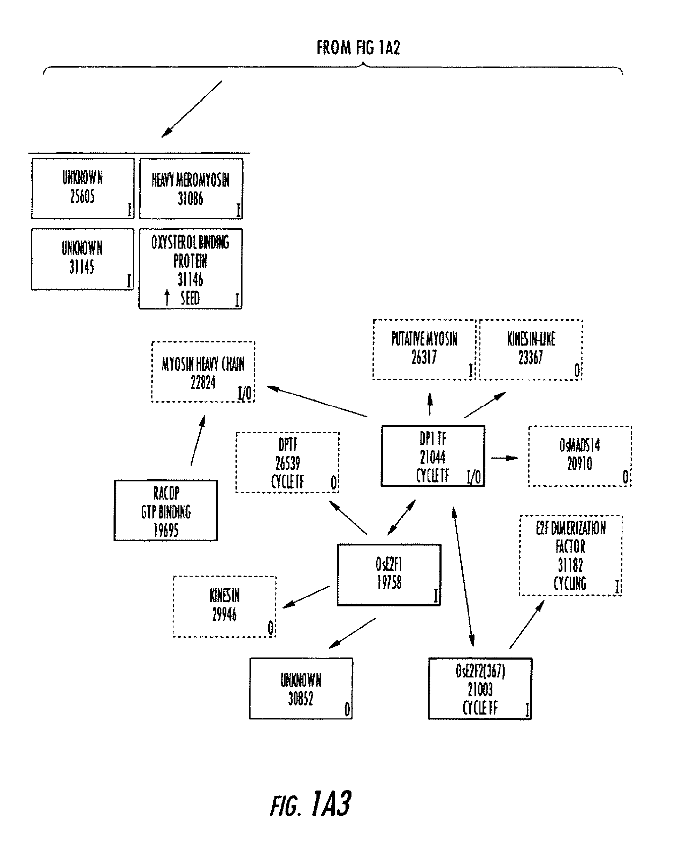

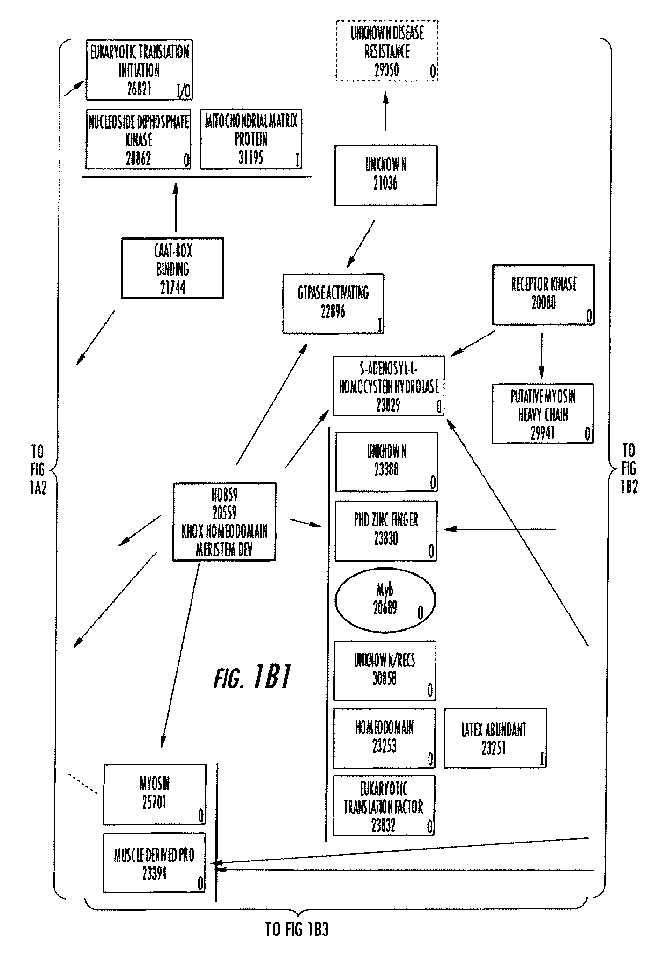

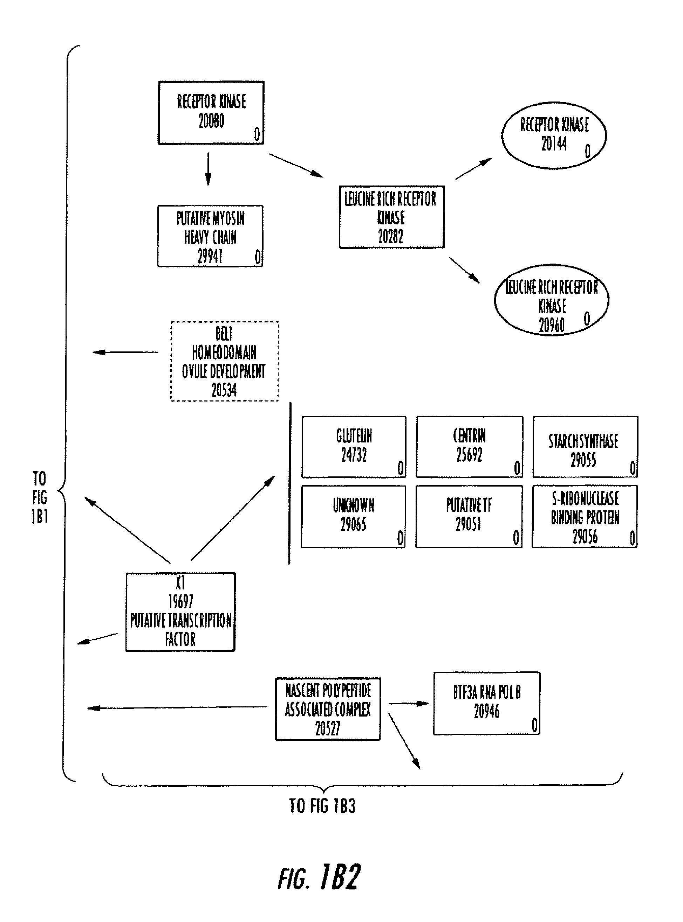

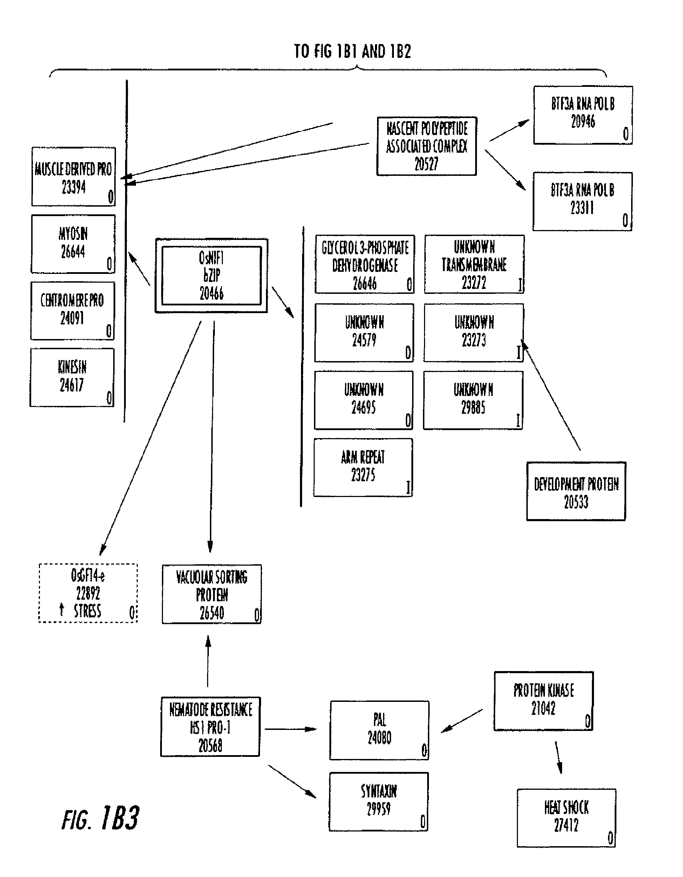

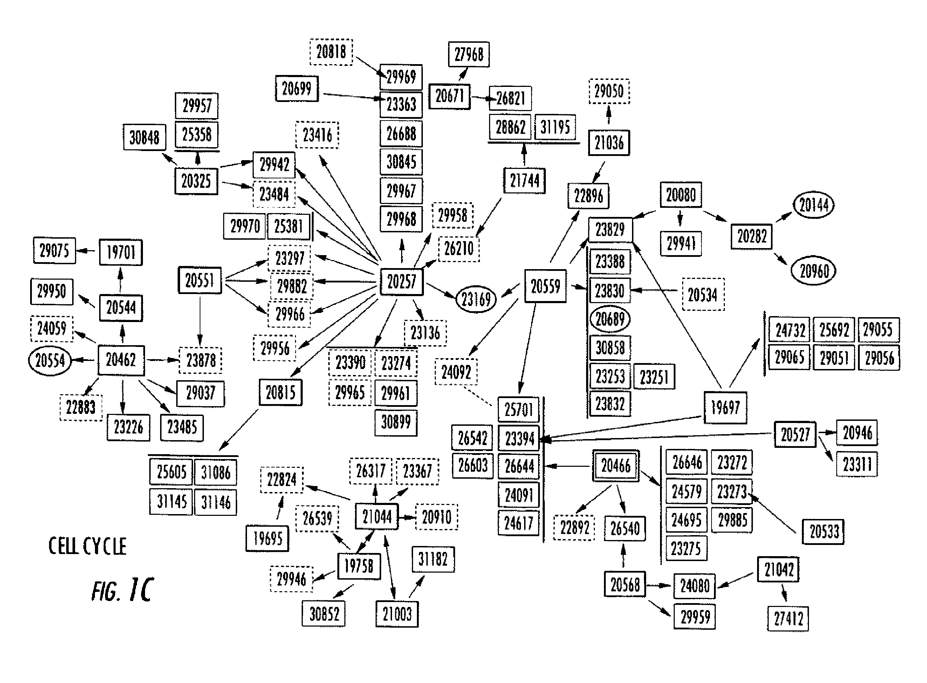

Figures 1A-1C are schematic representations of the interactions between various, non-limiting, cell proliferation-related proteins of the presently disclosed subject matter. Figures 1A and 1B represent the left and right halves, respectively, of Figure 1C. Arrows indicate interaction directions between DNA binding domain fused proteins (thick lined boxes or ovals) and activation domain fused proteins. Dotted boxes indicate previously published interactions. Ovals rather than boxes indicate that a protein fused to the DNA binding domain did not interact with other proteins. Circular arrows depict self-interactions. Dotted lines indicate amino acid similarity between proteins. The proteins listed in the Figure can be classified as follows: cell cycle (19758, 20257, 20235, 20462, 20551, 20815, 21003, 21044, 22824, 23136, 23274, 23297, 23367, 23390, 23394, 23484, 23829, 23878, 24091, 24092, 24617, 25692, 25701, 26210, 26317, 26539, 26542, 26603, 26644, 29882, 29941, 29946, 29956, 29958, 29959, 29965, 29966, 31086, and 31182); development (20466, 20533, 20534, 20559, 20689, 20699, 20910, and 31146); biotic stress (20568 and 29050); and abiotic stress (20466, 20554, 20818, 22892, and 23169).

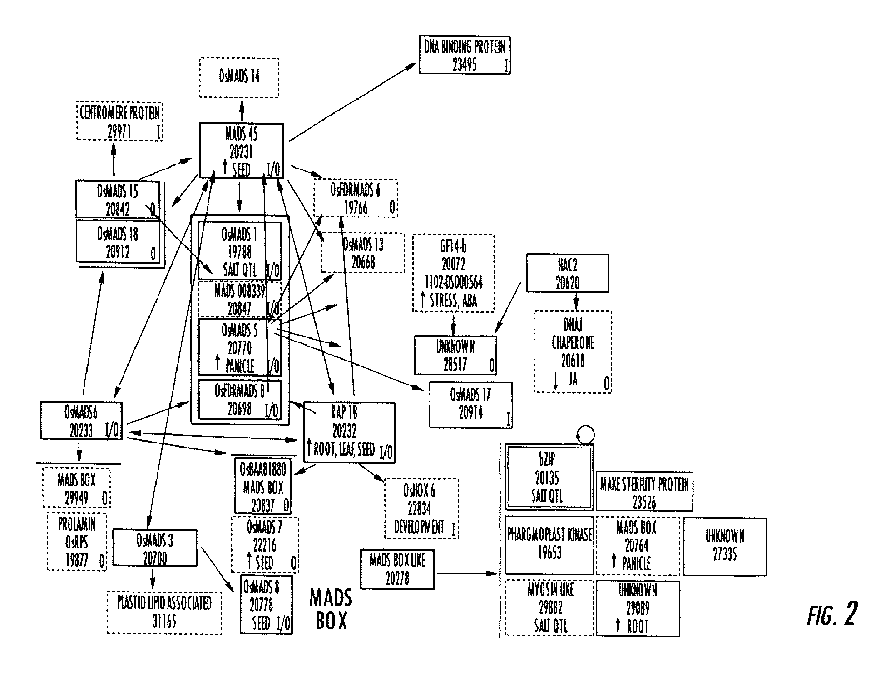

Figure 2 is a schematic representation of the interactions between various, non-limiting, cell proliferation-related proteins of the presently disclosed subject matter. Arrows indicate interaction direction between DNA binding domain fused proteins (thick lined boxes or ovals) and activation domain fused proteins. Dotted boxes indicate previously published interactions. Ovals rather than boxes indicate that a protein fused to the DNA binding domain did not interact with other proteins. Circular arrows depict self-interactions. Dotted lines indicate amino acid similarity between proteins. The proteins listed in the Figure can be classified as involved in development with the exception of the following: 19653, 20072 (abiotic stress), 20618 (cell cycle), 23495, 27335, 28517, 29089, 29971 (cell cycle), and 31165. Proteins that can be categorized in multiple categories include 20135 (development and abiotic stress) and 29882 (development and cell cycle).

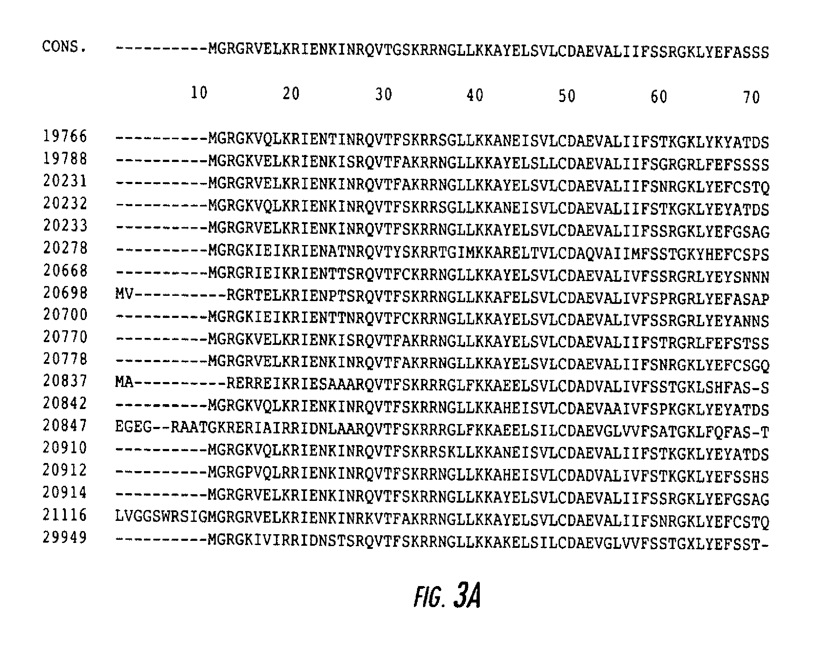

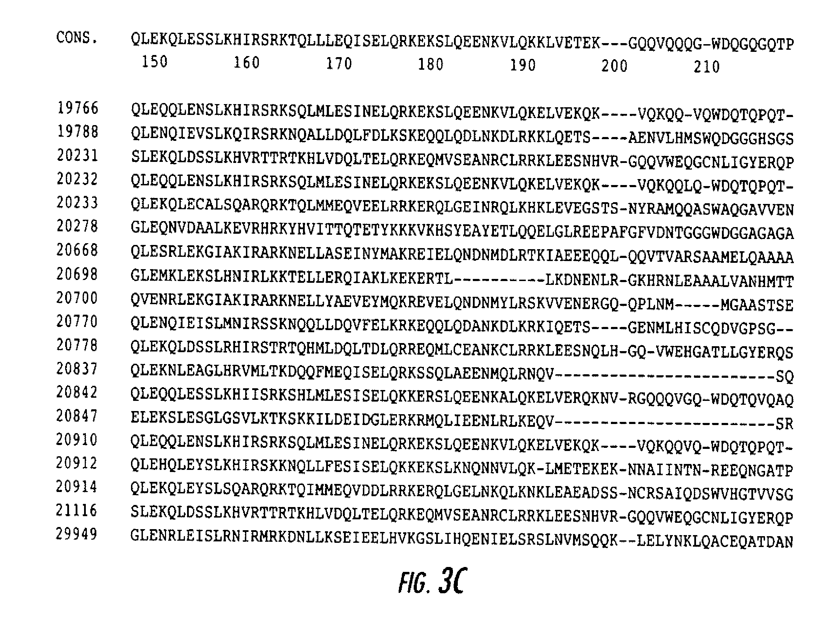

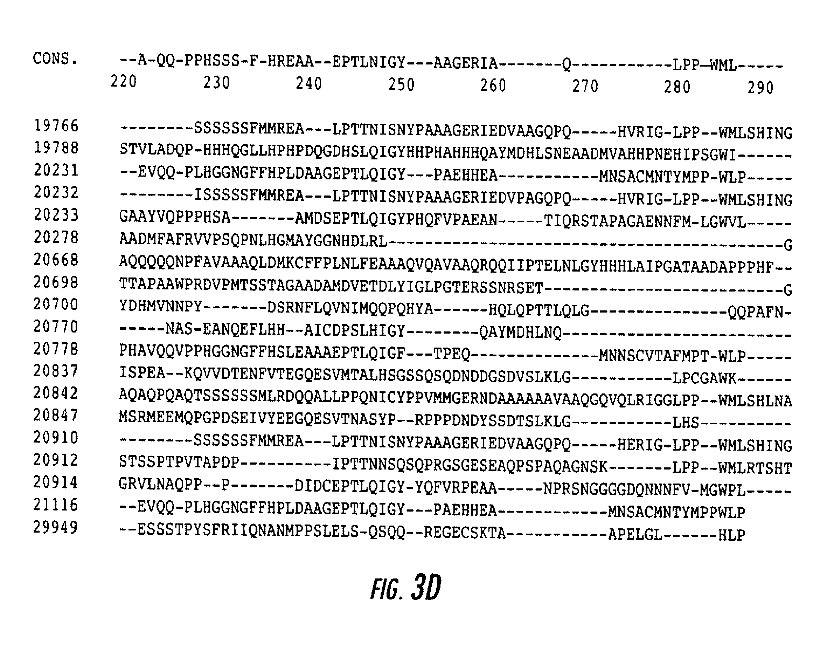

Figures 3A-3E depicts similarities between various cell proliferation-related proteins of the presently disclosed subject matter.
Figures 3A-3D are a schematic representation showing an amino acid alignment of various, non-limiting, cell proliferation-related proteins of the presently disclosed subject matter.

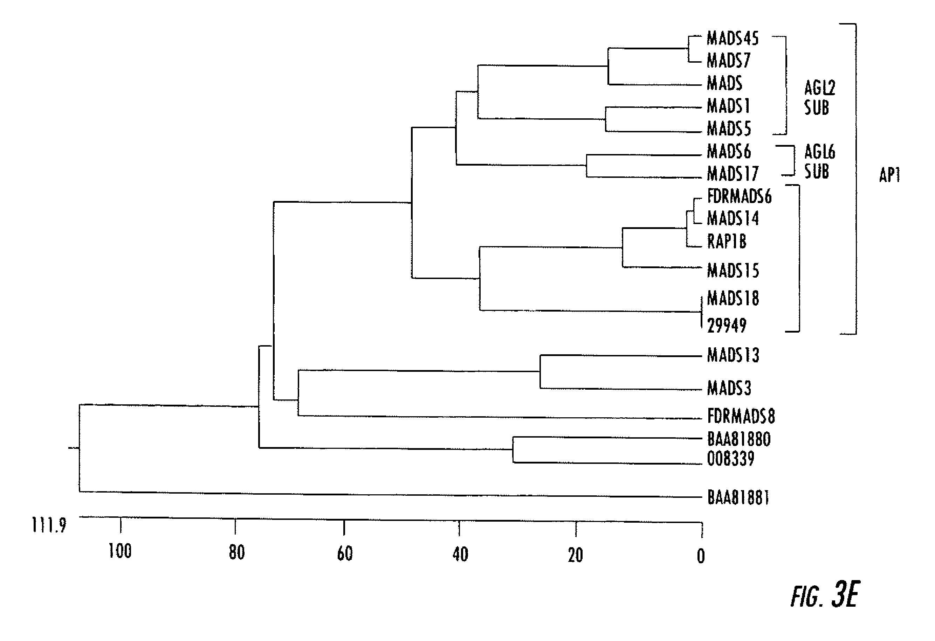

Figure 3E is a schematic representation showing a phylogenetic tree of the proteins for which amino acid sequence alignments are presented in Figures 3A-3D.

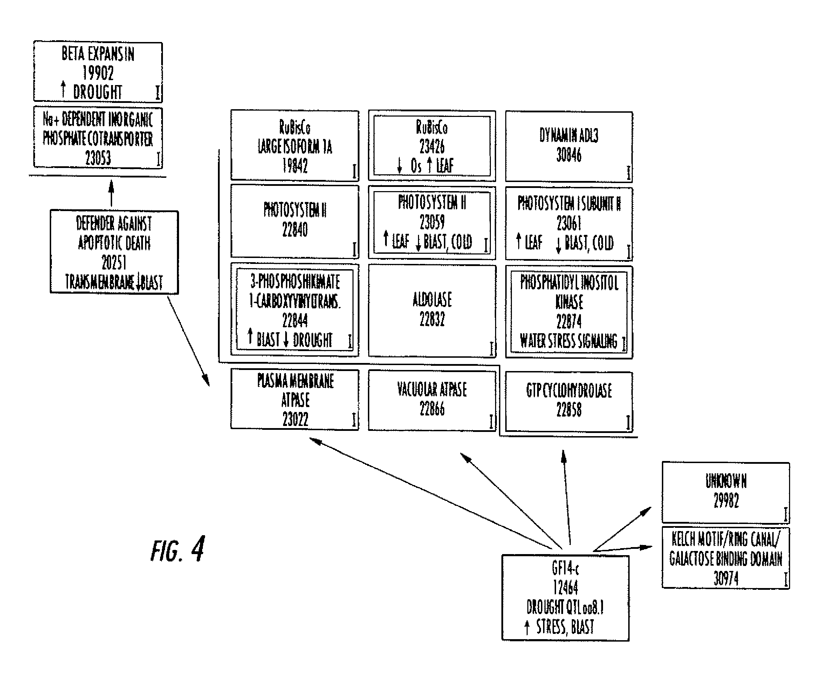

Figure 4 is a schematic representation of the interactions between various, non-limiting, cell proliferation-related proteins of the presently disclosed subject matter. Arrows indicate interaction direction between DNA binding domain fused proteins (thick lined boxes or ovals) and activation domain fused proteins. Dotted boxes indicate previously published interactions. Ovals rather than boxes indicate that a protein fused to the DNA binding domain did not interact with other proteins. Circular arrows depict self-interactions. Dotted lines indicate amino acid similarity between proteins. The proteins listed in the Figure can be classified as follows: biotic stress (20251); abiotic stress (12464, 19902, 22844, 22874, 23059, and 23426); and chloroplast (19842, 22832, 22840, 22844, 22858, 22874, 23059, 23061, 23426, and 30846).

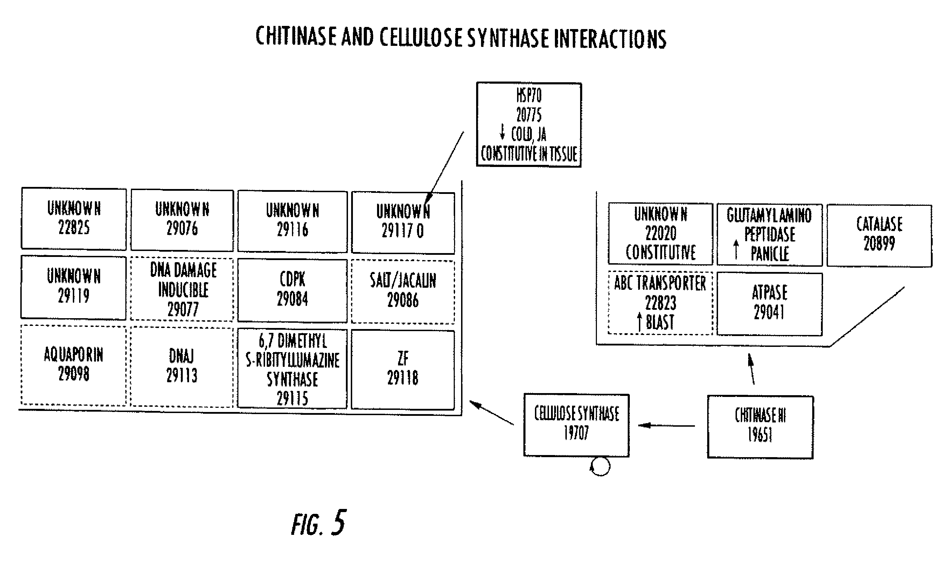

Figure 5 is a schematic representation of the interactions between various, non-limiting, cell proliferation-related proteins of the presently disclosed subject matter. Arrows indicate interaction direction between DNA binding domain fused proteins (thick lined boxes or ovals) and activation domain fused proteins. Dotted boxes indicate previously published interactions. Ovals rather than boxes indicate that a protein fused to the DNA binding domain did not interact with other proteins. Circular arrows depict self-interactions. Dotted lines indicate amino acid similarity between proteins. The proteins listed in the Figure can be classified as follows: development (glutamyl amino peptidase); biotic stress (19651, 20899, and 22823); abiotic stress (20775, 29077, 29098, 29086, and 29113).

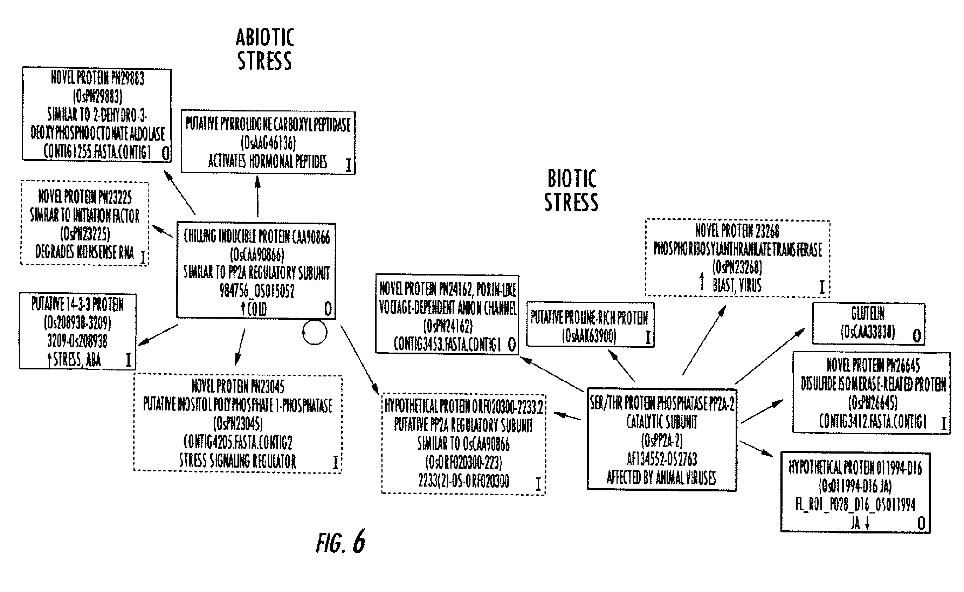

Figure 6 is a schematic representation of the interactions between various, non-limiting, cell proliferation-related proteins of the presently disclosed subject matter. Arrows indicate interaction direction between DNA binding domain fused proteins (thick lined boxes or ovals) and activation domain fused proteins. Dotted boxes indicate previously published interactions. Ovals rather than boxes indicate that a protein fused to the DNA binding domain did not interact with other proteins. Circular arrows depict self-interactions. Dotted lines indicate amino acid similarity between proteins. The proteins listed in the Figure can be classified as follows: biotic stress (ORF020300-2233.2, 23268, 011994-D16, and OsPP2-A) and abiotic stress (23225, OsCAA90866, and 3209-OS208938).

### Brief Description of the Sequence Listing

SEQ ID NOs: 1-340 present nucleic acid and amino acid sequences of the rice (*Oryza sativa*)polypeptides employed in the two hybrid assays disclosed hereinbelow. For these SEQ ID NOs., the odd numbered sequences are nucleic acid sequences, and the even numbered sequences are the deduced amino acid sequences of the nucleic acid sequence of the immediately preceding SEQ ID NO:. For example, SEQ ID NO: 2 is the deduced amino acid sequence of the nucleic acid sequence presented in SEQ ID NO: 1, SEQ ID NO: 4 is the deduced amino acid sequence of the nucleic acid sequence presented in SEQ ID NO: 3, SEQ ID NO: 6 is the deduced amino acid sequence of the nucleic acid sequence presented in SEQ ID NO: 5, etc. Further description of the SEQ ID NOs. is presented in the following Table:

| **SEQ ID NOs.** | **PN Number** | **Description** |
|---|---|---|
| 1, 2 | 21044 | Hypothetical Protein 018989-4003, Similar to *Triticum sp*. DP Protein |
| 3, 4 | 26539 | Novel Protein PN26539(AC087544), Probable DP |
| 5, 6 | 29946 | Novel Protein PN29946, Similar to *A. thaliana* Kinesin-Like Protein (GENBANK® Accession No. BAB11329.1; e=0.0) |
| 7, 8 | 30852 | Novel Protein PN30852 |
| 9, 10 | 21003 | *O*. *sativa* E2F2 Homolog (GENBANK® Accession Nos. AB041726; BAB20933) |
| 11, 12 | 22824 | Novel Protein PN22824, Myosin heavy chain |
| 13, 14 | 31182 | Novel Protein PN31183, *A. thaliana* DP-Like Protein (GENBANK® Accession No. CAC15483.1; 9e⁻⁵⁵) |
| 15, 16 | 23484 | Novel Protein PN23484, heavy meromyosin |
| 17, 18 | 29942 | Novel Protein PN29942, Fragment, zinc finger protein |
| 19, 20 | 29957 | Novel Protein PN29957, Fragment, unknown |
| 21, 22 | 30848 | Novel Protein PN30848, Fragment, RNA binding protein |
| 23, 24 | 30899 | Hypothetical Protein 000221-3976, Fragment, Similar to OsHP82 (GENBANK® Accession No. P33126; e=0.0) |
| 25, 26 | 29970 | Putative CorA-like Mg²⁺ Transporter Protein |
| 27, 28 | 20815 | Hypothetical Protein PN20815 Similar to *A. thaliana* Myosin Heavy Chain, Fragment |
| 29, 30 | 23274 | Novel Protein PN23274, Similar to *A. thaliana* ARM Repeat-Containing Protein |
| 31, 32 | 23390 | Novel Protein PN23390, Putative Kinesin-like Calmodulin Binding Protein, Fragment |
| 33, 34 | 26688 | Novel Protein PN26688, unknown |
| 35, 36 | 29882 | Novel Protein PN29882, Fragment, myosin heavy chain |
| 37, 38 | 29956 | Novel Protein PN29956, Fragment, nuclear matrix constituent |
| 39, 40 | 29958 | Novel Protein PN29958, Fragment, centromere homologue |
| 41, 42 | 29961 | Novel Protein PN29961, Fragment, Similar to *A*. *thaliana* Unknown Protein (GENBANK® Accession No. BAB02349) |
| 43, 44 | 29965 | Novel Protein PN29965, Fragment, Similar to *A*. *thaliana* Kinesin (Centromere Protein)-Like Heavy Chain-Like Protein (GENBANK® Accession No. BAB03114) |
| 45, 46 | 29966 | Novel Protein PN29966, Fragment, myosin heavy chain |
| 47, 48 | 29967 | Novel Protein PN29967, Fragment, unknown |
| 49, 50 | 29968 | Novel Protein PN29968, Similar to *A. thaliana* Unknown Protein (GENBANK® Accession No. BAB01990) |
| 51, 52 | 29969 | Novel Protein PN29969, Similar to *A. thaliana* Unknown Protein (GENBANK® Accession No. BAB01990) |
| 53, 54 | 30854 | Novel Protein PN30854, unknown |
| 55, 56 | 23495 | Novel protein PN23495 |
| 57, 58 | 22834 | Novel protein PN22834, similar to Oshox6, fragment |
| 59, 60 | 29949 | Novel protein PN29949 putative MADS protein |
| 61, 62 | 31165 | Novel protein PN31165 |
| 63, 64 | 20072 | Hypothetical protein 000564-1102 |
| 65, 66 | 29971 | Novel protein PN29971, fragment, similar to *A. thaliana* centromere protein (GENBANK® Accession No. NP_191066) |
| 67, 68 | 23169 | Hypothetical Protein 000221-3976, Fragment, Similar to OsHP82 (GENBANK® Accession No. P33126; e=0.0) |
| 69, 70 | 23251 | Novel Protein PN23251 |
| 71, 72 | 23388 | Novel Protein PN23388 |
| 73, 74 | 23829 | Novel Protein PN23829 Putative S-Adenosyl-L-Homocysteine Hydrolase (GENBANK® Accession No. P32112; e=0.0) |
| 75, 76 | 23830 | Novel Protein PN23830, Similar to *A. thaliana* Putative PHD-Finger Protein (GENBANK® Accession No. NP_566742.1; 2e⁻⁷³) |
| 77, 78 | 24092 | Novel Protein PN24092, Similar to *O. sativa* Putative Myosin |
| 79, 80 | 30858 | Novel Protein PN30858 |
| 81, 82 | 21036 | Hypothetical Protein 003181-3684 |
| 83, 84 | 22858 | Novel Protein 22858, Fragment, similar to *Arabidopsis* GTP Cyclohydrolase II (GENBANK® Accession No. BAB09512.1; e=0) |
| 85, 86 | 22874 | Novel Protein 22874, Fragment, similar to *Arabidopsis* Putative Phosphatidylinositol-4-phosphate 5-kinase (GENBANK® Accession No. NP_187603.1; 4e⁻¹⁸) |
| 87, 88 | 22866 | Novel Protein PN22866, Fragment, Similar to *A. Thaliana* Vacuolar ATP Synthase Subunit C (V- ATPase C subunit; Vacuolar proton pump C subunit) (GENBANK® Accession No. Q9SDS7; e⁻⁵²) |
| 89, 90 | 23022 | Novel Protein PN23022, Fragment, similar to *H*. *Vulgare* Plasma Membrane H⁺-ATPase (GENBANK® Accession No. CAC50884; e=0.0) |
| 91, 92 | 23061 | Hypothetical Protein OsContig3864, Similar to *H*. *vulgare* Photosystem I Reaction Center Subunit II, Chloroplast Precursor (GENBANK® Accession No. P36213; 6e⁻⁸⁷) |
| 93, 94 | 29982 | Novel Protein PN29982 |
| 95, 96 | 30846 | Novel Protein PN30846 |
| 97, 98 | 30974 | Novel Protein PN30974 |
| 99, 100 | 23053 | Novel Protein 23053, Fragment, Similar to *Arabidopsis* Putative Na+-Dependent Inorganic Phosphate Cotransporter (GENBANK® Accession No. NP_181341.1; e⁻¹⁰⁵) |
| 101, 102 | 23226 | Novel Protein PN23226, Callose synthase |
| 103, 104 | 23485 | Novel Protein PN23485, Similar to *Hordeum vulgare* Coproporphyrinogen III Oxidase, chloroplast precursor (GENBANK® Accession No. Q42840; e⁻¹⁶⁹) |
| 105, 106 | 29037 | Novel Protein PN29037 |
| 107, 108 | 29950 | Novel Protein PN29950 |
| 109, 110 | 20551 | Hypothetical Protein 003118-3674 Similar to *Lycopersicon esculentum* Calmodulin |
| 111, 112 | 24060 | L-aspartase-like protein-like |
| 113,114 | 23914 | RNA binding domain protein |
| 115, 116 | 23221 | Proline rich protein |
| 117, 118 | 24061 | Auxin induced protein-like |
| 119, 120 | 23949 | HSP70-like |
| 121, 122 | 29042 | Fibrillin-like |
| 123, 124 | 28982 | Archain delta COP-like |
| 125, 126 | 29984 | Novel Protein PN29950 |
| 127, 128 | 30844 | Novel protein PN30844 |
| 129, 130 | 30868 | NAD(P) binding domain protein |
| 131, 132 | 24292 | Gamma adaptin-like |
| 133, 1134 | 29983 | Novel protein PN29983 |
| 135, 136 | 30845 | Pectinesterase-like |
| 137, 138 | 31085 | Receptor-like protein kinase-like |
| 139, 140 | 20674 | Pyruvate orthophosphate dikinase-like |
| 141, 142 | 30870 | Isp-4 like |
| 143, 144 | 29997 | Xanthine dehydrogenase-like |
| 145, 146 | 30843 | Ubiquitin specific protease-like |
| 147, 148 | 30857 | Novel protein PN30857 |
| 149, 150 | 20115 | Ring zinc finger protein |
| 151, 152 | 22823 | Novel Protein PN22823, Similar to ABC Transporter Proteins (GENBANK® Accession Nos. T02187, AB043999.1, NP_171753; e=0) |
| 153, 154 | 22154 | Novel Protein PN22154, Similar to *A. thaliana* Glutamyl Aminopeptidase (GENBANK® Accession No. AL035525; e=0) |
| 155, 156 | 29041 | Novel Protein PN29041, Fragment, Similar to *A. thaliana* Putative ATPase (GENBANK® Accession No. AAG52137; e⁻¹⁷) |
| 157, 158 | 22020 | Novel Protein PN22020, Fragment, Similar to *A*. *thaliana* Putative Protein (GENBANK® Accession No. NP_197783; 3e⁻³⁴) |
| 159, 160 | 22825 | Novel Protein PN22825, Fragment |
| 161, 162 | 29076 | Novel Protein PN29076, Fragment |
| 163, 164 | 29077 | Novel Protein PN29077, Fragment, Similar to *A*. *thaliana* DNA-Damage Inducible Protein DDI1-Like (GENBANK® Accession No. BAB02792; 5e⁻⁹⁴) |
| 165, 166 | 29084 | Novel Protein PN29084, Fragment, Similar to Soybean (*Glycine max*) Calcium-Dependent Protein Kinase (GENBANK® Accession No. A43713, 2e⁻⁷⁹) |
| 167, 168 | 29115 | Novel Protein PN29115, Fragment, Similar to *A*. *thaliana* 6,7-Dimethyl-8-Ribityllumazine Synthase Precursor (GENBANK® Accession No. AAK93590, 6e⁻³⁷) |
| 169, 170 | 29116 | Novel Protein PN29116, Fragment |
| 171, 172 | 29117 | Novel Protein PN29117 |
| 173, 174 | 29118 | Novel Protein PN29118, Fragment |
| 175, 176 | 29119 | Novel Protein PN29119, Fragment |
| 177, 178 | 21639 | Hypothetical Protein ORF020300-2233.2, Putative PP2A Regulatory Subunit, Similar to OsCAA90866(AAD39930; 5e⁻⁹²) (GENBANK® Accession No. CAA90866; 5e⁻⁵³) |
| 179, 180 | 23268 | Novel Protein 23268, Similar to Phosphoribosylanthranilate Transferase, Chloroplast Precursor, Fragment (GENBANK® Accession No. AAB02913.1; 5e⁻⁹⁵) |
| 181, 182 | 26645 | Novel Protein PN26645, Putative Protein Disulfide Isomerase-Related Protein Precursor (GENBANK® Accession No. BAB09470.1; e⁻²⁸) |
| 183, 184 | 24162 | Novel Protein PN24162, Porin-like, Voltage-Dependent Anion Channel Protein (GENBANK® Accession No. NP_201551; 3e⁻⁸⁶) |
| 185, 186 | 20618 | Hypothetical Protein 011994-D16, Similar to *Z*. *mays* DnaJ protein (GENBANK® Accession No. T01643; e=0) |
| 187, 188 | 23045 | Novel Protein PN23045 |
| 189, 190 | 23225 | Novel Protein PN23225, Similar to *Tritticum aestivum* Initiation Factor (iso)4f p82 Subunit (GENBANK® Accession No. AAA74724; e=0) |
| 191, 192 | 29883 | Novel Protein PN29883, Fragment |
| 193, 194 | 19758 | *O. sativa* E2F Homolog (GENBANK® Accession Nos. AB041725; BAB20932) |
| 195, 196 | 23367 | *O. sativa* Kinesin-like Protein (GENBANK® Accession Nos. AC068924; AAG13527.1) |
| 197, 198 | 26317 | *O. sativa* Putative Myosin Heavy Chain (GENBANK® Accession Nos. AC091123; AAK72891) |
| 199, 200 | 20910 | *O. sativa* MADS Box Protein MADS14 (GENBANK® Accession Nos. AF058697, AAF19047) |
| 201, 202 | 20231 | *O. sativa* MADS Box Protein MADS45 (GENBANK® Accession Nos. U31994, AAB50180) |
| 203, 204 | 19695 | *O. sativa* Small GTP-Binding Protein RACDP (GENBANK® Accession Nos. AF218381; AAF28764) |
| 205, 20,6 | 20325 | *O. sativa* Cyclin OsS49462, Fragment (X82035) |
| 207, 208 | 25358 | Hypothetical Protein (GENBANK® Accession No. AAK39589) |
| 209, 210 | 20257 | *O. sativa* Cyclin OsCYCOS2 (GENBANK® Accession No. X82036) |
| 211, 212 | 23363 | *O. sativa* Hypothetical Protein 13324791 |
| 213, 214 | 26210 | *O. sativa* Putative CCAAT Displacement Protein |
| 215, 216 | 23297 | *O. sativa* Putative Myosin Heavy Chain |
| 217, 218 | 23416 | Chloroplast ATPase I Subunit |
| 219, 220 | 23136 | Hypothetical Protein BAA85200 Similar to Syntaxin Related Protein AtVam3p |
| 221, 222 | 25381 | Protein 13357265 Putative CorA-like Mg²⁺ Transporter Protein |
| 223, 224 | 20847 | *O. sativa* OS008339 MADS box transcription factor, fragment (GENBANK® Accession No. AJ293816) |
| 225, 226 | 19766 | *O. sativa* MADS-box protein FDRMADS6 (GENBANK® Accession Nos. AF139664, AAF66997) |
| 227, 228 | 20698 | *O. sativa* MADS-box protein FDRMADS8 (GENBANK® Accession Nos. AF141965, AAD38369) |
| 229, 230 | 19788 | *O. sativa* MADS box protein MADS1 (GENBANK® Accession Nos. AF204063, AAG35652) |
| 231, 232 | 20700 | *O. sativa* MADS box protein MADS3 (GENBANK® Accession Nos. L37528, AAA99964) |
| 233, 234 | 20770 | *O. sativa* MADS box protein MADS5 (GENBANK® Accession Nos. U78890, AAB71434) |
| 235, 236 | 20233 | *O. sativa* MADS box protein MADS6 (GENBANK® Accession Nos. U78782, AAB64250) |
| 237, 238 | 20668 | *O. sativa* MADS box protein MADS 13 (GENBANK® Accession Nos. AF151693, AAF13594) |
| 239, 240 | 20842 | *O. sativa* MADS box protein MADS15 (GENBANK® Accession Nos. AF058698, AAF19048) |
| 241, 242 | 20912 | *O. sativa* MADS box protein MADS18 (GENBANK® Accession Nos. AF091458, AAF04972) |
| 243, 244 | 20232 | *O. sativa* AP1-like MADS box protein RAP1B (GENBANK® Accession Nos. AB041020, BAA94342) |
| 245, 246 | 20837 | *O. sativa* MADS box-like protein (GENBANK® Accession Nos. AB003322, BAA81880) |
| 247, 248 | 21116 | *O. sativa* MADS box protein MADS7 (GENBANK® Accession Nos. U78891, AAC49816) |
| 249, 250 | 20778 | *O. sativa* MADS box protein MADS8 (GENBANK® Accession Nos. U78892, AAC49817) |
| 251, 252 | 20914 | *O. sativa* MADS box transcription factor MADS17 (GENBANK® Accession Nos. AF109153, AAF21900) |
| 253, 254 | 19877 | *O. sativa* Prolamin (GENBANK® Accession Nos. AF156714, AAF73991) |
| 255, 256 | 28517 | *O. sativa* Hypothetical protein BAB56078 (AP003106, BAB56078) |
| 257, 258 | 20559 | *O. sativa* Homeobox Protein HOS59, Fragment (GENBANK® Accession No. BAB55659.1) |
| 259, 260 | 22896 | *O. sativa* Hypothetical Protein, Similar to GTPase Activating Protein (GENBANK® Accession Nos. AF111710; AAD27557) |
| 261, 262 | 25701 | *O. sativa* Putative Myosin (GENBANK® Accession Nos. AC078840; AAG13633) |
| 263, 264 | 23253 | *O. sativa* Putative Homeodomain Protein OsAAK00972 (GENBANK® Accession Nos. AC079736; AAK00972.1) |
| 265, 266 | 23832 | *O. sativa* Putative Eukaryotic Translation Initiation Factor 3 Large Subunit (GENBANK® Accession Nos. AP002487; BAB07943.1) |
| 267, 268 | 20689 | *O. sativa* Probable Myb Factor (GENBANK® Accession No. T03830) |
| 269, 270 | 20466 | *O. sativa* bZIP Transcription Factor (GENBANK® Accession Nos. AB051294; BAB72061.1) |
| 271, 272 | 19697 | *O. sativa* Putative Transcription Factor X1 (GENBANK® Accession Nos. AF101045; AAF21887) |
| 273, 274 | 20080 | Hypothetical Protein 005792-3529 Similar to *O. sativa* Receptor Kinase (GENBANK® Accession Nos. AAK18840.1; 8e⁻⁰⁷) |
| 275, 276 | 20534 | Hypothetical Protein 018049-3655, Fragment, *O. sativa* Putative Homeodomain Transcription Factor, 3'-Partial (GENBANK® Accession Nos. AC092697; AAL58126.1) |
| 277, 278 | 12464 | *O. sativa* 14-3-3 Protein Homolog GF14-c (GENBANK® Accession No. U65957) |
| 279, 280 | 22844 | *O. sativa* 3-Phosphoshikimate 1-carboxyvinyltransferase (EPSP Synthase) (GENBANK® Accession Nos. AB052962; BAB61062.1) |
| 281, 282 | 22832 | *O. sativa* Fructose-Bisphosphate Aldolase, Chloroplast Precursor (GENBANK® Accession No. Q40677) |
| 283, 284 | 23426 | *O. sativa* Chloroplast Ribulose Bisphosphate Carboxylase, Large Chain (GENBANK® Accession Nos. D00207; P12089) |
| 285, 286 | 19842 | *O. sativa* Ribulose Bisphosphate Carboxylase/Oxygenase Activase, Large Isoform A1 (GENBANK® Accession Nos. AB034698, BAA97583) |
| 287, 288 | 23059 | OsContig4331, *O. sativa* Putative 33kDa Oxygen-Evolving Protein of Photosystem II (GENBANK® Accession No. BAB64069) |
| 289, 290 | 22840 | *O. sativa* Photosystem II 10 kDa Polypeptide (GENBANK® Accession Nos. U86018; T04177) |
| 291, 292 | 20251 | *O. sativa* Defender Against Apoptotic Death 1 (GENBANK® Accession Nos. D89727; BAA24104) |
| 293, 294 | 19902 | Beta-Expansin EXPB2 (GENBANK® Accession Nos. U95968; AAB61710) |
| 295, 296 | 20462 | Hypothetical Protein 006819-2510, Similar to Senescence-Related Protein 5 from *Hemerocallis* Hybrid Cultivar (GENBANK® Accession No. AAC34855.1; e⁻⁹⁷) |
| 297, 298 | 24059 | *O. sativa* Histone Deacetylase HD1 (GENBANK® Accession Nos. AF332875; AAK01712.1) |
| 299, 300 | 20544 | *O. sativa* Calreticulin Precursor (GENBANK® Accession Nos. AB021259; BAA88900) |
| 301, 302 | 22883 | *Oryza sativa* Low Temperature-Induced Protein 5 (GENBANK® Accession Nos. AB011368; BAA24979.1) |
| 303, 304 | 23878 | *Oryza sativa* Putative Myosin (GENBANK® Accession Nos. AC090120; AAL31066.1) |
| 305, 306 | 20554 | *O. sativa* DEHYDRIN RAB 16B (GENBANK® Accession No. P22911) |
| 307, 308 | 19701 | Soluble Starch Synthase (GENBANK® Accession Nos. AF165890; AAD49850) |
| 309, 310 | 20285 | OsSGT1 (GENBANK® Accession No. gil6581058) |
| 311, 312 | 20696 | Elicitor responsive protein (GENBANK® Accession No. gi\|11358958) |
| 313, 314 | 24063 | RAS GTPase (GENBANK® Accession No. gi\|730510) |
| 315, 316 | 20621 | Shaggy kinase (GENBANK® Accession No. gi\|13677093) |
| 317, 318 | 19651 | *O. sativa* Chitinase, Class III (GENBANK® Accession Nos. AF296279; AAG02504) |
| 319, 320 | 20899 | *O. sativa* Catalase A Isozyme (GENBANK® Accession Nos. D29966; BAA06232) |
| 321, 322 | 19707 | *O. sativa* Cellulose Synthase Catalytic Subunit, RSW1-Like (GENBANK® Accession Nos. AF030052; AAC39333) |
| 323, 324 | 29086 | *O. sativa* salT Gene Product (GENBANK® Accession Nos. AF001395; AAB53810.1) |
| 325, 326 | 29098 | *O. sativa* Aquaporin (GENBANK® Accession No. AF062393) |
| 327, 328 | 29113 | *O. sativa* DNAJ Homologue (GENBANK® Accession No. BAB70509.1) |
| 329, 330 | 20254 | *O. sativa* Serine/Threonine Protein Phosphatase PP2A- 2, Catalytic Subunit (GENBANK® Accession Nos. AF134552, AAD22116) |
| 331, 332 | 23266 | *O. sativa* Putative Proline-Rich Protein AAK63900 (GENBANK® Accession No. AC084884) |
| 333, 334 | 24775 | *O. sativa* Glutelin CAA33838 (GENBANK® Accession No. X15833) |
| 335, 336 | 20311 | *O. sativa* Chilling-Inducible Protein CAA90866 (GENBANK® Accession Nos. Z54153, CAA90866) |
| 337, 338 | 20215 | *O. sativa* Putative 14-3-3 Protein (GENBANK® Accession No. AAK38492) |
| 339, 340 | 23186 | *O. sativa* Putative Pyrrolidone Carboxyl Peptidase (GENBANK® Accession No. AAG46136) |
| 341, 342 | 25962 | putative protein kinase (GENBANK® Accession Nos: AC082645., AK18843) |
| 343, 344 | 27024 | Rice hypothetical protein (GENBANK® Accession Nos. AP000615, BAA85416) |
| 345, 346 | 20775 | Rice Hsp70 (GENBANK® Accession Nos. X67711, CAA47948) |

SEQ ID NO: 347 is a consensus sequence derived from the alignment depicted in Figures 3A-3D.

SEQ ID NO: 348 is an amino acid sequence of clone PN20278, as shown in Figures 3A-3D.

SEQ ID NO: 349 is an amino acid sequence of clone PN29949b, as shown in Figures 3A-3D.

### Detailed Description

The presently disclosed subject matter will be now be described more fully hereinafter with reference to the accompanying Examples, in which representative embodiments of the presently disclosed subject matter are shown. The presently disclosed subject matter can, however, be embodied in different forms and should not be construed as limited to the embodiments set forth herein. Rather, these embodiments are provided so that this disclosure will be thorough and complete, and will fully convey the scope of the presently disclosed subject matter to those skilled in the art.

All of the patents (including published patent applications) and publications (including GENBANK® sequence references), which are cited herein, are hereby incorporated by reference in their entireties to the same extent as if each were specifically stated to be incorporated by reference. Any inconsistency between these patents and publications and the present disclosure shall be resolved in favor of the present disclosure.

### I. General Considerations

A goal of functional genomics is to identify genes controlling expression of organismal phenotypes, and functional genomics employs a variety of methodologies including, but not limited to, bioinformatics, gene expression studies, gene and gene product interactions, genetics, biochemistry, and molecular genetics. For example, bioinformatics can assign function to a given gene by identifying genes in heterologous organisms with a high degree of similarity (homology) at the amino acid or nucleotide level. Studies of the expression of a gene at the mRNA or polypeptide levels can assign function by linking expression of the gene to an environmental response, a developmental process, or a genetic (mutational) or molecular genetic (gene overexpression or underexpression) perturbation. Expression of a gene at the mRNA level can be ascertained either alone (for example, by Northern analysis) or in concert with other genes (for example, by microarray analysis), whereas expression of a gene at the polypeptide level can be ascertained either alone (for example, by native or denatured polypeptide gel or immunoblot analysis) or in concert with other genes (for example, by proteomic analysis). Knowledge of polypeptide/polypeptide and polypeptide/DNA interactions can assign function by identifying polypeptides and nucleic acid sequences acting together in the same biological process. Genetics can assign function to a gene by demonstrating that DNA lesions (mutations) in the gene have a quantifiable effect on the organism, including, but not limited to, its development; hormone biosynthesis and response; growth and growth habit (plant architecture); mRNA expression profiles; polypeptide expression profiles; ability to resist diseases; tolerance of abiotic stresses (for example, drought conditions); ability to acquire nutrients; photosynthetic efficiency; altered primary and secondary metabolism; and the composition of various plant organs. Biochemistry can assign function by demonstrating that the polypeptide(s) encoded by the gene, typically when expressed in a heterologous organism, possesses a certain enzymatic activity, either alone or in combination with other polypeptides. Molecular genetics can assign function by overexpressing or underexpressing the gene in the native plant or in heterologous organisms, and observing quantifiable effects as disclosed in functional assignment by genetics above. In functional genomics, any or all of these approaches are utilized, often in concert, to assign functions to genes across any of a number of organismal phenotypes.

It is recognized by those skilled in the art that these different methodologies can each provide data as evidence for the function of a particular gene, and that such evidence is stronger with increasing amounts of data used for functional assignment: in one embodiment from a single methodology, in another embodiment from two methodologies, and in still another embodiment from more than two methodologies. In addition, those skilled in the art are aware that different methodologies can differ in the strength of the evidence provided for the assignment of gene function. Typically, but not always, a datum of biochemical, genetic, or molecular genetic evidence is considered stronger than a datum of bioinformatic or gene expression evidence. Finally, those skilled in the art recognize that, for different genes, a single datum from a single methodology can differ in terms of the strength of the evidence provided by each distinct datum for the assignment of the function of these different genes.

The objective of crop trait functional genomics is to identify crop trait genes of interest, for example, genes capable of conferring useful agronomic traits in crop plants. Such agronomic traits include, but are not limited to, enhanced yield, whether in quantity or quality; enhanced nutrient acquisition and metabolic efficiency; enhanced or altered nutrient composition of plant tissues used for food, feed, fiber, or processing; enhanced utility for agricultural or industrial processing; enhanced resistance to plant diseases; enhanced tolerance of adverse environmental conditions (abiotic stresses) including, but not limited to, drought, excessive cold, excessive heat, or excessive soil salinity or extreme acidity or alkalinity; and alterations in plant architecture or development, including changes in developmental timing. The deployment of such identified trait genes by either transgenic or non-transgenic means can materially improve crop plants for the benefit of agriculture.

Cereals are the most important crop plants on the planet in terms of both human and animal consumption. Genomic synteny (conservation of gene order within large chromosomal segments) is observed in rice, maize, wheat, barley, rye, oats, and other agriculturally important monocots, which facilitates the mapping and isolation of orthologous genes from diverse cereal species based on the sequence of a single cereal gene. Rice has the smallest (about 420 Mb) genome among the cereal grains, and has recently been a major focus of public and private genomic and EST sequencing efforts. See Goff et al., 2002.

### II. Definitions

Unless otherwise defined, all technical and scientific terms used herein have the same meaning as commonly understood by one of ordinary skill in the art to which the presently disclosed subject matter pertains. For clarity of the present specification, certain definitions are presented hereinbelow.

Following long-standing patent law convention, the terms "a" and "an" mean "one or more" when used in this application, including in the claims.

As used herein, the term "about", when referring to a value or to an amount of mass, weight, time, volume, concentration or percentage is meant to encompass variations of ±20% or ±10%, in another example ±5%, in another example ±1%, and in still another example ±0.1% from the specified amount, as such variations are appropriate to practice the presently disclosed subject matter. Unless otherwise indicated, all numbers expressing quantities of ingredients, reaction conditions, and so forth used in the specification and claims are to be understood as being modified in all instances by the term "about". Accordingly, unless indicated to the contrary, the numerical parameters set forth in this specification and attached claims are approximations that can vary depending upon the desired properties sought to be obtained by the presently disclosed subject matter.

As used herein, the terms "amino acid" and "amino acid residue" are used interchangeably and refer to any of the twenty naturally occurring amino acids, as well as analogs, derivatives, and congeners thereof; amino acid analogs having variant side chains; and all stereoisomers of any of any of the foregoing. Thus, the term "amino acid" is intended to embrace all molecules, whether natural or synthetic, which include both an amino functionality and an acid functionality and capable of being included in a polymer of naturally occurring amino acids.

An amino acid is formed upon chemical digestion (hydrolysis) of a polypeptide at its peptide linkages. The amino acid residues described herein are in one embodiment in the "L" isomeric form. However, residues in the "D" isomeric form can be substituted for any L-amino acid residue, as long as the desired functional property is retained by the polypeptide. NH₂ refers to the free amino group present at the amino terminus of a polypeptide. COOH refers to the free carboxy group present at the carboxy terminus of a polypeptide. In keeping with standard polypeptide nomenclature abbreviations for amino acid residues are shown in tabular form presented hereinabove.

It is noted that all amino acid residue sequences represented herein by formulae have a left-to-right orientation in the conventional direction of amino terminus to carboxy terminus. In addition, the phrases "amino acid" and "amino acid residue" are broadly defined to include modified and unusual amino acids.

Furthermore, it is noted that a dash at the beginning or end of an amino acid residue sequence indicates a peptide bond to a further sequence of one or more amino acid residues or a covalent bond to an amino-terminal group such as NH₂ or acetyl or to a carboxy-terminal group such as COOH.

As used herein, the terms "associated with" and "operatively linked" refer to two nucleic acid sequences that are related physically or functionally. For example, a promoter or regulatory DNA sequence is said to be "associated with" a DNA sequence that encodes an RNA or a polypeptide if the two sequences are operatively linked, or situated such that the regulator DNA sequence will affect the expression level of the coding or structural DNA sequence.

As used herein, the term "chimera" refers to a polypeptide that comprises domains or other features that are derived from different polypeptides or are in a position relative to each other that is not naturally occurring.

As used herein, the term "chimeric construct" refers to a recombinant nucleic acid molecule in which a promoter or regulatory nucleic acid sequence is operatively linked to, or associated with, a nucleic acid sequence that codes for an mRNA or which is expressed as a polypeptide, such that the regulatory nucleic acid sequence is able to regulate transcription or expression of the associated nucleic acid sequence. The regulatory nucleic acid sequence of the chimeric construct is not normally operatively linked to the associated nucleic acid sequence as found in nature.

As used herein, the term "co-factor" refers to a natural reactant, such as an organic molecule or a metal ion, required in an enzyme-catalyzed reaction. A co-factor can be, for example, NAD(P), riboflavin (including FAD and FMN), folate, molybdopterin, thiamin, biotin, lipoic acid, pantothenic acid and coenzyme A, S-adenosylmethionine, pyridoxal phosphate, ubiquinone, and menaquinone. In one embodiment, a co-factor can be regenerated and reused.

As used herein, the terms "coding sequence" and "open reading frame" (ORF) are used interchangeably and refer to a nucleic acid sequence that is transcribed into RNA such as mRNA, rRNA, tRNA, snRNA, sense RNA, or antisense RNA. In one embodiment, the RNA is then translated in vivo or in vitro to produce a polypeptide.

As used herein, the term "complementary" refers to two nucleotide sequences that comprise antiparallel nucleotide sequences capable of pairing with one another upon formation of hydrogen bonds between the complementary base residues in the antiparallel nucleotide sequences. As is known in the art, the nucleic acid sequences of two complementary strands are the reverse complement of each other when each is viewed in the 5' to 3' direction.

As is also known in the art, two sequences that hybridize to each other under a given set of conditions do not necessarily have to be 100% fully complementary. As used herein, the terms "fully complementary" and "100% complementary" refer to sequences for which the complementary regions are 100% in Watson-Crick base-pairing, i.e., that no mismatches occur within the complementary regions. However, as is often the case with recombinant molecules (for example, cDNAs) that are cloned into cloning vectors, certain of these molecules can have non-complementary overhangs on either the 5' or 3' ends that result from the cloning event. In such a situation, it is understood that the region of 100% or full complementarity excludes any sequences that are added to the recombinant molecule (typically at the ends) solely as a result of, or to facilitate, the cloning event. Such sequences are, for example, polylinker sequences, linkers with restriction enzyme recognition sites, etc.

As used herein, the terms "domain" and "feature", when used in reference to a polypeptide or amino acid sequence, refers to a subsequence of an amino acid sequence that has a particular biological function. Domains and features that have a particular biological function include, but are not limited to, ligand binding, nucleic acid binding, catalytic activity, substrate binding, and polypeptide-polypeptide interacting domains. Similarly, when used herein in reference to a nucleic acid sequence, a "domain", or "feature" is that subsequence of the nucleic acid sequence that encodes a domain or feature of a polypeptide.

As used herein, the term "enzyme activity" refers to the ability of an enzyme to catalyze the conversion of a substrate into a product. A substrate for the enzyme can comprise the natural substrate of the enzyme but also can comprise analogues of the natural substrate, which can also be converted by the enzyme into a product or into an analogue of a product. The activity of the enzyme is measured for example by determining the amount of product in the reaction after a certain period of time, or by determining the amount of substrate remaining in the reaction mixture after a certain period of time. The activity of the enzyme can also be measured by determining the amount of an unused co-factor of the reaction remaining in the reaction mixture after a certain period of time or by determining the amount of used co-factor in the reaction mixture after a certain period of time. The activity of the enzyme can also be measured by determining the amount of a donor of free energy or energy-rich molecule (e.g., ATP, phosphoenolpyruvate, acetyl phosphate, or phosphocreatine) remaining in the reaction mixture after a certain period of time or by determining the amount of a used donor of free energy or energy-rich molecule (e.g., ADP, pyruvate, acetate, or creatine) in the reaction mixture after a certain period of time.

As used herein, the term "expression cassette" refers to a nucleic acid molecule capable of directing expression of a particular nucleotide sequence in an appropriate host cell, comprising a promoter operatively linked to the nucleotide sequence of interest which is operatively linked to termination signals. It also typically comprises sequences required for proper translation of the nucleotide sequence. The coding region usually encodes a polypeptide of interest but can also encode a functional RNA of interest, for example antisense RNA or a non-translated RNA, in the sense or antisense direction. The expression cassette comprising the nucleotide sequence of interest can be chimeric, meaning that at least one of its components is heterologous with respect to at least one of its other components. The expression cassette can also be one that is naturally occurring but has been obtained in a recombinant form useful for heterologous expression. Typically, however, the expression cassette is heterologous with respect to the host; i.e., the particular DNA sequence of the expression cassette does not occur naturally in the host cell and was introduced into the host cell or an ancestor of the host cell by a transformation event. The expression of the nucleotide sequence in the expression cassette can be under the control of a constitutive promoter or of an inducible promoter that initiates transcription only when the host cell is exposed to some particular external stimulus. In the case of a multicellular organism such as a plant, the promoter can also be specific to a particular tissue, organ, or stage of development.

As used herein, the term "fragment" refers to a sequence that comprises a subset of another sequence. When used in the context of a nucleic acid or amino acid sequence, the terms "fragment" and "subsequence" are used interchangeably. A fragment of a nucleic acid sequence can be any number of nucleotides that is less than that found in another nucleic acid sequence, and thus includes, but is not limited to, the sequences of an exon or intron, a promoter, an enhancer, an origin of replication, a 5' or 3' untranslated region, a coding region, and a polypeptide binding domain. It is understood that a fragment or subsequence can also comprise less than the entirety of a nucleic acid sequence, for example, a portion of an exon or intron, promoter, enhancer, etc. Similarly, a fragment or subsequence of an amino acid sequence can be any number of residues that is less than that found in a naturally occurring polypeptide, and thus includes, but is not limited to, domains, features, repeats, etc. Also similarly, it is understood that a fragment or subsequence of an amino acid sequence need not comprise the entirety of the amino acid sequence of the domain, feature, repeat, etc. A fragment can also be a "functional fragment", in which the fragment retains a specific biological function of the nucleic acid sequence or amino acid sequence of interest. For example, a functional fragment of a transcription factor can include, but is not limited to, a DNA binding domain, a transactivating domain, or both. Similarly, a functional fragment of a receptor tyrosine kinase includes, but is not limited to a ligand binding domain, a kinase domain, an ATP binding domain, and combinations thereof.

As used herein, the term "gene" refers to a nucleic acid that encodes an RNA, for example, nucleic acid sequences including, but not limited to, structural genes encoding a polypeptide. The target gene can be a gene derived from a cell, an endogenous gene, a transgene, or exogenous genes such as genes of a pathogen, for example a virus, which is present in the cell after infection thereof. The cell containing the target gene can be derived from or contained in any organism, for example a plant, animal, protozoan, virus, bacterium, or fungus. The term "gene" also refers broadly to any segment of DNA associated with a biological function. As such, the term "gene" encompasses sequences including but not limited to a coding sequence, a promoter region, a transcriptional regulatory sequence, a non-expressed DNA segment that is a specific recognition sequence for regulatory proteins, a non-expressed DNA segment that contributes to gene expression, a DNA segment designed to have desired parameters, or combinations thereof. A gene can be obtained by a variety of methods, including cloning from a biological sample, synthesis based on known or predicted sequence information, and recombinant derivation from one or more existing sequences.

As is understood in the art, a gene comprises a coding strand and a non-coding strand. As used herein, the terms "coding strand" and "sense strand" are used interchangeably, and refer to a nucleic acid sequence that has the same sequence of nucleotide as an mRNA from which the gene product is translated. As is also understood in the art, when the coding strand and/or sense strand is used to refer to a DNA molecule, the coding/sense strand includes thymidine residues instead of the uridine residues found in the corresponding mRNA. Additionally, when used to refer to a DNA molecule, the coding/sense strand can also include additional elements not found in the mRNA including, but not limited to promoters, enhancers, and introns. Similarly, the terms "template strand" and "antisense strand" are used interchangeably and refer to a nucleic acid sequence that is complementary to the coding/sense strand.

As used herein, the terms "complementarity" and "complementary" refer to a nucleic acid that can form one or more hydrogen bonds with another nucleic acid sequence by either traditional Watson-Crick or other non-traditional types of interactions. In reference to the nucleic molecules of the presently disclosed subject matter, the binding free energy for a nucleic acid molecule with its complementary sequence is sufficient to allow the relevant function of the nucleic acid to proceed, in one embodiment, RNAi activity. For example, the degree of complementarity between the sense and antisense strands of the siRNA construct can be the same or different from the degree of complementarity between the antisense strand of the siRNA and the target nucleic acid sequence. Complementarity to the target sequence of less than 100% in the antisense strand of the siRNA duplex, including point mutations, is not well tolerated when these changes are located between the 3'-end and the middle of the antisense siRNA, whereas mutations near the 5'-end of the antisense siRNA strand can exhibit a small degree of RNAi activity (Elbashir et al., 2001). Determination of binding free energies for nucleic acid molecules is well known in the art. See e.g., Freier et al., 1986; Turner et al., 1987.

As used herein, the phrase "percent complementarity" refers to the percentage of contiguous residues in a nucleic acid molecule that can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementary). The terms "100% complementary", "fully complementary", and "perfectly complementary" indicate that all of the contiguous residues of a nucleic acid sequence can hydrogen bond with the same number of contiguous residues in a second nucleic acid sequence.

The term "gene expression" generally refers to the cellular processes by which a biologically active polypeptide is produced from a DNA sequence and exhibits a biological activity in a cell. As such, gene expression involves the processes of transcription and translation, but also involves post-transcriptional and post-translational processes that can influence a biological activity of a gene or gene product. These processes include, but are not limited to RNA syntheses, processing, and transport, as well as polypeptide synthesis, transport, and post-translational modification of polypeptides. Additionally, processes that affect protein-protein interactions within the cell can also affect gene expression as defined herein.

The terms "heterologous", "recombinant", and "exogenous", when used herein to refer to a nucleic acid sequence (e.g., a DNA sequence) or a gene, refer to a sequence that originates from a source foreign to the particular host cell or, if from the same source, is modified from its original form. Thus, a heterologous gene in a host cell includes a gene that is endogenous to the particular host cell but has been modified through, for example, the use of DNA shuffling or other recombinant techniques (for example, cloning the gene into a vector). The terms also include non-naturally occurring multiple copies of a naturally occurring DNA sequence. Thus, the terms refer to a DNA segment that is foreign or heterologous to the cell, or homologous to the cell but in a position or form within the host cell in which the element is not ordinarily found. Similarly, when used in the context of a polypeptide or amino acid sequence, an exogenous polypeptide or amino acid sequence is a polypeptide or amino acid sequence that originates from a source foreign to the particular host cell or, if from the same source, is modified from its original form. Thus, exogenous DNA segments can be expressed to yield exogenous polypeptides.

A "homologous" nucleic acid (or amino acid) sequence is a nucleic acid (or amino acid) sequence naturally associated with a host cell into which it is introduced.

As used herein, the terms "host cells" and "recombinant host cells" are used interchangeably and refer cells (for example, plant cells) into which the compositions of the presently disclosed subject matter (for example, an expression vector) can be introduced. Furthermore, the terms refer not only to the particular plant cell into which an expression construct is initially introduced, but also to the progeny or potential progeny of such a cell. Because certain modifications can occur in succeeding generations due to either mutation or environmental influences, such progeny might not, in fact, be identical to the parent cell, but are still included within the scope of the term as used herein.

The phrase "hybridizing specifically to" refers to the binding, duplexing, or hybridizing of a molecule only to a particular nucleotide sequence under stringent conditions when that sequence is present in a complex mixture (e.g., total cellular) DNA or RNA. The phrase "bind(s) substantially" refers to complementary hybridization between a probe nucleic acid and a target nucleic acid and embraces minor mismatches that can be accommodated by reducing the stringency of the hybridization media to achieve the desired detection of the target nucleic acid sequence.

As used herein, the term "inhibitor" refers to a chemical substance that inactivates or decreases the biological activity of a polypeptide such as a biosynthetic and catalytic activity, receptor, signal transduction polypeptide, structural gene product, or transport polypeptide. The term "herbicide" (or "herbicidal compound") is used herein to define an inhibitor applied to a plant at any stage of development, whereby the herbicide inhibits the growth of the plant or kills the plant.

An "isolated" nucleic acid molecule or protein, or biologically active portion thereof, is substantially free of other cellular material, or culture medium when produced by recombinant techniques, or substantially free of chemical precursors or other chemicals when chemically synthesized. Thus, the term "isolated nucleic acid" refers to a polynucleotide of genomic, cDNA, or synthetic origin or some combination thereof, which (1) is not associated with the cell in which the "isolated nucleic acid" is found in nature, or (2) is operatively linked to a polynucleotide to which it is not linked in nature. Similarly, the term "isolated polypeptide" refers to a polypeptide, in certain embodiments prepared from recombinant DNA or RNA, or of synthetic origin, or some combination thereof, which (1) is not associated with proteins that it is normally found with in nature, (2) is isolated from the cell in which it normally occurs, (3) is isolated free of other proteins from the same cellular source, (4) is expressed by a cell from a different species, or (5) does not occur in nature.

In certain embodiments, an "isolated" nucleic acid is free of sequences (e.g., protein encoding or regulatory sequences) that naturally flank the nucleic acid (i.e., sequences located at the 5' and 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb, or 0.1 kb of the nucleotide sequences that naturally flank the nucleic acid molecule in genomic DNA of the cell from which the nucleic acid is derived. A protein that is substantially free of cellular material includes preparations of protein or polypeptide having less than about 30%, 20%, 10%, or 5%, (by dry weight) of contaminating protein. When the protein of the presently disclosed subject matter, or biologically active portion thereof, is recombinantly produced, culture medium represents less than about 30%, 20%, 10%, or 5% (by dry weight) of chemical precursors or non-protein of interest chemicals. Thus, the term "isolated", when used in the context of an isolated DNA molecule or an isolated polypeptide, refers to a DNA molecule or polypeptide that, by the hand of man, exists apart from its native environment and is therefore not a product of nature. An isolated DNA molecule or polypeptide can exist in a purified form or can exist in a non-native environment such as, for example, in a transgenic host cell.

The term "isolated", when used in the context of an "isolated cell", refers to a cell that has been removed from its natural environment, for example, as a part of an organ, tissue, or organism.

As used herein, the term "mature polypeptide" refers to a polypeptide from which the transit peptide, signal peptide, and/or propeptide portions have been removed.

As used herein, the term "minimal promoter" refers to the smallest piece of a promoter, such as a TATA element, that can support any transcription. A minimal promoter typically has greatly reduced promoter activity in the absence of upstream or downstream activation. In the presence of a suitable transcription factor, a minimal promoter can function to permit transcription.

As used herein, the term "modified enzyme activity" refers to enzyme activity that is different from that which naturally occurs in a plant (i.e. enzyme activity that occurs naturally in the absence of direct or indirect manipulation of such activity by man). In one embodiment, a modified enzyme activity is displayed by a non-naturally occurring enzyme that is tolerant to inhibitors that inhibit the cognate naturally occurring enzyme activity.

As used herein, the term "modulate" refers to an increase, decrease, or other alteration of any, or all, chemical and biological activities or properties of a biochemical entity, e.g., a wild-type or mutant nucleic acid molecule. As such, the term "modulate" can refer to a change in the expression level of a gene, or a level of RNA molecule or equivalent RNA molecules encoding one or more proteins or protein subunits, or activity of one or more proteins or protein subunits is up regulated or down regulated, such that expression, level, or activity is greater than or less than that observed in the absence of the modulator. For example, the term "modulate" can mean "inhibit" or "suppress", but the use of the word "modulate" is not limited to this definition.

As used herein, the terms "inhibit", "suppress", "down regulate", and grammatical variants thereof are used interchangeably and refer to an activity whereby gene expression or a level of an RNA encoding one or more gene products is reduced below that observed in the absence of a nucleic acid molecule of the presently disclosed subject matter. In one embodiment, inhibition with a nucleic acid molecule (for example, a dsRNA, an antisense RNA, or an siRNA) results in a decrease in the steady state level of a target RNA. In another embodiment, inhibition with a a nucleic acid molecule (for example, a dsRNA, an antisense RNA, or an siRNA) results in an expression level of a target gene that is below that level observed in the presence of an inactive or attenuated molecule that is unable to mediate an RNAi response. In another embodiment, inhibition of gene expression with a nucleic acid molecule (for example, a dsRNA, an antisense RNA, or an siRNA) of the presently disclosed subject matter is greater in the presence of the a nucleic acid molecule than in its absence. In still another embodiment, inhibition of gene expression is associated with an enhanced rate of degradation of the mRNA encoded by the gene (for example, by RNAi mediated by an siRNA, a dsRNA, or an antisense RNA).

The term "modulation" as used herein refers to both upregulation (i.e., activation or stimulation) and downregulation (i.e., inhibition or suppression) of a response. Thus, the term "modulation", when used in reference to a functional property or biological activity or process (e.g., enzyme activity or receptor binding), refers to the capacity to upregulate (e.g., activate or stimulate), downregulate (e.g., inhibit or suppress), or otherwise change a quality of such property, activity, or process. In certain instances, such regulation can be contingent on the occurrence of a specific event, such as activation of a signal transduction pathway, and/or can be manifest only in particular cell types.

The term "modulator" refers to a polypeptide, nucleic acid, macromolecule, complex, molecule, small molecule, compound, species, or the like (naturally occurring or non-naturally occurring), or an extract made from biological materials such as bacteria, plants, fungi, or animal cells or tissues, that can be capable of causing modulation. Modulators can be evaluated for potential activity as inhibitors or activators (directly or indirectly) of a functional property, biological activity or process, or combination of them, (e.g., agonist, partial antagonist, partial agonist, inverse agonist, antagonist, anti-microbial agents, inhibitors of microbial infection or proliferation, and the like) by inclusion in assays. In such assays, many modulators can be screened at one time. The activity of a modulator can be known, unknown, or partially known.

Modulators can be either selective or non-selective. As used herein, the term "selective" when used in the context of a modulator (e.g., an inhibitor) refers to a measurable or otherwise biologically relevant difference in the way the modulator interacts with one molecule (e.g., a gene of interest) versus another similar but not identical molecule (e.g., a member of the same gene family as the gene of interest).

It must be understood that it is not required that the degree to which the interactions differ be completely opposite. Put another way, the term selective modulator encompasses not only those molecules that only bind to mRNA transcripts from a gene of interest and not those of related family members. The term is also intended to include modulators that are characterized by interactions with transcripts from genes of interest and from related family members that differ to a lesser degree. For example, selective modulators include modulators for which conditions can be found (such as the degree of sequence identity) that would allow a biologically relevant difference in the binding of the modulator to transcripts form the gene of interest versus transcripts from related genes.

When a selective modulator is identified, the modulator will bind to one molecule (for example an mRNA transcript of a gene of interest) in a manner that is different (for example, stronger) than it binds to another molecule (for example, an mRNA transcript of a gene related to the gene of interest). As used herein, the modulator is said to display "selective binding" or "preferential binding" to the molecule to which it binds more strongly.

As used herein, the term "mutation" carries its traditional connotation and refers to a change, inherited, naturally occurring or introduced, in a nucleic acid or polypeptide sequence, and is used in its sense as generally known to those of skill in the art.

As used herein, the term "native" refers to a gene that is naturally present in the genome of an untransformed plant cell. Similarly, when used in the context of a polypeptide, a "native polypeptide" is a polypeptide that is encoded by a native gene of an untransformed plant cell's genome.

As used herein, the term "naturally occurring" refers to an object that is found in nature as distinct from being artificially produced by man. For example, a polypeptide or nucleotide sequence that is present in an organism (including a virus) in its natural state, which has not been intentionally modified or isolated by man in the laboratory, is naturally occurring. As such, a polypeptide or nucleotide sequence is considered "non-naturally occurring" if it is encoded by or present within a recombinant molecule, even if the amino acid or nucleic acid sequence is identical to an amino acid or nucleic acid sequence found in nature.

As used herein, the terms "nucleic acid" and "nucleic acid molecule" refer to any of deoxyribonucleic acid (DNA), ribonucleic acid (RNA), oligonucleotides, fragments generated by the polymerase chain reaction (PCR), and fragments generated by any of ligation, scission, endonuclease action, and exonuclease action. Nucleic acids can be composed of monomers that are naturally occurring nucleotides (such as deoxyribonucleotides and ribonucleotides), or analogs of naturally occurring nucleotides (e.g., α-enantiomeric forms of naturally occurring nucleotides), or a combination of both. Modified nucleotides can have modifications in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Analogs of phosphodiester linkages include phosphorothioate, phosphorodithioate, phosphoroselenoate, phosphorodiselenoate, phosphoroanilothioate, phosphoranilidate, phosphoramidate, and the like. The term "nucleic acid" also includes so-called "peptide nucleic acids", which comprise naturally occurring or modified nucleic acid bases attached to a polyamide backbone. Nucleic acids can be either single stranded or double stranded.

The term "operatively linked", when describing the relationship between two nucleic acid regions, refers to a juxtaposition wherein the regions are in a relationship permitting them to function in their intended manner. For example, a control sequence "operatively linked" to a coding sequence is ligated in such a way that expression of the coding sequence is achieved under conditions compatible with the control sequences, such as when the appropriate molecules (e.g., inducers and polymerases) are bound to the control or regulatory sequence(s). Thus, in one embodiment, the phrase "operatively linked" refers to a promoter connected to a coding sequence in such a way that the transcription of that coding sequence is controlled and regulated by that promoter. Techniques for operatively linking a promoter to a coding sequence are well known in the art; the precise orientation and location relative to a coding sequence of interest is dependent, inter alia, upon the specific nature of the promoter.

Thus, the term "operatively linked" can refer to a promoter region that is connected to a nucleotide sequence in such a way that the transcription of that nucleotide sequence is controlled and regulated by that promoter region. Similarly, a nucleotide sequence is said to be under the "transcriptional control" of a promoter to which it is operatively linked. Techniques for operatively linking a promoter region to a nucleotide sequence are known in the art. The term "operatively linked" can also refer to a transcription termination sequence or other nucleic acid that is connected to a nucleotide sequence in such a way that termination of transcription of that nucleotide sequence is controlled by that transcription termination sequence. Additionally, the term "operatively linked" can refer to a enhancer, silencer, or other nucleic acid regulatory sequence that when operatively linked to an open reading frame modulates the expression of that open reading frame, either in a positive or negative fashion.

As used herein, the phrase "percent identical"," in the context of two nucleic acid or polypeptide sequences, refers to two or more sequences or subsequences that have in one embodiment 60%, in another embodiment 70%, in another embodiment 80%, in another embodiment 90%, in another embodiment 95%, and in still another embodiment at least 99% nucleotide or amino acid residue identity, respectively, when compared and aligned for maximum correspondence, as measured using one of the following sequence comparison algorithms or by visual inspection. The percent identity exists in one embodiment over a region of the sequences that is at least about 50 residues in length, in another embodiment over a region of at least about 100 residues, and in another embodiment, the percent identity exists over at least about 150 residues. In still another embodiment, the percent identity exists over the entire length of the sequences.

For sequence comparison, typically one sequence acts as a reference sequence to which test sequences are compared. When using a sequence comparison algorithm, test and reference sequences are input into a computer, subsequence coordinates are designated if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, for example, by the local homology algorithm disclosed in Smith & Waterman, 1981, by the homology alignment algorithm disclosed in Needleman & Wunsch, 1970, by the search for similarity method disclosed in Pearson & Lipman, 1988, by computerized implementations of these algorithms (GAP, BESTFIT, FASTA, and TFASTA in the GCG Wisconsin Package, available from Accelrys, Inc., San Diego, California, United States of America), or by visual inspection. See generally, Ausubel et al., 1988.

One example of an algorithm that is suitable for determining percent sequence identity and sequence similarity is the BLAST algorithm, which is described in Altschul et al., 1990. Software for performing BLAST analysis is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive valued threshold score T when aligned with a word of the same length in a database sequence. T is referred to as the neighborhood word score threshold. See generally, Altschul et al., 1990. These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when the cumulative alignment score falls off by the quantity X from its maximum achieved value, the cumulative score goes to zero or below due to the accumulation of one or more negative scoring residue alignments, or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M = 5, N = 4, and a comparison of both strands. For amino acid sequences, the BLASTP program uses as defaults a wordlength (W) of 3, an expectation (E) of 10, and the BLOSUM62 scoring matrix. See Henikoff & Henikoff, 1992.

In addition to calculating percent sequence identity, the BLAST algorithm also performs a statistical analysis of the similarity between two sequences (see e.g., Karlin & Altschul, 1993). One measure of similarity provided by the BLAST algorithm is the smallest sum probability (P(N)), which provides an indication of the probability by which a match between two nucleotide or amino acid sequences would occur by chance. For example, a test nucleic acid sequence is considered similar to a reference sequence if the smallest sum probability in a comparison of the test nucleic acid sequence to the reference nucleic acid sequence is in one embodiment less than about 0.1, in another embodiment less than about 0.01, and in still another embodiment less than about 0.001.

The phrase "hybridizing substantially to" refers to complementary hybridization between a probe nucleic acid molecule and a target nucleic acid molecule and embraces minor mismatches (for example, polymorphisms) that can be accommodated by reducing the stringency of the hybridization and/or wash media to achieve the desired hybridization.

"Stringent hybridization conditions" and "stringent hybridization wash conditions" in the context of nucleic acid hybridization experiments such as Southern and Northern blot analysis are both sequence- and environment-dependent. Longer sequences hybridize specifically at higher temperatures. An extensive guide to the hybridization of nucleic acids is found in Tijssen, 1993. Generally, high stringency hybridization and wash conditions are selected to be about 5°C lower than the thermal melting point (Tₘ) for the specific sequence at a defined ionic strength and pH. Typically, under "highly stringent conditions" a probe will hybridize specifically to its target subsequence, but to no other sequences. Similarly, medium stringency hybridization and wash conditions are selected to be more than about 5°C lower than the Tₘ for the specific sequence at a defined ionic strength and pH. Exemplary medium stringency conditions include hybridizations and washes as for high stringency conditions, except that the temperatures for the hybridization and washes are in one embodiment 8°C, in another embodiment 10°C, in another embodiment 12°C, and in still another embodiment 15°C lower than the Tₘ for the specific sequence at a defined ionic strength and pH.

The Tₘ is the temperature (under defined ionic strength and pH) at which 50% of the target sequence hybridizes to a perfectly matched probe. Very stringent conditions are selected to be equal to the Tₘ for a particular probe. An example of highly stringent hybridization conditions for Southern or Northern Blot analysis of complementary nucleic acids having more than about 100 complementary residues is overnight hybridization in 50% formamide with 1 mg of heparin at 42°C. An example of highly stringent wash conditions is 15 minutes in 0.1x standard saline citrate (SSC), 0.1% (w/v) SDS at 65°C. Another example of highly stringent wash conditions is 15 minutes in 0.2x SSC buffer at 65°C (see Sambrook and Russell, 2001 for a description of SSC buffer and other stringency conditions). Often, a high stringency wash is preceded by a lower stringency wash to remove background probe signal. An example of medium stringency wash conditions for a duplex of more than about 100 nucleotides is 15 minutes in 1X SSC at 45°C. Another example of medium stringency wash for a duplex of more than about 100 nucleotides is 15 minutes in 4-6X SSC at 40°C. For short probes (e.g., about 10 to 50 nucleotides), stringent conditions typically involve salt concentrations of less than about 1 M Na+ ion, typically about 0.01 to 1 M Na+ ion concentration (or other salts) at pH 7.0-8.3, and the temperature is typically at least about 30°C. Stringent conditions can also be achieved with the addition of destabilizing agents such as formamide. In general, a signal to noise ratio of 2-fold (or higher) than that observed for an unrelated probe in the particular hybridization assay indicates detection of a specific hybridization.

The following are examples of hybridization and wash conditions that can be used to clone homologous nucleotide sequences that are substantially similar to reference nucleotide sequences of the presently disclosed subject matter: a probe nucleotide sequence hybridizes in one example to a target nucleotide sequence in 7% sodium dodecyl sulfate (NaDS), 0.5M NaPO4, 1 mm ethylene diamine tetraacetic acid (EDTA) at 50°C followed by washing in 2X SSC, 0.1% NaDS at 50°C; in another example, a probe and target sequence hybridize in 7% NaDS, 0.5 M NaPO4, 1 mm EDTA at 50°C followed by washing in 1X SSC, 0.1 % NaDS at 50°C; in another example, a probe and target sequence hybridize in 7% NaDS, 0.5 M NaPO4, 1 mm EDTA at 50°C followed by washing in 0.5X SSC, 0.1% NaDS at 50°C; in another example, a probe and target sequence hybridize in 7% NaDS, 0.5 M NaPO4, 1 mm EDTA at 50°C followed by washing in 0.1X SSC, 0.1% NaDS at 50°C; in yet another example, a probe and target sequence hybridize in 7% NaDS, 0.5 M NaPO4, 1 mm EDTA at 50°C followed by washing in 0.1X SSC, 0.1% NaDS at 65°C. In one embodiment, hybridization conditions comprise hybridization in a roller tube for at least 12 hours at 42°C.

The term "phenotype" refers to the entire physical, biochemical, and physiological makeup of a cell or an organism, e.g., having any one trait or any group of traits. As such, phenotypes result from the expression of genes within a cell or an organism, and relate to traits that are potentially observable or assayable.

As used herein, the terms "polypeptide", "protein", and "peptide", which are used interchangeably herein, refer to a polymer of the 20 protein amino acids, or amino acid analogs, regardless of its size or function. Although "protein" is often used in reference to relatively large polypeptides, and "peptide" is often used in reference to small polypeptides, usage of these terms in the art overlaps and varies. The term "polypeptide" as used herein refers to peptides, polypeptides and proteins, unless otherwise noted. As used herein, the terms "protein", "polypeptide" and "peptide" are used interchangeably herein when referring to a gene product. The term "polypeptide" encompasses proteins of all functions, including enzymes. Thus, exemplary polypeptides include gene products, naturally occurring proteins, homologs, orthologs, paralogs, fragments, and other equivalents, variants and analogs of the foregoing.

The terms "polypeptide fragment" or "fragment", when used in reference to a reference polypeptide, refers to a polypeptide in which amino acid residues are deleted as compared to the reference polypeptide itself, but where the remaining amino acid sequence is usually identical to the corresponding positions in the reference polypeptide. Such deletions can occur at the amino-terminus or carboxy-terminus of the reference polypeptide, or alternatively both. Fragments typically are at least 5, 6, 8 or 10 amino acids long, at least 14 amino acids long, at least 20, 30, 40 or 50 amino acids long, at least 75 amino acids long, or at least 100, 150, 200, 300, 500 or more amino acids long. A fragment can retain one or more of the biological activities of the reference polypeptide. In certain embodiments, a fragment can comprise a domain or feature, and optionally additional amino acids on one or both sides of the domain or feature, which additional amino acids can number from 5, 10, 15, 20, 30, 40, 50, or up to 100 or more residues. Further, fragments can include a sub-fragment of a specific region, which sub-fragment retains a function of the region from which it is derived. In another embodiment, a fragment can have immunogenic properties.

As used herein, the term "pre-polypeptide" refers to a polypeptide that is normally targeted to a cellular organelle, such as a chloroplast, and still comprises a transit peptide.

As used herein, the term "primer" refers to a sequence comprising in one embodiment two or more deoxyribonucleotides or ribonucleotides, in another embodiment more than three, in another embodiment more than eight, and in yet another embodiment at least about 20 nucleotides of an exonic or intronic region. Such oligonucleotides are in one embodiment between ten and thirty bases in length.

The term "promoter" or "promoter region" each refers to a nucleotide sequence within a gene that is positioned 5' to a coding sequence and functions to direct transcription of the coding sequence. The promoter region comprises a transcriptional start site, and can additionally include one or more transcriptional regulatory elements. In one embodiment, a method of the presently disclosed subject matter employs a RNA polymerase III promoter.

A "minimal promoter" is a nucleotide sequence that has the minimal elements required to enable basal level transcription to occur. As such, minimal promoters are not complete promoters but rather are subsequences of promoters that are capable of directing a basal level of transcription of a reporter construct in an experimental system. Minimal promoters include but are not limited to the CMV minimal promoter, the HSV-tk minimal promoter, the simian virus 40 (SV40) minimal promoter, the human b-actin minimal promoter, the human EF2 minimal promoter, the adenovirus E1B minimal promoter, and the heat shock protein (hsp) 70 minimal promoter. Minimal promoters are often augmented with one or more transcriptional regulatory elements to influence the transcription of an operatively linked gene. For example, cell-type-specific or tissue-specific transcriptional regulatory elements can be added to minimal promoters to create recombinant promoters that direct transcription of an operatively linked nucleotide sequence in a cell-type-specific or tissue-specific manner

Different promoters have different combinations of transcriptional regulatory elements. Whether or not a gene is expressed in a cell is dependent on a combination of the particular transcriptional regulatory elements that make up the gene's promoter and the different transcription factors that are present within the nucleus of the cell. As such, promoters are often classified as "constitutive", "tissue-specific", "cell-type-specific", or "inducible", depending on their functional activities *in vivo* or *in vitro*. For example, a constitutive promoter is one that is capable of directing transcription of a gene in a variety of cell types. Exemplary constitutive promoters include the promoters for the following genes which encode certain constitutive or "housekeeping" functions: hypoxanthine phosphoribosyl transferase (HPRT), dihydrofolate reductase (DHFR; Scharfmann et al., 1991), adenosine deaminase, phosphoglycerate kinase (PGK), pyruvate kinase, phosphoglycerate mutase, the β-actin promoter (see e.g., Williams et al., 1993), and other constitutive promoters known to those of skill in the art. "Tissue-specific" or "cell-type-specific" promoters, on the other hand, direct transcription in some tissues and cell types but are inactive in others. Exemplary tissue-specific promoters include those promoters described in more detail hereinbelow, as well as other tissue-specific and cell-type specific promoters known to those of skill in the art.

When used in the context of a promoter, the term "linked" as used herein refers to a physical proximity of promoter elements such that they function together to direct transcription of an operatively linked nucleotide sequence

The term "transcriptional regulatory sequence" or "transcriptional regulatory element", as used herein, each refers to a nucleotide sequence within the promoter region that enables responsiveness to a regulatory transcription factor. Responsiveness can encompass a decrease or an increase in transcriptional output and is mediated by binding of the transcription factor to the DNA molecule comprising the transcriptional regulatory element. In one embodiment, a transcriptional regulatory sequence is a transcription termination sequence, alternatively referred to herein as a transcription termination signal.

The term "transcription factor" generally refers to a protein that modulates gene expression by interaction with the transcriptional regulatory element and cellular components for transcription, including RNA Polymerase, Transcription Associated Factors (TAFs), chromatin-remodeling proteins, and any other relevant protein that impacts gene transcription.

As used herein, "significance" or "significant" relates to a statistical analysis of the probability that there is a non-random association between two or more entities. To determine whether or not a relationship is "significant" or has "significance", statistical manipulations of the data can be performed to calculate a probability, expressed as a "p-value". Those p-values that fall below a user-defined cutoff point are regarded as significant. In one example, a p-value less than or equal to 0.05, in another example less than 0.01, in another example less than 0.005, and in yet another example less than 0.001, are regarded as significant.

The term "purified" refers to an object species that is the predominant species present (i.e., on a molar basis it is more abundant than any other individual species in the composition). A "purified fraction" is a composition wherein the object species comprises at least about 50 percent (on a molar basis) of all species present. In making the determination of the purity of a species in solution or dispersion, the solvent or matrix in which the species is dissolved or dispersed is usually not included in such determination; instead, only the species (including the one of interest) dissolved or dispersed are taken into account. Generally, a purified composition will have one species that comprises more than about 80 percent of all species present in the composition, more than about 85%, 90%, 95%, 99% or more of all species present. The object species can be purified to essential homogeneity (contaminant species cannot be detected in the composition by conventional detection methods) wherein the composition consists essentially of a single species. A skilled artisan can purify a polypeptide of the presently disclosed subject matter using standard techniques for protein purification in light of the teachings herein. Purity of a polypeptide can be determined by a number of methods known to those of skill in the art, including for example, amino-terminal amino acid sequence analysis, gel electrophoresis, and mass-spectrometry analysis.

A "reference sequence" is a defined sequence used as a basis for a sequence comparison. A reference sequence can be a subset of a larger sequence, for example, as a segment of a full-length nucleotide or amino acid sequence, or can comprise a complete sequence. Generally, when used to refer to a nucleotide sequence, a reference sequence is at least 200, 300 or 400 nucleotides in length, frequently at least 600 nucleotides in length, and often at least 800 nucleotides in length. Because two proteins can each (1) comprise a sequence (i.e., a portion of the complete protein sequence) that is similar between the two proteins, and (2) can further comprise a sequence that is divergent between the two proteins, sequence comparisons between two (or more) proteins are typically performed by comparing sequences of the two proteins over a "comparison window" (defined hereinabove) to identify and compare local regions of sequence similarity.

The term "regulatory sequence" is a generic term used throughout the specification to refer to polynucleotide sequences, such as initiation signals, enhancers, regulators, promoters, and termination sequences, which are necessary or desirable to affect the expression of coding and non-coding sequences to which they are operatively linked. Exemplary regulatory sequences are described in Goeddel, 1990, and include, for example, the early and late promoters of simian virus 40 (SV40), adenovirus or cytomegalovirus immediate early promoter, the lac system, the trp system, the TAC or TRC system, T7 promoter whose expression is directed by T7 RNA polymerase, the major operator and promoter regions of phage lambda, the control regions for fd coat protein, the promoter for 3-phosphoglycerate kinase or other glycolytic enzymes, the promoters of acid phosphatase, e.g., Pho5, the promoters of the yeast a-mating factors, the polyhedron promoter of the baculovirus system and other sequences known to control the expression of genes of prokaryotic or eukaryotic cells or their viruses, and various combinations thereof. The nature and use of such control sequences can differ depending upon the host organism. In prokaryotes, such regulatory sequences generally include promoter, ribosomal binding site, and transcription termination sequences. The term "regulatory sequence" is intended to include, at a minimum, components whose presence can influence expression, and can also include additional components whose presence is advantageous, for example, leader sequences and fusion partner sequences.

In certain embodiments, transcription of a polynucleotide sequence is under the control of a promoter sequence (or other regulatory sequence) that controls the expression of the polynucleotide in a cell-type in which expression is intended. It will also be understood that the polynucleotide can be under the control of regulatory sequences that are the same or different from those sequences which control expression of the naturally occurring form of the polynucleotide.

The term "reporter gene" refers to a nucleic acid comprising a nucleotide sequence encoding a protein that is readily detectable either by its presence or activity, including, but not limited to, luciferase, fluorescent protein (e.g., green fluorescent protein), chloramphenicol acetyl transferase, β-galactosidase, secreted placental alkaline phosphatase, β-lactamase, human growth hormone, and other secreted enzyme reporters. Generally, a reporter gene encodes a polypeptide not otherwise produced by the host cell, which is detectable by analysis of the cell(s), e.g., by the direct fluorometric, radioisotopic or spectrophotometric analysis of the cell(s) and typically without the need to kill the cells for signal analysis. In certain instances, a reporter gene encodes an enzyme, which produces a change in fluorometric properties of the host cell, which is detectable by qualitative, quantitative, or semiquantitative function or transcriptional activation. Exemplary enzymes include esterases, β-lactamase, phosphatases, peroxidases, proteases (tissue plasminogen activator or urokinase) and other enzymes whose function can be detected by appropriate chromogenic or fluorogenic substrates known to those skilled in the art or developed in the future.

As used herein, the term "sequencing" refers to determining the ordered linear sequence of nucleic acids or amino acids of a DNA or protein target sample, using conventional manual or automated laboratory techniques.

As used herein, the term "substantially pure" refers to that the polynucleotide or polypeptide is substantially free of the sequences and molecules with which it is associated in its natural state, and those molecules used in the isolation procedure. The term "substantially free" refers to that the sample is in one embodiment at least 50%, in another embodiment at least 70%, in another embodiment 80% and in still another embodiment 90% free of the materials and compounds with which is it associated in nature.

As used herein, the term "target cell" refers to a cell, into which it is desired to insert a nucleic acid sequence or polypeptide, or to otherwise effect a modification from conditions known to be standard in the unmodified cell. A nucleic acid sequence introduced into a target cell can be of variable length. Additionally, a nucleic acid sequence can enter a target cell as a component of a plasmid or other vector or as a naked sequence.

As used herein, the term "transcription" refers to a cellular process involving the interaction of an RNA polymerase with a gene that directs the expression as RNA of the structural information present in the coding sequences of the gene. The process includes, but is not limited to, the following steps: (a) the transcription initiation; (b) transcript elongation; (c) transcript splicing; (d) transcript capping; (e) transcript termination; (f) transcript polyadenylation; (g) nuclear export of the transcript; (h) transcript editing; and (i) stabilizing the transcript.

As used herein, the term "transcription factor" refers to a cytoplasmic or nuclear protein which binds to a gene, or binds to an RNA transcript of a gene, or binds to another protein which binds to a gene or an RNA transcript or another protein which in turn binds to a gene or an RNA transcript, so as to thereby modulate expression of the gene. Such modulation can additionally be achieved by other mechanisms; the essence of a "transcription factor for a gene" pertains to a factor that alters the level of transcription of the gene in some way.

The term "transfection" refers to the introduction of a nucleic acid, e.g., an expression vector, into a recipient cell, which in certain instances involves nucleic acid-mediated gene transfer. The term "transformation" refers to a process in which a cell's genotype is changed as a result of the cellular uptake of exogenous nucleic acid. For example, a transformed cell can express a recombinant form of a polypeptide of the presently disclosed subject matter or antisense expression can occur from the transferred gene so that the expression of a naturally occurring form of the gene is disrupted.

The term "vector" refers to a nucleic acid capable of transporting another nucleic acid to which it has been linked. One type of vector that can be used in accord with the presently disclosed subject matter is an episome, i.e., a nucleic acid capable of extra-chromosomal replication. Other vectors include those capable of autonomous replication and expression of nucleic acids to which they are linked. Vectors capable of directing the expression of genes to which they are operatively linked are referred to herein as "expression vectors". In general, expression vectors of utility in recombinant DNA techniques are often in the form of plasmids. In the present specification, "plasmid" and "vector" are used interchangeably as the plasmid is the most commonly used form of vector. However, the presently disclosed subject matter is intended to include such other forms of expression vectors which serve equivalent functions and which become known in the art subsequently hereto.

The term "expression vector" as used herein refers to a DNA sequence capable of directing expression of a particular nucleotide sequence in an appropriate host cell, comprising a promoter operatively linked to the nucleotide sequence of interest which is operatively linked to transcription termination sequences. It also typically comprises sequences required for proper translation of the nucleotide sequence. The construct comprising the nucleotide sequence of interest can be chimeric. The construct can also be one that is naturally occurring but has been obtained in a recombinant form useful for heterologous expression. The nucleotide sequence of interest, including any additional sequences designed to effect proper expression of the nucleotide sequences, can also be referred to as an "expression cassette".

The terms "heterologous gene", "heterologous DNA sequence", "heterologous nucleotide sequence", "exogenous nucleic acid molecule", or "exogenous DNA segment", as used herein, each refer to a sequence that originates from a source foreign to an intended host cell or, if from the same source, is modified from its original form. Thus, a heterologous gene in a host cell includes a gene that is endogenous to the particular host cell but has been modified, for example by mutagenesis or by isolation from native transcriptional regulatory sequences. The terms also include non-naturally occurring multiple copies of a naturally occurring nucleotide sequence. Thus, the terms refer to a DNA segment that is foreign or heterologous to the cell, or homologous to the cell but in a position within the host cell nucleic acid wherein the element is not ordinarily found.

Two nucleic acids are "recombined" when sequences from each of the two nucleic acids are combined in a progeny nucleic acid. Two sequences are "directly" recombined when both of the nucleic acids are substrates for recombination. Two sequences are "indirectly recombined" when the sequences are recombined using an intermediate such as a cross over oligonucleotide. For indirect recombination, no more than one of the sequences is an actual substrate for recombination, and in some cases, neither sequence is a substrate for recombination.

As used herein, the term "regulatory elements" refers to nucleotide sequences involved in controlling the expression of a nucleotide sequence. Regulatory elements can comprise a promoter operatively linked to the nucleotide sequence of interest and termination signals. Regulatory sequences also include enhancers and silencers. They also typically encompass sequences required for proper translation of the nucleotide sequence.

As used herein, the term "significant increase" refers to an increase in activity (for example, enzymatic activity) that is larger than the margin of error inherent in the measurement technique, in one embodiment an increase by about 2 fold or greater over a baseline activity (for example, the activity of the wild type enzyme in the presence of the inhibitor), in another embodiment an increase by about 5 fold or greater, and in still another embodiment an increase by about 10 fold or greater.

As used herein, the terms "significantly less" and "significantly reduced" refer to a result (for example, an amount of a product of an enzymatic reaction) that is reduced by more than the margin of error inherent in the measurement technique, in one embodiment a decrease by about 2 fold or greater with respect to a baseline activity (for example, the activity of the wild type enzyme in the absence of the inhibitor), in another embodiment, a decrease by about 5 fold or greater, and in still another embodiment a decrease by about 10 fold or greater.

As used herein, the terms "specific binding" and "immunological cross-reactivity" refer to an indicator that two molecules are substantially similar. An indication that two nucleic acid sequences or polypeptides are substantially similar is that the polypeptide encoded by the first nucleic acid is immunologically cross reactive with, or specifically binds to, the polypeptide encoded by the second nucleic acid. Thus, a polypeptide is typically substantially similar to a second polypeptide, for example, where the two polypeptides differ only by conservative substitutions.

The phrase "specifically (or selectively) binds to an antibody," or "specifically (or selectively) immunoreactive with," when referring to a polypeptide or peptide, refers to a binding reaction which is determinative of the presence of the polypeptide in the presence of a heterogeneous population of polypeptides and other biologics. Thus, under designated immunoassay conditions, the specified antibodies bind to a particular polypeptide and do not bind in a significant amount to other polypeptides present in the sample. Specific binding to an antibody under such conditions can require an antibody that is selected for its specificity for a particular polypeptide. For example, antibodies raised to the polypeptide with the amino acid sequence encoded by any of the nucleic acid sequences of the presently disclosed subject matter can be selected to obtain antibodies specifically immunoreactive with that polypeptide and not with other polypeptides except for polymorphic variants. A variety of immunoassay formats can be used to select antibodies specifically immunoreactive with a particular polypeptide. For example, solid phase ELISA immunoassays, Western blots, or immunohistochemistry are routinely used to select monoclonal antibodies specifically immunoreactive with a polypeptide. See Harlow & Lane, 1988, for a description of immunoassay formats and conditions that can be used to determine specific immunoreactivity. Typically a specific or selective reaction will be at least twice background signal or noise and more typically more than 10 to 100 times background.

As used herein, the term "subsequence" refers to a sequence of nucleic acids or amino acids that comprises a part of a longer sequence of nucleic acids or amino acids (e.g., polypeptide), respectively.

As used herein, the term "substrate" refers to a molecule that an enzyme naturally recognizes and converts to a product in the biochemical pathway in which the enzyme naturally carries out its function; or is a modified version of the molecule, which is also recognized by the enzyme and is converted by the enzyme to a product in an enzymatic reaction similar to the naturally-occurring reaction.

As used herein, the term "suitable growth conditions" refers to growth conditions that are suitable for a certain desired outcome, for example, the production of a recombinant polypeptide or the expression of a nucleic acid molecule.

As used herein, the term "transformation" refers to a process for introducing heterologous DNA into a plant cell, plant tissue, or plant. Transformed plant cells, plant tissue, or plants are understood to encompass not only the end product of a transformation process, but also transgenic progeny thereof.

As used herein, the terms "transformed", "transgenic", and "recombinant" refer to a host organism such as a bacterium or a plant into which a heterologous nucleic acid molecule has been introduced. The nucleic acid molecule can be stably integrated into the genome of the host or the nucleic acid molecule can also be present as an extrachromosomal molecule. Such an extrachromosomal molecule can be auto-replicating. Transformed cells, tissues, or plants are understood to encompass not only the end product of a transformation process, but also transgenic progeny thereof. A "non-transformed," "non-transgenic", or "non-recombinant" host refers to a wild-type organism, e.g., a bacterium or plant, which does not contain the heterologous nucleic acid molecule.

As used herein, the term "viability" refers to a fitness parameter of a plant. Plants are assayed for their homozygous performance of plant development, indicating which polypeptides are essential for plant growth.

### III. Nucleic Acids and Polypeptides

In one aspect, the presently disclosed subject matter provides an isolated nucleic acid molecule encoding a cell proliferation-related polypeptide, wherein the polypeptide binds to a fragment of a protein selected from the group consisting of OsE2F1, Os018989-4003, OsE2F2, OsS49462, OsCYCOS2, OsMADS45, OsRAP1B, OsMADS6, OsFDRMADS8, OsMADS3, OsMADS5, OsMADS15, OsHOS59, OsGF14-c, OsDAD1, Os006819-2510, OsCRTC, OsSGT1, OsERP, OsCHIB1, OsCS, OsPP2A-2, and OsCAA90866. In certain embodiments, the isolated nucleic acid molecule is derived from rice (i.e., *Oryza sativa*).

As used herein, the phrase "cell proliferation-related polypeptide" refers to a protein or polypeptide (note that these two terms are used interchangeably throughout) that is involved in cell proliferation, particularly plant cell proliferation. Such a polypeptide can be involved in an increase in cell proliferation; conversely, such a polypeptide can be involved in the abrogation or inhibition of cell proliferation. Moreover, the polypeptide can be involved in cell proliferation only, for example, when the cell is exposed to a stress (e.g., biotic or abiotic stress). In addition, the polypeptide can be involved in cell proliferation only when the cell is differentiating or developing. A "cell proliferation-related polypeptide" of the presently disclosed subject matter is identified by the ability of an increase or decrease in the level of expression of such a polypeptide in a cell to modulate the rate of that cell's proliferation, whether alone or together with some other stimuli (e.g., presence of growth factor, presence of stress).

As used herein, term "binds" means that a cell proliferation-related polypeptide preferentially interacts with a stated target molecule. In some embodiments, that interaction allows a biological read-out (e.g., a positive in the yeast two-hybrid system). In some embodiments, that interaction is measurable (e.g., a K_{D} of at least 10⁻⁵ M).

Disclosed herein are rice (*O. sativa*)-derived cDNAs encoding plant proteins that interact with OsE2F1, Os018989-4003, OsE2F2, OsS49462, OsCYCOS2, OsMADS45, OsRAP1B, OsMADS6, OsFDRMADS8, OsMADS3, OsMADS5, OsMADS15, OsHOS59, OsGF14-c, OsDAD1, Os006819-2510, OsCRTC, OsSGT1, OsERP, OsCHIB1, OsCS, OsPP2A-2, and OsCAA90866 in the yeast two-hybrid system. All of the cell proliferation-related proteins of the invention are related, and many interact with one another. Figures 1-6 are schematic representations showing the interrelatedness of the different cell proliferation-related proteins of the invention.

In certain embodiments, the presently disclosed subject matter provides an isolated nucleic acid molecule comprising a nucleotide sequence substantially similar to the nucleotide sequence of the nucleic acid molecule encoding a cell proliferation-related polypeptide disclosed herein.

In a broad sense, the term "substantially similar", as used herein with respect to a nucleotide sequence, refers to a nucleotide sequence corresponding to a reference nucleotide sequence (i.e., a nucleotide sequence of a nucleic acid molecule encoding a cell proliferation-related protein of the presently disclosed subject matter), wherein the corresponding sequence encodes a polypeptide having substantially the same structure as the polypeptide encoded by the reference nucleotide sequence. In some embodiments, the substantially similar nucleotide sequence encodes the polypeptide encoded by the reference nucleotide sequence (i.e., although the nucleotide sequence is different, the encoded protein has the same amino acid sequence). In some embodiments, "substantially similar" refers to nucleotide sequences having at least 50% sequence identity, or at least 60%, 70%, 80% or 85%, or at least 90% or 95%, or at least 96%, 97% or 99% sequence identity, compared to a reference sequence containing nucleotide sequences encoding one of the cell proliferation-related proteins of the presently disclosed subject matter (e.g., the proteins described below in the Examples).

"Substantially similar" also refers to nucleotide sequences having at least 50% identity, or at least 80% identity, or at least 95% identity, or at least 99% identity, to a region of nucleotide sequence encoding a BIOPATH protein and/or an Functional Protein Domain (FPD), wherein the nucleotide sequence comparisons are conducted using GAP analysis as described herein. The term "substantially similar" is specifically intended to include nucleotide sequences wherein the sequence has been modified to optimize expression in particular cells.

A polynucleotide including a nucleotide sequence "substantially similar" to the reference nucleotide sequence hybridizes to a polynucleotide including the reference nucleotide sequence in one embodiment in 7% sodium dodecyl sulfate (SDS), 0.5 M NaPO₄, 1 mM ethylenediamine teatraacetic acid (EDTA) at 50°C with washing in 2X standard saline citrate (SSC), 0.1% SDS at 50°C, in another embodiment in 7% sodium dodecyl sulfate (SDS), 0.5 M NaPO₄, 1 mM EDTA at 50°C with washing in 1X SSC, 0.1% SDS at 50°C, in another embodiment in 7% sodium dodecyl sulfate (SDS), 0.5 M NaPO₄, 1 mM EDTA at 50°C with washing in 0.5X SSC, 0.1 % SDS at 50°C, or in 7% sodium dodecyl sulfate (SDS), 0.5 M NaPO₄, 1 mM EDTA at 50°C with washing in 0.1X SSC, 0.1% SDS at 50°C, or in still another embodiment in 7% sodium dodecyl sulfate (SDS), 0.5 M NaPO₄, 1 mM EDTA at 50°C with washing in 0.1X SSC, 0.1% SDS at 65°C.

The term "substantially similar", when used herein with respect to a protein or polypeptide, refers to a protein or polypeptide corresponding to a reference protein (i.e., a cell proliferation-related protein of the presently disclosed subject matter), wherein the protein has substantially the same structure and function as the reference protein, where only changes in amino acids sequence that do not materially affect the polypeptide function occur. When used for a protein or an amino acid sequence the percentage of identity between the substantially similar and the reference protein or amino acid sequence is at least 30%, or at least 40%, 50%, 60%, 70%, 80%, 85%, or 90%, or at least 95%, or at least 99% with every individual number falling within this range of at least 30% to at least 99% also being part of the presently disclosed subject matter, using default GAP analysis parameters with the GCG Wisconsin Package SEQWEB® application of GAP, based on the algorithm of Needleman & Wunsch, 1970.

In one embodiment, the polypeptide is involved in a function such as abiotic stress tolerance, disease resistance, enhanced yield or nutritional quality or composition. In one embodiment, the polypeptide is involved in drought resistance.

In one embodiment, isolated polypeptides comprise the amino acid sequences set forth in even numbered SEQ ID NOs: 2-192, and variants having conservative amino acid modifications. The term "conservative modified variants" refers to polypeptides that can be encoded by nucleic acid sequences having degenerate codon substitutions wherein at least one position of one or more selected (or all) codons is substituted with mixed-base and/or deoxyinosine residues (Batzer et al., 1991; Ohtsuka et al., 1985; Rossolini et al., 1994). Additionally, one skilled in the art will recognize that individual substitutions, deletions, or additions to a nucleic acid, peptide, polypeptide, or polypeptide sequence that alters, adds, or deletes a single amino acid or a small percentage of amino acids in the encoded sequence is a "conservative modification" where the modification results in the substitution of an amino acid with a chemically similar amino acid. Conservative modified variants provide similar biological activity as the unmodified polypeptide. Conservative substitution tables listing functionally similar amino acids are known in the art. See Creighton, 1984.

The term "conservatively modified variant" also refers to a peptide having an amino acid residue sequence substantially similar to a sequence of a polypeptide of the presently disclosed subject matter in which one or more residues have been conservatively substituted with a functionally similar residue. Examples of conservative substitutions include the substitution of one non-polar (hydrophobic) residue such as isoleucine, valine, leucine or methionine for another; the substitution of one polar (hydrophilic) residue for another such as between arginine and lysine, between glutamine and asparagine, between glycine and serine; the substitution of one basic residue such as lysine, arginine or histidine for another; or the substitution of one acidic residue, such as aspartic acid or glutamic acid for another.

Amino acid substitutions, such as those which might be employed in modifying the polypeptides described herein, are generally based on the relative similarity of the amino acid side-chain substituents, for example, their hydrophobicity, hydrophilicity, charge, size, and the like. An analysis of the size, shape and type of the amino acid side-chain substituents reveals that arginine, lysine and histidine are all positively charged residues; that alanine, glycine and serine are all of similar size; and that phenylalanine, tryptophan and tyrosine all have a generally similar shape. Therefore, based upon these considerations, arginine, lysine and histidine; alanine, glycine and serine; and phenylalanine, tryptophan and tyrosine; are defined herein as biologically functional equivalents. Other biologically functionally equivalent changes will be appreciated by those of skill in the art.

In making biologically functional equivalent amino acid substitutions, the hydropathic index of amino acids can be considered. Each amino acid has been assigned a hydropathic index on the basis of their hydrophobicity and charge characteristics, these are: isoleucine (+ 4.5); valine (+ 4.2); leucine (+ 3.8); phenylalanine (+ 2.8); cysteine (+ 2.5); methionine (+ 1.9); alanine (+ 1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The importance of the hydropathic amino acid index in conferring interactive biological function on a protein is generally understood in the art (Kyte & Doolittle, 1982, incorporated herein by reference). It is known that certain amino acids can be substituted for other amino acids having a similar hydropathic index or score and still retain a similar biological activity. Substitutions of amino acids involve amino acids for which the hydropathic indices are in one embodiment within ±2 of the original value, in another embodiment within ±1 of the original value, and in still another embodiment within ±0.5 of the original value in making changes based upon the hydropathic index.

It is also understood in the art that the substitution of like amino acids can be made effectively on the basis of hydrophilicity. U.S. Pat. No. 4,554,101, incorporated herein by reference, states that the greatest local average hydrophilicity of a protein, as governed by the hydrophilicity of its adjacent amino acids, correlates with its immunogenicity and antigenicity, i.e. with a biological property of the protein. It is understood that an amino acid can be substituted for another having a similar hydrophilicity value and still obtain a biologically equivalent protein.

As detailed in U.S. Patent No. 4,554,101, the following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4).

Substitutions of amino acids involve amino acids for which the hydrophilicity values are in one embodiment within ±2 of the original value, in another embodiment within ±1 of the original value, and in still another embodiment within ±0.5 of the original value in making changes based upon similar hydrophilicity values.

While discussion has focused on functionally equivalent polypeptides arising from amino acid changes, it will be appreciated that these changes can be effected by alteration of the encoding DNA, taking into consideration also that the genetic code is degenerate and that two or more codons can code for the same amino acid.

In one embodiment, the polypeptide is expressed in a specific location or tissue of a plant. In one embodiment, the location or tissue includes, but is not limited to, epidermis, vascular tissue, meristem, cambium, cortex, or pith. In another embodiment, the location or tissue is leaf or sheath, root, flower, and developing ovule or seed. In another embodiment, the location or tissue can be, for example, epidermis, root, vascular tissue, meristem, cambium, cortex, pith, leaf, or flower. In yet another embodiment, the location or tissue is a seed.

The polypeptides of the presently disclosed subject matter, fragments thereof, or variants thereof, can comprise any number of contiguous amino acid residues from a polypeptide of the presently disclosed subject matter, wherein the number of residues is selected from the group of integers consisting of from 10 to the number of residues in a full-length polypeptide of the presently disclosed subject matter. In one embodiment, the portion or fragment of the polypeptide is a functional polypeptide. The presently disclosed subject matter includes active polypeptides having specific activity of at least in one embodiment 20%, in another embodiment 30%, in another embodiment 40%, in another embodiment 50%, in another embodiment 60%, in another embodiment 70%, in another embodiment 80%, in another embodiment 90%, and in still another embodiment 95% that of the native (non-synthetic) endogenous polypeptide. Further, the substrate specificity (k_{cat}/Kₘ) can be substantially similar to the native (non-synthetic), endogenous polypeptide. Typically the Kₘ will be at least in one embodiment 30%, in another embodiment 40%, in another embodiment 50% of the native, endogenous polypeptide; and in another embodiment at least 60%, in another embodiment 70%, in another embodiment 80%, and in yet another embodiment 90% of the native, endogenous polypeptide. Methods of assaying and quantifying measures of activity and substrate specificity are well known to those of skill in the art.

The isolated polypeptides of the presently disclosed subject matter can elicit production of an antibody specifically reactive to a polypeptide of the presently disclosed subject matter when presented as an immunogen. Therefore, the polypeptides of the presently disclosed subject matter can be employed as immunogens for constructing antibodies immunoreactive to a polypeptide of the presently disclosed subject matter for such purposes including, but not limited to, immunoassays or polypeptide purification techniques. Immunoassays for determining binding are well known to those of skill in the art and include, but are not limited to, enzyme-linked immunosorbent assays (ELISAs) and competitive immunoassays.

### IV. The Yeast Two-Hybrid System

The yeast two-hybrid system is a well known system which is based on the finding that most eukaryotic transcription activators are modular (see e.g., Gyuris et al., 1993; Bartel & Fields, 1997; Feys et al., 2001). The yeast two-hybrid system uses: 1) a plasmid that directs the synthesis of a "bait" (a known protein which is brought to the yeast's DNA by being fused to a DNA binding domain); 2) one or more reporter genes ("reporters") with upstream binding, sites for the bait; and 3) a plasmid that directs the synthesis of proteins fused to activation domains and other useful moieties ("activation tagged proteins", or "prey").

In all of the Examples described below, an automated, high-throughput yeast two-hybrid assay technology (provided by Myriad Genetics Inc., Salt Lake City, Utah, United States of America) was used to search for protein interactions with the bait proteins. Briefly, the target protein (e.g., OsE2F1) was expressed in yeast as a fusion to the DNA-binding domain of the yeast Ga14p polypeptide. DNA encoding the target protein or a fragment of this protein was amplified from cDNA by PCR or prepared from an available clone. The resulting DNA fragment was cloned by ligation or recombination into a DNA-binding domain vector (e.g., pGBT9, pGBT.C, pAS2-1) such that an in-frame fusion between the Ga14p and target protein sequences was created. The resulting construct, the target gene construct, was introduced by transformation into a haploid yeast strain.

A screening protocol was then used to search the individual baits against two activation domain libraries of assorted peptide motifs of greater than five million cDNA clones. The libraries were derived from RNA isolated from leaves, stems, and roots of rice plants grown in normal conditions, plus tissues from plants exposed to various stresses (input trait library), and from various seed stages, callus, and early and late panicle (output trait library). To screen, a library of activation domain fusions (i.e., *O. sativa* cDNA cloned into an activation domain vector) was introduced by transformation into a haploid yeast strain of the opposite mating type. The yeast strain that carried the activation domain constructs contained one or more Ga14p-responsive reporter genes, the expression of which can be monitored. Non-limiting examples of some yeast reporter strains include Y190, PJ69, and CBY14a.

Yeast carrying the target gene construct was combined with yeast carrying the activation domain library. The two yeast strains mated to form diploid yeast and were plated on media that selected for expression of one or more Ga14p-responsive reporter genes. Thus, both hybrid proteins (i.e., the target "bait" protein and the activation domain "prey" protein) were expressed in a yeast reporter strain where an interaction between the test proteins results in transcription of the reporter genes *TRP1 and LEU2,* allowing growth on selective medium lacking tryptophan and leucine. Colonies that arose after incubation were selected for further characterization. The activation domain plasmid was isolated from each colony obtained in the two-hybrid search. The sequence of the insert in this construct was obtained by sequence analysis (e.g., Sanger's dideoxy nucleotide chain termination method; see Ausubel et al., 1988, including updates up to 2002). Thus, the identity of positives obtained from these searches was determined by sequence analysis against proprietary and public (e.g., GENBANK®) nucleic acid and protein databases.

Interaction of the activation domain fusion with the target protein was confirmed by testing for the specificity of the interaction. The activation domain construct was co-transformed into a yeast reporter strain with either the original target protein construct or a variety of other DNA-binding domain constructs. Expression of the reporter genes in the presence of the target protein but not with other test proteins indicated that the interaction was genuine.

To further characterize the genes encoding the interacting proteins, the nucleic acid sequences of the baits and preys were compared with nucleic acid sequences present on Torrey Mesa Research Institute (TMRI)'s proprietary GENECHIP® Rice Genome Array (Affymetrix, Santa Clara, California, United States of America; see Zhu et al., 2001). The rice genome array contained 25-mer oligonucleotide probes with sequences corresponding to the 3' ends of 21,000 predicted open reading frames found in approximately 42,000 contigs that make up the rice genome map (see Goff et al., 2002). Sixteen different probes were used to measure the expression level of each nucleic acid. The sequences of the probes are available at http://tmri.org/gene_exp_web/. The calculated expression value was determined based on the observed expression level minus the noise background associated with each probe. Experiments included evaluating the differential gene expression from various plant tissues comprising seed, root, leaf and stem, panicle, and pollen. Gene expression was also measured in plants exposed to environmental cold (i.e., 14°C), osmotic pressure (growth media supplemented with 260 mM mannitol), drought (media supplemented with 25% polyethylene glycol 8000), salt (media supplemented with 150 mM NaCl), abscisic acid (ABA)-inducible stresses (media supplemented with 50 uM ABA; see Chen et al., 2002), infection by the fungal pathogen *Magnaporthe grisea,* and treatment with plant hormones (jasmonic acid (JA; 100 µM), gibberellin (GA3; 50 µM), and abscisic acid) and with herbicides benzylamino purine (BAP; 10 µM), 2,4- dichlorophenoxyacetic acid (2,4-D;2 mg/!),andBL2 (10 µM)).

Many of the cell proliferation-related proteins of the presently disclosed subject matter interact with one another.

### V. Controlling and Modulating the Expression of Nucleic Acid Molecules

### A. General Considerations

One aspect of the presently disclosed subject matter provides compositions and methods for modulating (i.e. increasing or decreasing) the level of nucleic acid molecules and/or polypeptides of the presently disclosed subject matter in plants. In particular, the nucleic acid molecules and polypeptides of the presently disclosed subject matter are expressed constitutively, temporally, or spatially (e.g., at developmental stages), in certain tissues, and/or quantities, which are uncharacteristic of non-recombinantly engineered plants. Therefore, the presently disclosed subject matter provides utility in such exemplary applications as altering the specified characteristics identified above.

The isolated nucleic acid molecules of the presently disclosed subject matter are useful for expressing a polypeptide of the presently disclosed subject matter in a recombinantly engineered cell such as a bacterial, yeast, insect, mammalian, or plant cell. Expressing cells can produce the polypeptide in a non-natural condition (e.g., in quantity, composition, location and/or time) because they have been genetically altered to do so. Those skilled in the art are knowledgeable in the numerous expression systems available for expression of nucleic acids encoding a polypeptide of the presently disclosed subject matter.

In another aspect, the presently disclosed subject matter features a cell proliferation-related polypeptide encoded by a nucleic acid molecule disclosed herein. In certain embodiments, the cell proliferation-related polypeptide is isolated.

The presently disclosed subject matter further provides a method for modifying (i.e. increasing or decreasing) the concentration or composition of a polypeptide of the presently disclosed subject matter in a plant or part thereof. Modification can be effected by increasing or decreasing the concentration and/or the composition (i.e. the ration of the polypeptides of the presently disclosed subject matter) in a plant. The method comprises introducing into a plant cell an expression cassette comprising a nucleic acid molecule of the presently disclosed subject matter as disclosed above to obtain a transformed plant cell or tissue, and culturing the transformed plant cell or tissue. The nucleic acid molecule can be under the regulation of a constitutive or inducible promoter. The method can further comprise inducing or repressing expression of a nucleic acid molecule of a sequence in the plant for a time sufficient to modify the concentration and/or composition in the plant or plant part.

A plant or plant part having modified expression of a nucleic acid molecule of the presently disclosed subject matter can be analyzed and selected using methods known to those skilled in the art including, but not limited to, Southern blotting, DNA sequencing, or PCR analysis using primers specific to the nucleic acid molecule and detecting amplicons produced therefrom.

In general, a concentration or composition is increased or decreased by at least in one embodiment 5%, in another embodiment 10%, in another embodiment 20%, in another embodiment 30%, in another embodiment 40%, in another embodiment 50%, in another embodiment 60%, in another embodiment 70%, in another embodiment 80%, and in still another embodiment 90% relative to a native control plant, plant part, or cell lacking the expression cassette.

### B. Modulation of Expression of Nucleic Acid Molecules

The compositions of the presently disclosed subject matter include plant nucleic acid molecules, and the amino acid sequences of the polypeptides or partial-length polypeptides encoded by nucleic acid molecules comprising an open reading frame. These sequences can be employed to alter the expression of a particular gene corresponding to the open reading frame by decreasing or eliminating expression of that plant gene or by overexpressing a particular gene product. Methods of this embodiment of the presently disclosed subject matter include stably transforming a plant with a nucleic acid molecule of the presently disclosed subject matter that includes an open reading frame operatively linked to a promoter capable of driving expression of that open reading frame (sense or antisense) in a plant cell. By "portion" or "fragment", as it relates to a nucleic acid molecule that comprises an open reading frame or a fragment thereof encoding a partial-length polypeptide having the activity of the full length polypeptide, is meant a sequence having in one embodiment at least 80 nucleotides, in another embodiment at least 150 nucleotides, and in still another embodiment at least 400 nucleotides. If not employed for expression, a "portion" or "fragment" means in representative embodiments at least 9, or 12, or 15, or at least 20, consecutive nucleotides (e.g., probes and primers or other oligonucleotides) corresponding to the nucleotide sequence of the nucleic acid molecules of the presently disclosed subject matter. Thus, to express a particular gene product, the method comprises introducing into a plant, plant cell, or plant tissue an expression cassette comprising a promoter operatively linked to an open reading frame so as to yield a transformed differentiated plant, transformed cell, or transformed tissue. Transformed cells or tissue can be regenerated to provide a transformed differentiated plant. The transformed differentiated plant or cells thereof can express the open reading frame in an amount that alters the amount of the gene product in the plant or cells thereof, which product is encoded by the open reading frame. The presently disclosed subject matter also provides a transformed plant prepared by the methodsa disclosed herein, as well as progeny and seed thereof.

The presently disclosed subject matter further includes a nucleotide sequence that is complementary to one (hereinafter "test" sequence) that hybridizes under stringent conditions to a nucleic acid molecule of the presently disclosed subject matter, as well as an RNA molecule that is transcribed from the nucleic acid molecule. When hybridization is performed under stringent conditions, either the test or nucleic acid molecule of presently disclosed subject matter can be present on a support: e.g., on a membrane or on a DNA chip. Thus, either a denatured test or nucleic acid molecule of the presently disclosed subject matter is first bound to a support and hybridization is effected for a specified period of time at a temperature of, in one embodiment, between 55°C and 70°C, in 2X SSC containing 0.1% SDS, followed by rinsing the support at the same temperature but with a buffer having a reduced SSC concentration. Depending upon the degree of stringency required, such reduced concentration buffers are typically 1X SSC containing 0.1% SDS, 0.5X SSC containing 0.1% SDS, or 0.1X SSC containing 0.1 % SDS.

In a further embodiment, the presently disclosed subject matter provides a transformed plant host cell, or one obtained through breeding, capable of over-expressing, under-expressing, or having a knockout of a polypeptide-encoding gene and/or its gene product(s). The plant cell is transformed with at least one such expression vector wherein the plant host cell can be used to regenerate plant tissue or an entire plant, or seed there from, in which the effects of expression, including overexpression and underexpression, of the introduced sequence or sequences can be measured *in vitro* or *in planta*.

In another aspect, the presently disclosed subject matter features an isolated cell proliferation-related polypeptide, wherein the polypeptide binds to a fragment of a protein selected from the group consisting of OsE2F1, Os018989-4003, OsE2F2, OsS49462, OsCYCOS2, OsMADS45, OsRAP1B, OsMADS6, OsFDRMADS8, OsMADS3, OsMADS5, OsMADS15, OsHOS59, OsGF14-c, OsDAD1, Os006819-2510, OsCRTC, OsSGT1, OsPN31085, OsCHIB1, OsCS, OsPP2A-2, and OsCAA90866. In some embodiments, the presently disclosed subject matter features an isolated polypeptide comprising or consisting of an amino acid sequence substantially similar to the amino acid sequence of an isolated cell proliferation-related polypeptide of the presently disclosed subject matter.

Because the proteins of the presently disclosed subject matter have a roll in cell proliferation, in certain embodiments, a cell introduced with a nucleic acid molecule of the presently disclosed subject matter has a different cell proliferation rate as compared to a cell not introduced with the nucleic acid molecule.

In another aspect, the presently disclosed subject matter features a method for modulating the proliferation of a plant cell comprising introducing an isolated nucleic acid molecule encoding a cell proliferation-related polypeptide into the plant cell, wherein the polypeptide binds to a fragment of a protein selected from the group consisting of OsE2F1, Os018989-4003, OsE2F2, OsS49462, OsCYCOS2, OsMADS45, OsRAP1B, OsMADS6, OsFDRMADS8, OsMADS3, OsMADS5, OsMADS15, OsHOS59, OsGF14-c, OsDAD1, Os006819-2510, OsCRTC, OsSGT1, OsERP, OsCHIB1, OsCS, OsPP2A-2, and OsCAA90866, wherein the polypeptide is expressed by the cell.

In another aspect, the presently disclosed subject matter features a method for modulating the proliferation of a plant cell comprising introducing an isolated nucleic acid molecule encoding a cell proliferation-related polypeptide into the plant cell, wherein the polypeptide binds to a fragment of a protein selected from the group consisting of OsE2F1, Os018989-4003, OsE2F2, OsS49462, OsCYCOS2, OsMADS45, OsRAP1B, OsMADS6, OsFDRMADS8, OsMADS3, OsMADS5, OsMADS15, OsHOS59, OsGF14-c, OsDAD1, Os006819-2510, OsCRTC, OsSGT1, OsERP, OsCHIB1, OsCS, OsPP2A-2, and OsCAA90866, wherein expression of the polypeptide encoded by the nucleic acid molecule is reduced in the cell.

As discussed herein, all of the cell proliferation-related proteins described herein affect cell proliferation, either under normal conditions, under adverse conditions (e.g., when the plant is exposed to biotic or abiotic stress), or when the plant is developing and differentiating. Accordingly, by changing the amount of a cell proliferation-related protein of the presently disclosed subject matter in a plant cell, the proliferation of that plant cell can be modulated.

In some situations, increasing expression of a cell proliferation-related protein of the presently disclosed subject matter in a cell will cause that cell to increase its rate of proliferation, either alone or in response to some stimulus (e.g., stress or growth hormone). In other situations, increasing expression of a cell proliferation-related protein of the presently disclosed subject matter in a cell causes that cell to reduce its rate of proliferation. Similarly, decreasing the expression of a cell proliferation-related protein of the presently disclosed subject matter in a cell can increase or decrease that cell's rate of proliferation. What is relevant is that the rate of proliferation of the cell changes if the level of expression of a cell proliferation-related protein of the presently disclosed subject matter is either increased or decreased.

Increasing the level of expression of a cell proliferation-related protein of the presently disclosed subject matter in a cell is a relatively simple matter. For example, overexpression of the protein can be accomplished by transforming the cell with a nucleic acid molecule encoding the protein according to standard methods such as those described above.

Reducing the level of expression of a cell proliferation-related protein of the presently disclosed subject matter in a cell is likewise simply accomplished using standard methods. For example, an antisense RNA or DNA oligonucleotide that is complementary to the sense strand (i.e., the mRNA strand) of a nucleic acid molecule encoding the protein can be administered to the cell to reduce expression of that protein in that cell (see e.g., Agrawal, 1993; U.S. Patent No. 5,929,226).

The modulation in expression of the nucleic acid molecules of the presently disclosed subject matter can be achieved, for example, in one of the following ways:

### 1. "Sense" Suppression

Alteration of the expression of a nucleotide sequence of the presently disclosed subject matter, in one embodiment reduction of its expression, is obtained by "sense" suppression (referenced in e.g., Jorgensen et al., 1996). In this case, the entirety or a portion of a nucleotide sequence of the presently disclosed subject matter is comprised in a DNA molecule. The DNA molecule can be operatively linked to a promoter functional in a cell comprising the target gene, in one embodiment a plant cell, and introduced into the cell, in which the nucleotide sequence is expressible. The nucleotide sequence is inserted in the DNA molecule in the "sense orientation", meaning that the coding strand of the nucleotide sequence can be transcribed. In one embodiment, the nucleotide sequence is fully translatable and all the genetic information comprised in the nucleotide sequence, or portion thereof, is translated into a polypeptide. In another embodiment, the nucleotide sequence is partially translatable and a short peptide is translated. In one embodiment, this is achieved by inserting at least one premature stop codon in the nucleotide sequence, which brings translation to a halt. In another embodiment, the nucleotide sequence is transcribed but no translation product is made. This is usually achieved by removing the start codon, i.e. the "ATG", of the polypeptide encoded by the nucleotide sequence. In a further embodiment, the DNA molecule comprising the nucleotide sequence, or a portion thereof, is stably integrated in the genome of the plant cell. In another embodiment, the DNA molecule comprising the nucleotide sequence, or a portion thereof, is comprised in an extrachromosomally replicating molecule.

In transgenic plants containing one of the DNA molecules disclosed immediately above, the expression of the nucleotide sequence corresponding to the nucleotide sequence comprised in the DNA molecule can be reduced. The nucleotide sequence in the DNA molecule in one embodiment is at least 70% identical to the nucleotide sequence the expression of which is reduced, in another embodiment is at least 80% identical, in another embodiment is at least 90% identical, in another embodiment is at least 95% identical, and in still another embodiment is at least 99% identical.

### 2. "Antisense" Suppression

In another embodiment, the alteration of the expression of a nucleotide sequence of the presently disclosed subject matter, for example the reduction of its expression, is obtained by "antisense" suppression. The entirety or a portion of a nucleotide sequence of the presently disclosed subject matter is comprised in a DNA molecule. The DNA molecule can be operatively linked to a promoter functional in a plant cell, and introduced in a plant cell, in which the nucleotide sequence is expressible. The nucleotide sequence is inserted in the DNA molecule in the "antisense orientation", meaning that the reverse complement (also called sometimes non-coding strand) of the nucleotide sequence can be transcribed. In one embodiment, the DNA molecule comprising the nucleotide sequence, or a portion thereof, is stably integrated in the genome of the plant cell. In another embodiment the DNA molecule comprising the nucleotide sequence, or a portion thereof, is comprised in an extrachromosomally replicating molecule. Several publications describing this approach are cited for further illustration (Green et al., 1986; van der Krol et al., 1991; Powell et al., 1989; Ecker & Davis, 1986).

In transgenic plants containing one of the DNA molecules disclosed immediately above, the expression of the nucleotide sequence corresponding to the nucleotide sequence comprised in the DNA molecule can be reduced. The nucleotide sequence in the DNA molecule is in one embodiment at least 70% identical to the nucleotide sequence the expression of which is reduced, in another embodiment at least 80% identical, in another embodiment at least 90% identical, in another embodiment at least 95% identical, and in still another embodiment at least 99% identical.

### 3. Homologous Recombination

In another embodiment, at least one genomic copy corresponding to a nucleotide sequence of the presently disclosed subject matter is modified in the genome of the plant by homologous recombination as further illustrated in Paszkowski et al., 1988. This technique uses the ability of homologous sequences to recognize each other and to exchange nucleotide sequences between respective nucleic acid molecules by a process known in the art as homologous recombination. Homologous recombination can occur between the chromosomal copy of a nucleotide sequence in a cell and an incoming copy of the nucleotide sequence introduced in the cell by transformation. Specific modifications are thus accurately introduced in the chromosomal copy of the nucleotide sequence. In one embodiment, the regulatory elements of the nucleotide sequence of the presently disclosed subject matter are modified. Such regulatory elements are easily obtainable by screening a genomic library using the nucleotide sequence of the presently disclosed subject matter, or a portion thereof, as a probe. The existing regulatory elements are replaced by different regulatory elements, thus altering expression of the nucleotide sequence, or they are mutated or deleted, thus abolishing the expression of the nucleotide sequence. In another embodiment, the nucleotide sequence is modified by deletion of a part of the nucleotide sequence or the entire nucleotide sequence, or by mutation. Expression of a mutated polypeptide in a plant cell is also provided in the presently disclosed subject matter. Recent refinements of this technique to disrupt endogenous plant genes have been disclosed (Kempin et al., 1997 and Miao & Lam, 1995).

In one embodiment, a mutation in the chromosomal copy of a nucleotide sequence is introduced by transforming a cell with a chimeric oligonucleotide composed of a contiguous stretch of RNA and DNA residues in a duplex conformation with double hairpin caps on the ends. An additional feature of the oligonucleotide is for example the presence of 2'-O-methylation at the RNA residues. The RNA/DNA sequence is designed to align with the sequence of a chromosomal copy of a nucleotide sequence of the presently disclosed subject matter and to contain the desired nucleotide change. For example, this technique is further illustrated in U.S. Patent No. 5,501,967 and Zhu et al., 1999.

### 4. Ribozymes

In a further embodiment, an RNA coding for a polypeptide of the presently disclosed subject matter is cleaved by a catalytic RNA, or ribozyme, specific for such RNA. The ribozyme is expressed in transgenic plants and results in reduced amounts of RNA coding for the polypeptide of the presently disclosed subject matter in plant cells, thus leading to reduced amounts of polypeptide accumulated in the cells. This method is further illustrated in U.S. Patent No. 4,987,071.

### 5. Dominant-Negative Mutants

In another embodiment, the activity of a polypeptide encoded by the nucleotide sequences of the presently disclosed subject matter is changed. This is achieved by expression of dominant negative mutants of the polypeptides in transgenic plants, leading to the loss of activity of the endogenous polypeptide.

### 6. Aptamers

In a further embodiment, the activity of polypeptide of the presently disclosed subject matter is inhibited by expressing in transgenic plants nucleic acid ligands, so-called aptamers, which specifically bind to the polypeptide. Aptamers can be obtained by the SELEX (Systematic Evolution of Ligands by Exponential Enrichment) method. In the SELEX method, a candidate mixture of single stranded nucleic acids having regions of randomized sequence is contacted with the polypeptide and those nucleic acids having an increased affinity to the target are partitioned from the remainder of the candidate mixture. The partitioned nucleic acids are amplified to yield a ligand-enriched mixture. After several iterations a nucleic acid with optimal affinity to the polypeptide is obtained and is used for expression in transgenic plants. This method is further illustrated in U.S. Patent No. 5,270,163.

### 7. Zinc Finger Polypeptides

A zinc finger polypeptide that binds a nucleotide sequence of the presently disclosed subject matter or to its regulatory region can also be used to alter expression of the nucleotide sequence. In alternative embodiments, transcription of the nucleotide sequence is reduced or increased. Zinc finger polypeptides are disclosed in, for example, Beerli et al., 1998, or in WO 95/19431, WO 98/54311, or WO 96/06166, all incorporated herein by reference in their entirety.

### 8. dsRNA

Alteration of the expression of a nucleotide sequence of the presently disclosed subject matter can also be obtained by double stranded RNA (dsRNA) interference (RNAi) as disclosed, for example, in WO 99/32619, WO 99/53050, or WO 99/61631, all incorporated herein by reference in their entireties. In one embodiment, the alteration of the expression of a nucleotide sequence of the presently disclosed subject matter, in one embodiment the reduction of its expression, is obtained by dsRNA interference. The entirety, or in one embodiment a portion, of a nucleotide sequence of the presently disclosed subject matter, can be comprised in a DNA molecule. The size of the DNA molecule is in one embodiment from 100 to 1000 nucleotides or more; the optimal size to be determined empirically. Two copies of the identical DNA molecule are linked, separated by a spacer DNA molecule, such that the first and second copies are in opposite orientations. In one embodiment, the first copy of the DNA molecule is the reverse complement (also known as the non-coding strand) and the second copy is the coding strand; in another embodiment, the first copy is the coding strand, and the second copy is the reverse complement. The size of the spacer DNA molecule is in one embodiment 200 to 10,000 nucleotides, in another embodiment 400 to 5000 nucleotides, and in yet another embodiment 600 to 1500 nucleotides in length. The spacer is in one embodiment a random piece of DNA, in another embodiment a random piece of DNA without homology to the target organism for dsRNA interference, and in still another embodiment a functional intron that is effectively spliced by the target organism. The two copies of the DNA molecule separated by the spacer are operatively linked to a promoter functional in a plant cell, and introduced in a plant cell in which the nucleotide sequence is expressible. In one embodiment, the DNA molecule comprising the nucleotide sequence, or a portion thereof, is stably integrated in the genome of the plant cell. In another embodiment, the DNA molecule comprising the nucleotide sequence, or a portion thereof, is comprised in an extrachromosomally replicating molecule. Several publications describing this approach are cited for further illustration (Waterhouse et al., 1998; Chuang & Meyerowitz, 2000; Smith et al., 2000).

In another non-limiting example, RNA interference (RNAi) or post-transcriptional gene silencing (PTGS) can be employed to reduce the level of expression of a cell proliferation-related protein of the presently disclosed subject matter in a cell. As used herein, the terms "RNA interference" and "post-transcriptional gene silencing" are used interchangeably and refer to a process of sequence-specific modulation of gene expression mediated by a small interfering RNA (siRNA; see generally Fire et al., 1998), resulting in null or hypomorphic phenotypes. Thus, because described herein are nucleotide sequences encoding the cell proliferation-related proteins of the presently disclosed subject matter, RNAi can be readily designed. Indeed, constructs encoding an RNAi molecule have been developed which continuously synthesize an RNAi molecule, resulting in prolonged repression of expression of the targeted gene (Brummelkamp et al., 2002).

In transgenic plants containing one of the DNA molecules disclosed immediately above, the expression of the nucleotide sequence corresponding to the nucleotide sequence comprised in the DNA molecule is in one embodiment reduced. In one embodiment, the nucleotide sequence in the DNA molecule is at least 70% identical to the nucleotide sequence the expression of which is reduced, in another embodiment it is at least 80% identical, in another embodiment it is at least 90% identical, in another embodiment it is at least 95% identical, and in still another embodiment it is at least 99% identical.

### 9. Insertion of a DNA Molecule (Insertional Mutagenesis)

In one embodiment, a DNA molecule is inserted into a chromosomal copy of a nucleotide sequence of the presently disclosed subject matter, or into a regulatory region thereof. In one embodiment, such DNA molecule comprises a transposable element capable of transposition in a plant cell, such as, for example, Ac/Ds, Em/Spm, mutator. Alternatively, the DNA molecule comprises a T-DNA border of an *Agrobacterium* T-DNA. The DNA molecule can also comprise a recombinase or integrase recognition site that can be used to remove part of the DNA molecule from the chromosome of the plant cell. Methods of insertional mutagenesis using T-DNA, transposons, oligonucleotides, or other methods known to those skilled in the art are also encompassed. Methods of using T-DNA and transposon for insertional mutagenesis are disclosed in Winkler & Feldmann, 1989, and Martienssen, 1998, incorporated herein by reference in their entireties.

### 10. Deletion Mutagenesis

In yet another embodiment, a mutation of a nucleic acid molecule of the presently disclosed subject matter is created in the genomic copy of the sequence in the cell or plant by deletion of a portion of the nucleotide sequence or regulator sequence. Methods of deletion mutagenesis are known to those skilled in the art. See e.g., Miao & Lam, 1995.

In yet another embodiment, a deletion is created at random in a large population of plants by chemical mutagenesis or irradiation and a plant with a deletion in a gene of the presently disclosed subject matter is isolated by forward or reverse genetics. Irradiation with fast neutrons or gamma rays is known to cause deletion mutations in plants (Silverstone et al., 1998; Bruggemann et al., 1996; Redei & Koncz, 1992). Deletion mutations in a gene of the presently disclosed subject matter can be recovered in a reverse genetics strategy using PCR with pooled sets of genomic DNAs as has been shown in *C. elegans* (Liu et al., 1999). A forward genetics strategy involves mutagenesis of a line bearing a trait of interest followed by screening the M2 progeny for the absence of the trait. Among these mutants would be expected to be some that disrupt a gene of the presently disclosed subject matter. This could be assessed by Southern blotting or PCR using primers designed for a gene of the presently disclosed subject matter with genomic DNA from these mutants.

### 11. Overexpression in a Plant Cell

In yet another embodiment, a nucleotide sequence of the presently disclosed subject matter encoding a polypeptide is overexpressed. Examples of nucleic acid molecules and expression cassettes for over-expression of a nucleic acid molecule of the presently disclosed subject matter are disclosed above. Methods known to those skilled in the art of over-expression of nucleic acid molecules are also encompassed by the presently disclosed subject matter.

In one embodiment, the expression of the nucleotide sequence of the presently disclosed subject matter is altered in every cell of a plant. This can be obtained, for example, though homologous recombination or by insertion into a chromosome. This can also be obtained, for example, by expressing a sense or antisense RNA, zinc finger polypeptide or ribozyme under the control of a promoter capable of expressing the sense or antisense RNA, zinc finger polypeptide, or ribozyme in every cell of a plant. Constitutive, inducible, tissue-specific, cell type-specific, or developmentally-regulated expression are also within the scope of the presently disclosed subject matter and result in a constitutive, inducible, tissue-specific, or developmentally-regulated alteration of the expression of a nucleotide sequence of the presently disclosed subject matter in the plant cell. Constructs for expression of the sense or antisense RNA, zinc finger polypeptide, or ribozyme, or for over-expression of a nucleotide sequence of the presently disclosed subject matter, can be prepared and transformed into a plant cell according to the teachings of the presently disclosed subject matter, for example, as disclosed herein.

### C. Construction of Plant Expression Vectors

Further encompassed within the presently disclosed subject matter is a recombinant vector comprising an expression cassette according to the embodiments of the presently disclosed subject matter. Also encompassed are plant cells comprising expression cassettes according to the present disclosure, and plants comprising these plant cells. In one embodiment, the plant is a dicot. In another embodiment, the plant is a gymnosperm. In another embodiment, the plant is a monocot. In one embodiment, the monocot is a cereal. In one embodiment, the cereal is, for example, maize, wheat, barley, oats, rye, millet, sorghum, triticale, secale, einkorn, spelt, emmer, teff, milo, flax, gramma grass, *Tripsacum* or *teosinte*. In another embodiment, the cereal is sorghum.

In one embodiment, the expression cassette is expressed throughout the plant. In another embodiment, the expression cassette is expressed in a specific location or tissue of a plant. In one embodiment, the location or tissue includes, but is not limited to, epidermis, root, vascular tissue, meristem, cambium, cortex, pith, leaf, flower, and combinations thereof. In another embodiment, the location or tissue is a seed.

In one embodiment, the expression cassette is involved in a function including, but not limited to, disease resistance, yield, biotic or abiotic stress resistance, nutritional quality, carbon metabolism, photosynthesis, signal transduction, cell growth, reproduction, disease processes (for example, pathogen resistance), gene regulation, and differentiation. In one embodiment, the polypeptide is involved in a function such as biotic or abiotic stress tolerance, enhanced yield or proliferation, disease resistance, or nutritional composition.

For example, a nucleic acid molecule of the presently disclosed subject matter can be introduced, under conditions for expression, into a host cell such that the host cell transcribes and translates the nucleic acid molecule to produce a cell proliferation-related polypeptide. By "under conditions for expression" is meant that a nucleic acid molecule is positioned in the cell such that it will be expressed in that cell. For example, a nucleic acid molecule can be located downstream of a promoter that is active in the cell, such that the promoter will drive the expression of the polypeptide encoded for by the nucleic acid molecule in the cell. Any regulatory sequence (e.g., promoter, enhancer, inducible promoter) can be linked to the nucleic acid molecule; alternatively, the nucleic acid molecule can include its own regulatory sequence(s) such that it will be expressed (i.e., transcribed and/or translated) in a cell.

Where the nucleic acid molecule of the presently disclosed subject matter is introduced into a cell under conditions of expression, that nucleic acid molecule can be included in an expression cassette. Thus, the presently disclosed subject matter further provides a host cell comprising an expression cassette comprising a nucleic acid molecule encoding a cell proliferation-related polypeptide as disclosed herein. Such an expression cassette can include, in addition to the nucleic acid molecule encoding a cell proliferation-related polypeptide of the presently disclosed subject matter, at least one regulatory sequence (e.g., a promoter and/or an enhancer).

As such, coding sequences intended for expression in transgenic plants can be first assembled in expression cassettes operatively linked to a suitable promoter expressible in plants. The expression cassettes can also comprise any further sequences required or selected for the expression of the transgene. Such sequences include, but are not limited to, transcription terminators, extraneous sequences to enhance expression such as introns, vital sequences, and sequences intended for the targeting of the gene product to specific organelles and cell compartments. These expression cassettes can then be easily transferred to the plant transformation vectors disclosed below. The following is a description of various components of typical expression cassettes.

### 1. Promoters

The selection of the promoter used in expression cassettes can determine the spatial and temporal expression pattern of the transgene in the transgenic plant. Selected promoters can express transgenes in specific cell types (such as leaf epidermal cells, mesophyll cells, root cortex cells) or in specific tissues or organs (roots, leaves, or flowers, for example) and the selection can reflect the desired location for accumulation of the gene product. Alternatively, the selected promoter can drive expression of the gene under various inducing conditions. Promoters vary in their strength; i.e., their abilities to promote transcription. Depending upon the host cell system utilized, any one of a number of suitable promoters can be used, including the gene's native promoter. The following are non-limiting examples of promoters that can be used in expression cassettes.

In one non-limiting example, a plant promoter fragment can be employed that will direct expression of the gene in all tissues of a regenerated plant. Such promoters are referred to herein as "constitutive" promoters and are active under most environmental conditions and states of development or cell differentiation. Examples of constitutive promoters include the cauliflower mosaic virus (CaMV) 35S transcription initiation region, the 1'- or 2'-promoter derived from T-DNA of *Agrobacterium tumefaciens*, and other transcription initiation regions from various plant genes known to those of ordinary skill in the art. Such genes include for example, the *AP2* gene, *ACT11* from *Arabidopsis* (Huang et al., 1996), *Cat3* from *Arabidopsis* (GENBANK® Accession No. U43147; Zhong et al., 1996), the gene encoding stearoyl-acyl carrier protein desaturase from *Brassica napus* (GENBANK® Accession No. X74782; Solocombe et al., 1994), *GPc1* from maize (BENBANK® Accession No. X15596; Martinez et al., 1989), and *Gpc2* from maize (GENBANK® Accession No. U45855; Manjunath et al., 1997).

Alternatively, the plant promoter can direct expression of the nucleic acid molecules of the presently disclosed subject matter in a specific tissue or can be otherwise under more precise environmental or developmental control. Examples of environmental conditions that can effect transcription by inducible promoters include anaerobic conditions, elevated temperature, or the presence of light. Such promoters are referred to herein as "inducible", "cell type-specific", or "tissue-specific" promoters. Ordinary skill in the art will recognize that a tissue-specific promoter can drive expression of operatively linked sequences in tissues other than the target tissue. Thus, as used herein a tissue-specific promoter is one that drives expression preferentially in the target tissue, but can also lead to some expression in other tissues as well.

Examples of promoters under developmental control include promoters that initiate transcription only (preferentially) in certain tissues, such as fruit, seeds, or flowers. Promoters that direct expression of nucleic acids in ovules, flowers, or seeds are particularly useful in the presently disclosed subject matter. As used herein a seed-specific or preferential promoter is one that directs expression specifically or preferentially in seed tissues. Such promoters can be, for example, ovule-specific, embryo-specific, endosperm-specific, integument-specific, seed coat-specific, or some combination thereof. Examples include a promoter from the ovule-specific *BEL1* gene described in Reiser et al., 1995 (GENBANK® Accession No. U39944). Non-limiting examples of seed specific promoters are derived from the following genes: *MAC1* from maize (Sheridan et al., 1996), *Cat3* from maize (GENBANK® Accession No. L05934; Abler et al., 1993), the gene encoding oleosin 18 kD from maize (GENBANK® Accession No. J05212; Lee et al., 1994), *vivparous-1* from *Arabidopsis* (GENBANK® Accession No. U93215), the gene encoding oleosin from *Arabidopsis* (GENBANK® Accession No. Z17657), *Atmycl* from *Arabidopsis* (Urao et al., 1996), the 2s seed storage protein gene family from *Arabidopsis* (Conceicao et al., 1994) the gene encoding oleosin 20 kD from *Brassica napus* (GENBANK® Accession No. M63985), *napA* from *Brassica napus* (GENBANK® Accession No. J02798; Josefsson et al., 1987), the *napin* gene family from *Brassica napus* (Sjodahl et al., 1995), the gene encoding the 2S storage protein from *Brassica napus* (Dasgupta et al., 1993), the genes encoding oleosin A (GENBANK® Accession No. U09118) and oleosin B (GENBANK® Accession No. U09119) from soybean, and the gene encoding low molecular weight sulphur rich protein from soybean (Choi et al., 1995).

Alternatively, particular sequences that provide the promoter with desirable expression characteristics, or the promoter with expression enhancement activity, could be identified and these or similar sequences introduced into the sequences via cloning or via mutation. It is further contemplated that these sequences can be mutagenized in order to enhance the expression of transgenes in a particular species.

Furthermore, it is contemplated that promoters combining elements from more than one promoter can be employed. For example, U.S. Patent No. 5,491,288 discloses combining a Cauliflower Mosaic Virus (CaMV) promoter with a histone promoter. Thus, the elements from the promoters disclosed herein can be combined with elements from other promoters.

### a. Constitutive Expression: the Ubiquitin Promoter

Ubiquitin is a gene product known to accumulate in many cell types and its promoter has been cloned from several species for use in transgenic plants (e.g., sunflower - Binet et al., 1991; maize - Christensen et al., 1989; and *Arabidopsis* - Callis et al., 1990; Norris et al., 1993). The maize ubiquitin promoter has been developed in transgenic monocot systems and its sequence and vectors constructed for monocot transformation are disclosed in the patent publication EP 0 342 926 (to Lubrizol) which is herein incorporated by reference. Taylor et al., 1993, describes a vector (pAHC25) that comprises the maize ubiquitin promoter and first intron and its high activity in cell suspensions of numerous monocotyledons when introduced via microprojectile bombardment. The *Arabidopsis* ubiquitin promoter is suitable for use with the nucleotide sequences of the presently disclosed subject matter. The ubiquitin promoter is suitable for gene expression in transgenic plants, both monocotyledons and dicotyledons. Suitable vectors are derivatives of pAHC25 or any of the transformation vectors disclosed herein, modified by the introduction of the appropriate ubiquitin promoter and/or intron sequences.

### b. Constitutive Expression: the CaMV 35S Promoter

Construction of the plasmid pCGN1761 is disclosed in the published patent application EP 0 392 225 (Example 23), which is hereby incorporated by reference. pCGN1761 contains the "double" CaMV 35S promoter and the *tml* transcriptional terminator with a unique EcoRI site between the promoter and the terminator and has a pUC-type backbone. A derivative of pCGN1761 is constructed which has a modified polylinker that includes Notl and XhoI sites in addition to the existing EcoRI site. This derivative is designated pCGN1761ENX. pCGN1761ENX is useful for the cloning of cDNA sequences or coding sequences (including microbial ORF sequences) within its polylinker for the purpose of their expression under the control of the 35S promoter in transgenic plants. The entire 35S promoter-coding sequence-tml terminator cassette of such a construction can be excised by Hindlll, Sphl, SalI, and Xbal sites 5' to the promoter and Xbal, BamHI and BglI sites 3' to the terminator for transfer to transformation vectors such as those disclosed below. Furthermore, the double 35S promoter fragment can be removed by 5' excision with HindIII, Sphl, SalI, Xbal, or PstI, and 3' excision with any of the polylinker restriction sites (EcoRI, NotI or XhoI) for replacement with another promoter. If desired, modifications around the cloning sites can be made by the introduction of sequences that can enhance translation. This is particularly useful when overexpression is desired. For example, pCGN1761 ENX can be modified by optimization of the translational initiation site as disclosed in Example 37 of U.S. Patent No. 5,639,949, incorporated herein by reference.

### c. Constitutive Expression: the Actin Promoter

Several isoforms of actin are known to be expressed in most cell types and consequently the actin promoter can be used as a constitutive promoter. In particular, the promoter from the rice *Actl* gene has been cloned and characterized (McElroy et al., 1990). A 1.3 kilobase (kb) fragment of the promoter was found to contain all the regulatory elements required for expression in rice protoplasts. Furthermore, numerous expression vectors based on the *ActI* promoter have been constructed specifically for use in monocotyledons (McElroy et al., 1991). These incorporate the *ActI*-intron 1, *AdhI* 5' flanking sequence (from the maize alcohol dehydrogenase gene) and Adhl-intron 1 and sequence from the CaMV 35S promoter. Vectors showing highest expression were fusions of 35S and *ActI* intron or the *ActI* 5' flanking sequence and the *ActI* intron. Optimization of sequences around the initiating ATG (of the β-glucuronidase (GUS) reporter gene) also enhanced expression. The promoter expression cassettes disclosed in McElroy et al., 1991, can be easily modified for gene expression and are particularly suitable for use in monocotyledonous hosts. For example, promoter-containing fragments are removed from the McElroy constructions and used to replace the double 35S promoter in pCGN1761ENX, which is then available for the insertion of specific gene sequences. The fusion genes thus constructed can then be transferred to appropriate transformation vectors. In a separate report, the rice *ActI* promoter with its first intron has also been found to direct high expression in cultured barley cells (Chibbar et al., 1993).

### d. Inducible Expression: PR-1 Promoters

The double 35S promoter in pCGN1761 ENX can be replaced with any other promoter of choice that will result in suitably high expression levels. By way of example, one of the chemically regulatable promoters disclosed in U.S. Patent No. 5,614,395, such as the tobacco PR-1a promoter, can replace the double 35S promoter. Alternately, the *Arabidopsis* PR-1 promoter disclosed in Lebel et al., 1998, can be used. The promoter of choice can be excised from its source by restriction enzymes, but can alternatively be PCR-amplified using primers that carry appropriate terminal restriction sites. Should PCR-amplification be undertaken, the promoter can be re-sequenced to check for amplification errors after the cloning of the amplified promoter in the target vector. The chemically/pathogen regulatable tobacco PR-1a promoter is cleaved from plasmid pCIB1004 (for construction, see example 21 of EP 0 332 104, which is hereby incorporated by reference) and transferred to plasmid pCGN1761 ENX (Uknes et al., 1992). pCIB1004 is cleaved with Ncol and the resulting 3' overhang of the linearized fragment is rendered blunt by treatment with T4 DNA polymerase. The fragment is then cleaved with *Hind*III and the resultant PR-1 a promoter-containing fragment is gel purified and cloned into pCGN1761ENX from which the double 35S promoter has been removed. This is accomplished by cleavage with Xhol and blunting with T4 polymerase, followed by cleavage with Hindlll, and isolation of the larger vector-terminator containing fragment into which the pCIB1004 promoter fragment is cloned. This generates a pCGN 1761 ENX derivative with the PR-1 a promoter and the *tml* terminator and an intervening polylinker with unique EcoRI and Notl sites. The selected coding sequence can be inserted into this vector, and the fusion products (i.e. promoter-gene-terminator) can subsequently be transferred to any selected transformation vector, including those disclosed herein. Various chemical regulators can be employed to induce expression of the selected coding sequence in the plants transformed according to the presently disclosed subject matter, including the benzothiadiazole, isonicotinic acid, and salicylic acid compounds disclosed in U.S. Patent Nos. 5,523,311 and 5,614,395.

### e. Inducible Expression: an Ethanol-Inducible Promoter

A promoter inducible by certain alcohols or ketones, such as ethanol, can also be used to confer inducible expression of a coding sequence of the presently disclosed subject matter. Such a promoter is for example the *alcA* gene promoter from *Aspergillus nidulans* (Caddick et al., 1998). In *A. nidulans*, the *alcA* gene encodes alcohol dehydrogenase I, the expression of which is regulated by the AlcR transcription factors in presence of the chemical inducer. For the purposes of the presently disclosed subject matter, the CAT coding sequences in plasmid palcA:CAT comprising a *alcA* gene promoter sequence fused to a minimal 35S promoter (Caddick et al., 1998) are replaced by a coding sequence of the presently disclosed subject matter to form an expression cassette having the coding sequence under the control of the *alcA* gene promoter. This is carried out using methods known in the art.

### f. Inducible Expression: a Glucocorticoid-Inducible Promoter

Induction of expression of a nucleic acid sequence of the presently disclosed subject matter using systems based on steroid hormones is also provided. For example, a glucocorticoid-mediated induction system is used (Aoyama & Chua, 1997) and gene expression is induced by application of a glucocorticoid, for example a synthetic glucocorticoid, for example dexamethasone, at a concentration ranging in one embodiment from 0.1 mM to 1 mM, and in another embodiment from 10 mM to 100 mM. For the purposes of the presently disclosed subject matter, the luciferase gene sequences Aoyama & Chua are replaced by a nucleic acid sequence of the presently disclosed subject matter to form an expression cassette having a nucleic acid sequence of the presently disclosed subject matter under the control of six copies of the GAL4 upstream activating sequences fused to the 35S minimal promoter. This is carried out using methods known in the art. The trans-acting factor comprises the GAL4 DNA-binding domain (Keegan et al., 1986) fused to the transactivating domain of the herpes viral polypeptide VP16 (Triezenberg et al., 1988) fused to the hormone-binding domain of the rat glucocorticoid receptor (Picard et al., 1988). The expression of the fusion polypeptide is controlled either by a promoter known in the art or disclosed herein. A plant comprising an expression cassette comprising a nucleic acid sequence of the presently disclosed subject matter fused to the 6x GAL4/minimal promoter is also provided. Thus, tissue- or organ-specificity of the fusion polypeptide is achieved leading to inducible tissue- or organ-specificity of the nucleic acid sequence to be expressed.

### g. Root Specific Expression

Another pattern of gene expression is root expression. A suitable root promoter is the promoter of the maize metallothionein-like (MTL) gene disclosed in de Framond, 1991, and also in U.S. Patent No. 5,466,785, each of which is incorporated herein by reference. This "MTL" promoter is transferred to a suitable vector such as pCGN1761ENX for the insertion of a selected gene and subsequent transfer of the entire promoter-gene-terminator cassette to a transformation vector of interest.

### h. Wound-Inducible Promoters

Wound-inducible promoters can also be suitable for gene expression. Numerous such promoters have been disclosed (e.g., Xu et al., 1993; Logemann et al., 1989; Rohrmeier & Lehle, 1993; Firek et al., 1993; Warner et al., 1993) and all are suitable for use with the presently disclosed subject matter. Logemann et al. describe the 5' upstream sequences of the dicotyledonous potato *wunI* gene. Xu et al. show that a wound-inducible promoter from the dicotyledon potato (*pin2*) is active in the monocotyledon rice. Further, Rohrmeier & Lehle describe the cloning of the maize *Wipl* cDNA that is wound induced and which can be used to isolate the cognate promoter using standard techniques. Similarly, Firek et al. and Warner et al. have disclosed a wound-induced gene from the monocotyledon *Asparagus officinalis*, which is expressed at local wound and pathogen invasion sites. Using cloning techniques well known in the art, these promoters can be transferred to suitable vectors, fused to the genes pertaining to the presently disclosed subject matter, and used to express these genes at the sites of plant wounding.

### i. Pith-Preferred Expression

PCT International Publication WO 93/07278, which is herein incorporated by reference, describes the isolation of the maize *trpA* gene, which is preferentially expressed in pith cells. The gene sequence and promoter extending up to -1726 basepairs (bp) from the start of transcription are presented. Using standard molecular biological techniques, this promoter, or parts thereof, can be transferred to a vector such as pCGN1761 where it can replace the 35S promoter and be used to drive the expression of a foreign gene in a pith-preferred manner. In fact, fragments containing the pith-preferred promoter or parts thereof can be transferred to any vector and modified for utility in transgenic plants.

### j. Leaf-Specific Expression

A maize gene encoding phosphoenol carboxylase (PEPC) has been disclosed by Hudspeth & Grula, 1989. Using standard molecular biological techniques, the promoter for this gene can be used to drive the expression of any gene in a leaf-specific manner in transgenic plants.

### k. Pollen-Specific Expression

WO 93/07278 describes the isolation of the maize calcium-dependent protein kinase (CDPK) gene that is expressed in pollen cells. The gene sequence and promoter extend up to 1400 bp from the start of transcription. Using standard molecular biological techniques, this promoter or parts thereof can be transferred to a vector such as pCGN1761 where it can replace the 35S promoter and be used to drive the expression of a nucleic acid sequence of the presently disclosed subject matter in a pollen-specific manner.

### 2. Transcriptional Terminators

A variety of 5' and 3' transcriptional regulatory sequences are available for use in the presently disclosed subject matter. Transcriptional terminators are responsible for the termination of transcription and correct mRNA polyadenylation. The 3' nontranslated regulatory DNA sequence includes from in one embodiment about 50 to about 1,000, and in another embodiment about 100 to about 1,000, nucleotide base pairs and contains plant transcriptional and translational termination sequences. Appropriate transcriptional terminators and those that are known to function in plants include the CaMV 35S terminator, the *tml* terminator, the nopaline synthase terminator, the pea rbcS E9 terminator, the terminator for the T7 transcript from the octopine synthase gene of *Agrobacterium tumefaciens*, and the 3' end of the protease inhibitor I or II genes from potato or tomato, although other 3' elements known to those of skill in the art can also be employed. Alternatively, a gamma coixin, oleosin 3, or other terminator from the genus *Coix* can be used.

Non-limiting 3' elements include those from the nopaline synthase gene of *Agrobacterium tumefaciens* (Bevan et al., 1983), the terminator for the T7 transcript from the octopine synthase gene of *Agrobacterium tumefaciens*, and the 3' end of the protease inhibitor I or II genes from potato or tomato.

As the DNA sequence between the transcription initiation site and the start of the coding sequence (i.e., the untranslated leader sequence, also referred to as the 5' untranslated region) can influence gene expression, a particular leader sequence can also be employed. Non-limiting leader sequences are contemplated to include those that include sequences predicted to direct optimum expression of the operatively linked gene; i.e., to include a consensus leader sequence that can increase or maintain mRNA stability and prevent inappropriate initiation of translation. The choice of such sequences will be known to those of skill in the art in light of the present disclosure. Sequences that are derived from genes that are highly expressed in plants are useful in the presently disclosed subject matter.

Thus, a variety of transcriptional terminators are available for use in expression cassettes. These are responsible for termination of transcription and correct mRNA polyadenylation. Appropriate transcriptional terminators are those that are known to function in plants and include the CaMV 35S terminator, the *tml* terminator, the nopaline synthase terminator, and the pea *rbcS* E9 terminator. These can be used in both monocotyledons and dicotyledons. In addition, a gene's native transcription terminator can be used.

### 3. Other Sequences for the Enhancement or Regulation of Expression

Numerous sequences have been found to enhance gene expression from within the transcriptional unit and these sequences can be used in conjunction with the genes of the presently disclosed subject matter to increase their expression in transgenic plants.

Other sequences that have been found to enhance gene expression in transgenic plants include intron sequences (e.g., from *Adh1, bronze1, actin1, actin 2* (PCT International Publication No. WO 00/760067), or the sucrose synthase intron), and viral leader sequences (e.g., from Tobacco Mosaic Virus (TMV), Maize Chlorotic Mottle Virus (MCMV), or Alfalfa Mosaic Virus (AMV)). For example, a number of non-translated leader sequences derived from viruses are known to enhance the expression of operatively linked nucleic acids. Specifically, leader sequences from Tobacco Mosaic Virus (TMV), Maize Chlorotic Mottle Virus (MCMV), and Alfalfa Mosaic Virus (AMV) have been shown to be effective in enhancing expression (e.g., Gallie et al., 1987; Skuzeski et al., 1990). Other leaders known in the art include, but are not limited to picornavirus leaders, for example, encephalomyocarditis virus (EMCV) leader (encephalomyocarditis 5' noncoding region; Elroy-Stein et al., 1989); potyvirus leaders (e.g., Tobacco Etch Virus (TEV) leader and Maize Dwarf Mosaic Virus (MDMV) leader); human immunoglobulin heavy-chain binding protein (BiP) leader (Macejak et al., 1991); untranslated leader from the coat protein mRNA of AMV (AMV RNA 4; Jobling & Gehrke, 1987); TMV leader (Gallie et al., 1989); and maize chlorotic mottle virus leader (Lommel et al., 1991). See also, Della-Cioppa et al., 1987. Regulatory elements such as *Adh* intron 1 (Callis et al., 1987), sucrose synthase intron (Vasil et al., 1989) or TMV omega element (Gallie et al., 1989), can further be included where desired. Non-limiting examples of enhancers include elements from the CaMV 35S promoter, octopine synthase genes (Ellis et al., 1987), the rice actin I gene, the maize alcohol dehydrogenase gene (Callis et al., 1987), the maize shrunken I gene (Vasil et al., 1989), TMV omega element (Gallie et al., 1989) and promoters from non-plant eukaryotes (e.g., yeast; Ma et al., 1988).

A number of non-translated leader sequences derived from viruses are also known to enhance expression, and these are particularly effective in dicotyledonous cells. Specifically, leader sequences from Tobacco Mosaic Virus (TMV; the "W-sequence"), Maize Chlorotic Mottle Virus (MCMV), and Alfalfa Mosaic Virus (AMV) have been shown to be effective in enhancing expression (see e.g., Gallie et al., 1987; Skuzeski et al., 1990). Other leader sequences known in the art include, but are not limited to, picornavirus leaders, for example, EMCV (encephalomyocarditis virus) leader (5' noncoding region; see Elroy-Stein et al., 1989); potyvirus leaders, for example, from Tobacco Etch Virus (TEV; see Allison et al., 1986); Maize Dwarf Mosaic Virus (MDMV; see Kong & Steinbiss 1998); human immunoglobulin heavy-chain binding polypeptide (BiP) leader (Macejak & Sarnow, 1991); untranslated leader from the coat polypeptide mRNA of alfalfa mosaic virus (AMV; RNA 4; see Jobling & Gehrke, 1987); tobacco mosaic virus (TMV) leader (Gallie et al., 1989); and Maize Chlorotic Mottle Virus (MCMV) leader (Lommel et al., 1991). See also, Della-Cioppa et al., 1987.

In addition to incorporating one or more of the aforementioned elements into the 5' regulatory region of a target expression cassette of the presently disclosed subject matter, other elements can also be incorporated. Such elements include, but are not limited to, a minimal promoter. By minimal promoter it is intended that the basal promoter elements are inactive or nearly so in the absence of upstream or downstream activation. Such a promoter has low background activity in plants when there is no transactivator present or when enhancer or response element binding sites are absent. One minimal promoter that is particularly useful for target genes in plants is the Bz1 minimal promoter, which is obtained from the *bronze1* gene of maize. The Bz1 core promoter is obtained from the "myc" mutant Bz1-luciferase construct pBz1LucR98 via cleavage at the *Nhe*l site located at positions -53 to -58 (Roth et al., 1991). The derived Bz1 core promoter fragment thus extends from positions -53 to +227 and includes the Bz1 intron-1 in the 5' untranslated region. Also useful for the presently disclosed subject matter is a minimal promoter created by use of a synthetic TATA element. The TATA element allows recognition of the promoter by RNA polymerase factors and confers a basal level of gene expression in the absence of activation (see generally, Mukumoto et al., 1993; Green, 2000.

### 4. Targeting of the Gene Product Within the Cell

Various mechanisms for targeting gene products are known to exist in plants and the sequences controlling the functioning of these mechanisms have been characterized in some detail. For example, the targeting of gene products to the chloroplast is controlled by a signal sequence found at the amino terminal end of various polypeptides that is cleaved during chloroplast import to yield the mature polypeptides (see e.g., Comai et al., 1988). These signal sequences can be fused to heterologous gene products to affect the import of heterologous products into the chloroplast (Van den Broeck et al., 1985). DNA encoding for appropriate signal sequences can be isolated from the 5' end of the cDNAs encoding the ribulose-1,5-bisphosphate carboxylase/oxygenase (RUBISCO) polypeptide, the chlorophyll a/b binding (CAB) polypeptide, the 5-enol-pyruvyl shikimate-3-phosphate (EPSP) synthase enzyme, the GS2 polypeptide and many other polypeptides which are known to be chloroplast localized. See also, the section entitled "Expression With Chloroplast Targeting" in Example 37 of U.S. Patent No. 5,639,949, herein incorporated by reference.

Other gene products can be localized to other organelles such as the mitochondrion and the peroxisome (e.g., Unger et al., 1989). The cDNAs encoding these products can also be manipulated to effect the targeting of heterologous gene products to these organelles. Examples of such sequences are the nuclear-encoded ATPases and specific aspartate amino transferase isoforms for mitochondria. Targeting cellular polypeptide bodies has been disclosed by Rogers et al., 1985.

In addition, sequences have been characterized that control the targeting of gene products to other cell compartments. Amino terminal sequences are responsible for targeting to the endoplasmic reticulum (ER), the apoplast, and extracellular secretion from aleurone cells (Koehler & Ho, 1990). Additionally, amino terminal sequences in conjunction with carboxy terminal sequences are responsible for vacuolar targeting of gene products (Shinshi et al., 1990).

By the fusion of the appropriate targeting sequences disclosed above to transgene sequences of interest it is possible to direct the transgene product to any organelle or cell compartment. For chloroplast targeting, for example, the chloroplast signal sequence from the RUBISCO gene, the CAB gene, the EPSP synthase gene, or the GS2 gene is fused in frame to the amino terminal ATG of the transgene. The signal sequence selected can include the known cleavage site, and the fusion constructed can take into account any amino acids after the cleavage site that are required for cleavage. In some cases this requirement can be fulfilled by the addition of a small number of amino acids between the cleavage site and the transgene ATG or, alternatively, replacement of some amino acids within the transgene sequence. Fusions constructed for chloroplast import can be tested for efficacy of chloroplast uptake by *in vitro* translation of *in vitro* transcribed constructions followed by *in vitro* chloroplast uptake using techniques disclosed by Bartlett et al., 1982 and Wasmann et al., 1986. These construction techniques are well known in the art and are equally applicable to mitochondria and peroxisomes.

The above-disclosed mechanisms for cellular targeting can be utilized not only in conjunction with their cognate promoters, but also in conjunction with heterologous promoters so as to effect a specific cell-targeting goal under the transcriptional regulation of a promoter that has an expression pattern different from that of the promoter from which the targeting signal derives.

### D. Construction of Plant Transformation Vectors

### 1. Introduction

Numerous transformation vectors available for plant transformation are known to those of ordinary skill in the plant transformation art, and the genes pertinent to the presently disclosed subject matter can be used in conjunction with any such vectors. The selection of vector will depend upon the selected transformation technique and the target species for transformation. For certain target species, different antibiotic or herbicide selection markers might be employed. Selection markers used routinely in transformation include the *nptll* gene, which confers resistance to kanamycin and related antibiotics (Messing & Vieira, 1982; Bevan et al., 1983); the *bar* gene, which confers resistance to the herbicide phosphinothricin (White et al., 1990; Spencer et al., 1990); the *hph* gene, which confers resistance to the antibiotic hygromycin (Blochinger & Diggelmann, 1984); the *dhfr* gene, which confers resistance to methotrexate (Bourouis & Jarry, 1983); the EPSP synthase gene, which confers resistance to glyphosate (U.S. Patent Nos. 4,940,935 and 5,188,642); and the mannose-6-phosphate isomerase gene, which provides the ability to metabolize mannose (U.S. Patent Nos. 5,767,378 and 5,994,629).

The compositions of the presently disclosed subject matter include plant nucleic acid molecules, and the amino acid sequences of the polypeptides or partial-length polypeptides encoded by nucleic acid molecules comprising an open reading frame. These sequences can be employed to alter the expression of a particular gene corresponding to the open reading frame by decreasing or eliminating expression of that plant gene or by overexpressing a particular gene product. Methods of this embodiment of the presently disclosed subject matter include stably transforming a plant with a nucleic acid molecule of the presently disclosed subject matter that includes an open reading frame operatively linked to a promoter capable of driving expression of that open reading frame (sense or antisense) in a plant cell. By "portion" or "fragment", as it relates to a nucleic acid molecule that comprises an open reading frame or a fragment thereof encoding a partial-length polypeptide having the activity of the full length polypeptide, is meant a sequence having in one embodiment at least 80 nucleotides, in another embodiment at least 150 nucleotides, and in still another embodiment at least 400 nucleotides. If not employed for expression, a "portion" or "fragment" means in representative embodiments at least 9, or 12, or 15, or at least 20, consecutive nucleotides (e.g., probes and primers or other oligonucleotides) corresponding to the nucleotide sequence of the nucleic acid molecules of the presently disclosed subject matter. Thus, to express a particular gene product, the method comprises introducing into a plant, plant cell, or plant tissue an expression cassette comprising a promoter operatively linked to an open reading frame so as to yield a transformed differentiated plant, transformed cell, or transformed tissue. Transformed cells or tissue can be regenerated to provide a transformed differentiated plant. The transformed differentiated plant or cells thereof can express the open reading frame in an amount that alters the amount of the gene product in the plant or cells thereof, which product is encoded by the open reading frame. The presently disclosed subject matter also provides a transformed plant prepared by the methodsa disclosed herein, as well as progeny and seed thereof.

The presently disclosed subject matter further includes a nucleotide sequence that is complementary to one (hereinafter "test" sequence) that hybridizes under stringent conditions to a nucleic acid molecule of the presently disclosed subject matter, as well as an RNA molecule that is transcribed from the nucleic acid molecule. When hybridization is performed under stringent conditions, either the test or nucleic acid molecule of presently disclosed subject matter can be present on a support: e.g., on a membrane or on a DNA chip. Thus, either a denatured test or nucleic acid molecule of the presently disclosed subject matter is first bound to a support and hybridization is effected for a specified period of time at a temperature of, in one embodiment, between 55°C and 70°C, in 2X SSC containing 0.1% SDS, followed by rinsing the support at the same temperature but with a buffer having a reduced SSC concentration. Depending upon the degree of stringency required, such reduced concentration buffers are typically 1X SSC containing 0.1% SDS, 0.5X SSC containing 0.1% SDS, or 0.1X SSC containing 0.1 % SDS.

In a further embodiment, the presently disclosed subject matter provides a transformed plant host cell, or one obtained through breeding, capable of over-expressing, under-expressing, or having a knockout of a polypeptide-encoding gene and/or its gene product(s). The plant cell is transformed with at least one such expression vector wherein the plant host cell can be used to regenerate plant tissue or an entire plant, or seed there from, in which the effects of expression, including overexpression and underexpression, of the introduced sequence or sequences can be measured *in vitro* or *in planta*.

In another aspect, the presently disclosed subject matter features an isolated cell proliferation-related polypeptide, wherein the polypeptide binds to a fragment of a protein selected from the group consisting of OsE2F1, Os018989-4003, OsE2F2, OsS49462, OsCYCOS2, OsMADS45, OsRAP1B, OsMADS6, OsFDRMADS8, OsMADS3, OsMADS5, OsMADS15, OsHOS59, OsGF14-c, OsDAD1, Os006819-2510, OsCRTC, OsSGT1, OsPN31085, OsCHIB1, OsCS, OsPP2A-2, and OsCAA90866. In some embodiments, the presently disclosed subject matter features an isolated polypeptide comprising or consisting of an amino acid sequence substantially similar to the amino acid sequence of an isolated cell proliferation-related polypeptide of the presently disclosed subject matter.

Because the proteins of the presently disclosed subject matter have a roll in cell proliferation, in certain embodiments, a cell introduced with a nucleic acid molecule of the presently disclosed subject matter has a different cell proliferation rate as compared to a cell not introduced with the nucleic acid molecule.

In another aspect, the presently disclosed subject matter features a method for modulating the proliferation of a plant cell comprising introducing an isolated nucleic acid molecule encoding a cell proliferation-related polypeptide into the plant cell, wherein the polypeptide binds to a fragment of a protein selected from the group consisting of OsE2F1, Os018989-4003, OsE2F2, OsS49462, OsCYCOS2, OsMADS45, OsRAP1B, OsMADS6, OsFDRMADS8, OsMADS3, OsMADS5, OsMADS15, OsHOS59, OsGF14-c, OsDAD1, Os006819-2510, OsCRTC, OsSGT1, OsERP, OsCHIB1, OsCS, OsPP2A-2, and OsCAA90866, wherein the polypeptide is expressed by the cell.

In another aspect, the presently disclosed subject matter features a method for modulating the proliferation of a plant cell comprising introducing an isolated nucleic acid molecule encoding a cell proliferation-related polypeptide into the plant cell, wherein the polypeptide binds to a fragment of a protein selected from the group consisting of OsE2F1, Os018989-4003, OsE2F2, OsS49462, OsCYCOS2, OsMADS45, OsRAP1B, OsMADS6, OsFDRMADS8, OsMADS3, OsMADS5, OsMADS15, OsHOS59, OsGF14-c, OsDAD1, Os006819-2510, OsCRTC, OsSGT1, OsERP, OsCHIB1, OsCS, OsPP2A-2, and OsCAA90866, wherein expression of the polypeptide encoded by the nucleic acid molecule is reduced in the cell.

As discussed herein, all of the cell proliferation-related proteins described herein affect cell proliferation, either under normal conditions, under adverse conditions (e.g., when the plant is exposed to biotic or abiotic stress), or when the plant is developing and differentiating. Accordingly, by changing the amount of a cell proliferation-related protein of the presently disclosed subject matter in a plant cell, the proliferation of that plant cell can be modulated.

In some situations, increasing expression of a cell proliferation-related protein of the presently disclosed subject matter in a cell will cause that cell to increase its rate of proliferation, either alone or in response to some stimulus (e.g., stress or growth hormone). In other situations, increasing expression of a cell proliferation-related protein of the presently disclosed subject matter in a cell causes that cell to reduce its rate of proliferation. Similarly, decreasing the expression of a cell proliferation-related protein of the presently disclosed subject matter in a cell can increase or decrease that cell's rate of proliferation. What is relevant is that the rate of proliferation of the cell changes if the level of expression of a cell proliferation-related protein of the presently disclosed subject matter is either increased or decreased.

Increasing the level of expression of a cell proliferation-related protein of the presently disclosed subject matter in a cell is a relatively simple matter. For example, overexpression of the protein can be accomplished by transforming the cell with a nucleic acid molecule encoding the protein according to standard methods such as those described above.

Once a nucleic acid sequence of the presently disclosed subject matter has been cloned into an expression system, it is transformed into a plant cell. The receptor and target expression cassettes of the presently disclosed subject matter can be introduced into the plant cell in a number of art-recognized ways. Methods for regeneration of plants are also well known in the art. For example, Ti plasmid vectors have been utilized for the delivery of foreign DNA, as well as direct DNA uptake, liposomes, electroporation, microinjection, and microprojectiles. In addition, bacteria from the genus *Agrobacterium* can be utilized to transform plant cells. Below are descriptions of representative techniques for transforming both dicotyledonous and monocotyledonous plants, as well as a representative plastid transformation technique.

Transformation of a plant can be undertaken with a single DNA molecule or multiple DNA molecules (i.e., co-transformation), and both these techniques are suitable for use with the expression cassettes of the presently disclosed subject matter. Numerous transformation vectors are available for plant transformation, and the expression cassettes of the presently disclosed subject matter can be used in conjunction with any such vectors. The selection of vector will depend upon the transformation technique and the species targeted for transformation.

A variety of techniques are available and known for introduction of nucleic acid molecules and expression cassettes comprising such nucleic acid molecules into a plant cell host. These techniques include, but are not limited to transformation with DNA employing *A. tumefaciens* or *A. rhizogenes* as the transforming agent, liposomes, PEG precipitation, electroporation, DNA injection, direct DNA uptake, microprojectile bombardment, particle acceleration, and the like (see e.g., EP 0 295 959 and EP 0 138 341; see also below). However, cells other than plant cells can be transformed with the expression cassettes of the presently disclosed subject matter. A general descriptions of plant expression vectors and reporter genes, and *Agrobacterium* and *Agrobacterium*-mediated gene transfer, can be found in Gruber et al., 1993, incorporated herein by reference in its entirety.

Expression vectors containing genomic or synthetic fragments can be introduced into protoplasts or into intact tissues or isolated cells. In some embodiments, expression vectors are introduced into intact tissue. "Plant tissue" includes differentiated and undifferentiated tissues or entire plants, including but not limited to roots, stems, shoots, leaves, pollen, seeds, tumor tissue, and various forms of cells and cultures such as single cells, protoplasts, embryos, and callus tissues. The plant tissue can be in plants or in organ, tissue, or cell culture. General methods of culturing plant tissues are provided, for example, by Maki et al., 1993 and by Phillips et al. 1988. In some embodiments, expression vectors are introduced into maize or other plant tissues using a direct gene transfer method such as microprojectile-mediated delivery, DNA injection, electroporation, or the like. In some embodiments, expression vectors are introduced into plant tissues using microprojectile media delivery with a biolistic device (see e.g., Tomes et al., 1995). The vectors of the presently disclosed subject matter can not only be used for expression of structural genes but can also be used in exon-trap cloning or in promoter trap procedures to detect differential gene expression in varieties of tissues (Lindsey et al., 1993; Auch & Reth, 1990).

In some embodiments, the binary type vectors of the Ti and Ri plasmids of *Agrobacterium spp* are employed. Ti-derived vectors can be used to transform a wide variety of higher plants, including monocotyledonous and dicotyledonous plants including, but not limited to soybean, cotton, rape, tobacco, and rice (Pacciotti et al., 1985: Byrne et al., 1987; Sukhapinda et al., 1987; Lorz et al., 1985; Potrykus, 1985; Park et al., 1985: Hiei et al., 1994). The use of T-DNA to transform plant cells has received extensive study and is amply described (European Patent Application No. EP 0 120 516; Hoekema, 1985; Knauf et al., 1983; and An et al., 1985, each of which is incorporated by reference in its entirety). For introduction into plants, the nucleic acid molecules of the presently disclosed subject matter can be inserted into binary vectors as described in the examples.

Other transformation methods are available to those skilled in the art, such as direct uptake of foreign DNA constructs (see European Patent Application No. EP 0 295 959), electroporation (Fromm et al., 1986), or high velocity ballistic bombardment of plant cells with metal particles coated with the nucleic acid constructs (Kline et al., 1987; U.S. Patent No. 4,945,050). Once transformed, the cells can be regenerated using techniques familiar to those of skill in the art. Of particular relevance are the recently described methods to transform foreign genes into commercially important crops, such as rapeseed (De Block et al., 1989), sunflower (Everett et al., 1987), soybean (McCabe et al., 1988; Hinchee et al., 1988; Chee et al., 1989; Christou et al., 1989; European Patent Application No. EP 0 301 749), rice (Hiei et al., 1994), and corn (Gordon Kamm et al., 1990; Fromm et al., 1990).

Of course, the choice of method might depend on the type of plant, i.e., monocotyledonous or dicotyledonous, targeted for transformation. Suitable methods of transforming plant cells include, but are not limited to microinjection (Crossway et al., 1986), electroporation (Riggs et al., 1986), Agrobacterium-mediated transformation (Hinchee et al., 1988), direct gene transfer (Paszkowski et al., 1984), and ballistic particle acceleration using devices available from Agracetus, Inc. (Madison, Wisconsin, United States of America) and BioRad (Hercules, California, United States of America). See e.g., U.S. Patent No. 4,945,050; McCabe et al., 1988; Weissinger et al., 1988; Sanford et al., 1987 (onion); Christou et al., 1988 (soybean); McCabe et al., 1988 (soybean); Datta et al., 1990 (rice); Klein et al., 1988 (maize); Fromm et al., 1990 (maize); Gordon-Kamm et al., 1990 (maize); Svab et al., 1990 (tobacco chloroplast); Koziel et al., 1993 (maize); Shimamoto et al., 1989 (rice); Christou et al., 1991 (rice); European Patent Application EP 0 332 581 (orchardgrass and other Pooideae); Vasil et al., 1993 (wheat); Weeks et al., 1993 (wheat). In one embodiment, the protoplast transformation method for maize is employed (see European Patent Application EP 0 292 435; U. S. Patent No. 5,350,689).

### 2. Vectors Suitable for Agrobacterium Transformation

*Agrobacterium tumefaciens* cells containing a vector comprising an expression cassette of the presently disclosed subject matter, wherein the vector comprises a Ti plasmid, are useful in methods of making transformed plants. Plant cells are infected with an *Agrobacterium tumefaciens* as described above to produce a transformed plant cell, and then a plant is regenerated from the transformed plant cell. Numerous *Agrobacterium* vector systems useful in carrying out the presently disclosed subject matter are known to ordinary skill in the art.

Many vectors are available for transformation using *Agrobacterium tumefaciens*. These typically carry at least one T-DNA border sequence and include vectors such as pBIN19 (Bevan, 1984). Below, the construction of two typical vectors suitable for *Agrobacterium* transformation is disclosed.

### a. pCIB200 and pCIB2001

The binary vectors pCIB200 and pCIB2001 are used for the construction of recombinant vectors for use with *Agrobacterium* and are constructed in the following manner. pTJS75kan is created by *Narl* digestion of pTJS75 (Schmidhauser & Helinski, 1985) allowing excision of the tetracycline-resistance gene, followed by insertion of an *Acc*I fragment from pUC4K carrying an NPTII sequence (Messing & Vieira, 1982: Bevan et al., 1983: McBride & Summerfelt, 1990). Xhol linkers are ligated to the *EcoR*V fragment of PCIB7 which contains the left and right T-DNA borders, a plant selectable *nos*/*nptll* chimeric gene and the pUC polylinker (Rothstein et al., 1987), and the *Xhol*-digested fragment are cloned into *SalI*-digested pTJS75kan to create pCIB200 (see also EP 0 332 104, example 19). pCIB200 contains the following unique polylinker restriction sites: *EcoR*I, *Sst*I, *Kpn*I, *Bgl*II, *Xba*I, and *Sal*I. pCIB2001 is a derivative of pCIB200 created by the insertion into the polylinker of additional restriction sites. Unique restriction sites in the polylinker of pCIB2001 are *EcoR*I, *Sst*I, *Kpn*I, *Bgl*ll, *Xba*l, *Sal*l, *Mlu*l, *Bcl*l, *Avr*ll, *Apa*l, *Hpa*l, and *Stul*. pCIB2001, in addition to containing these unique restriction sites, also has plant and bacterial kanamycin selection, left and right T-DNA borders for Agrobacterium-mediated transformation, the RK2-derived *trfA* function for mobilization between *E. coli* and other hosts, and the *OriT* and *OriV* functions also from RK2. The pCIB2001 polylinker is suitable for the cloning of plant expression cassettes containing their own regulatory signals.

### b. pCIB10 and Hygromycin Selection Derivatives Thereof

The binary vector pCIB10 contains a gene encoding kanamycin resistance for selection in plants, T-DNA right and left border sequences, and incorporates sequences from the wide host-range plasmid pRK252 allowing it to replicate in both *E*. *coli* and *Agrobacterium.* Its construction is disclosed by Rothstein et al., 1987. Various derivatives of pCIB10 can be constructed which incorporate the gene for hygromycin B phosphotransferase disclosed by Gritz & Davies, 1983. These derivatives enable selection of transgenic plant cells on hygromycin only (pCIB743), or hygromycin and kanamycin (pCIB715, pCIB717).

### 3. Vectors Suitable for non-Agrobacterium Transformation

Transformation without the use of *Agrobacterium tumefaciens* circumvents the requirement for T-DNA sequences in the chosen transformation vector, and consequently vectors lacking these sequences can be utilized in addition to vectors such as the ones disclosed above that contain T-DNA sequences. Transformation techniques that do not rely on *Agrobacterium* include transformation via particle bombardment, protoplast uptake (e.g., polyethylene glycol (PEG) and electroporation), and microinjection. The choice of vector depends largely on the species being transformed. Below, the construction of typical vectors suitable for non-*Agrobacterium* transformation is disclosed.

### a. pCIB3064

pCIB3064 is a pUC-derived vector suitable for direct gene transfer techniques in combination with selection by the herbicide BASTA® (glufosinate ammonium or phosphinothricin). The plasmid pCIB246 comprises the CaMV 35S promoter in operational fusion to the *E. coli* β-glucuronidase (GUS) gene and the CaMV 35S transcriptional terminator and is disclosed in the PCT International Publication WO 93/07278. The 35S promoter of this vector contains two ATG sequences 5' of the start site. These sites are mutated using standard PCR techniques in such a way as to remove the ATGs and generate the restriction sites *Ssp*l and *Pvu*ll. The new restriction sites are 96 and 37 bp away from the unique *Sal*I site and 101 and 42 bp away from the actual start site. The resultant derivative of pCIB246 is designated pCIB3025. The GUS gene is then excised from pCIB3025 by digestion with *Sal*I and *Sac*I, the termini rendered blunt and religated to generate plasmid pCIB3060. The plasmid pJIT82 is obtained from the John Innes Centre, Norwich, England, and the 400 bp *Sma*I fragment containing the *bar* gene from *Streptomyces viridochromogenes* is excised and inserted into the *Hpa*l site of pCIB3060 (Thompson et al., 1987). This generated pCIB3064, which comprises the *bar* gene under the control of the CaMV 35S promoter and terminator for herbicide selection, a gene for ampicillin resistance (for selection in *E. coli*) and a polylinker with the unique sites *Sph*l, *Pst*l, *Hind*lll, and *BamHl*. This vector is suitable for the cloning of plant expression cassettes containing their own regulatory signals.

### b. pSOG19 and pSOG35

pSOG35 is a transformation vector that utilizes the *E. coli* dihydrofolate reductase (DHFR) gene as a selectable marker conferring resistance to methotrexate. PCR is used to amplify the 35S promoter (-800 bp), intron 6 from the maize Adh1 gene (-550 bp), and 18 bp of the GUS untranslated leader sequence from pSOG10. A 250-bp fragment encoding the *E*. *coli* dihydrofolate reductase type II gene is also amplified by PCR and these two PCR fragments are assembled with a *Sac*I-*Pst*I fragment from pB1221 (BD Biosciences Clontech, Palo Alto, California, United States of America) that comprises the pUC19 vector backbone and the nopaline synthase terminator. Assembly of these fragments generates pSOG19 that contains the 35S promoter in fusion with the intron 6 sequence, the GUS leader, the DHFR gene, and the nopaline synthase terminator. Replacement of the GUS leader in pSOG19 with the leader sequence from Maize Chlorotic Mottle Virus (MCMV) generates the vector pSOG35. pSOG19 and pSOG35 carry the pUC gene for ampicillin resistance and have *Hind*III, *Sph*I, *Pst*I, and *EcoR*I sites available for the cloning of foreign substances.

### 4. Selectable Markers for Transformation Approaches

Methods using either a form of direct gene transfer or *Agrobacterium-*mediated transfer usually, but not necessarily, are undertaken with a selectable marker that can provide resistance to an antibiotic (e.g., kanamycin, hygromycin, or methotrexate) or a herbicide (e.g., phosphinothricin). The choice of selectable marker for plant transformation is not, however, critical to the presently disclosed subject matter.

For certain plant species, different antibiotic or herbicide selection markers can be employed. Selection markers used routinely in transformation include the *nptll* gene, which confers resistance to kanamycin and related antibiotics (Messing & Vierra, 1982; Bevan et al., 1983), the *bar* gene, which confers resistance to the herbicide phosphinothricin (White et al., 1990, Spencer et al., 1990), the *hph* gene, which confers resistance to the antibiotic hygromycin (Blochinger & Diggelmann, 1984), and the *dhfr* gene, which confers resistance to methotrexate (Bourouis & Jarry, 1983).

Selection markers resulting in positive selection, such as a phosphomannose isomerase (PMI) gene (described in PCT International Publication No. WO 93/05163) can also be used. Other genes that can be used for positive selection are described in PCT International Publication No. WO 94/20627 and encode xyloisomerases and phosphomanno-isomerases such as mannose-6-phosphate isomerase and mannose-1-phosphate isomerase; phosphomanno mutase; mannose epimerases such as those that convert carbohydrates to mannose or mannose to carbohydrates such as glucose or galactose; phosphatases such as mannose or xylose phosphatase, mannose-6-phosphatase and mannose-1-phosphatase, and permeases that are involved in the transport of mannose, or a derivative or a precursor thereof, into the cell. An agent is typically used to reduce the toxicity of the compound to the cells, and is typically a glucose derivative such as methyl-3-O-glucose or phloridzin. Transformed cells are identified without damaging or killing the non-transformed cells in the population and without co-introduction of antibiotic or herbicide resistance genes. As described in PCT International Publication No. WO 93/05163, in addition to the fact that the need for antibiotic or herbicide resistance genes is eliminated, it has been shown that the positive selection method is often far more efficient than traditional negative selection.

As noted above, one vector useful for direct gene transfer techniques in combination with selection by the herbicide BASTA® (or phosphinothricin) is pCIB3064. This vector is based on the plasmid pCIB246, which comprises the CaMV 35S promoter operatively linked to the *E. coli* β-glucuronidase (GUS) gene and the CaMV 35S transcriptional terminator, and is described in PCT International Publication No. WO 93/07278. One gene useful for conferring resistance to phosphinothricin is the bar gene from *Streptomyces viridochromogenes* (Thompson et al., 1987). This vector is suitable for the cloning of plant expression cassettes containing their own regulatory signals.

As noted above, an additional transformation vector is pSOG35, which utilizes the *E. coli* dihydrofolate reductase (DHFR) gene as a selectable marker conferring resistance to methotrexate. Polymerase chain reaction (PCR) was used to amplify the 35S promoter (about 800 basepairs (bp)), intron 6 from the maize *Adh1* gene (about 550 bp), and 18 bp of the GUS untranslated leader sequence from pSOG10. A 250 bp fragment encoding the *E. coli* dihydrofolate reductase type II gene was also amplified by PCR and these two PCR fragments are assembled with a Sacl-Pstl fragment from pBI221 (BD Biosciences - Clontech, Palo Alto, California, United States of America), which comprised the pUC19 vector backbone and the nopaline synthase terminator. Assembly of these fragments generated pSOG19, which contains the 35S promoter in fusion with the intron 6 sequence, the GUS leader, the *DHFR* gene and the nopaline synthase terminator. Replacement of the *GUS* leader in pSOG19 with the leader sequence from Maize Chlorotic Mottle Virus (MCMV) generated the vector pSOG35. pSOG19 and pSOG35 carry the pUC-derived gene for ampicillin resistance, and have Hindlll, Sphl, Pstl and EcoRl sites available for the cloning of foreign sequences.

Binary backbone vector pNOV2117 contains the T-DNA portion flanked by the right and left border sequences, and including the POSITECH^{™} (Syngenta Corp., Wilmington, Delaware, United States of America) plant selectable marker and the "candidate gene" gene expression cassette. The POSITECH^{™} plant selectable marker confers resistance to mannose and in this instance consists of the maize ubiquitin promoter driving expression of the *PMI* (phosphomannose isomerase) gene, followed by the cauliflower mosaic virus transcriptional terminator.

### 5. Vector Suitable for Chloroplast Transformation

For expression of a nucleotide sequence of the presently disclosed subject matter in plant plastids, plastid transformation vector pPH143 (PCT International Publication WO 97/32011, example 36) is used. The nucleotide sequence is inserted into pPH143 thereby replacing the protoporphyrinogen oxidase (Protox) coding sequence. This vector is then used for plastid transformation and selection of transformants for spectinomycin resistance. Alternatively, the nucleotide sequence is inserted in pPH143 so that it replaces the *aadH* gene. In this case, transformants are selected for resistance to PROTOX inhibitors.

### 6. Transformation of Plastids

In another embodiment, a nucleotide sequence of the presently disclosed subject matter is directly transformed into the plastid genome. Plastid transformation technology is described in U.S. Patent Nos. 5,451,513; 5,545,817; and 5,545,818; and in PCT International Publication No. WO 95/16783; and in McBride et al., 1994. The basic technique for chloroplast transformation involves introducing regions of cloned plastid DNA flanking a selectable marker together with the gene of interest into a suitable target tissue, e.g., using biolistics or protoplast transformation (e.g., calcium chloride or PEG mediated transformation). The 1 to 1.5 kilobase (kb) flanking regions, termed targeting sequences, facilitate orthologous recombination with the plastid genome and thus allow the replacement or modification of specific regions of the plastome. Initially, point mutations in the chloroplast 16S rRNA and *rps12* genes conferring resistance to spectinomycin and/or streptomycin are utilized as selectable markers for transformation (Svab et al., 1990; Staub et al., 1992). This resulted in stable homoplasmic transformants at a frequency of approximately one per 100 bombardments of target leaves. The presence of cloning sites between these markers allowed creation of a plastid targeting vector for introduction of foreign genes (Staub et al., 1993). Substantial increases in transformation frequency are obtained by replacement of the recessive rRNA or r-protein antibiotic resistance genes with a dominant selectable marker, the bacterial *aadA* gene encoding the spectinomycin-detoxifying enzyme aminoglycoside-3N-adenyltransferase (Staub et al., 1993). Other selectable markers useful for plastid transformation are known in the art and encompassed within the scope of the presently disclosed subject matter. Typically, approximately 15-20 cell division cycles following transformation are required to reach a homoplastidic state.

Plastid expression, in which genes are inserted by orthologous recombination into all of the several thousand copies of the circular plastid genome present in each plant cell, takes advantage of the enormous copy number advantage over nuclear-expressed genes to permit expression levels that can readily exceed 10% of the total soluble plant protein. In one embodiment, a nucleotide sequence of the presently disclosed subject matter is inserted into a plastid targeting vector and transformed into the plastid genome of a desired plant host. Plants homoplastic for plastid genomes containing a nucleotide sequence of the presently disclosed subject matter are obtained, and are in one embodiment capable of high expression of the nucleotide sequence.

An example of plastid transformation follows. Seeds of *Nicotiana tabacum* c.v. 'Xanthi nc' are germinated seven per plate in a 1" circular array on T agar medium and bombarded 12-14 days after sowing with 1 µm tungsten particles (M10, Biorad, Hercules, California, United States of America) coated with DNA from plasmids pPH143 and pPH145 essentially as disclosed (Svab & Maliga, 1993). Bombarded seedlings are incubated on T medium for two days after which leaves are excised and placed abaxial side up in bright light (350-500 µmol photons/m²/s) on plates of RMOP medium (Svab et al., 1990) containing 500 µg/ml spectinomycin dihydrochloride (Sigma, St. Louis, Missouri, United States of America). Resistant shoots appearing underneath the bleached leaves three to eight weeks after bombardment are subcloned onto the same selective medium, allowed to form callus, and secondary shoots isolated and subcloned. Complete segregation of transformed plastid genome copies (homoplasmicity) in independent subclones is assessed by standard techniques of Southern blotting (Sambrook & Russell, 2001). *BamHl*/*EcoRl-*digested total cellular DNA (Mettler, 1987) is separated on 1% Tris-borate-EDTA (TBE) agarose gels, transferred to nylon membranes (Amersham Biosciences, Piscataway, New Jersey, United States of America) and probed with ³²P-labeled random primed DNA sequences corresponding to a 0.7 kb *BamH*I/*Hind*III DNA fragment from pC8 containing a portion of the *rps7*/*12* plastid targeting sequence. Homoplasmic shoots are rooted aseptically on spectinomycin-containing MS/IBA medium (McBride et al., 1994) and transferred to the greenhouse.

### 7. Transformation of Dicotyledons

Transformation techniques for dicotyledons are well known in the art and include *Agrobacterium*-based techniques and techniques that do not require *Agrobacterium*. Non-*Agrobacterium* techniques involve the uptake of exogenous genetic material directly by protoplasts or cells. This can be accomplished by PEG or electroporation-mediated uptake, particle bombardment-mediated delivery, or microinjection. Examples of these techniques are disclosed in Paszkowski et al., 1984; Potrykus et al., 1985; Reich et al., 1986; and Klein et al., 1987. In each case the transformed cells are regenerated to whole plants using standard techniques known in the art.

*Agrobacterium*-mediated transformation is a useful technique for transformation of dicotyledons because of its high efficiency of transformation and its broad utility with many different species. *Agrobacterium* transformation typically involves the transfer of the binary vector carrying the foreign DNA of interest (e.g., pCIB200 or pCIB2001) to an appropriate *Agrobacterium* strain which can depend on the complement of *vir* genes carried by the host *Agrobacterium* strain either on a co-resident Ti plasmid or chromosomally (e.g., strain CIB542 for pCIB200 and pCIB2001 (Uknes et al., 1993). The transfer of the recombinant binary vector to *Agrobacterium* is accomplished by a triparental mating procedure using *E. coli* carrying the recombinant binary vector, a helper E. *coli* strain that carries a plasmid such as pRK2013 and which is able to mobilize the recombinant binary vector to the target *Agrobacterium* strain. Alternatively, the recombinant binary vector can be transferred to *Agrobacterium* by DNA transformation (Höfgen & Willmitzer, 1988).

Transformation of the target plant species by recombinant *Agrobacterium* usually involves co-cultivation of the *Agrobacterium* with explants from the plant and follows protocols well known in the art. Transformed tissue is regenerated on selectable medium carrying the antibiotic or herbicide resistance marker present between the binary plasmid T-DNA borders.

Another approach to transforming plant cells with a gene involves propelling inert or biologically active particles at plant tissues and cells. This technique is disclosed in U.S. Patent Nos. 4,945,050; 5,036,006; and 5,100,792; all to Sanford et al. Generally, this procedure involves propelling inert or biologically active particles at the cells under conditions effective to penetrate the outer surface of the cell and afford incorporation within the interior thereof. When inert particles are utilized, the vector can be introduced into the cell by coating the particles with the vector containing the desired gene. Alternatively, the target cell can be surrounded by the vector so that the vector is carried into the cell by the wake of the particle. Biologically active particles (e.g., dried yeast cells, dried bacterium, or a bacteriophage, each containing DNA sought to be introduced) can also be propelled into plant cell tissue.

### 8. Transformation of Monocotyledons

Transformation of most monocotyledon species has now also become routine. Exemplary techniques include direct gene transfer into protoplasts using PEG or electroporation, and particle bombardment into callus tissue. Transformations can be undertaken with a single DNA species or multiple DNA species (i.e. co-transformation), and both these techniques are suitable for use with the presently disclosed subject matter. Co-transformation can have the advantage of avoiding complete vector construction and of generating transgenic plants with unlinked loci for the gene of interest and the selectable marker, enabling the removal of the selectable marker in subsequent generations, should this be regarded as desirable. However, a disadvantage of the use of co-transformation is the less than 100% frequency with which separate DNA species are integrated into the genome (Schocher et al., 1986).

Patent Applications EP 0 292 435, EP 0 392 225, and WO 93/07278 describe techniques for the preparation of callus and protoplasts from an elite inbred line of maize, transformation of protoplasts using PEG or electroporation, and the regeneration of maize plants from transformed protoplasts. Gordon-Kamm et al., 1990 and Fromm et al., 1990 have published techniques for transformation of A188-derived maize line using particle bombardment. Furthermore, WO 93/07278 and Koziel et al., 1993 describe techniques for the transformation of elite inbred lines of maize by particle bombardment. This technique utilizes immature maize embryos of 1.5-2.5 mm length excised from a maize ear 14-15 days after pollination and a PDS-1000He Biolistic particle delivery device (DuPont Biotechnology, Wilmington, Delaware, United States of America) for bombardment.

Transformation of rice can also be undertaken by direct gene transfer techniques utilizing protoplasts or particle bombardment. Protoplast-mediated transformation has been disclosed for Japonica-types and *Indica-*types (Zhang et al., 1988; Shimamoto et al., 1989; Datta et al., 1990) of rice. Both types are also routinely transformable using particle bombardment (Christou et al., 1991). Furthermore, WO 93/21335 describes techniques for the transformation of rice via electroporation. Casas et al., 1993 discloses the production of transgenic sorghum plants by microprojectile bombardment.

Patent Application EP 0 332 581 describes techniques for the generation, transformation, and regeneration of Pooideae protoplasts. These techniques allow the transformation of *Dactylis* and wheat. Furthermore, wheat transformation has been disclosed in Vasil et al., 1992 using particle bombardment into cells of type C long-term regenerable callus, and also by Vasil et al., 1993 and Weeks et al., 1993 using particle bombardment of immature embryos and immature embryo-derived callus.

A representative technique for wheat transformation, however, involves the transformation of wheat by particle bombardment of immature embryos and includes either a high sucrose or a high maltose step prior to gene delivery. Prior to bombardment, embryos (0.75-1 mm in length) are plated onto MS medium with 3% sucrose (Murashige & Skoog, 1962) and 3 mg/l 2,4-dichlorophenoxyacetic acid (2,4-D) for induction of somatic embryos, which is allowed to proceed in the dark. On the chosen day of bombardment, embryos are removed from the induction medium and placed onto the osmoticum (i.e. induction medium with sucrose or maltose added at the desired concentration, typically 15%). The embryos are allowed to plasmolyze for 2-3 hours and are then bombarded. Twenty embryos per target plate are typical, although not critical. An appropriate gene-carrying plasmid (such as pCIB3064 or pSG35) is precipitated onto micrometer size gold particles using standard procedures. Each plate of embryos is shot with the DuPont BIOLISTICS® helium device using a burst pressure of about 1000 pounds per square inch (psi) using a standard 80 mesh screen. After bombardment, the embryos are placed back into the dark to recover for about 24 hours (still on osmoticum). After 24 hours, the embryos are removed from the osmoticum and placed back onto induction medium where they stay for about a month before regeneration. Approximately one month later the embryo explants with developing embryogenic callus are transferred to regeneration medium (MS + 1 mg/liter NAA, 5 mg/liter GA), further containing the appropriate selection agent (10 mg/l BASTA® in the case of pCIB3064 and 2 mg/l methotrexate in the case of pSOG35). After approximately one month, developed shoots are transferred to larger sterile containers known as "GA7s" which contain half-strength MS, 2% sucrose, and the same concentration of selection agent.

Transformation of monocotyledons using *Agrobacterium* has also been disclosed. See WO 94/00977 and U.S. Patent No. 5,591,616, both of which are incorporated herein by reference. See also Negrotto et al., 2000, incorporated herein by reference. Zhao et al., 2000 specifically discloses transformation of sorghum with *Agrobacterium.* See also U.S. Patent No. 6,369,298.

Rice (*Oryza sativa*) can be used for generating transgenic plants. Various rice cultivars can be used (Hiei et al., 1994; Dong et al., 1996; Hiei et al., 1997). Also, the various media constituents disclosed below can be either varied in quantity or substituted. Embryogenic responses are initiated and/or cultures are established from mature embryos by culturing on MS-CIM medium (MS basal salts, 4.3 g/liter; B5 vitamins (200 x), 5 ml/liter; Sucrose, 30 g/liter; proline, 500 mg/liter; glutamine, 500 mg/liter; casein hydrolysate, 300 mg/liter; 2,4-D (1 mg/ml), 2 ml/liter; pH adjusted to 5.8 with 1 N KOH; Phytagel, 3 g/liter). Either mature embryos at the initial stages of culture response or established culture lines are inoculated and co-cultivated with the *Agrobacterium tumefaciens* strain LBA4404 (*Agrobacterium*) containing the desired vector construction. *Agrobacterium* is cultured from glycerol stocks on solid YPC medium (plus 100 mg/L spectinomycin and any other appropriate antibiotic) for about 2 days at 28°C. *Agrobacterium* is resuspended in liquid MS-CIM medium. The *Agrobacterium* culture is diluted to an OD₆₀₀ of 0.2-0.3 and acetosyringone is added to a final concentration of 200 µM. Acetosyringone is added before mixing the solution with the rice cultures to induce *Agrobacterium* for DNA transfer to the plant cells. For inoculation, the plant cultures are immersed in the bacterial suspension. The liquid bacterial suspension is removed and the inoculated cultures are placed on co-cultivation medium and incubated at 22°C for two days. The cultures are then transferred to MS-CIM medium with ticarcillin (400 mg/liter) to inhibit the growth of *Agrobacterium.* For constructs utilizing the PMI selectable marker gene (Reed et al., 2001), cultures are transferred to selection medium containing mannose as a carbohydrate source (MS with 2% mannose, 300 mg/liter ticarcillin) after 7 days, and cultured for 3-4 weeks in the dark. Resistant colonies are then transferred to regeneration induction medium (MS with no 2,4-D, 0.5 mg/liter IAA, 1 mg/liter zeatin, 200 mg/liter TIMENTIN®, 2% mannose, and 3% sorbitol) and grown in the dark for 14 days. Proliferating colonies are then transferred to another round of regeneration induction media and moved to the light growth room. Regenerated shoots are transferred to GA7 containers with GA7-1 medium (MS with no hormones and 2% sorbitol) for 2 weeks and then moved to the greenhouse when they are large enough and have adequate roots. Plants are transplanted to soil in the greenhouse (To generation) grown to maturity and the T₁ seed is harvested.

### E. Growth and Screening of Transformed Cells

Transgenic plant cells are then placed in an appropriate selective medium for selection of transgenic cells, which are then grown to callus. Shoots are grown from callus and plantlets generated from the shoot by growing in rooting medium. The various constructs normally are joined to a marker for selection in plant cells. Conveniently, the marker can be resistance to a biocide (for example, an antibiotic including, but not limited to kanamycin, G418, bleomycin, hygromycin, chloramphenicol, herbicide, or the like). The particular marker used is designed to allow for the selection of transformed cells (as compared to cells lacking the DNA that has been introduced). Components of DNA constructs including transcription cassettes of the presently disclosed subject matter are prepared from sequences that are native (endogenous) or foreign (exogenous) to the host. As used herein, the terms "foreign" and "exogenous" refer to sequences that are not found in the wild-type host into which the construct is introduced, or alternatively, have been isolated from the host species and incorporated into an expression vector. Heterologous constructs contain in one embodiment at least one region that is not native to the gene from which the transcription initiation region is derived.

To confirm the presence of the transgenes in transformed cells and plants, a variety of assays can be performed. Such assays include, for example, "molecular biological" assays well known to those of skill in the art, such as Southern and Northern blotting, *in situ* hybridization and nucleic acid-based amplification methods such as PCR or RT-PCR; "biochemical" assays, such as detecting the presence of a protein product, e.g., by immunological means (enzyme-linked immunosorbent assays (ELISAs) and Western blots) or by enzymatic function; plant part assays, such as seed assays; and also by analyzing the phenotype of the whole regenerated plant, e.g., for disease or pest resistance.

DNA can be isolated from cell lines or any plant parts to determine the presence of the preselected nucleic acid segment through the use of techniques well known to those skilled in the art. Note that intact sequences will not always be present, presumably due to rearrangement or deletion of sequences in the cell.

The presence of nucleic acid elements introduced through the methods of this presently disclosed subject matter can be determined by the polymerase chain reaction (PCR). Using this technique, discreet fragments of nucleic acid are amplified and detected by gel electrophoresis. This type of analysis permits one to determine whether a preselected nucleic acid segment is present in a stable transformant. It is contemplated that using PCR techniques it would be possible to clone fragments of the host genomic DNA adjacent to an introduced preselected DNA segment.

Positive proof of DNA integration into the host genome and the independent identities of transformants can be determined using the technique of Southern hybridization. Using this technique, specific DNA sequences that are introduced into the host genome and flanking host DNA sequences can be identified. Hence, the Southern hybridization pattern of a given transformant serves as an identifying characteristic of that transformant. In addition, it is possible through Southern hybridization to demonstrate the presence of introduced preselected DNA segments in high molecular weight DNA: e.g., to confirm that the introduced preselected DNA segment has been integrated into the host cell genome. Southern hybridization provides certain information that can also be obtained using PCR, e.g., the presence of a preselected DNA segment, but can also demonstrate integration of an exogenous nucleic acid molecule into the genome and can characterize each individual transformant.

It is contemplated that using the techniques of dot or slot blot hybridization, which are modifications of Southern hybridization techniques, the same information that is derived from PCR could be obtained (e.g., the presence of a preselected DNA segment).

Both PCR and Southern hybridization techniques can be used to demonstrate transmission of a preselected DNA segment to progeny. In most instances, the characteristic Southern hybridization pattern for a given transformant will segregate in progeny as one or more Mendelian genes (Spencer et al., 1990; Laursen et al., 1994), indicating stable inheritance of the gene. The non-chimeric nature of the callus and the parental transformants (R₀) can be suggested by germline transmission and the identical Southern blot hybridization patterns and intensities of the transforming DNA in callus, R₀ plants, and R₁ progeny that segregated for the transformed gene.

Whereas certain DNA analysis techniques can be conducted using DNA isolated from any part of a plant, specific RNAs might only be expressed in particular cells or tissue types and hence it can be necessary to prepare RNA for analysis from these tissues. PCR techniques can also be used for detection and quantitation of RNA produced from introduced preselected DNA molecules. In this application of PCR, it is first necessary to reverse transcribe RNA into complementary DNA (cDNA) using an enzyme such as a reverse transcriptase, and then through the use of conventional PCR techniques, to amplify the resulting cDNA.

In some instances, PCR techniques might not demonstrate the integrity of the RNA product. Further information about the nature of the RNA product can be obtained by Northern blotting. This technique demonstrates the presence of an RNA species and additionally gives information about the integrity of that RNA. The presence or absence of an RNA species can also be determined using dot or slot blot Northern hybridizations using techniques known in the art. These techniques are modifications of Northern blotting and typically demonstrate only the presence or absence of an RNA species.

Thus, Southern blotting and PCR can be used to detect the presence of a DNA molecule of interest. Expression can be evaluated by specifically identifying the protein products of the introduced preselected DNA segments or evaluating the phenotypic changes brought about by their expression.

Assays for the production and identification of specific proteins can make use of physical-chemical, structural, functional, or other properties of the proteins. Unique physical-chemical or structural properties allow the proteins to be separated and identified by electrophoretic procedures, such as native or denaturing gel electrophoresis or isoelectric focusing, or by chromatographic techniques such as ion exchange or gel exclusion chromatography. The unique structures of individual proteins offer opportunities for use of specific antibodies to detect the presence of individual proteins using art-recognized techniques such as an ELISA assay. Combinations of approaches can be employed to gain additional information, such as Western blotting, in which antibodies are used to locate individual gene products that have been separated by electrophoretic techniques and transferred to a solid support. Additional techniques can be employed to confirm the identity of the product of interest, such as evaluation by amino acid sequencing following purification. Although these are among the most commonly employed, other procedures known to the skilled artisan can also be used.

Assay procedures can also be used to identify the expression of proteins by their functions, especially the ability of enzymes to catalyze specific chemical reactions involving specific substrates and products. These reactions can be followed by providing and quantifying the loss of substrates or the generation of products of the reactions by physical or chemical procedures. Examples are as varied as the enzyme to be analyzed, and are known in the art for many different enzymes.

The expression of a gene product can also be determined by evaluating the phenotypic results of its expression. These assays also can take many forms including, but not limited to analyzing changes in the chemical composition, morphology, or physiological properties of the plant. Morphological changes can include greater stature or thicker stalks. Changes in the response of plants or plant parts to imposed treatments are typically evaluated under carefully controlled conditions termed bioassays.

As such, protein expression levels can be measured by any standard method. For example, antibodies (monoclonal or polyclonal) can be generated by standard methods that specifically bind to a cell proliferation-related protein of the presently disclosed subject matter (see methods for making antibodies in, e.g., Ausubel et al., 1988, including updates up to 2002; Harlow & Lane, 1988). Using such a cell proliferation-related protein-specific antibody, protein levels can be determined by any immunological method including, without limitation, Western blotting, immunoprecipitation, and ELISA.

Another non-limiting method for measuring protein level is by measuring mRNA levels. For example, total mRNA can be isolated from a cell introduced with a nucleic acid molecule of the presently disclosed subject matter (or with an antisense of such a nucleic acid molecule) and from an untreated cell. Northern blotting analysis using the nucleic acid molecule that was introduced to the treated cell as a probe can indicate if the treated cell expresses the nucleic acid molecule at a different level (at both the mRNA and polypeptide levels) as compared to the untreated cell.

Changes in cell proliferation rates (either in unchallenged cells and plants, or in cells and plants challenged with, for example, exposure to salt or pathogen-infection) can be readily determined by counting the cells by any standard method. For example, cells can be manually counted using a hemacytometer or microscope. Callus growth and plant growth can be measured by weight and/or height. Individual cell growth can be determined by any standard cell proliferation assay (e.g., ³H incorporation).

The presently disclosed subject matter further includes the manipulation of cell and plant proliferation by modulation of the expression of more than one of the cell proliferation-related proteins described herein. For example, an increase in the level of expression of a first cell proliferation-related protein coupled with a decrease in the level of expression of a second the cell proliferation-related protein can result in a greater change in the proliferation rate of a cell (or plant including such a cell) than either the increase in the level of expression of a first cell proliferation-related protein of the decrease in the level of expression of a second the cell proliferation-related protein alone. The presently disclosed subject matter has provided numerous cell proliferation-related proteins and their interrelations with one another. Manipulation of expression of one or more of the cell proliferation-related proteins of the presently disclosed subject matter enables the development of genetically engineered plants (i.e., transgenic plants) that have superior growth rates either in favorable conditions, under differentiation, or under stress (e.g., biotic or abiotic stress).

### VI. Plants, Breeding, and Seed Production

### A. Plants

A host cell is any type of cell including, without limitation, a bacterial cell, a yeast cell, a plant cell, an insect cell, and a mammalian cell. Numerous such cells are commercially available, for example, from the American Type Culture Collection, Manassas, Virginia, United States of America.

In certain embodiments, the cell is a plant cell, which can be regenerated to form a transgenic plant. Thus, the presently disclosed subject matter provides a transformed (transgenic) plant cell, *in planta* or *ex planta*, including a transformed plastid or other organelle (e.g., nucleus, mitochondria or chloroplast). As used herein, a "transgenic plant" is a plant having one or more plant cells that contain an exogenous nucleic acid molecule (e.g., a nucleic acid molecule encoding a cell proliferation-related polypeptide of the presently disclosed subject matter). Thus, a transgenic plant can comprise a nucleic acid molecule comprising a foreign nucleic acid sequence (i.e. a nucleic acid sequence derived from a different plant species). Alternatively or in addition, a transgenic plant can comprise a nucleic acid molecule comprising a nucleic acid sequence from the same plant species, wherein the nucleic acid sequence has been isolated from that plant species. In the latter example, the nucleic acid sequence can be the same or different from the wild-type sequence, and can optionally include regulatory sequences that are the same or different from those that are found in the naturally occurring plant.

The presently disclosed subject matter can be used for transforming cells of any plant species, including, but not limited to from corn (*Zea mays*), *Brassica* sp. (e.g., *B. napus, B. rapa, B. juncea*), particularly those *Brassica* species useful as sources of seed oil, alfalfa (*Medicago sativa*), rice (*Oryza sativa*), rye (*Secale cereale*), sorghum (*Sorghum bicolor*, *Sorghum vulgare*), millet (e.g., pearl millet (*Pennisetum glaucum*)), proso millet (*Panicum miliaceum*), foxtail millet (*Setaria italica*), finger millet (*Eleusine coracana*)), sunflower (*Helianthus annuus*), safflower (*Carthamus tinctorius*), wheat (*Triticum aestivum*), soybean (*Glycine max*), tobacco (*Nicotiana tabacum*), potato (*Solanum tuberosum*), peanut (*Arachis hypogaea*), cotton (*Gossypium barbadense, Gossypium hirsutum*), sweet potato (*Ipomoea batatus*), cassava (*Manihot esculenta*), coffee (*Cofea* spp.), coconut (*Cocos nucifera*), pineapple (*Ananas comosus*), citrus trees (*Citrus* spp.), cocoa (*Theobroma cacao*), tea (*Camellia sinensis*), banana (*Musa* spp.), avocado (*Persea ultilane*), fig (*Ficus casica*), guava (*Psidium guajava*), mango (*Mangifera indica*), olive (*Olea europaea*), papaya (*Carica papaya*), cashew (*Anacardium occidentale*), macadamia (*Macadamia integrifolia*), almond (*Prunus amygdalus*), sugar beets (*Beta vulgaris*), sugarcane (*Saccharum* spp.), oats, duckweed (*Lemna*), barley, vegetables, ornamentals, and conifers.

Duckweed (*Lemna*, see PCT International Publication No. WO 00/07210) includes members of the family *Lemnaceae*. There are known four genera and 34 species of duckweed as follows: genus *Lemna (L. aequinoctialis, L. disperma, L. ecuadoriensis, L. gibba, L. japonica, L. minor, L. miniscula, L. obscura, L. perpusilla, L. tenera, L. trisulca, L.turionifera, L. valdiviana*); genus *Spirodela (S. intermedia, S. polyrrhiza, S. punctata);* genus *Woffia (Wa. Angusta, Wa. Arrhiza, Wa. Australina, Wa. Borealis, Wa. Brasiliensis, Wa. Columbiana, Wa. Elongata, Wa. Globosa, Wa. Microscopica, Wa. Neglecta)* and genus *Wofiella (W1. ultila, W1. ultilanen, W1. gladiata, W1. ultila, W1. lingulata, W1. repunda, W1. rotunda, and W1. neotropica)*. Any other genera or species of *Lemnaceae*, if they exist, are also aspects of the presently disclosed subject matter. In one embodiment, *Lemna gibba* is employed in the presently disclosed subject matter, and in other embodiments, *Lemna minor* and *Lemna miniscula* are employed. *Lemna* species can be classified using the taxonomic scheme described by Landolt, 1986.

Vegetables within the scope of the presently disclosed subject matter include tomatoes (*Lycopersicon esculentum*), lettuce (e.g., *Lactuca sativa*), green beans (*Phaseolus vulgaris*), lima beans (*Phaseolus limensis*), peas (*Lathyrus* spp.), and members of the genus *Cucumis* such as cucumber (C. *sativus*), cantaloupe (*C. cantalupensis*), and musk melon (*C. melo*). Ornamentals include azalea (*Rhododendron* spp.), hydrangea (*Macrophylla hydrangea*), hibiscus (*Hibiscus rosasanensis*), roses (*Rosa* spp.), tulips (*Tulipa* spp.), daffodils (*Narcissus* spp.), petunias (*Petunia hybrida*), carnations (*Dianthus caryophyllus*), poinsettias (*Euphorbia pulcherrima*), and chrysanthemums. Conifers that can be employed in practicing the presently disclosed subject matter include, for example, pines such as loblolly pine (*Pinus taeda*), slash pine (*Pinus elliotii*), ponderosa pine (*Pinus ponderosa*), lodgepole pine (*Pinus contorta*), and Monterey pine (*Pinus radiata*), Douglas-fir (*Pseudotsuga menziesii*); Western hemlock (*Tsuga ultilane*); Sitka spruce *(Picea glauca*); redwood (*Sequoia sempervirens*); true firs such as silver fir (*Abies amabilis*) and balsam fir (*Abies balsamea*); and cedars such as Western red cedar (*Thuja* p*licata*) and Alaska yellow-cedar (*Chamaecyparis nootkatensis*).

Leguminous plants that can be employed in the presently disclosed subject matter include beans and peas. Representative beans include guar, locust bean, fenugreek, soybean, garden beans, cowpea, mungbean, lima bean, fava bean, lentils, chickpea, etc. Legumes include, but are not limited to *Arachis* (e.g., peanuts), *Vicia* (e.g., crown vetch, hairy vetch, adzuki bean, mung bean, and chickpea), *Lupinus* (e.g., lupine, trifolium), *Phaseolus* (e.g., common bean and lima bean), *Pisum* (e.g., field bean), *Melilotus* (e.g., clover), *Medicago* (e.g., alfalfa), *Lotus* (e.g., trefoil), *lens* (e.g., lentil), and false indigo. Non-limiting forage and turf grass for use in the methods of the presently disclosed subject matter include alfalfa, orchard grass, tall fescue, perennial ryegrass, creeping bent grass, and redtop.

Other plants within the scope of the presently disclosed subject matter include Acacia, aneth, artichoke, arugula, blackberry, canola, cilantro, clementines, escarole, eucalyptus, fennel, grapefruit, honey dew, jicama, kiwifruit, lemon, lime, mushroom, nut, okra, orange, parsley, persimmon, plantain, pomegranate, poplar, radiata pine, radicchio, Southern pine, sweetgum, tangerine, triticale, vine, yams, apple, pear, quince, cherry, apricot, melon, hemp, buckwheat, grape, raspberry, chenopodium, blueberry, nectarine, peach, plum, strawberry, watermelon, eggplant, pepper, cauliflower, Brassica, e.g., broccoli, cabbage, ultilan sprouts, onion, carrot, leek, beet, broad bean, celery, radish, pumpkin, endive, gourd, garlic, snapbean, spinach, squash, turnip, ultilane, and zucchini.

Ornamental plants within the scope of the presently disclosed subject matter include impatiens, Begonia, Pelargonium, Viola, Cyclamen, Verbena, Vinca, Tagetes, Primula, Saint Paulia, Agertum, Amaranthus, Antihirrhinum, Aquilegia, Cineraria, Clover, Cosmo, Cowpea, Dahlia, Datura, Delphinium, Gerbera, Gladiolus, Gloxinia, Hippeastrum, Mesembryanthemum, Salpiglossos, and Zinnia.

In certain embodiments, transgenic plants of the presently disclosed subject matter are crop plants and in particular cereals. Such crop plants and cereals include, but are not limited to corn, alfalfa, sunflower, rice, *Brassica,* canola, soybean, barley, soybean, sugarbeet, cotton, safflower, peanut, sorghum, wheat, millet, and tobacco.

The presently disclosed subject matter also provides plants comprising the disclosed compositions. In one embodiment, the plant is characterized by a modification of a phenotype or measurable characteristic of the plant, the modification being attributable to the expression cassette. In one embodiment, the modification involves, for example, nutritional enhancement, increased nutrient uptake efficiency, enhanced production of endogenous compounds, or production of heterologous compounds. In another embodiment, the modification includes having increased or decreased resistance to an herbicide, an abiotic stress, or a pathogen. In another embodiment, the modification includes having enhanced or diminished requirement for light, water, nitrogen, or trace elements. In another embodiment, the modification includes being enriched for an essential amino acid as a proportion of a polypeptide fraction of the plant. In another embodiment, the polypeptide fraction can be, for example, total seed polypeptide, soluble polypeptide, insoluble polypeptide, water-extractable polypeptide, and lipid-associated polypeptide. In another embodiment, the modification includes overexpression, underexpression, antisense modulation, sense suppression, inducible expression, inducible repression, or inducible modulation of a gene.

### B. Breeding

The plants obtained via transformation with a nucleic acid sequence of the presently disclosed subject matter can be any of a wide variety of plant species, including monocots and dicots; however, the plants used in the method for the presently disclosed subject matter are selected in one embodiment from the list of agronomically important target crops set forth hereinabove. The expression of a gene of the presently disclosed subject matter in combination with other characteristics important for production and quality can be incorporated into plant lines through breeding. Breeding approaches and techniques are known in the art. See e.g., Welsh, 1981; Wood, 1983; Mayo, 1987; Singh, 1986; Wricke & Weber, 1986.

The genetic properties engineered into the transgenic seeds and plants disclosed above are passed on by sexual reproduction or vegetative growth and can thus be maintained and propagated in progeny plants. Generally, the maintenance and propagation make use of known agricultural methods developed to fit specific purposes such as tilling, sowing, or harvesting. Specialized processes such as hydroponics or greenhouse technologies can also be applied. As the growing crop is vulnerable to attack and damage caused by insects or infections as well as to competition by weed plants, measures are undertaken to control weeds, plant diseases, insects, nematodes, and other adverse conditions to improve yield. These include mechanical measures such as tillage of the soil or removal of weeds and infected plants, as well as the application of agrochemicals such as herbicides, fungicides, gametocides, nematicides, growth regulants, ripening agents, and insecticides.

Use of the advantageous genetic properties of the transgenic plants and seeds according to the presently disclosed subject matter can further be made in plant breeding, which aims at the development of plants with improved properties such as tolerance of pests, herbicides, or biotic or abiotic stress, improved nutritional value, increased yield or proliferation, or improved structure causing less loss from lodging or shattering. The various breeding steps are characterized by well-defined human intervention such as selecting the lines to be crossed, directing pollination of the parental lines, or selecting appropriate progeny plants.

Depending on the desired properties, different breeding measures are taken. The relevant techniques are well known in the art and include, but are not limited to, hybridization, inbreeding, backcross breeding, multiline breeding, variety blend, interspecific hybridization, aneuploid techniques, etc. Hybridization techniques can also include the sterilization of plants to yield male or female sterile plants by mechanical, chemical, or biochemical means. Cross-pollination of a male sterile plant with pollen of a different line assures that the genome of the male sterile but female fertile plant will uniformly obtain properties of both parental lines. Thus, the transgenic seeds and plants according to the presently disclosed subject matter can be used for the breeding of improved plant lines that, for example, increase the effectiveness of conventional methods such as herbicide or pesticide treatment or allow one to dispense with said methods due to their modified genetic properties. Alternatively new crops with improved stress tolerance can be obtained, which, due to their optimized genetic "equipment", yield harvested product of better quality than products that were not able to tolerate comparable adverse developmental conditions (for example, drought).

Additionally, The presently disclosed subject matter also provides a transgenic plant, a seed from such a plant, and progeny plants from such a plant including hybrids and inbreds. In representative embodiments, transgenic plants are transgenic maize, soybean, barley, alfalfa, sunflower, canola, soybean, cotton, peanut, sorghum, tobacco, sugarbeet, rice, wheat, rye, turfgrass, millet, sugarcane, tomato, or potato.

A transformed (transgenic) plant of the presently disclosed subject matter includes a plant, the genome of which is augmented by an exogenous nucleic acid molecule, or in which a gene has been disrupted, e.g., to result in a loss, a decrease, or an alteration in the function of the product encoded by the gene, which plant can also have increased yields and/or produce a better-quality product than the corresponding wild-type plant. The nucleic acid molecules of the presently disclosed subject matter are thus useful for targeted gene disruption, as well as for use as markers and probes.

The presently disclosed subject matter also provides a method of plant breeding, e.g., to prepare a crossed fertile transgenic plant. The method comprises crossing a fertile transgenic plant comprising a particular nucleic acid molecule of the presently disclosed subject matter with itself or with a second plant, e.g., one lacking the particular nucleic acid molecule, to prepare the seed of a crossed fertile transgenic plant comprising the particular nucleic acid molecule. The seed is then planted to obtain a crossed fertile transgenic plant. The plant can be a monocot or a dicot. In a particular embodiment, the plant is a cereal plant.

The crossed fertile transgenic plant can have the particular nucleic acid molecule inherited through a female parent or through a male parent. The second plant can be an inbred plant. The crossed fertile transgenic can be a hybrid. Also included within the presently disclosed subject matter are seeds of any of these crossed fertile transgenic plants.

### C. Seed Production

Some embodiments of the presently disclosed subject matter also provide seed and isolated product from plants that comprise an expression cassette comprising a promoter sequence operatively linked to an isolated nucleic acid as disclosed herein. In some embodiments, the isolated nucleic acid molecule is selected from the group consisting of:
a. a nucleic acid molecule encoding a polypeptide comprising an amino acid sequence of one of even numbered SEQ ID NOs: 2-192;
b. a nucleic acid molecule comprising a nucleic acid sequence of one of odd numbered SEQ ID NOs:1-191;
c. a nucleic acid molecule that has a nucleic acid sequence at least 90% identical to the nucleic acid sequence of the nucleic acid molecule of (a) or (b) ;
d. a nucleic acid molecule that hybridizes to (a) or (b) under conditions of hybridization selected from the group consisting of:
   i. 7% sodium dodecyl sulfate (SDS), 0.5 M NaPO₄, 1 mM ethylenediamine tetraacetic acid (EDTA) at 50°C with a final wash in 2X standard saline citrate (SSC), 0.1 % SDS at 50°C;
   ii. 7% SDS, 0.5 M NaPO₄, 1 mM EDTA at 50°C with a final wash in 1X SSC, 0.1% SDS at 50°C;
   iii. 7% SDS, 0.5 M NaPO₄, 1 mM EDTA at 50°C with a final wash in 0.5X SSC, 0.1 % SDS at 50°C;
   iv. 7% sodium dodecyl sulfate (SDS), 0.5 M NaPO₄, 1 mM EDTA at 50°C with a final wash in 0.1X SSC, 0.1% SDS at 50°C; and
   v. 7% sodium dodecyl sulfate (SDS), 0.5 M NaPO₄, 1 mM EDTA at 50°C with a final wash in 0.1X SSC, 0.1% SDS at 65°C;
e. a nucleic acid molecule comprising a nucleic acid sequence fully complementary to (a); and
f. a nucleic acid molecule comprising a nucleic acid sequence that is the full reverse complement of (a).

In one embodiment the isolated product comprises an enzyme, a nutritional polypeptide, a structural polypeptide, an amino acid, a lipid, a fatty acid, a polysaccharide, a sugar, an alcohol, an alkaloid, a carotenoid, a propanoid, a steroid, a pigment, a vitamin, or a plant hormone.

Embodiments of the presently disclosed subject matter also relate to isolated products produced by expression of an isolated nucleic acid containing a nucleotide sequence selected from the group consisting of:
(a) a nucleotide sequence that hybridizes under conditions of hybridization of 45°C in 1 M NaCl, followed by a final washing step at 50°C in 0.1 M NaCl to a nucleotide sequence listed in odd numbered sequences of SEQ ID NOs:1-191, or a fragment, domain, or feature thereof;
(b) a nucleotide sequence encoding a polypeptide that is an ortholog of a polypeptide listed in even numbered sequences of SEQ ID NOs: 2-192, or a fragment, domain, or feature thereof;
(c) a nucleotide sequence complementary (for example, fully complementary) to (a) or (b); and
(d) a nucleotide sequence that is the reverse complement (for example, its full reverse complement) of (a) or (b) according to the present disclosure.

In one embodiment, the product is produced in a plant. In another embodiment, the product is produced in cell culture. In another embodiment, the product is produced in a cell-free system. In one embodiment, the product comprises an enzyme, a nutritional polypeptide, a structural polypeptide, an amino acid, a lipid, a fatty acid, a polysaccharide, a sugar, an alcohol, an alkaloid, a carotenoid, a propanoid, a steroid, a pigment, a vitamin, or a plant hormone. In another embodiment, the product is polypeptide comprising an amino acid sequence listed in even numbered sequences of SEQ ID NOs: 2-192, or ortholog thereof. In one embodiment, the polypeptide comprises an enzyme.

In seed production, germination quality and uniformity of seeds are essential product characteristics. As it is difficult to keep a crop free from other crop and weed seeds, to control seedborne diseases, and to produce seed with good germination, fairly extensive and well-defined seed production practices have been developed by seed producers who are experienced in the art of growing, conditioning, and marketing of pure seed. Thus, it is common practice for the farmer to buy certified seed meeting specific quality standards instead of using seed harvested from his own crop. Propagation material to be used as seeds is customarily treated with a protectant coating comprising herbicides, insecticides, fungicides, bactericides, nematicides, molluscicides, or mixtures thereof. Customarily used protectant coatings comprise compounds such as captan, carboxin, thiram (tetramethylthiuram disulfide; TMTD®; available from R. T. Vanderbilt Company, Inc., Norwalk, Connecticut, United States of America), methalaxyl (APRON XL®; available from Syngenta Corp., Wilmington, Delaware, United States of America), and pirimiphos-methyl (ACTELLIC®; available from Agriliance, LLC, St. Paul, Minnesota, United States of America). If desired, these compounds are formulated together with further carriers, surfactants, and/or application-promoting adjuvants customarily employed in the art of formulation to provide protection against damage caused by bacterial, fungal, or animal pests. The protectant coatings can be applied by impregnating propagation material with a liquid formulation or by coating with a combined wet or dry formulation. Other methods of application are also possible such as treatment directed at the buds or the fruit.

The presently disclosed subject matter will be further described by reference to the following detailed examples. These examples are provided for purposes of illustration only, and are not intended to be limiting unless otherwise specified.

### Examples

The following Examples have been included to illustrate modes of the presently disclosed subject matter. In light of the present disclosure and the general level of skill in the art, those of skill will appreciate that the following Examples are intended to be exemplary only and that numerous changes, modifications, and alterations can be employed without departing from the scope of the presently disclosed subject matter.

### Example I

Plant growth is accomplished two ways: by cell growth and by cell division, each of which is respectively controlled by the G1 phases and the M phases of the cell cycle. Cyclins are proteins that play an active role in controlling nuclear cell division cycles, and regulate cyclin dependent kinases (CDKs), which are essential for cell cycle progression in eukaryotes. John et al., 2001 teaches that all cyclins interact with the catalytic subunit of cyclin-dependent protein kinases (CDK), and the two proteins (i.e., the cyclin and CDK), along with the CDK activating subunit, in turn phosphorylate substrates on serine or threonine residues, thereby controlling a chain of events that advance the cell through the various phases of the cell cycle.

Eukaryotic cells have multiple classes of cyclins, each of which is required for specific regulatory steps during the cell cycle. Activity and substrate specificity of the cyclin-CDK enzyme complex is determined by the specific cyclin subunit associated with the CDK catalytic subunit. Thus, the association of CDKs with specific cyclins is a key regulatory mechanism that advances the cell through the various stages of the cell cycle. Cell cycle progression involves changes in abundance of individual cyclins, due to changing rates of their transcription or proteolysis, with consequent changes in the substrates of CDK through the cell cycle. Cyclin accumulation is particularly important in terminating the G1 phase, when such accumulation raises CDK activity and starts events leading to DNA replication.

Cyclins are essential for CDK activation and their binding to specific individual proteins is thought to provide potential substrates to CDKs (John et al., 2001). Thus, the yeast two-hybrid approach was thought to be a useful method to dissect cyclin-mediated cell cycle events. Cyclin and CDK complex substrates include CDK inhibitors, kinases and phosphatases, enzymes that control DNA replication, the cytoskeletal structures necessary for chromosome movement during mitosis, and compounds of the ubiquitin-dependent pathway for degradation of proteins, all of which participate in key steps of the cell cycle. High levels of CDK activity alternate with high levels of proteolytic activity, which is responsible for the turnover of cyclins and CDK inhibitors.

The eukaryotic cell cycle has a growth phase and a reproductive phase, the latter involving replication of chromosomes and their subsequent distribution to daughter cells. Cyclins are well conserved, and thus have been comparatively well characterized in plants. However, while the basic mechanisms of cell cycle control and the key genes that mediate cell cycle progression are highly conserved in eukaryotes (reviewed in Potuschak & Doerner, 2001; John et al., 2001), some pathways regulating cell proliferation in plants are different from those in animals partly because plants are sessile and require developmental flexibility to respond to a spectrum of environmental changes (e.g., flexible growth rates and patterns to exploit their environment optimally, cell division and expansion being essential to responding to environmental changes). Therefore, the pathways regulating cell proliferation in plants are likely different from those in animals. In higher plants, the cell cycle is coupled with developmental phase changes that are regulated by a complex gene network. (CDK-cyclin complexes and their involvement in cell cycle progression are reviewed by John et al., 2001). Plant cyclins and their associations with CDKs and substrate proteins are important and serve as key regulatory mechanisms that control proliferation in response to the many environmental and developmental cues that affect plant growth and development. The role of cyclin-CDK complexes in regulation of the plant cell cycle is reviewed in John et al., 2001 and Potuschak & Doerner, 2001.

This Example provides newly characterized rice proteins interacting with *O. sativa* E2F Homolog (OsE2F1) and identified by means of a yeast two-hybrid assay technology. One of the interactors found is a rice DP homolog similar to *Triticum sp.* DP Protein. This interactor was named Hypothetical Protein 018989-4003 (Os018989-4003) and was also used as a bait in the yeast two-hybrid screen.

In animals, members of the E2F transcription factor family regulate the expression of genes required for progression through the cell cycle, such as genes coding for several regulatory proteins and for enzymes involved in nucleotide and DNA synthesis. Specifically, E2F/DP complexes are important regulators of the G1/S transition (reviewed by Trimarchi & Lees, 2002), at which checkpoint cells either initiate the S phase or undergo arrest of the cell cycle. E2F transcriptional activity results from the concerted action of a family of E2F-like proteins that form heterodimers. Based on sequence homology and functional properties of the genes that encode them, at least six E2F (E2F1 - E2F6) and two DP (DP1 and DP2) proteins have been identified in mammals as components of E2F complexes existing in all possible combinations. E2F subgroups (E2F1, E2F2 and E2F3, versus E2F4 and E2F5) are functionally distinct from each other, and are thought to act in opposition to one another to mediate the activation or the repression of cell cycle regulator genes, thereby promoting either cellular proliferation or cell cycle arrest and terminal differentiation. Additionally, E2F activity is regulated by interactions with other cellular proteins including the three members of the retinoblastoma (RB) protein family pRB, p107 and p130, which bind to E2F and negatively regulate its transcriptional activity, and by indirect binding of cyclins and cyclin-dependent kinases (CDKs). Phosphorylation of RB proteins by G1-specific CDKs releases the E2F heterodimer from the RB protein in late G1 to S phase, and the resulting "free E2F" induces the expression of many genes implicated in cellular proliferation, including cell cycle regulators and enzymes required for DNA synthesis. Individual E2F-DP complexes elicit different transcriptional responses depending on the identity of the E2F subunits and the proteins that are associated with the complex. These observations lend support to the yeast two-hybrid approach as a method to dissect E2F-mediated cell cycle control.

A number of cDNAs encoding E2F or DP homologs have been isolated from plants and characterized, including three E2F and two DP proteins from *Arabidopsis thaliana* (Magyar et al., 2000; reviewed in Kosugi & Ohashi, 2002). Plant E2Fs share high sequence similarity but no distinguishable similarity with the animal E2F proteins, though they slightly resemble E2F-4 and E2F-5. However, evidence is accumulating that plant E2F-like genes are functionally equivalent to their mammalian homologs and that the G1/S transition in plants is at least partly under the control of regulators similar to those found in animals, such as D-type cyclins, Rb-related proteins, and E2F and DP homologs. Like animal E2Fs, plant E2F proteins can bind to the consensus binding sites of the animal E2F and their DNA-binding activities can be stimulated by human and plant DP proteins. They can also bind human RB or plant RB-like proteins. However, their properties, including transactivation, subcellular localization, and functional differences, have not been well characterized (Kosugi & Ohashi, 2002). One study indicates that, unlike animal E2Fs, the *Arabidopsis* E2F and DP are not predominantly localized to the nucleus, but rather their nuclear localization is controlled by an interaction with some DPs andor other proteins (Kosugi & Ohashi, 2002). Based on these findings, Kosugi & Ohashi, 2002 suggests that the function of plant E2F and DP proteins is primarily controlled by their nuclear localization mediated by the interaction with specific partner proteins, and that this difference in the regulation of the E2F/RB pathway between plants and animals can reflect differences in cell cycle regulation.

The protein interactions involving the rice E2F and DP homologs identified in this Example are aimed at elucidating the mechanisms of E2F-mediated cell cycle regulation in plants. Proteins that participate in cell cycle regulation in rice are targets for genetic manipulation or for compounds that modify their level or activity, thereby modulating the plant cell cycle. The identification of genes encoding these proteins, as described herein, allows genetic manipulation of crops or application of compounds to modulate the plant cell cycle and effect agronomically desirable changes in plant development or growth.

### Results

OsE2F1 was found to interact with four novel rice proteins: two DP-like proteins (Os018989-4003 and OsPN26539); a kinesin-like protein (OsPN29946) with a putative microtubule motor function in events occurring in the G1/S transition phase of the cell cycle; and a protein of unknown function (OsPN30852).

The novel DP protein Os018989-4003 (as either bait or prey in the yeast two-hybrid screen) interacted with rice E2F homolog OsE2F1 (described above) and with two splicing variants of rice E2F2 homolog, OsE2F2 (annotated in the public domain) and OsE2F2 (367) (identified in this study). The OsE2F2 (367) variant also interacted with another novel DP-like protein, OsPN31182. Other interactors identified for the DP protein Os018989-4003 include rice kinesin-like protein (OsAAG13527); MADS box protein MADS14 (OsMADS14), with a known role in flower development; putative myosin heavy chain (OsAAK72891), which likely functions as an actin motor in cell-cycle-dependent cytoskeletal dynamic events; and another myosin heavy-chain-like protein, the novel protein OsPN22824.

The interacting proteins of this Example are listed in Tables 1 and 2 below, followed by detailed information on each protein and a discussion of the significance of the interactions. A diagram of the some of the interactions described in this Example is provided in Figure 1. The nucleotide sequences (from which the amino acid sequences can be deduced) of the proteins of this Example are provided in odd numbered SEQ ID NOs: 1-11, and 193-199.

Some of the proteins identified represent novel rice proteins previously uncharacterized. Based on their predicted biological function and on the ability of the prey proteins to specifically interact with rice E2F homolog OsE2F1and DP homolog Os018989-4003, the interacting proteins are likely involved in the E2F-mediated regulation of the cell cycle.

**Table 1**

| Interacting Proteins Identified for OsE2F1 (E2F Homolog) | | | |
|---|---|---|---|
| The names of the clones of the proteins used as baits and found as preys protein name are given. Nucleotide/protein sequence accession numbers for the proteins of this Example (or related proteins) are shown in parentheses under the protein name. The bait and prey coordinates (Coord) are the amino acids encoded by the bait fragment(s) used in the search and by the interacting prey clone(s), respectively. The source is the library from which each prey clone was retrieved. | | | |
| **Gene Name** | **Protein Name** **(GENBANK® Accession No.)** | **Bait Coord** | **Prey Coord** **(source)** |
| **BAIT PROTEIN** | | | |
| OsE2F1 PN19758 (SEQ ID NO : 194) | *O. sativa* E2F Homolog (AB041725; BAB20932) | 300-437& | |

| **INTERACTORS** | | | |
|---|---|---|---|
| Os018989- 4003* PN21044 (SEQ ID NO : 2) | Hypothetical Protein 018989-4003, Similar to *Triticum sp.* DP Protein | 100-250 | 9-179 177-294 (Output Trait) |
| OsPN26539 (SEQ ID NO : 4) | Novel Protein PN26539 (AC087544), Probable DP | 100-250 | 2x 66-346 2x 194-346 82-253 (Output Trait) |
| OsPN29946 (SEQ ID NO: 6) | Novel Protein PN29946, Similar to *A. thaliana* Kinesin-Like Protein (BAB11329.1; e=0.0) | 100-250 | 2x 173-470 (Output Trait) |
| OsPN30852 (SEQ ID NO: 8) | Novel Protein PN30852 | 100-250 | 45-86 (Output Trait) |

| | | | |
|---|---|---|---|
| & Self-activating clone, i.e., it activates the reporter genes in the two-hybrid system in the absence of a prey protein, and thus it was not used in the search * This protein was also used as a bait in this Example (see Table 2). | | | |

**Table 2**

| Interacting Proteins Identified for Os018989-4003 (Hvpothetical Protein 018989-4003, Similar to *Triticum sp.* DP Protein) | | | |
|---|---|---|---|
| **Gene Name** | **Protein Name** **(GENBANK® Accession No.)** | **Bait Coord** | **Prey Coord** **(Source)** |
| **BAIT PROTEIN** | | | |
| Os018989-4003 PN21044 (SEQ ID NO: 2) | Hypothetical Protein 018989-4003, Similar to *Triticum sp.* DP Protein | | |

| **INTERACTORS** | | | |
|---|---|---|---|
| OsE2F1 PN19758 (SEQ ID NO: 194) | *O. sativa* E2F Homolog (AB041725; BAB20932) | 90-220 | 191-436 (Output Trait) 95-276 (Input Trait) |
| OsE2F2# PN21003 (SEQ ID NO: 10) | *O. sativa* E2F2 Homolog (AB041726; BAB20933) | 90-220 | 90-358 (Input Trait) |
| OsAAG13527 PN23367 (SEQ ID NO: 196) | *O. sativa* Kinesin-like Protein (AC068924; AAG13527.1) | 90-220 | 668-859 (Output Trait) |
| OsAAK72891 PN26317 (SEQ ID NO: 198) | *O. sativa* Putative Myosin Heavy Chain (AC091123; AAK72891) | 90-220 | 342-638 322-549 (Input Trait) 339-651 (Output Trait) |
| OsMADS14* PN20910 (SEQ ID NO: 200) | *O. sativa* MADS Box Protein MADS14 (AF058697, AAF19047) | 90-220 | 54-180 (Output Trait) |
| OsPN22824& (SEQ ID NO: 12) | Novel Protein PN22824, Myosin heavy chain | 90-220 | 2x 393-494 (Output Trait) |

| | | | |
|---|---|---|---|
| # A splicing variant of the OsE2F2 sequence, OsE2F2 (367), was used as a bait; its interactions are shown below in Table 3 * Additional interactions identified for OsMADS14 are listed below on Table 4 & Additional interactions identified for PN22824 are listed below on Table 5 | | | |

**Table 3**

| Interacting Proteins Identified for OsE2F2 (E2F2 Homolog, Alternative Transcript, 367) | | | |
|---|---|---|---|
| **Gene Name** | **Protein Name** **(GENBANK® Accession No.)** | **Bait Coord** | **Prey Coord** **(source)** |
| **BAIT PROTEIN** | | | |
| OsE2F2 (367) PN21003 (SEQ ID NO: 10) | E2F2 Homolog, Alt. Transcript (367) (AB041726; BAB20933) | 180-368 | |

| **INTERACTORS** | | | |
|---|---|---|---|
| Os018989- 4003 PN21044 (SEQ ID NO: 2) | Hypothetical Protein 018989-4003, Similar to *Triticum sp.* DP Protein | 1-368 | 69-294 (Input Trait) |
| OsPN31182 (SEQ ID NO: 14) | Novel Protein PN31182, *A. thaliana* DP-Like Protein (CAC15483.1; 9e⁻⁵⁵) | | 124-324 72-255 156-334 (Input Trait) |

**Table 4**

| Additional interactions identified for OsMADS14 | | | |
|---|---|---|---|
| **Gene Name** | **Protein Name** **(GENBANK® Accession No.)** | **Bait Coord** | **Prey Coord** **(source)** |
| **PREY PROTEIN** | | | |
| OsMADS14 PN20910 (SEQ ID NO: 200) | *O. sativa* MADS Box Protein MADS14 (AF058697, AAF19047) | 50-198 | 124-223 82-197 (output trait) |

| **BAIT PROTEIN** | | | |
|---|---|---|---|
| OsMADS45 PN20231 (1905929- OS000555) (SEQ ID NO: 202) | *O. sativa* MADS Box Protein MADS45 (U31994, AAB50180) | | |

**Table 5**

| Additional interactions identified for OsPN22824 | | | |
|---|---|---|---|
| **Gene Name** | **Protein Name** **(GENBANK® Accession No.)** | **Bait Coord** | **Prey Coord** **(source)** |
| **PREY PROTEIN** | | | |
| OsPN22824 (SEQ ID NO: 12) | Novel Protein PN22824 | 1-198 | 301-500 (Input Trait) |

| **BAIT PROTEIN** | | | |
|---|---|---|---|
| OsRACD PN19695 (SEQ ID NO: 204) | *O. sativa* Small GTP-Binding Protein RACDP (AF218381; AAF28764) | | |

### Two-hybrid system using OsE2F1as bait

OsE2F1 (GENBANK® Accession No. BAB20932; Kosugi & Ohashi, 2002) is a 436-amino acid protein that is a member of the E2F transcription factor family. It contains a transcription factor E2F/dimerization partner (TDP) signature (amino acids 108 to 333), as predicted by analysis of the amino acid sequence (3.1e⁻³⁵ prediction value). E2F proteins function as heterodimers with transcription factors called DP proteins (Wu et al., 1995). These transcriptional complexes regulate the transcription of genes encoding proteins required for progression through the cell cycle. Consistent with the interactions of E2F transcription factors with DP proteins documented in the literature are those identified in this Example between the rice orthologs of these proteins. It is likely that the Os018989-4003-OsE2F1 interaction represents a step in cell cycle control in rice. This interaction was identified for both Os018989-4003 and OsE2F1 used as bait.

The bait fragment used in the yeast two-hybrid screen encoded amino acids 100 to 250 of OsE2F1.

OsE2F1 was found to interact with Os018989-4003, a protein of 294 amino acids that includes the presence of a transcription factor E2F/dimerization partner (TDP) signature (amino acids 100 to 294, 3.2e⁻¹⁷). E2F transcription factors form heterodimers with DP proteins; the resulting E2F/DP transcriptional complexes function as transcriptional activators of genes required for progression through the cell cycle (Wu et al., 1995). The activity of E2F/DP complexes is normally regulated by association with negative regulators of the retinoblastoma protein (pRB) family such as pRB, p107, and p130, and with other cellular proteins including cyclins and cyclin-dependent kinases (CDKs). Wu et al., 1995 also demonstrated that the binding specificity of the various E2F/DP complexes towards pRB or p107 is mediated by the E2F subunit. In agreement with the presence of the TDP signature, a BLAST analysis of the amino acid sequence of Os018989-4003 against the Genpept database indicated that this protein shares 62.5% identity with *Triticum sp.* DP protein (GENBANK® Accession No. CAC19034, 62.5%, e⁻⁹¹). These analyses thus indicate that Os018989-4003 is a rice DP homolog.

Os018989-4003 was also used as a bait in the yeast two-hybrid screen. Its interactions are shown in Table 2 and discussed later in this Example.

OsE2F1 was also found to interact with novel protein OsPN26539. A BLAST analysis of the nucleotide sequence of the prey clone OsPN26539 identified the gene potentially encoding novel protein PN26539 on rice chromosome 10 clone nbxb0046P18A (GENBANK® Accession No. 26539). A BLAST analysis of the 346-amino acid sequence of OsPN26539 indicated that this protein is similar to a putative protein (GENBANK® Accession No. NP_568116.1, 61% identity, 2e⁻¹⁰³), Transcription Factor-Like Protein (GENBANK® Accession No. T48364, 56% identity, 6e⁻⁹⁶), and DP-Like Protein (GENBANK® Accession No. CAC15483, 53% identity, e⁻⁵⁵), all from *A. thaliana*. The DP-like protein is AtDPa, one of the two distinct DP-related proteins (AtDPa and AtDPb) identified in *Arabidopsis* by Magyar et al., 2000. These authors showed that AtDPa and AtDPb heterodimerize *in vitro* with the *Arabidopsis* E2F-related proteins AtE2Fa and AtE2Fb identified by the same group. They also found that the *AtDPa* and *AtE2Fa* genes are transcribed in a cell cycle-dependent manner, being predominantly produced in actively dividing cells, with highest transcript levels in early S phase cells. The novel protein OsPN26539 is thus likely a rice DP transcription factor.

OsE2F1 was also found to interact with novel protein OsPN29946. A BLAST analysis of the 614-amino acid sequence of OsPN29946 indicated that this protein is similar to kinesin-like protein (GENBANK® Accession No. BAB11329.1, 70.9% identity, e = 0.0) from *A. thaliana*. Kinesins are molecular motors, molecules that hydrolyze ATP and use the derived energy to generate motor force. Molecular motors are involved in diverse cellular functions such as vesicle and organelle transport, cytoskeleton dynamics, morphogenesis, polarized growth, cell movements, spindle formation, chromosome movement, nuclear fusion, and signal transduction. Three families of non-plant molecular motors (kinesins, dyneins, and myosins) have been characterized. Kinesins and dyneins use microtubules, while myosins use actin filaments as tracks to transport materials intracellularly. A large number (about 40) of kinesin and myosin motors have been identified in *A. thaliana*, although little is known about plant molecular motors and their roles in cell division, cell expansion, cytoplasmic streaming, cell-to-cell communication, membrane trafficking, and morphogenesis. Calcium, through the calcium binding protein calmodulin, is thought to play a key role in regulating the function of both microtubule- and actin-based motors in plants (molecular motors are reviewed in Reddy, 2001). The kinesin-like calmodulin (CaM) binding protein (KCBP), a minus end-directed microtubule motor protein unique to plants, has been implicated in cell division. During nuclear envelope breakdown and anaphase, activated KCBP promotes the formation of a converging bipolar spindle by sliding and bundling microtubules, while KCBP activity is down-regulated by Ca²⁺ and CaM during metaphase and telophase (Vos et al., 2000). The prey protein OsPN29946 is a kinesin-like protein likely involved in microtubule movements and its association with OsE2F1 suggests that this interaction can represent a step in the control of cell-cycle dependent events involving cytoskeleton organization.

OsE2F1was also found to interact with novel protein OsPN30852. A BLAST analysis of the 86-amino acid sequence of OsPN30852 indicated that this protein is similar to an unknown protein from *A. thaliana* (GENBANK® Accession No. AAK48957.1, 80% identity, 4e⁻³¹). Analysis of gene expression in plants indicated that this gene is up-regulated by stress and by abscisic acid and jasmonic acid (JA).

### Two-hybrid system using Os018989-4003 as bait

Hypothetical protein Os018989-4003, which is similar to *Triticum sp*. DP Protein, was used as bait in the two-hybrid assay. This protein is described as an interactor for OsE2F1 earlier in this Example. The bait clone used in the screen encoded amino acids 90 to 220 of Os018989-4003.

The bait fragment encoding amino acids 90 to 220 of Os18989-4003 was found to interact with OsE2F1 (see description above). The interaction of Os018989-4003 with OsE2F1 confirms the interaction between the same proteins in the reverse bait and prey roles described earlier in this Example.

Os18989-4003 was also found to interact with OsE2F2. OsE2F2 is a protein of 393 amino acids that includes a transcription factor E2F/dimerization partner (TDP; amino acids 74 to 300). A BLAST analysis indicated that this protein is the rice E2F homolog (GENBANK® Accession No. BAB20933, 100% identity, e = 0.0), a member of the E2F transcription factor family. E2F transcription factor family members have been described herein. OsE2F2 is translated from one of two alternatively spliced mRNA species (identified in this study) and, like other E2F family members, it likely regulates transcription of genes encoding proteins involved in cell cycle progression in rice.

The splicing variant of OsE2F2, OsE2F2 (367), has a sequence of 367 amino acids that includes a predicted transcription factor E2F/dimerization partner (TDP; amino acids 84 to 310, e⁻³⁹ prediction value). A BLAST analysis of its amino acid sequence determined that it is the rice E2F homolog (GENBANK® Accession No. BAB20933, 100% identity, e = 0.0). OsE2F2 (367) was also used as a bait in this study and found to interact with the following two DP proteins (these interactions are shown in Table 3):
a) Hypothetical protein 018989-4003 (Os018989-4003, described above), which is similar to *Triticum sp*. DP Protein. The OsE2F2 (367)-Os018989-4003 interaction validated the interaction between the same DP protein, namely 018989-4003, and OsE2F2.
b) Protein PN31182 (OsPN31182), which is similar to *A. thaliana* DP-Like Protein. OsPN31182 is a novel protein of 379 amino acids. A BLAST analysis indicated that the amino acid sequence of OsPN31182 is similar to *A. thaliana* Putative Protein (top hit, GENBANK® Accession No. NP_568116.1, 70% identity, 5e⁻¹⁰⁸) and DP-Like Protein (third hit, GENBANK® Accession No. CAC15483.1, 50% identity, 9e⁻⁵⁵), and to DP-like proteins from other organisms. OsPN31182 is thus a novel rice DP protein.

DP proteins heterodimerize with E2F transcription factors to regulate the transcription of genes encoding proteins that are important for cell cycle progression. This notion is consistent with the interactions identified here between the rice E2F homolog OsE2F2 (367) and the DP-like proteins Os018989-4003 and OsPN31182. It is likely that these interactions participate in cell cycle progression in rice.

Os18989-4003 was also found to interact with OsAAG13527, an 859-amino acid protein determined by BLAST analysis to be the rice Kinesin-Like Protein (GENBANK® Accession No. AAG13527.1, 100% identity, e = 0.0). Kinesins are molecular motors associated with microtubule movement during diverse cellular events, and have been described herein.

Os18989-4003 was also found to interact with the putative myosin heavy chain protein OsAAK72891. A BLAST analysis of the OsAAK72891 amino acid sequence determined that this protein is the rice Putative Myosin Heavy Chain (GENBANK® Accession No. AAK72891.1, 100% identity, e = 0.0).

Members of the myosin family participate in many types of cellular motility in all eukaryotic cells. Myosins are cytoskeletal proteins that function as molecular motors to generate movement and mechanical force in ATP-dependent interactions with actin filaments in various cellular events. The superfamily of myosin proteins has been divided into at least 14 classes (designated I to XIV) on the basis of their conserved ATPase- and actin-binding regions, each myosin containing tail domains believed to be responsible for the specific subcellular localization and function of these motors (reviewed in Reichelt et al., 1999). Molecular motors are involved in diverse cellular functions such as vesicle and organelle transport, cytoskeleton dynamics, morphogenesis, polarized growth, cell movements, spindle formation, chromosome movement, nuclear fusion, and signal transduction (molecular motors are reviewed in Reddy, 2001). While the role of myosins in animal and unicellular organisms is well established in muscular contraction, cytokinesis, and membrane-associated functions such as vesicle transport and membrane dynamics, little is known about myosins and other molecular motors in plants and their roles in cell division, cell expansion, cytoplasmic streaming, cell-to-cell communication, membrane trafficking, and morphogenesis (Reddy, 2001).

Myosins in higher plants are thought to participate as motors in intracellular transport of organelles and vesicles associated with cytoplasmic streaming and in tip-growing cells of pollen tubes (reviewed in Yokota et al., 1999b). The active sliding of myosin heavy chain along actin filaments provides the motor force for cytoplasmic streaming (i.e., the constant movement of the cytoplasm and suspended organelles, membrane systems and molecules which is observed in plant cells), and the myosin activity is regulated by calcium through the calcium-binding protein calmodulin (Yokota et al., 1999a; Yokota et al., 1999b). The function of cytoplasmic streaming and the mechanisms of its biochemical regulation are not known, although it is thought to facilitate the exchange of materials within the cell and between the cell an its environment. Specific movement and anchoring of some organelles is also known to depend on actin filaments and is thus thought to involve myosin, but these mechanisms have not been documented (myosins are discussed in Buchanan et al., 2002, at page 221). Additionally, Reichelt et al., 1999 localized a plant myosin VIII at the post-cytokinetic cell wall, suggesting a role for this protein in cytokinesis, specifically in maturation of the cell plate and reestablishment of cytoplasmic actin cables at sites of intercellular communication. Based on current knowledge of plant myosins, the rice heavy chain myosin OsAAK72891 can be a cytoskeletal component that participates in cytoplasmic streaming events in a cell-cycle-dependent manner.

Os18989-4003 was also found to interact with OsMADS14 (GENBANK® Accession No. AF058697), a 246-amino acid protein that includes a MADS box domain (amino acids 1 to 61). Moon et al. report that OsMADS14 is homologous to the maize AP1 homolog ZAP1 and classify it as a member of the SQUAMOSA-like (SQUA) subfamily in the AP1/AGL9 family of MADS box genes, which control the specification of meristem and organ identity in developing flowers (Moon et al., 1999). OsMADS14 was expressed from the early through the later stages of flower development, with transcripts detectable in sterile lemmas, paleas/lemmas, stamens, and carpels of mature flowers. Moon et al. suggested that this gene regulates a very early stage of flower development, based on their observation that transgenic plants ectopically expressing OsMADS14 exhibit extreme early flowering and dwarfism (Moon et al., 1999). MADS box proteins are known to regulate transcription as heterodimers or ternary complexes that include other MADS box proteins, and these interactions are thought to occur through the K box present in MADS proteins (Lim et al., 2000, Moon et al., 1999).

Because MADS box proteins are known to mediate various plant developmental processes as heterodimers or trimers, and given the involvement of the DP protein Os018989-4003 in the regulation of genes required for cell cycle progression, it is likely the interaction between the MADS box protein OsMADS14 and Os018989-4003 represents a newly characterized interaction that regulates transcription of genes associated with plant development in rice.

OsMADS14 was also found to interact with the MADS box protein OsMADS45 (GENBANK® Accession No. AAB50180; see Table 4). OsMADS45 is a 249-amino acid protein that includes a MADS box domain (amino acids 1 to 61) and two coiled coils (amino acids 83 to 117 and amino acids 152 to 176); the coiled coils are likely part of a K-box predicted between amino acids 73 and 176. The OsMADS45 gene, identified by Greco et al., 1997, encodes a protein highly homologous to the products of *Arabidopsis AGL2* and *AGL4* MADS box genes. Temporal and spatial RNA expression patterns suggest that the rice OsMADS45 and *Arabidopsis* AGL2 and AGL4 play similar roles in flower development (Greco et al., 1997), specifically in the development of all floral organs by acting as intermediates between the meristem identity and organ identity genes (Savidge et al., 1995).

A BLAST analysis comparing the nucleotide sequence of OsMADS45 against TMRI's GENECHIP® Rice Genome Array sequence database identified probeset OS014912_f_at (6e⁻⁶⁴ expectation value) and probeset OS000555_f_at (6e⁻⁶⁰) as the closest matches. Analysis of gene indicated that these genes are expressed early in seed development.

Os18989-4003 was also found to interact with OsPN22824, a 500-amino acid protein fragment. A BLAST analysis of the OsPN22824 amino acid sequence revealed no high similarity with any of the proteins in the Genpept database. The most similar amino acid sequences are six plant proteins of unknown function, the top hit being *A. thaliana* Expressed Protein (GENBANK® Accession No. NP_564015.1, 33% identity, 5e⁻⁴⁵), and *A*. *thaliana* Myosin Heavy-Chain-Like (seventh hit, GENBANK® Accession No. BAA97502, 29% identity, e⁻⁰¹⁶). In agreement with these results, the most similar protein in Myriad's database is human Myosin, Heavy Chain llx/d, Skeletal Muscle (MyHC-lix/d; 23% identity, e = 0.004).

OsPN22824 was also found to interact with rice Small GTP-Binding Protein RACDP (OsRACD; GENBANK® Accession No. AAF28764; see Table 5). OsRACD is a 197-amino acid protein that includes an ATP/GTP-binding site motif A (P-loop, amino acids 13 to 20) and a prenyl group binding site (CAAX box, amino acids 194 to 197). Analysis of the amino acid sequence by SMART identified a Rho (Ras homology) signature (amino acids 9 to 180, 6e⁻¹¹⁶), while analysis by Pfam predicted nearly the same region to be a Ras family signature (amino acids 8 to 197, 2.3e⁻⁷⁸). These predictions indicate that OsRACD is a member of the Rho subfamily of Ras-like small GTPases. Hydrolysis of GTP to GDP is an important step in many intracellular signal transduction pathways that control various cellular processes such as cell growth and development, apoptosis, lipid metabolism, cytoarchitecture, membrane trafficking, and transcriptional regulation (Aznar & Lacal, 2001). The rice OsRACD protein has not been described, however, other members of the Rho subfamily have been characterized. Cdc42, Rac, and Rho isoforms regulate the assembly and disassembly of the actin cytoskeleton in response to extracellular signals (Tapon & Hall, 1997). Plant small GTPase Rac homologs are components of the oxidative burst associated with disease resistance (Ono et al., 2001; Dwyer et al., 1996). OsRACD is a rice GTPase that likely participates in signal transduction involving GTP hydrolysis, and its association with the myosin-like protein OsPN22824 suggests that this GTPase activity occurs during events related to organization of the actin cytoskeleton as part of either plant development and/or response to pathogen invasion.

### Summary

OsE2F1 interacts with four novel rice proteins, two of which are DP-like proteins (Os018989-4003 and OsPN26539). In addition, the DP prey protein Os018989-4003 interacts with the E2F2 homolog splicing variant OsE2F2 (367) and, when used as bait, with both rice OsE2F1 and OsE2F2 homologs. OsE2F2 (367) also interacts with another novel DP-like protein, OsPN31182. The identification of these new DP proteins interacting with E2F proteins in rice is in accord with the presence of E2F and DP homologs identified previously in plants (reviewed in Kosugi & Ohashi, 2002). Plant E2F and DP proteins exhibit binding activities similar to those of animal E2F transcription factors, which function as heterodimeric complexes with DP or other E2F-like proteins (reviewed in Trimarchi & Lees, 2002; Magyar et al., 2000). The associations between the rice E2F and DP homologs identified in this Example are consistent with the subunit composition of E2F/DP transcription factors and provide further evidence that plant E2F-like genes are functionally equivalent to their mammalian homologs. It is likely that these interactions participate in cell cycle progression in rice.

Animal E2F/DP transcription factors play a central role in the control of the G1/S transition through integration of the activities of important regulators of the cell cycle with the transcription apparatus. The G1/S control point in plants is thought to be at least partly regulated by molecules similar to those found in animals, such as D-type cyclins, RB-related proteins, and E2F-like proteins (reviewed in Magyar et al., 2000). The G1 phase, which precedes the S phase, is a period of intense biochemical activity in which cells expand, double in size, and synthesize molecules and structures, including microtubules and other cytoskeletal structures, in preparation for cell division. The end of G1 is an important checkpoint in the control of cell cycle progression, at which the control system either arrests the cycle or triggers initiation of S phase (the plant cell cycle phases are discussed in Raven et al., 1999). OsE2F1 and the DP protein Os018989-4003 were found to interact with several cytoskeletal structural proteins, and this finding supports the notion that the rice E2F/DP transcription factor has a role in controlling events related to cell cycle progression. Two of these interactors are kinesin-like proteins: a novel rice kinesin-like protein (OsPN29946, interactor for OsE2F1) and rice kinesin-like protein annotated in the public domain (OsAAG13527, interactor for Os018989-4003).

Two additional cytoskeletal components interacting with the DP protein Os018989-4003 are myosin heavy-chain proteins: putative myosin heavy chain (OsAAK72891) and a novel rice myosin heavy-chain-like protein (OsPN22824). Kinesins and myosins are molecular motors that use microtubules (in the case of kinesins) or actin filaments (in the case of myosins) as cytoskeletal tracks to transport cargo materials intracellularly. Molecular motors, including kinesins, myosins and dyneins, have been well characterized in non-plant organisms and implicated in a variety of cellular functions such as vesicle and organelle transport, cytoskeleton dynamics, morphogenesis, polarized growth, cell movements, spindle formation, chromosome movement, nuclear fusion, and signal transduction. In contrast, the roles of the many kinesins and myosins identified in plants are largely unknown (molecular motors are reviewed in Reddy, 2001). A few studies suggest that myosin heavy-chain in higher plants participates in intracellular transport of organelles and vesicles (along actin filaments) associated with cytoplasmic streaming and in tip-growing cells of pollen tubes (reviewed in Yokota et al., 1999b). An unconventional class VIII plant myosin has been implicated in maturation of the cell plate at cytokinesis (Reichelt et al., 1999). However, the function and regulation of plant motors in cell division, cell expansion, cytoplasmic streaming, cell-to-cell communication, membrane trafficking, and morphogenesis remains to be elucidated (Reddy, 2001). Based on functional homology with animal and plant E2F proteins, which are known to participate in regulation of the G1/S transition phase, it appears that the interactions of the rice OsE2F1 and DP protein Os018989-4003 with the kinesin-like and myosin-like prey proteins identified herein represent transcriptional regulation of cell-cycle-dependent events involving cytoskeleton organization/function and possibly occurring during the G1/S transition.

Cell cycle regulators in plants must couple control of cell cycle phases to the environmental and developmental factors that affect plant growth and development. In agreement with this notion, the DP protein Os018989-4003 interacts with a protein known to regulate plant development, the MADS box protein MADS14 (OsMADS14), which in turn interacts with the MADS box protein OsMADS45. MADS box proteins mediate various plant developmental processes and, like other transcription factors, function as heterodimers or ternary complexes (for reviews, see Riechmann & Meyerowitz, 1997; Moon et al., 1999; Theissen et al., 2000). Additional interactions identified for MADS box proteins are discussed below in Example IV. The products of MADS box genes interact with each other and with other gene products participating in the genetic control of various plant development processes, with regulatory interactions (activation, repression) between the different genes/groups of genes within this network. Likewise, E2F-like proteins regulate transcription as heterodimeric complexes, and their activity is regulated by interactions with other cellular proteins (Trimarchi & Lees, 2002; Kosugi & Ohashi, 2002). Given the presumed involvement of the DP protein Os018989-4003 in the regulation of genes required for cell cycle progression, it is likely that the interaction between the DP protein Os018989-4003, possibly in heterodimer form with OsE2F1 or OsE2F2 and the MADS box protein OsMADS14, is involved in transcriptional regulation of genes important in plant development in a cell-cycle dependent fashion in rice, and that these developmental processes can occur during the G1/S phase of the cell cycle.

The fourth interactor identified for E2F1 is a protein of unknown function (OsPN30852). However, based on its association with rice E2F1 and on the presumed role of the latter in regulation of cell cycle progression, it is likely that OsPN30852 is involved in cell cycle regulation.

The rice proteins found to interact with the rice E2F and DP homologs OsE2F1 and Os018989-4003 appear to be involved in regulation of the cell cycle/plant development. Some of these interactors are newly characterized rice proteins, and their interactions with OsE2F1 and Os018989-4003 represent molecular mechanisms for E2F-mediated transcriptional regulation of the cell cycle in rice that have not been previously described.

### Example II

This Example provides newly characterized rice proteins interacting with rice cyclin OsS49462 and cyclin OsCYCOS2 identified by means of yeast two-hybrid assays.

As discussed in Example I, cyclins are regulatory proteins required to activate cyclin-dependent protein kinases (CDKs). Cyclins are classified into two groups: mitotic cyclins, which include A-type and B-type cyclins (also known as S and M cyclins, respectively), which are essential for the control of the cell cycle at the G2/M (mitosis) transition, and G1 cyclins, which include D- and E-type cyclins, which are essential for the control of the cell cycle at the G1/S (start) transition. G2/M cyclins accumulate steadily during G2 and are abruptly destroyed as cells exit from mitosis (at the end of the M-phase).

B-type cyclins contain a large conserved central domain, the cyclin box, which interacts with the kinase subunit, and a domain called mitotic destruction box, which mediates cyclin degradation late in mitosis. B-type cyclins are expressed specifically in late G2 and early M phase of the cell cycle. They regulate the cell cycle progression from G2 to mitosis during plant development, and Myb-type transcription factors can be involved in this regulation (reviewed by Doonan et al., 1997). B-type cyclins of rice plants accumulate steadily during G2 and then are rapidly degraded at mitosis (Umeda et al., 1999). The B-type cyclins OsS49462 and OsCYCOS2 share 75.1% sequence identity at the amino acid level and are both encoded by mRNAs of 1.6 kb, as reported by Sauter et al., 1995. Expression of OsCYCOS2 is induced by the plant hormone gibberellin (GA) in the intercalary meristem of deepwater rice (*Oryza sativa L.*) internodes, and that the time course of OsCYCOS2 induction is compatible with a role for both cyclins in regulating the G2/M phase transition (Sauter et al., 1995). GA promotes rapid internodal growth in this plant subspecies, and this growth occurs through signaling events requiring cell cycle induction at the G2/M transition. Thus, GA promotes the activity of p34cdc2/CDC28-like histone H1 protein kinase, an enzyme known to regulate mitosis, and that the increase in this protein kinase activity is mediated by OSCYCOS2. The cyclins were expressed in the intercalary meristem and the elongation zone of the internode, but the GA-induced increase in transcript levels was restricted to the meristem only (Sauter et al., 1995).

Thus, OsS49462 and OsCYCOS2 are B-type mitotic cyclins that regulate the cell cycle progression from G2 to mitosis. The protein interactions involving OsS49462 and OsCYCOS2 identified in this Example are useful for elucidating the mechanisms of cell cycle regulation in plants. Proteins that participate in cell cycle regulation in rice can be targets for genetic manipulation or for compounds that modify their level or activity, thereby modulating the plant cell cycle. The identification of genes encoding these proteins can allow genetic manipulation of crops or application of compounds to effect agronomically desirable changes in plant development or growth.

### Results

Cyclin OsS49462 was found to interact with a rice hypothetical protein of unknown function (OsPN25358) and with four novel rice proteins: a putative RNA-binding protein (OsPN30848) and a zinc finger protein (OsPN29942), a myosin-like protein (OsPN23484) and an unknown protein (OsPN29957). Two of these proteins (OsPN23484 and OsPN29942) also interact with the second bait, cyclin OsCYCOS2.

Cyclin OsCYCOS2 was found to interact with seven known rice proteins and with 18 novel rice proteins. The known interactors include a putative CCAAT displacement protein whose function as a transcriptional regulator is cell cycle-dependent (PN2621 0); a putative myosin heavy chain, a cytoskeletal protein that likely functions as a molecular motor to move actin filaments in events related to cell polarity or cytokinesis (PN23297); a chloroplast ATPase I subunit (PN23416); a syntaxin related protein (PN23136); a heat shock protein (PN23169); a cora-like Mg transporter (PN25381) and a hypothetical protein of unknown function (PN23363). Among the novel interactors identified are several proteins with putative roles in cytoskeletal function: four putative myosin heavy-chain proteins (PN23484, PN20815, OsPN29882, and OsPN29966); two kinesin-like proteins with a putative microtubule motor function during cell division (the calmodulin-binding protein OsPN23390 and the centromere/kinetochore protein OsPN29965); a spectrin-like protein with a presumed actin-binding function/nuclear matrix protein (OsPN29956); a putative Mg transporter (OsPN29970), a centromere homolog (PN29958) and a zinc finger protein (PN29942). Other novel interactors include a protein similar to *A. thaliana* ARM repeat-containing protein with a possible role in cell adhesion and/or signaling (OsPN23274); a chaperone heat shock protein (PN30899); and 6 proteins of unknown function (OsPN29961, OsPN29969, OsPN26688, OsPN29967, OsPN29968, OsPN30854), two of which (OsPN23484 and OsPN29942) also interact with the cyclin OsS49462 bait.

The interacting proteins of the Example are listed in Table 6 and Table 7 below, followed by detailed information on each protein and a discussion of the significance of the interactions. The nucleotide and amino acid sequences of the proteins of this Example are provided in SEQ ID NOs: 15-53 and 209-221.

Some of the proteins identified represent rice proteins previously uncharacterized. Based on their predicted biological function and on the ability of the prey proteins to specifically interact with cyclin OsS49462 and cyclin OsCYCOS2, the interacting proteins are likely part of a protein network involved in the cyclin-mediated regulation of the cell cycle.

**Table 6**

| Interacting Proteins Identified for OsS49462 (Cyclin OsS49462, fragment) | | | |
|---|---|---|---|
| The names of the clones of the proteins used as baits and found as preys are given. Nucleotide/protein sequence accession numbers for the proteins of the Example (or related proteins) are shown in parentheses under the protein name. The bait and prey coordinates (Coord) are the amino acids encoded by the bait fragment(s) used in the search and by the interacting prey clone(s), respectively. The source is the library from which each prey clone was retrieved. | | | |
| **Gene Name** | **Protein Name** **(GENBANK® Accession No.)** | **Bait Coord** | **Prey Coord** **(source)** |
| **BAIT PROTEIN** | | | |
| OsS49462 PN20325 (6331703- OS002997) (SEQ ID NO: 206) | *O. sativa* Cyclin OsS49462, Fragment (X82035) | 1-243 50-150 100-243 | |

| **INTERACTORS** | | | |
|---|---|---|---|
| PN25358 13786464 (SEQ ID NO: 208) | Hypothetical Protein AAK39589 | 1 to 100 | 2x303-472 (output trait) |
| OsPN23484 Novel (CONTIG1447_FAS TA.CONTIG1) (SEQ ID NO: 16) | Novel Protein PN23484, heavy meromyosin | 1 to 100 | 111-194 (output trait) |
| OsPN29942 novel (SEQ ID NO: 18) | Novel Protein PN29942, Fragment, zinc finger protein | 1 to 100 | 11-182 (output trait) |
| OsPN29957 novel (SEQ ID NO: 20) | Novel Protein PN29957, Fragment, unknown | 1 to 100 | 2x51-288 28-214 (output trait) |
| OsPN30848 novel (SEQ ID NO: 22) | Novel Protein PN30848, Fragment, RNA binding protein | 1 to 100 | 365-476 (input trait) |

**Table 7**

| Interacting Proteins Identified for OsCYCOS2 (*O. sativa* Cyclin OsCYCOS2) | | | |
|---|---|---|---|
| The names of the clones of the proteins used as baits and found as preys are given. Nucleotide/protein sequence accession numbers for the proteins of the Example (or related proteins) are shown in parentheses under the protein name. The bait and prey coordinates (Coord) are the amino acids encoded by the bait fragment(s) used in the search and by the interacting prey clone(s), respectively. The source is the library from which each prey clone was retrieved. | | | |
| **Gene Name** | **Protein Name** **(GENBANK® Accession No.)** | **Bait Coord** | **Prey Coord** **(Source)** |
| **BAIT PROTEIN** | | | |
| OsCYCOS2 PN20257 (1694891- OS003088 (SEQ ID NO: 210) | *O. sativa* Cyclin OsCYCOS2 (X82036) | 1-150 100-275 140-350 300-420 1-420 | |

| **INTERACTORS** | | | |
|---|---|---|---|
| PN30899 417154 (SEQ ID NO: 24) | Hypothetical Protein 000221- 3976 Similar to OsHP82, Fragment | 50-233 | 4 to 228 (output trait) |
| PN29970 (SEQ ID NO: 26) | Putative CorA-like Mg²⁺ Transporter Protein | 50 to 233 | 1-158 (output trait) |
| PN23363 13324791 (SEQ ID NO: 212) | *O. sativa* Hypothetical Protein 13324791 | 50 to 233 | 50-148 (input trait) |
| PN26210 13702813 (SEQ ID NO: 214) | *O. sativa* Putative CCAAT Displacement Protein | 170 to 310 | 422 to 646 2x364 to 613 (output trait) |
| 15451591 PN23297 (SEQ ID NO: 216) | *O. sativa* Putative Myosin Heavy Chain | 50 to 233 | 980 to 1160 (input trait) |
| PN23416 11466783 (SEQ ID NO: 218) | Chloroplast ATPase I Subunit | 50 to 233 | 130 to 176 (input trait) |
| PN23136 5922624 (SEQ ID NO: 220) | Hypothetical Protein BAA85200 Similar to Syntaxin Related Protein AtVam3p | 50 to 233 | 66 to 191 (output trait) |
| PN20815 Novel (3210- OS_ORF019753) (SEQ ID NO: 28) | Hypothetical Protein PN20815 Similar to *A. thaliana* Myosin Heavy Chain, Fragment | 170 to 310 | 1 to 134 (output trait) |
| OsPN23274 Novel (CONTIG697.FAST A.CONTIG2/ CONTIG697.FASTA. CONTIG1) (SEQ ID NO: 30) | Novel Protein PN23274, Similar to *A. thaliana* ARM Repeat-Containing Protein | 50 to 233 | 6x79 to 210 (input trait) |
| OsPN23390 novel (SEQ ID NO: 32) | Novel Protein PN23390, Putative Kinesin-like Calmodulin Binding Protein, Fragment | 50 to 233 | 595 to 845 576 to 738 (output trait) |
| OsPN23484 Novel (CONTIG1447.FAST A.CONTIG1) (SEQ ID NO: 16) | Novel Protein PN23484, heavy meromyosin | 170 to 310 | 77 to 233 2x64 to 212 90 to 245 (output trait) |
| OsPN26688 Novel (CONTIG3772.FAST A.CONTIG1) (SEQ ID NO: 34) | Novel Protein PN26688, unknown | 50 to 233 | 132 to 225 (input trait) |
| OsPN29882 novel (SEQ ID NO: 36) | Novel Protein PN29882, Fragment, myosin heavy chain | 50 to 233 | 107 to 273 (output trait) |
| OsPN29942 Novel (CONTIG3164.FAST A.CONTIG1) (SEQ ID NO: 18) | Novel Protein PN29942, Fragment, zinc finger protein | 170 to 310 | 1 to 159 (output trait) |
| OsPN29956 novel (SEQ ID NO: 38) | Novel Protein PN29956, Fragment, nuclear matrix constituent | 50 to 233 | 2x96 to 235 2 to 373 (output trait) |
| OsPN29958 novel (SEQ ID NO: 40) | Novel Protein PN29958, Fragment, centromere homologue | 50 to 233 | 3 to 304 (output trait) |
| OsPN29961 novel (SEQ ID NO: 42) | Novel Protein PN29961, Fragment, Similar to *A. thaliana* Unknown Protein BAB02349 | 50 to 233 | 10 to 215 (output trait) |
| OsPN29965 novel (SEQ ID NO: 44) | Novel Protein PN29965, Fragment, Similar to *A. thaliana* Kinesin (Centromere Protein)- Like Heavy Chain-Like Protein BAB03114 | 50 to 233 | 12 to 124 (output trait) |
| OsPN29966 novel (SEQ ID NO: 46) | Novel Protein PN29966, Fragment, myosin heavy chain | 50 to 233 | 8 to 216 (output trait) |
| OsPN29967 novel (SEQ ID NO: 48) | Novel Protein PN29967, Fragment, unknown | 50 to 233 | 3x16 to 174 (output trait) |
| OsPN29968 novel (SEQ ID NO: 50) | Novel Protein PN29968, Similar to *A. thaliana* Unknown Protein BAB01990 | 50 to 233 | 12 to 113 (output trait) |
| OsPN29969 novel (SEQ ID NO: 52) | Novel Protein PN29969, Similar to *A. thaliana* Unknown Protein BAB01990 | 50 to 233 | 2x16 to 123 (output trait) |
| OsPN25381 13357265 (SEQ ID NO: 222) | Protein 13357265 Putative CorA-like Mg²⁺ Transporter Protein | 50 to 233 | 30-218 (output trait) |
| OsPN30854 Novel (CONTIG962.FAST A.CONTIG1) (SEQ ID NO: 54) | Novel Protein PN30854, unknown | 170 to 310 | 100 to 169 (output trait) |
| OsPN30899 novel (SEQ ID NO: 24) | Novel Protein PN30899, DNAJ | 50 to 233 | 4 to 228 (output trait) |

### Two-hybrid system using OsS49462 as bait

The bait OsS49462 (GENBANK® Accession No. X82035; Sauter et al., 1995) is a 242-amino acid protein that contains a cyclin, N-terminal domain (amino acids 1 to 105, 7.1e-⁴⁹) and a cyclin C-terminal domain (amino acids 107 to 227, e⁻⁵⁰), as determined by analysis of the amino acid sequence. Like OsCYCOS2 (described as a bait below in this Example), OsS49462 is a rice B-type cyclin protein.

A BLAST analysis comparing the nucleotide sequence of OSS49462 against TMRI's GENECHIP® Rice Genome Array sequence database identified probeset OS002997.1_s_at (e = 0 expectation value) as the closest match. Analysis of gene expression indicated that this gene is not specifically expressed in several different tissue types and is not specifically induced by a broad range of plant stresses, herbicides, or applied hormones.

The bait protein encoding amino acids 1 to 100 of OsS49462 (which contains the cyclin, N-terminal domain) was found to interact with hypothetical protein AAK39589 (PN25358). Two prey clones encoding amino acids 303 to 472 of PN25358 were retrieved from the output trait library. PN25358 is a 472-amino acid protein that includes a transmembrane domain (amino acids 403 to 419), as predicted by analysis of the amino acid sequence. A BLAST analysis against the Genpept database determined that it is similar to a rice unknown protein (GENBANK® Accession No. AAK39589, e = 0) and to an *A. thaliana* putative protein (GENBANK® Accession No. NP_199010.1, 64% identity, 7e⁻¹⁶¹). BLAST analysis of the PN25358 amino acid sequence against Myriad's proprietary database found no significant similarities for this protein. Since PN25358 interacts with OsS49462, it might be involved in cell cycle regulation.

The bait protein encoding amino acids 1 to 100 of OsS49462 was also found to interact with novel protein OsPN23484. (One prey clone encoding amino acids 111 to 194 of OsPN23484 was retrieved from the output trait library) BLAST analysis suggests that PN23484 is a heavy meromyosin protein. Novel protein OsPN23484 also interacts with the bait OsCYCOS2 (described below in this Example). This observation validates the OsS49462-OsPN23484 interaction and suggests that OsPN23484 plays a broad role in regulation by cyclins and thus in the control of cell cycle progression.

The bait protein encoding amino acids 1 to 100 of OsS49462 was also found to interact with a fragment of the novel protein OsPN29942 (one prey clone encoding amino acids 11 to 182 of OsPN29942 was retrieved from the output trait library). OsPN29942 is a protein for which the complete amino acid sequence is not known. Analysis of the available 183 amino acids identified a BTB/POZ domain (amino acids 1 to 85). This domain is found primarily at the N terminus of zinc finger proteins and is evolutionarily conserved from *Drosophila* to mammals (Zollman, et al., 1994). This region can affect the DNA-binding activity of zinc finger proteins (Bradwell et al., 1994). A BLAST analysis against the Genpept database indicated that OsPN29942 shares 62% identity with an unknown protein from *A. thaliana* (GENBANK® Accession No. AAF00643, 5e⁻⁵³).

OsPN29942 also interacts with the bait OsCYCOS2 as described later in this Example. This observation validates the OsS49462-OsPN29942 interaction and suggests that OsPN29942 plays a broad role in regulation by cyclins and thus in the control of cell cycle progression.

The bait protein encoding amino acids 1 to 100 of OsS49462 was also found to interact with OsPN29957. Three prey clones, two encoding amino acids 51 to 288 and one encoding amino acids 28 to 214 of OsPN29957 were retrieved from the output trait library. OsPN29957 is a protein for which the complete amino acid sequence is not known. Upon analysis of the available 328 amino acids. A BLAST analysis against the Genpept database indicated that OsPN29957 shares 69% identity with an *A. thaliana* unknown protein (GENBANK® Accession No. NP_175186, e⁻²²). The available information makes it difficult to determine the function of OsPN29957. Discovery of the complete amino acid sequence is likely to clarify the biological role of this protein and of its interaction with OsS49462.

The bait protein encoding amino acids 1 to 100 of OsS49462 was also found to interact with PN30848 (one prey clone encoding amino acids 365 to 476 of OsPN30848 was retrieved from the input trait library). OsPN30848 is a protein for which the complete amino acid sequence is not known. Analysis of the available 497 amino acids identified two putative RNA-binding regions (amino acids 162 to 169 and amino acids 243 to 250). A BLAST analysis against the Genpept database indicated that OsPN30848 shares 50% identity with two *A. thaliana* putative RNA-binding proteins (GENBANK® Accession No. NP_190834, 2e⁻⁹⁷ and GENBANK® Accession No. AAK32943, e⁻⁹⁴) and another *A. thaliana* protein similar to nucleolin (GENBANK® Accession No. AAB62861, 46% identity, 5e⁻⁸⁹. Nucleolin is important for ribosome biogenesis and possesses RNA-binding activity. The similarity of OsPN30848 and nucleolin suggests a similar role for OsPN30848. The interaction of OsPN30848 with OsS49462 can alter cell cycle progression by regulating this activity.

A BLAST analysis comparing the nucleotide sequence of OsPN30848 against TMRI's GENECHIP® Rice Genome Array sequence database identified probeset OS_ORF013388_at (e⁻¹⁰⁸ expectation value) as the closest match. Gene expression analysis indicated that this gene is not specifically expressed in several different tissue types and is not specifically induced by a broad range of plant stresses, herbicides, or applied hormones.

### Two-hybrid system using OsCYCOS2 as bait

The 419-amino acid protein OsCYCOS2 (GENBANK® Accession No. X82036; Sauter et al., 1995) is a G2/M type cyclin. Analysis of the OsCYCOS2 amino acid sequence identified two cyclin domains spanning amino acids 200 to 284 (2.7e-²⁶) and amino acids 297 to 379 (1.29e⁻²²). Type G2/M cyclins regulate the cell cycle progression from G2 to mitosis during plant development. The role of these proteins has been discussed earlier in this Example with regard to the bait OsS49462.

A BLAST analysis comparing the nucleotide sequence of OsCYCOS2 against TMRI's GENECHIP® Rice Genome Array sequence database identified probeset OS003088.1 _at (e = 0 expectation value) as the closest match. Gene expression analysis indicated that this gene is specifically expressed in panicle.

The bait encoding amino acids 50 to 233 of OsCYCOS2 was found to interact with a fragment of the hypothetical protein 00221-3976 (PN30899). One prey clone encoding amino acids 4 to 228 of PN30899 was retrieved from the input trait library. BLAST analysis indicates that PN30899 is most likely a heat shock (chaperone) protein (*Oryza sativa* protein 417154 HSP82). While heat shock proteins (HSPs) have been ascribed a main role in the plant stress response, some of these proteins are designated as HSPs solely based on sequence homology and their functions in plants have not been demonstrated *in vitro*. Indeed, some HSPs are expressed throughout development. HSPs function as molecular chaperones that promote proper protein folding and can have roles not related to the stress response. HSP70 proteins, for instance, are essential for normal cell function. They are ATP-dependent molecular chaperones that can interact with many different proteins, given their role in protein folding, unfolding, assembly, and disassembly. These topics are discussed in Buchanan et al., 2002. The heat shock protein HSP70 in sea urchin cells has been proposed to have a chaperone role in tubulin folding when localized on centrosomes, and in the assembling and disassembling of the mitotic apparatus when localized on the fibres of spindles and asters (Agueli et al., 2001).

PN30899 also interacts with homeobox protein HOS59, fragment (OsHOS59; see Example IV). Most proteins containing a homeobox domain are known to be sequence-specific DNA-binding transcription factors, some of which have important roles in development. A BLAST analysis comparing the nucleotide sequence of PN30899 against TMRI's GENECHIP® Rice Genome Array sequence database identified probeset OS000221_at (e = 0 expectation value) as the closest match. Gene expression analysis indicated that this gene is not specifically expressed in several different tissue types and is not specifically induced by a broad range of plant stresses, herbicides, or applied hormones.

The bait encoding amino acids 50 to 233 of OsCYCOS2 was also found to interact with the putative Cor-A-like Mg²⁺ transporter protein, PN29970. One prey clone encoding amino acids 1 to 158 of PN29970 was retrieved from the output trait library. The constitutively expressed CorA protein is the primary magnesium cation (Mg²⁺) influx system of Bacteria and Archaea. CorA is ubiquitous in these organisms, forming a distinct family of transport proteins that comprises at least 22 members, as determined by genomic sequence analysis, and with 6 more distant members in the yeasts (Kehres et al., 1998). The similarity of PN29970 to a CorA protein suggests that this prey protein can function as an ion pump in events of the cell cycle regulated by OsCYCOS2.

The bait encoding amino acids 50 to 233 of OsCYCOS2 was also found to interact with hypothetical protein AAK18839 (PN23363) (GENBANK® Accession No. AC082645), a 286-amino acid protein in which no domains, motifs, or signatures have been clearly identified. (One prey clone encoding amino acids 50 to 148 of PN23363 was retrieved from the input trait library.) A BLAST analysis of the Genpept database indicates identity with an *O. sativa* unknown protein (GENBANK® Accession No. AAK18839, 3e⁻⁸¹). A BLAST analysis comparing the nucleotide sequence of PN23363 against TMRI's GENECHIP® Rice Genome Array sequence database identified probeset OS_ORF005240_at (e⁻¹⁷⁵ expectation value) as the closest match. Gene expression analysis indicated that this gene is not specifically expressed in several different tissue types and is not specifically induced by a broad range of plant stresses, herbicides, or applied hormones.

A bait fragment encoding amino acids 170 to 310 of OsCYCOS2 was found to interact with the putative CCAAT displacement protein PN26210. Three prey clones, one encoding amino acids 422 to 646 and two encoding amino acids 364 to 613, of PN26210 were retrieved from the output trait library. PN26210 is a 687-amino acid protein that includes a transmembrane domain (amino acids 621 to 367), as predicted by analysis of the amino acid sequence. The analysis also predicted three coiled coils (amino acids 60 to 345, 381 to 445, and 489 to 643), although with prediction significance below threshold. Coiled coils participate in protein interactions in many types of proteins. A leucine zipper (amino acids 321 to 342) was also identified, which is known in transcription factors to facilitate dimer formation. Moreover, BLAST analysis of the amino acid sequence indicated that PN26210 is the same as Oryza sativa protein 13702813. CCAAT displacement proteins (known as CDP, Cut, or Cux in the literature) belong to a highly conserved family of transcriptional regulators (reviewed by Nepveu, 2001). These proteins have multiple DNA-binding domains that include one Cut homeodomain and one, two or three Cut repeats. The combination of these domains determines their distinct DNA-binding activities, which are elevated during proliferation and reduced during terminal differentiation. The CCAAT motif is found in the promoters of many eukaryotic genes, and CCAAT displacement proteins typically act as transcriptional repressors by directly binding to the promoters of genes that are important during development, but they can also function as transcriptional activators. CDP/Cut was found to be a component of the promoter complex HiNF-D, which is believed to promote the transcriptional induction of histone H4 genes at the G1/S phase transition of the cell cycle and to attenuate H4 gene transcription at later cell cycle stages in humans. The regulatory effect of CDP/Cut on transcription is thought to vary depending on the proteins with which it interacts (Nepveu, *supra*).

The bait encoding amino acids 50 to 233 of OsCYCOS2 was also found to interact with the putative myosin heavy chain protein PN23297. (One prey clone encoding amino acids 980 to 1160 of PN23297 was retrieved from the input trait library.) PN23297 (*Oryza sativa* protein 15451591) is a 1601-amino acid protein that includes an ATP/GTP-binding site motif A (P-loop) (amino acids 267 to 274). Analysis of the protein sequence clearly indicates that this protein is some form of myosin chain, being similar to many myosin-like proteins and myosin heavy chain proteins including myosin-like protein (GENBANK® Accession No. NP_195046, e = 0.0) and myosin heavy chain (GENBANK® Accession No. T05200, e = 0.0) from *A. thaliana*. While myosin is best known for its role in muscle contraction, this protein participates in other cellular events. In plants, for example, myosin heavy chain can participate in cytoplasmic streaming that occurs in tobacco and lily pollen tubes (Yokota et al., 1999a; Yokota et al., 1999b). Cruz et al., 1998 present evidence that myosin assembly is important for mitosis. Specifically, myosin II-deficient yeast cells undergo cell cycle arrest at the G2/M transition, a phase regulated by OsCYCOS2. Furthermore, Xia et al., 1996 demonstrate that *A. thaliana* myosin heavy chain is among the proteins that play a role in cell cycle regulation as well as in cytoskeleton function and in the establishment of cell polarity. The similarity of PN23297 to myosin heavy chain proteins suggests that this prey protein is a cytoskeletal component that can participate in events relating to cell polarity and cytokinesis.

Putative myosin heavy chain PN23297 also interacts with hypothetical protein 003118-3674 similar to *Lycopersicon esculentum* calmodulin (Os003118-3674). Os003118-3674 is a 148-amino acid protein with two EF-hand calcium-binding domains (amino acids 22 to 34 and 93 to 105). In agreement with the observation that Os003118-3674 includes EF-hand calcium-binding domains, BLAST analysis of the Genpept database indicates that this protein shares 72% identity with *A. thaliana* putative calmodulin (GENBANK® Accession No. NP_1764705, e⁻⁵⁷), although the top score in this search is *A. thaliana* putative serine/threonine kinase (GENBANK® Accession No. NP_172695.1, 76% identity, 7e⁻⁶⁰). Therefore, this calmodulin-like protein can possess kinase activity. A BLAST analysis comparing the nucleotide sequence of putative myosin heavy chain PN23297 against TMRI's GENECHIP® Rice Genome Array sequence database identified probeset OS005818_at (e⁻⁶ expectation value) as the closest match. The expectation value is too low for this probeset to be a reliable indicator of the gene expression of PN23297.

A bait fragment encoding amino acids 50 to 233 of OsCYCOS2 was also found to interact with the Chloroplast ATPase I subunit PN23415. One prey clone encoding amino acids 130 to 176 of PN23416 was retrieved from the input trait library. This protein shares the rice ATPase I subunit (GENBANK® Accession No. NP_039379; protein 11466783). ATPases are essential cellular energy converters that transduce the chemical energy of ATP hydrolysis from transmembrane ionic electrochemical potential differences. The plant ATPases are present in chloroplasts, mitochondria and vacuoles. In the chloroplast, ATPases produce ATP that can be used as chemical energy in photosynthetic processes. The prey protein PN23416 is a chloroplast ATPase. A BLAST analysis comparing the nucleotide sequence of PN23416 against TMRI's GENECHIP® Rice Genome Array sequence database identified probeset OS003787_at (e=0 expectation value) as the closest match. Gene expression analysis that this gene is not specifically expressed in several different tissue types and is not specifically induced by a broad range of plant stresses, herbicides, or applied hormones.

A bait fragment encoding amino acids 50 to 233 of OsCYCOS2 was also found to interact with the hypothetical protein BAA85200 (i.e., PN23136), which is similar to the syntaxin related protein AtVam3p. One prey clone encoding amino acids 66 to 191 of PN23136 was retrieved from the output trait library. PN23136 is *Oryza sativa* protein 5922624 (BAA85200) and is similar to AtVam3p. AtVam3p, the product of the AtVAM3 gene, is a syntaxin-related molecule implicated in vacuolar assembly in *A. thaliana*. This protein is expressed in various tissues including roots, leaves, inflorescence stems, flower buds, and young siliques, and AtVAM3 transcripts are abundant in undifferentiated cells in the meristematic region (Sato, et al., 1997). The AtVam3p protein is one of the t-SNARE membrane proteins that mediate protein cargo trafficking inside vesicles between the organelles of the plant endomembrane system. TheAtVAM3p has been localized not only to the vacuolar membrane, but also on the prevacuolar compartment in *Arabidopsis* cells and has been suggested to also have a role in post-Golgi trafficking (Sanderfoot et al., 1999). The similarity of PN23136 to a t-SNARE membrane protein and its association with OsCYCOS2 suggests that this prey protein can be involved in protein trafficking associated with the endomembrane system during the cell cycle.

A bait fragment encoding amino acids 170 to 310 of OsCYCOS2 was also found to interact with a fragment of the hypothetical protein PN20815, which is similar to the *A. thaliana* myosin heavy chain fragment. (One prey clone encoding amino acids 1 to 134 of PN20815 was retrieved from the output trait library.) PN20815 is a 496-amino acid protein. Analysis of the amino acid sequence determined that there is a possible cleavage site between amino acids 61 and 62, although no N-terminal signal peptide appears to be present. Its similarity to *A. thaliana* myosin heavy chain (GENBANK® Accession No. AALL11549, 4e⁻¹¹⁴) suggests that PN20815 might be a cytoskeletal component and can therefore participate in events relating to cell polarity and cytokinesis. Myosin assembly is important for mitosis. Myosin proteins have been discussed herein with regard to the interacting protein PN23297.

A bait fragment encoding amino acids 50 to 233 of OsCYCOS2 was also found to interact with novel protein PN23274. Six prey clones encoding amino acids 79 to 210 of OsPN23274, a region that includes the putative leucine zipper in PN23274, were retrieved from the input trait library. A BLAST analysis against the public databases indicated that the 680-amino acid protein OsPN23274 is similar to *A. thaliana* putative arm repeat containing protein (GENBANK® Accession No. NP_174228, e⁻⁸⁰) and to *Brassica napus* putative arm repeat containing protein 1 (ARC1; GENBANK® Accession No. T08872, e⁻⁵⁶). Analysis of the OsPN23274 protein sequence predicted that it has an armadillo/plakoglobin ARM repeat profile (amino acids 346 to 386; 1.8e⁻⁰⁹). Two other ARM-repeat domains were identified with much lower prediction significance (amino acids 431 to 471, e = 1.2; and amino acids 507 to 548, e = 35). ARM motifs are tandemly repeated sequences of approximately 50 amino acid residues that occur in a wide variety of eukaryotic proteins (Peifer et al., 1994; Groves 1999; Hatzfeld, 1999; Huber et al., 1997). The ARM repeat was first identified in the *Drosophila* protein armadillo that is involved in segment polarity and cell adhesion (Peifer et al., 1990). ARM repeats are found in the mammalian Wnt pathway proteins beta-catenin (an armadillo homolog), plakoglobin, Adenomatous Polyposis Coli (APC) tumor suppressor protein (Huber et al., *supra*), and other proteins. The ARM repeats in Armadillo family members mediate various protein interactions representing steps in signaling events that result in control of cell adhesion, cytoskeletal alterations, and transcription (reviewed by Hatzfeld, 1999). Furthermore, analysis of the protein sequence identified a SecD SecF domain (Bolhuis et al., 1998) between amino acids 316 and 531, although with poor prediction significance (e = 9). This domain is necessary for secretion of some proteins. Also predicted is a leucine zipper (amino acids 65 to 86), a domain known to facilitate protein interactions, particularly in transcription factors. The predicted leucine zipper is of interest when considering that beta-catenin is known to participate in transcriptional regulation. Given its similarity to an ARM repeat protein and its interaction with OsCYCOS2, the prey protein OsPN23274 has a likely role in cell adhesion associated with cytoskeletal alterations occurring at the G2/M transition.

A BLAST analysis comparing the nucleotide sequence of OsPN23274 against TMRI's GENECHIP® Rice Genome Array sequence database identified probeset OS017669_at (4e⁻⁷⁰ expectation value) as the closest match. Gene expression analysis that this gene is not specifically expressed in several different tissue types and is not specifically induced by a broad range of plant stresses, herbicides, or applied hormones.

A bait fragment encoding amino acids 50 to 233 of OsCYCOS2 was also found to interact with a fragment of the novel protein PN23390, a putative kinesin-like calmodulin-binding protein (OsPN23390). Two prey clones, encoding amino acids 595 to 845 and 576 to 738, of OsPN23390 were retrieved from the output trait library. Kinesins are molecular motors, molecules that hydrolyze ATP and use the derived energy to generate motor force. Molecular motors are involved in diverse cellular functions such as vesicle and organelle transport, cytoskeleton dynamics, morphogenesis, polarized growth, cell movements, spindle formation, chromosome movement, nuclear fusion, and signal transduction. Three families of non-plant molecular motors (kinesins, dyneins, and myosins) have been characterized. Kinesins and dyneins use microtubules, while myosins use actin filaments as tracks to transport materials intracellularly. A large number (about 40) of kinesin and myosin motors have been identified in *A. thaliana*, although little is known about plant molecular motors and their roles in cell division, cell expansion, cytoplasmic streaming, cell-to-cell communication, membrane trafficking, and morphogenesis. Calcium, through the calcium binding protein calmodulin, is thought to play a key role in regulating the function of both microtubule- and actin-based motors in plants (molecular motors are reviewed in Reddy, 2001). The kinesin-like calmodulin (CaM) binding protein (KCBP), a minus end-directed microtubule motor protein unique to plants, has been implicated in cell division. During nuclear envelope breakdown and anaphase, activated KCBP promotes the formation of a converging bipolar spindle by sliding and bundling microtubules, while KCBP activity is down-regulated by Ca²⁺ and CaM during metaphase and telophase (Vos et al., 2000). The association of OsPN23390 with OsCYCOS2 suggests that the prey protein is involved in microtubule movement during cell division events mediated by the cyclin. The presence of a calmodulin-binding domain indicates that its activity is regulated by calmodulin.

OsCYCOS2 was also found to interact with the novel protein PN23484. The bait fragment used in the search encodes amino acids 170 to 310 of OsCYCOS2. Four prey clones, one encoding amino acids 77 to 233, two encoding amino acids 64 to 212, and one encoding amino acids 90 to 245, of OsPN23484 were retrieved from the output trait library. As already discussed above, OsPN23484 also interacts with the bait OsS49462. This observation validates the OsCYCOS2- OsPN23484 interaction and suggests that OsPN29942 plays a broad role in regulation by cyclins and thus in the control of cell cycle progression.

The bait fragment encoding amino acids 50 to 233 of OsCYCOS2 was also found to interact with novel protein OsPN26688. One prey clone encoding amino acids 132 to 255 of OsPN26688 was retrieved from the input trait library. OsPN26688 is a novel 251-amino acid protein of unknown function. The lack of information about OsPN26688 makes it difficult to determine its function and the significance of the OsCYCOS2-OsPN26688 interaction. However, the discovery of this interaction links OsPN26688 to control of the cell cycle in rice.

A BLAST analysis comparing the nucleotide sequence of OsPN26688 against TMRI's GENECHIP® Rice Genome Array sequence database identified probeset OS005073.1_at (e = 0 expectation value) as the closest match. Gene expression analysis indica ted that this gene is not specifically expressed in several different tissue types and is not specifically induced by a broad range of plant stresses, herbicides, and applied hormones.

OsCYCOS2 was also found to interact with novel protein PN29882. This protein is similar to myosin proteins. The bait fragment used in the search encodes amino acids 50 to 233 of OsCYCOS2. One prey clone encoding amino acids 107 to 273 of OsPN29882 was retrieved from the output trait library.

OsPN29882 also interacts with MADS box-like protein BAA8188 (OsBAA81881; see Example III). MADS box transcription factors, encoded by members of the large MADS-box family of genes, participate in signal transduction and developmental control in plants, animals, yeast, and fungi. In plants, they are important regulators of genes implicated in flower and fruit development. This links cell cycling controlled by OsCYCOS2 to development controlled by MADS box proteins.

OsPN29882 also was found to interact with a ser/thr kinase/calmodulin that also interacted with PN23297 (see description above). The ser/thr kinase/calmodulin can serve as part of the CDK complex with OsCYCOS2 to activate myosin substrates during mitosis.

A bait fragment encoding amino acids 170 to 310 of OsCYCOS2 (a region that includes the cyclin domain) was found to interact with a fragment of the novel protein PN29942 This protein is discussed earlier in this Example as an interactor for the bait OsS49462. One prey clone encoding amino acids 1 to 159 of OsPN29942 was retrieved from the output trait library. This region spans the putative BTB/POZ domain that was identified in OsPN29942.

A bait fragment encoding amino acids 50-233 of OsCYCOS2 was found to interact with a fragment of the novel protein OsPN29956. OsPN29956 is a novel protein for which only a partial sequence is known. Analysis of the available 374 amino acids indicated that OsPN29956 includes a spectrin repeat (amino acids 167 to 209). In agreement with the observations that OsPN29956 is a nuclear protein with a spectrin repeat, a BLAST analysis revealed that OsPN29956 shares amino acid sequence with nuclear matrix constituent protein 1 from *A. thaliana* (35% identity, GENBANK® Accession No. BAB10684, 4e⁻⁵⁵). Therefore, there is strong evidence that OsPN29956 is a nuclear matrix protein, and the interaction between OsCYCOS2 and OsPN29956 can represent a step in cell cycle control through modulation of nuclear events.

Three prey clones were retrieved from the output trait library. Two of these encode amino acids 96 to 235 and one encodes amino acids 2 to 373 of OsPN29956. All three prey clones include the spectrin repeat that is present in OsPN29956. Spectrin repeats are also found in several proteins involved in cytoskeletal structure, such as actin-binding proteins (Hartwig, 1995). Actin-binding proteins of the superfamily of spectrins are ubiquitous proteins present in all animal and in plant cells. Spectrin-like epitopes have been localized mainly at the plasma membrane in several plant species and different cell types, but also in secretory vesicles, in the nuclei of various plant tissues, and in gravitropically tip-growing rhizoids and protonemata of characean algae, where they were found to be associated with the actin-organized aggregate of endoplasmic reticulum and correlated with active tip growth (Braun, 2001). Studies indicate the presence of spectrin-based membrane skeleton in higher plant cells and demonstrate the ability of these proteins to interact with other components of the membrane skeleton such as actin and calmodulin (Bisikirska et al., 1997). Therefore, OsPN29956 could be a spectrin-like cytoskeleton protein that binds actin or calmodulin during events related to cell division.

A bait fragment encoding amino acids 50-233 of OsCYCOS2 was also found to interact with a fragment of protein PN29958. One prey clone encoding amino acids 3 to 304 of OsPN29958 was retrieved from the output trait library. BLAST analysis suggests that this is a centromere homologue (e-10) and is also homologous to the tobacco NT3 salinity tolerance protein (e-12). The BLAST results suggest a role for PN29958 in the centromere and also in salinity tolerance.

A bait fragment encoding amino acids 50-233 of OsCYCOS2 was also found to interact with protein PN29961, which is similar to *A. thaliana* protein BAB02349. One prey clone encoding amino acids 10 to 215 of OsPN29961 was retrieved from the output trait library.

A bait fragment encoding amino acids 50-233 of OsCYCOS2 was also found to interact with protein OsPN29965. One prey clone encoding amino acids 12 to 124 of OsPN29965 was retrieved from the output trait library. OsPN29965 is similar to *A. thaliana* kinesin (centromere protein). In animal cells, cytokinesis begins shortly after the sister chromatids move to the spindle poles. The centromere is a region of the chromosome to which the spindle fibers attach for the separation of the replicated chromatids in mitosis and meiosis. The kinetochores are the main sites of interaction between spindle microtubules and chromosomes; they are protein-rich structures associated with centromeric DNA and form on each sister chromatid at opposite sides of the paired centromeric region. Various proteins have been localized to animal kinetochores, including dynein and kinesin, but the protein composition of plant kinetocores has yet to be elucidated (Buchanan et al., 2002). The kinetochore-associated kinesin-like protein CENP-E binds to kinetochores during mitosis and has been shown to be essential for chromosome bioriented spindle attachment in mammalian cells (McEwen et al., 2001). Like CENP-E, the Drosophila kinesin-like motor protein CENP-meta similar to the vertebrate CENP-E, is a component of centromeric/kinetochore regions of Drosophila chromosomes and is required for maintenance of metaphase chromosome alignment (Yucel, 2000). The inner centromere protein (INCENP) of animal cells has been implicated in both chromosome segregation and cytokinesis by promoting dissolution of sister chromatid cohesion and the assembly of the central spindle (Kaitna et al., 2000). Kinesin-like calmodulin-binding proteins (KCBP) that are regulated by Ca²⁺/calmodulin have been isolated from dicot (*A. thaliana*) as well as from monocot plants (maize). These motor proteins contain a highly conserved C-terminal region that includes the motor domain and the calmodulin-binding domain, which suggests that the KCBP is ubiquitous and highly conserved in all flowering plants (Abdel-Ghany et al., 2000). Plant KCBP localizes to and is involved in establishing mitotic microtubule (MT) arrays during different stages of cell division, and Ca²⁺/calmodulin regulates the formation of these MT arrays (Kao et al., 2000).

The association of OsPN29965 with OsCYCOS2 suggests that the prey protein is involved in microtubule movement during cell division events mediated by the cyclin. OsPN29965 likely represents a novel centromere- kinetochore-associated protein in plants.

A bait fragment encoding amino acids 50-233 of OsCYCOS2 was also found to interact with a fragment of the novel protein OsPN29966. (One prey clone encoding amino acids 8 to 216 of OsPN29966 was retrieved from the output trait library.) PN29966 is similar to other myosin proteins also described earlier in this Example. It also interacted with the ser/thr kinase calmodulin (see above).

A bait fragment encoding amino acids 50-233 of OsCYCOS2 was also found to interact with a fragment of the protein PN29967. Three prey fragments encoding amino acids 16 to 174 of OsPN29967 were retrieved from the output trait library. OsPN29967 is a novel protein for which only a partial sequence is known. Analysis of the available 176 amino acids predicted a cleavable signal peptide (amino acids 1 to 37) and a leucine zipper (amino acids 123 to 144). The leucine zipper domain supports the notion that this protein participates in protein-protein interactions. A BLAST analysis against the Genpept database determined that OsPN29967 shares 40% amino acid sequence identity with an *A. thaliana* unknown protein (GENBANK® Accession No. CAB10357, 2e⁻¹⁴), for which no information is available other than the nucleotide sequence of the gene encoding this protein.

A bait fragment encoding amino acids 50-233 of OsCYCOS2 was also found to interact with the novel protein OsPN29968, which is sijmilar to the unknown *A. thaliana* protein BAB01990. One prey clone encoding amino acids 12 to 113 of OsPN29968 was retrieved from the output trait library. A BLAST analysis comparing the nucleotide sequence of OsPN29968 against TMRI's GENECHIP® Rice Genome Array sequence database identified probeset OS006631.1_at (e⁻⁹⁵ expectation value) as the closest match. Gene expression analysis indicated that this gene is specifically expressed in seed.

A bait fragment encoding amino acids 50-233 of OsCYCOS2 was also found to interact with a fragment of the novel protein PN29969, which is similar to the *A. thaliana* unknown protein BAB01990. Two prey clones encoding amino acids 16 to 123 of OsPN29969 were retrieved from the output trait library. OsPN29969 is a novel protein for which the complete amino acid sequence is not known. Analysis of the available 123 amino acids identified a tropomyosin signature (amino acids 75 to 91), which suggests that OsPN29969 might be a novel structural protein. Tropomyosins are a family of closely related proteins present in muscle and non-muscle cells. In striated muscle, tropomyosin mediates the interactions between the troponin complex and actin so as to regulate muscle contraction, while the role of this protein in smooth muscle and non-muscle tissues is not clear (Smilie, 1979; McLeod, 1986). Based on the interaction of OsPN29969 with OsCYCOS2, this protein is likely to be involved in mediating interactions between actin and other proteins during the G2/M transition. Thus, the interaction between OsCYCOS2 and OsPN29969 can represent a step in the control of the cell cycle through modulation of the nuclear matrix.

A bait fragment encoding amino acids 50-233 of OsCYCOS2 was also found to interact with the putative Cor-A-like Mg²⁺ transporter protein PN25381. One prey clone encoding amino acids 30 to 218 of OsPN25381 was retrieved from the output trait library. This protein is *Oryza sativa* protein 13357265. The constitutively expressed CorA protein is the primary magnesium cation (Mg²⁺) influx system of *Bacteria* and *Archaea*. CorA is ubiquitous in these organisms, forming a distinct family of transport proteins that comprises at least 22 members, as determined by genomic sequence analysis, and with 6 more distant members in the yeasts (Kehres et al., 1998). The similarity of PN25381 to a CorA protein suggests that this prey protein can function as an ion pump in events of the cell cycle regulated by OsCYCOS2.

A bait fragment encoding amino acids 170 to 310 of OsCYCOS2 was found to interact with novel protein PN30854. One prey clone encoding amino acids 100 to 169 of OsPN30854 was retrieved from the output trait library. OsPN30854 is a 169-amino acid protein. A BLAST analysis against the Genpept database indicated that OsPN30854 shares 67% identity with *A. thaliana* protein AT5g03660/F17C15_80 (GENBANK® Accession No. AAL06894, 9e⁻⁴²). The interaction of PN30854 with OsCYCOS2 suggests that it plays some role in cell cycle regulation. A BLAST analysis comparing the nucleotide sequence of OsPN30854 against TMRI's GENECHIP® Rice Genome Array sequence database identified probeset OS009560_r_at (2e⁻¹⁶ expectation value) as the closest match. The expectation value is too low for this probeset to be a reliable indicator of the gene expression of OsPN30854.

A bait fragment encoding amino acids 50 to 233 of OsCYCOS2 was found to interact with a fragment of novel protein PN30899, which is similar to *A. thaliana* protein NP_199769. This protein is similar to DNAJ, a type of chaperone. Heat shock protein chaperones and potential roles in cell cycling have been discussed herein. One prey clone encoding amino acids 4 to 228 of OsPN30899 was retrieved from the output trait library.

### Summary

M cyclins complexed with protein kinases commit the cell to mitosis at the G2-to-M transition. The synthesis of M cyclins in late G2 prepares the cell for mitosis, and increase of mitotic CDK activity at the G2-to-M transition initiates mitosis and cytokinesis. Mitosis, the stage in the cell cycle at which the duplicated chromosomes are separated into two nuclei, and cytokinesis, the division of one cell into two cells, are accomplished by means of cytoskeletal structures. Mitosis depends on the mitotic spindle, a bipolar arrangement of mostly microtubules, but also actin and associated proteins, that interact with chromosomes and other proteins that participate in chromosome movement. Cytokinesis depends on the phragmoplast, an organelle consisting of actin, myosin, and microtubules which gives rise to a plate in the center of the plant cell between the reforming nuclei and shapes the growing plate into a partition in the form of a new cell wall. Actin filaments, microtubules, and intermediate filaments are filamentous protein polymers comprising the cytoskeleton of eukaryotic cells. Accessory proteins are the motors and joints that link, move and modify the actin and tubulin scaffolding to stabilize the cytoskeleton, create polarities and move chromosomes during cell division, lower polymer concentration by binding (i.e., proteins that bind soluble actin), and link the cytoskeleton to other cellular components such as biosynthetic or signaling enzymes. Many different accessory proteins mediate the function of the cytoskeleton by interacting with the polymers, including the motor proteins myosin, dynein and kinesin, as well as other proteins that cross-link (or bundle) cytoskeletal polymers of the same type. The dynamic behavior and polarity of actin and microtubules, enhanced by energy derived from hydrolysis of nucleoside triphosphates, is responsible for the movements of cytoplasm and organelles during the different phases of the cell cycle.

Mitosis starts with the initiation of chromosome condensation and the disassembly of the nuclear envelope that separates nuclear matrix from cytoplasm. Cells become fully competent for mitosis when the condensed chromosomes are aligned along a plane in the center of the cell, each chromosome comprising two chromatids (daughter strands) attached to each other and connected by microtubules to opposite ends of the cell. Chromosome segregation then initiates with the severing of the link between sister chromatids. The centromere is a region of the chromosome to which the spindle fibers attach for the separation of the replicated chromatids. The kinetochores, the main sites of interaction between spindle microtubules and chromosomes, are protein-rich structures that attach to centromeric DNA and serve as attachment points for the spindle microtubules, which congregate the chromosomes along a plate and subsequently pull apart the sister chromatids to opposite cell poles. Various proteins have been localized to animal kinetocores, including dynein and kinesin, but the protein composition of plant kinetocores has yet to be elucidated. (The plant cell cycle and cytoskeleton structure are discussed in detail in Buchanan et al., 2002). The concentrations of cyclins in the plant cell are thought to be important in mediating CDK activity at the cytoskeleton, chromosomes, spindle, nuclear envelope, and phragmoplast (John et al., 2001).

The interactions identified in this Example for OsCYCOS2 with several cytoskeletal structural proteins in consistent with the role of the cyclin in controlling events related to cell division. Five of these prey proteins-PN23484, PN23297, PN20815, OsPN29882, and OsPN29966--are putative myosin heavy-chain proteins. Previous reports on the role of *Arabidopsis* myosin heavy chain protein in cell cycle control and cytoskeleton function Xia et al., 1996; Cruz et al., 1998) suggest that the putative myosin prey proteins identified here likely function as actin motors during the establishment of cell polarity at mitosis or during cytokinesis. The observation by Cruz et al. that myosin is required in yeast cells for the G2/M transition supports the notion that the interactions of OsCYCOS2 with the myosin heavy chain proteins regulate the cell cycle at this transition point. It is interesting that PN23297, PN29882 and PN29966 also interact with a ser/thr kinase/calmodulin-like protein (Os003118-3674). Kinases regulate the activity of CDK-cyclin complexes, and while no evidence exists that all three proteins--OsCYCOS2, putative myosin heavy chain PN23297 (or other myosins), and the kinase Os003118-3674--interact at the same time, the possibility that Os003118-3674 possesses kinase activity increases the likelihood that this interaction propagates a signaling event.

Other cytoskeletal proteins interacting with OsCYCOS2 include a spectrin-like protein with a presumed actin-binding function nuclear matrix constituent, and its interaction with OsCYCOS2 can represent a step in cell cycle control through modulation of nuclear events (OsPN29956).

Additional interactors with a motor function are the kinesin-like proteins OsPN23390 and OsPN29965. Kinesins in both animals and plants are implicated in the formation of mitotic spindles (Buchanan et al., 2002; Vos et al., 2000). Plant kinesin-like proteins regulated by calmodulin are involved in microtubule array formation during cell division (Kao et al., 2000). Based on these reports and on their interactions with OsCYCOS2, we postulate that the prey proteins OsPN23390 and OsPN29965 function as microtubule motor proteins during the formation of the mitotic spindle. The calmodulin-regulated OsPN23390 can be involved in microtubule array formation, while the similarity of OsPN29965 to a centromere protein suggests that this prey protein is a novel kinesin component of the centromeric/kinetochore regions of rice chromosomes with a putative role in chromosome alignment. The interactions of the cyclin protein with all these cytoskeletal proteins represent a newly characterized mechanism for control of cell division in rice.

OsCYCOS2 also interacts with PN23416, a protein similar to chloroplast ATPase I subunit. The interactions of the cyclin with microtubule- and actin-motor proteins is consistent with the presence of the ATPase prey protein. ATPases hydrolyze ATP to provide energy used by the motor proteins to generate force and directional movement associated with microtubules and actin filaments during mitosis.

Another prey protein, OsPN23274, is similar to *A. thaliana* ARM repeat-containing protein. The interactions of the ARM repeat domain with diverse binding partners reflect diverse functions for ARM repeat-containing proteins. These molecules combine structural roles as adhesion (cell-contact) and cytoskeleton-associated proteins with signaling roles by generating and transducing signals affecting gene expression (Hatzfeld, 1999). The interaction of OsPN23274 with the cyclin suggests that the prey protein is likely involved in cell adhesion associated with the cytoskeletal alterations occurring during the transition from the G2 to M phase, although a role in signaling can be coupled with this function.

Another interactor for OsCYCOS2 is PN26210, a putative CCAAT displacement protein with a role as a transcriptional regulator. During replication, chromosomal DNA remains organized in chromatin, a complex composed mainly of histone proteins. Histone gene expression (RNA) and protein accumulation are strongly stimulated in early S phase to double histone cellular content for the assembly of newly replicated DNA. CCAAT displacement proteins (CDPs) are thought to function as transcriptional activators of histone gene expression at the G1/S phase transition and as attenuators of histone gene transcription at later cell cycle stages in humans (Nepveu, 2001). The dependence of the DNA-binding activity of these proteins on the cell cycle validates the interaction of a putative CCAAT displacement protein with a cyclin. Perhaps this interaction participates in a mechanism in which OsCYCOS2 sequesters PN26210 and prevents it from participating in gene regulation. It is also worth noting that the function of CDPs is regulated by posttranslational modifications (Nepveu, A., *supra*), specifically, the DNA-binding activity, and consequently, the transcriptional activity of CDP is inhibited by phosphorylation of either cut repeats or the cut homeodomain. Given that cyclins interact with cyclin-dependent kinases, it is tempting to speculate that the function of the OsCYCOS2-PN26210 interaction is, alternatively; to allow the posttranslational phosphorylation of PN26210 as part of the process leading to down-regulation of histone transcription during the G2/M phase.

Three membrane transport proteins were also found to interact with OsCYCOS2. PN23136 is similar to a t-SNARE membrane protein, a family of proteins involved in protein cargo trafficking among the organelles of the plant endomembrane system (Sanderfoot et al., 1999). The ER system, which gives rise to the endomembrane system, is a dynamic network whose organization changes during the cell cycle. During mitosis, the ER undergoes a series of rearrangements that result in regulation of spindle activities and cell plate assembly through control of local calcium concentrations (Buchanan et al., 2002). The interaction of PN23136 with OsCYCOS2 points to a role for the prey protein in mediating protein trafficking associated with the dynamic behavior of the ER endomembrane system during mitosis. The other two transporters found to interact with OsCYCOS2 are putative CorA-like magnesium cation transporter that can function as a membrane-spanning pump to regulate turgor pressure or transmit solutes during cytokinesis.

Finally, OsCYCOS2 interacts with the putative heat shock prey proteins PN23169 and PN30899. HSPs act as molecular chaperones and, while these proteins in plants have been mainly linked to the stress response, some are not related to stress and their functions remain to be defined (Buchanan et al., 2002). In the context of all the interactions identified for OsCYCOS2, we speculate that PN30899 and PN23169 act as a molecular glue to hold together interacting proteins. An alternative role for this prey protein can be deduced by functional homology with animal heat shock proteins whose chaperone roles in tubulin folding or mitotic structures assembly/disassembly depends on their localization on centrosomes or spindle fibers, respectively (Agueli et al., 2001). These are functions associated with the phase of the cell cycle controlled by OsCYCOS2.

Proteins that participate in cell cycle regulation can be targets for genetic manipulation or for compounds that modify their level or activity, thereby modulating the plant cell cycle. The identification of genes encoding these proteins in rice can allow the development of methods for controlling plant growth, specifically, cell proliferation and differentiation, to facilitate or retard plant development and promote regeneration. Such methods can involve the application of compounds to crops or the engineering of plants in which the level and/or activity of a protein associated with cell cycle regulation is modulated for a time and under conditions sufficient to modify or control cell division.

One application for the results of this Example would involve modifying plant growth in the presence of one or more environmental conditions including increased or decreased temperatures, salinity, drought or nutrients, or exposure to disease. For example, in case that a limited amount of water is available following winter rain, it can be necessary to restrain plant growth so that water resources are not exhausted before the valuable portion of the crop has developed. Chemical agents that reduce water transpiration have been found to have persisting adverse side effects on subsequent growth. By contrast, modulation of the expression or activity of proteins regulating the cell cycle could result in reduced growth without toxic side effects. Methods have been proposed for controlling plant cell growth by modulating the level and or catalytic activity of proteins having a cyclin-related kinase function to facilitate plant regeneration and development in cereal crops (see U.S. Patent No. 6,087,175).

### Example III

This Example provides a network of proteins interacting with rice MADS box protein MADS45 (OsMADS45), AP1-like MADS box protein (OsRAP1B), MADS box protein MADS6 (OsMADS6), MADS-box protein FDRMADS8 (OsFDRMADS8), MADS box protein MADS3 (OsMADS3), MADS box protein MADS5 (OsMADS5), and MADS box protein MADS15 (OsMADS15). Almost all the proteins of the network, identified by means of yeast two-hybrid assays, are MADS box transcription factors.

MADS box transcription factors, encoded by members of the large MADS-box family of genes, include a conserved sequence-specific DNA-binding/dimerization domain designated as the MADS box. These proteins participate in signal transduction and developmental control in plants, animals, yeast, and fungi. In angiosperms, many MADS box proteins display primarily floral-specific expression and are important regulators of genes implicated in flower and fruit development, most notably in the determination of meristem and floral organ identity. Floral development is conserved among divergent species of flowering plants such as *Arabidopsis thaliana* and maize, which indicates that MADS box genes are part of a highly conserved process that has evolved from an ancient flowering plant (the evolution and function of these genes is reviewed in Ng & Yanofsky, 2001; Theissen et al., 2000; and specifically in rice and maize, in Munster et al., 2001). Plant MADS box genes are organized into several phylogenetically distinct gene groups--AGAMOUS (AG), APETALA3 (AP3)/PISTILLATA (PI) and APETALA1 (AP1)/ AG-LIKE (AGL)9 - each group containing genes that share similar functions in regulating different aspects of flower development, including early acting meristem identity genes controlling the transition from vegetative to reproductive development and floral meristem development, late acting floral organ identity genes, and genes mediating between these two functions (reviews by Purugganan et al., 1995; Theissen et al., 2000). MADS box genes interact with each other and with other genes participating in the genetic control of flower development, with regulatory interactions (activation, repression) between the different genes/groups of genes within this network. In addition to flower development, several MADS box genes are involved in the control of ovule and seed development, vegetative growth, root development, fruit development and dehiscence, embryogenesis, or symbiotic induction (Moon et al., 1999; Riechmann & Meyerowitz, 1997; Theissen et al., 2000). Investigation of MADS box transcription factors and the proteins with which they interact in specific pathways can thus elucidate these biological processes at the molecular level.

The biological relevance of such interactions is further underlined by the fact that these proteins are known to regulate transcription as heterodimers or ternary complexes that include other MADS box proteins (Lim et al., 2000). These interactions have been reported to occur through the K box (Sung et al., 2001; Lim et al., 2000) and to be enhanced by a region immediately downstream of the K domain. Plant MADS box proteins consist of a MADS box domain, an I region, a K domain, and a C-terminal region. The K box is a domain characteristic of plant MADS box proteins that sets them apart from their animal and fungal counterparts, which indicates that plant MADS box factors can have different criteria for interaction (Davies et al., 1996). The K box is commonly found C-terminal to MADS box domains and is thought to serve as a dimerization moiety by forming coiled-coil structures known to facilitate protein interactions. The high potential for protein-protein interactions makes MADS box proteins suitable candidates for two-hybrid assays. However, though many MADS box proteins have been isolated from monocots including maize, sorghum, orchid and rice, few interactions between the MADS box proteins have been investigated (Moon et al., 1999). The protein interactions identified in this Example are aimed at elucidating the molecular mechanisms of plant development regulation by MADS box proteins in rice. The identification and characterization of protein interactions involving MADS box transcription factors in a major crop such as rice has important applications in agriculture. Knowledge of the complex genetic system controlling flower morphogenesis in cereals could be exploited for the development of genetically engineered plants characterized as having a phenotype of modulated development, for example, early or delayed flowering.

A yeast two-hybrid search (as has been described above) led to the identification of a network of rice proteins comprised mainly of MADS box transcription factors that interact as heterodimers, some of which represent interactions not previously described. Some of the interactors are previously identified proteins including the MADS box proteins Os008339, OsFDRMADS6, OsMADS7, OsMADS8, OsMADS13, OsMADS14, OsMADS17, OsMADS18, OsBAA81880, and the same proteins used as baits in these interaction studies, OsMADS45, OsRAP1B, OsMADS6, OsFDRMADS8, OsMADS1, OsMADS3, OsMADS5, and OsMADS15. An additional interactor is the seed storage protein prolamin (OsRP5). The search also led to the identification of six novel rice proteins: the MADS box protein OsPN29949 (interactor for OsMADS6); a putative transcriptional regulator, OsPN23495 (interactor for OsMADS45); a putative hox protein, OsPN22834 (interactor for OsRAP1B); a protein of unknown function, OsPN31165 (interactor for OsMADS3); a 14-3-3-like protein, Os000564-1102 (interactor for OsMADS5); and a putative centromere protein, OsPN29971 (interactor for OsMADS15).

To determine the relationships among the interacting MADS box proteins, an analysis of the amino acid sequence alignment of the regions encoded by the interacting clones was performed. From these alignments, a phylogenetic tree was constructed.

The interacting proteins of the Example are listed in Tables 8-14, followed by detailed information on each protein and a discussion of the significance of the interactions. A diagram of the interactions is shown in Figure 2. The nucleotide and amino acid sequences of the proteins of this Example are provided in SEQ ID NOs: 55-66, 199-202, and 223-256. An analysis of the amino acid sequence alignments is shown in Figures 3A-3D, and phylogenetic tree is shown in Figure 3E.

The ability of the interacting proteins to interact with the bait proteins OsMADS45, OsRAP1B, OsMADS6, OsFDRMADS8, OsMADS1, OsMADS3, OsMADS5, and OsMADS15, and the known or predicted biological functions of the interacting proteins indicate thatthe interacting proteins are involved in transcriptional regulation of genes associated with flower development in rice, except for prolamin, with a presumed role in seed development. Some of the interactions and proteins identified in this Example have not been previously described and represent a novel observation.

### Tables 8-14 Interacting Proteins Identified in the Yeast Two-Hybrid Screen for the Bait Proteins OsMADS45, OsRAP1B, OsMADS6, OsFDRMADS8, OsMADS3, OsMADS5, and OsMADS15

The names of the clones of the proteins used as baits and found as preys are given. Nucleotide/protein sequence accession numbers for the proteins of the Example (or related proteins) are shown in parentheses under the protein name. The bait and prey coordinates (Coord) are the amino acids encoded by the bait fragment(s) used in the search and by the interacting prey clone(s), respectively. The source is the library from which each prey clone was retrieved.

**Table 8**

| Interacting Proteins Identified for OsMADS45 (MADS box protein MADS45) | | | |
|---|---|---|---|
| **Gene Name** | **Protein Name** **(GENBANK® Accession No.)** | **Bait Coord** | **Prey Coord** **(Source)** |
| **BAIT PROTEIN** | | | |
| OsMADS45 PN20231 (1905929- OS000555) (SEQ ID NO: 202) | *O. sativa* MADS box protein MADS45 (U31994, AAB50180) | 1-250* 100-250* 150-250* | |

| **INTERACTORS** | | | |
|---|---|---|---|
| Os008339 PN20847(AJ293816- OS0083339) (SEQ ID NO: 224) | *O. sativa* OS008339 MADS box transcription factor, fragment (AJ293816) | 50-198 | 30-178 (input trait) |
| OsFDRMADS6 PN19766 (SEQ ID NO: 226) | *O. sativa* MADS-box protein FDRMADS6 (AF139664, AAF66997) | 50-198 | 3x 115-246 93-244 (output trait) |
| OsFDRMADS8 PN20698 (SEQ ID NO: 228) | *O. sativa* MADS-box protein FDRMADS8 (AF141965, AAD38369) | 50-198 | 2x 104-233 63-186 (output trait) |
| OsMADS1 PN19788 (11493806- OS015136) (SEQ ID NO: 230) | *O. sativa* MADS box protein MADS1 (AF204063, AAG35652) | 50-198 | 3x 82-241 2x 71-257 (output trait) |
| OsMADS3 PN20700 (SEQ ID NO: 232) | *O. sativa* MADS box protein MADS3 (L37528, AAA99964) | 50-198 | 48-177 (output trait) |
| OsMADS5 PN20770 (SEQ ID NO: 234) | *O. sativa* MADS box protein MADS5 (U78890, AAB71434) | 50-198 | 113-225 (output trait) |
| OsMADS6 PN20233 (SEQ ID NO: 236) | *O. sativa* MADS box protein MADS6 (U78782, AAB64250) | 50-198 | 70-250 (output trait) |
| OsMADS13 PN20668 (SEQ ID NO: 238) | *O. sativa* MADS box protein MADS13 (AF151693, AAF13594) | 50-198 | 2x 75-263 (output trait) |
| OsMADS14 PN20910 (SEQ ID NO: 200) | *O. sativa* MADS box protein MADS14 (AF058697, AAF19047) | 50-198 | 124-223 82-197 (output trait) |
| OsMADS15 PN20842 (SEQ ID NO: 240) | *O. sativa* MADS box protein MADS15 (AF058698, AAF19048) | 50-198 | 2x 92-237 (output trait) |
| OsMADS18 PN20912 (SEQ ID NO: 242) | *O. sativa* MADS box protein MADS18 (AF091458, AAF04972) | 50-198 | 57-224 82-154 (output trait) |
| OsPN23495 (SEQ ID NO: 56) | Novel protein PN23495 | 50-198 | 39-165 12-198 (input trait) |
| OsRAP1B PN20232(7592641- OS000556) (SEQ ID NO: 244) | *O. sativa* AP1-like MADS box protein RAP1B (AB041020, BAA94342) | 50-198 | 1-158 (output trait) |

| | | | |
|---|---|---|---|
| * Self-activating clone, i.e., it activates the reporter genes in the two-hybrid system in the absence of a prey protein, and thus it was not used in the search. | | | |

**Table 9**

| Interacting Proteins Identified for OsRAP1B (*O. sativa* AP1-like MADS box protein RAP1B) | | | |
|---|---|---|---|
| **Gene Name** | **Protein Name** **(GENBANK® Accession No.)** | **Bait Coord** | **Prey Coord** **(Source)** |
| **BAIT PROTEIN** | | | |
| OsRAP1B PN20232 (SEQ ID NO: 244) | *O. sativa* AP1-like MADS box protein RAP1B(AB041020, BAA94342) | | |

| **INTERACTORS** | | | |
|---|---|---|---|
| Os008339 PN20847 (SEQ ID NO: 224) | *O. sativa* OS008339 MADS box transcription factor, fragment (AJ293816) | 1-150 | 3x 32-162 (input trait) |
| OsBAA81880 PN20837 (52957- OS011794) (SEQ ID NO: 246) | *O. sativa* MADS box-like protein (AB003322, BAA81880) | 125-235 | 2-168 24-203 (output trait) |
| OsFDRMADS6 PN19766 (SEQ ID NO: 226) | *O. sativa* MADS-box protein FDRMADS6 (AF139664, AAF66997) | 1-247 | 1-186 (output trait) |
| | | 100-247 | 100-246 (output trait) |
| OsFDRMADS8 PN20698 (SEQ ID NO: 228) | *O. sativa* MADS-box protein FDRMADS8 (AF141965, AAD38369) | 100-247 | 4x 69-233 (input trait) 94-230 (output trait) |
| | | 1-247 | 53-233 (output trait) |
| OsMADS1 PN19788 (SEQ ID NO: 230) | *O. sativa* MADS box protein MADS1 (AF204063, AAG35652) | 1-247 | 4x 100-231 (input trait) 95-257 (output trait) |
| | | 100-247 | 2x 95-257 (input trait) |
| | | 65-200 | 4x 74-172 (input trait) |
| | | 125-235 | 73-239 (output trait) |
| OsMADS5 PN20770 (SEQ ID NO: 234) | *O. sativa* MADS box protein MADS5 (U78890, AAB71434) | 30-180 | 106-225 (input trait) 121-225 (output trait) |
| | | 1-247 | 2x 109-225 (output trait) |
| | | 125-235 | 2x 108-225 (output trait) |
| OsMADS6 PN20233 (SEQ ID NO: 236) | *O. sativa* MADS box protein MADS6 (U78782, AAB64250) | 1-247 | 116-250 (output trait) |
| OsMADS7 PN21116 (SEQ ID NO: 248) | *O. sativa* MADS box protein MADS7 (U78891, AAC49816) | 1-247 | 5x 1-250 (output trait) |
| OsMADS8 PN20778 (SEQ ID NO: 250) | *O. sativa* MADS box protein MADS8 (U78892, AAC49817) | 1-247 | 6x 107-248 (output trait) 75-248 (input trait) |
| | | 30-180 | 109-248 74-183 (output trait) |
| | | 100-247 | 127-248 (output trait) |
| | | 125-235 | 2x 79-248 (output trait) |
| OsMADS17 PN20914 (SEQ ID NO: 252) | *O. sativa* MADS box transcription factor MADS17 (AF109153, AAF21900) | 1-247 | 106-249 (input trait) |
| OsMADS45 PN20231 (SEQ ID NO: 202) | *O. sativa O.* MADS box protein MADS45 (U31994, AAB50180) | 1-247 | 96-249 (input trait) 3x 75-249 (output trait) |
| | | 30-180 | 61-248 (output trait) |
| | | 125-235 | 4x 98-249 3x 69-249 (output trait) |
| OsPN22834 (SEQ ID NO: 58) | Novel protein PN22834, similar to Oshox6, fragment | 1-247 | 2x 112-278 (input trait) |

**Table 10**

| Interacting Proteins Identified for OsMADS6 (*O. sativa* MADS box protein MADS6) | | | |
|---|---|---|---|
| **Gene Name** | **Protein Name** **(GENBANK® Accession No.)** | **Bait Coord** | **Prey Coord** **(Source)** |
| **BAIT PROTEIN** | | | |
| OsMADS6 PN20233 (SEQ ID NO: 236) | *O. sativa* MADS box protein MADS6 (U78782, AAB64250) | 1-251* 100-251 * | |

| **INTERACTORS** | | | |
|---|---|---|---|
| Os008339 PN20847 (SEQ ID NO: 224) | *O. sativa* OS008339 MADS box transcription factor, fragment (AJ293816) | 50-200 | 108-226 (output trait) |
| OsBAA81880 PN20837 (SEQ ID NO: 246) | *O. sativa* MADS box-like protein (AB003322, BAA81880) | 50-200 | 2x 120-228 (output trait) |
| OsFDRMADS8 PN20698 (SEQ ID NO: 228) | *O. sativa* MADS-box protein FDRMADS8 (AF141965, AAD38369) | 50-200 | 91-233 (output trait) |
| OsMADS1 PN19788 (SEQ ID NO: 230) | *O. sativa* MADS box protein MADS1 (AF204063, AAG35652) | 50-200 | 3x 70-257 (output trait) |
| OsMADS5 PN20770 (SEQ ID NO: 234) | *O. sativa* MADS box protein MADS5 (U78890, AAB71434) | 50-200 | 61-171 (output trait) |
| OsMADS7 PN21116 (SEQ ID NO: 248) | *O. sativa* MADS box protein MADS7 (U78891, AAC49816) | 50-200 | 95-259 (output trait) |
| OsMADS8 PN20778 (SEQ ID NO: 250) | *O. sativa* MADS box protein MADS8 (U78892, AAC49817) | 50-200 | 2x 79-248 75-238 (output trait) |
| OsMADS15 PN20842 (SEQ ID NO: 240) | *O. sativa* OSMADS15 (AF058698, AAF19048) | 50-200 | 73-183 1-176 (output trait) |
| OsMADS18 PN20912 (SEQ ID NO: 242) | *O. sativa* MADS box transcription factor MADS18 (AF091458, AAF04972) | 50-200 | 64-249 (output trait) |
| OsMADS45 PN20231 (SEQ ID NO: 202) | *O. sativa O. sativa* MADS box protein MADS45 (U31994, AAB50180) | 50-200 | 83-234 (output trait) |
| OsPN29949 (SEQ ID NO: 60) | Novel protein PN29949 putative MADS protein | 50-200 | 118-241 109-193 (output trait) |
| OsRAP1B PN20232 (SEQ ID NO: 244) | *O. sativa* AP1-like MADS box protein RAP1B (AB041020, BAA94342) | 50-200 | 1-188 (input trait) 1-179 (output trait) |
| OsRP5 PN19877 (SEQ ID NO: 254) | *O. sativa* Prolamin (AF156714, AAF73991) | 50-200 | 13-140 (output trait) |

| | | | |
|---|---|---|---|
| * Self-activating clone, i.e., it activates the reporter genes in the two-hybrid system in the absence of a prey protein, and thus it was not used in the search. NOTE: Interactions of OsMADS6 with OsMADS14 and with OsMADS17, identified through a yeast two-hybrid system, are reported in the literature (Moon et al., 1999). | | | |

**Table 11**

| Interacting Proteins Identified for OsFDRMADS8 (*O. sativa* MADS box protein FDRMADS8) | | | |
|---|---|---|---|
| **Gene Name** | **Protein Name** **(GENBANK® Accession No.)** | **Bait Coord** | **Prey Coord** **(Source)** |
| **BAIT PROTEIN** | | | |
| OsFDRMADS8 PN20698 (SEQ ID NO: 228) | *O. sativa* MADS-box protein FDRMADS8 (AF141965, AAD38369) | | |

| **INTERACTORS** | | | |
|---|---|---|---|
| OsMADS45 PN20231 (SEQ ID NO: 202) | *O. sativa* MADS box protein MADS45 (U31994, AAB50180) | 60-160 | 3x 56-249 (output trait) |

**Table 12**

| Interacting Proteins Identified for OsMADS3 (*O. sativa* MADS box protein MADS3) | | | |
|---|---|---|---|
| **Gene Name** | **Protein Name** **(GENBANK® Accession No.)** | **Bait Coord** | **Prey Coord** **(Source)** |
| **BAIT PROTEIN** | | | |
| OsMADS3 PN20700 (SEQ ID NO: 232) | *O. sativa* MADS box protein MADS3 (L37528, AAA99964) | 120-210* 120-237* | |

| **INTERACTORS** | | | |
|---|---|---|---|
| OsMADS8 PN20778 (SEQ ID NO: 250) | *O. sativa* MADS box protein MADS8 (U78892, AAC49817) | 70-170 | 61-248 (input trait) 6-159 68-245 (output trait) |
| OsMADS45 PN20231 (SEQ ID NO: 202) | *O. sativa O. sativa* MADS box protein MADS45 (U31994, AAB50180) | 70-170 | 48-249 (input trait) 4x 2-214 57-249 (output trait) |
| OsPN31165 (SEQ ID NO: 62) | Novel protein PN31165 | 70-170 | 58-252 (input trait) |

| | | | |
|---|---|---|---|
| * Self-activating clone, i.e., it activates the reporter genes in the two-hybrid system in the absence of a prey protein, and thus it was not used in the search. | | | |

**Table 13**

| Interacting Proteins Identified for OsMADS5 (*O*. *sativa* MADS box protein MADS5) | | | |
|---|---|---|---|
| **Gene Name** | **Protein Name** **(GENBANK® Accession No.)** | **Bait Coord** | **Prey Coord** **(Source)** |
| **BAIT PROTEIN** | | | |
| OsMADS5 PN20770 (SEQ ID NO: 234) | *O. sativa* MADS box protein MADS5 (U78890, AAB71434) | 100-226 | |

| **INTERACTORS** | | | |
|---|---|---|---|
| OsFDRMADS6 PN19766 (SEQ ID NO: 226) | *O. sativa* MADS-box protein FDRMADS6 (AF139664, AAF66997) | 50-160 | 74-246 (output trait) |
| OsMADS13 PN20668 (SEQ ID NO: 238) | *O. sativa* MADS box protein MADS13 (AF151693, AAF13594) | 50-160 | 2x 69-230 (output trait) |
| OsMADS17 PN20914 (SEQ ID NO: 252) | *O. sativa* MADS box transcription factor MADS17 (AF109153, AAF21900) | 50-160 | 51-248 (output trait) |
| Os000564-1102 PN20072 (SEQ ID NO: 64) | Hypothetical protein 000564-1102 | 50-160 | 72-172 (output trait) |
| OsBAB56078 PN28517 (SEQ ID NO: 256) | *O. sativa* Hypothetical protein BAB56078 (AP003106, BAB56078) | 50-160 | 51-155 (output trait) |

**Table 14**

| Interacting Proteins Identified for OsMADS15 (*O. sativa* MADS box protein MADS15) | | | |
|---|---|---|---|
| **Gene Name** | **Protein Name** **(GENBANK® Accession No.)** | **Bait Coord** | **Prey Coord** **(Source)** |
| **BAIT PROTEIN** | | | |
| OsMADS15 PN20842 (SEQ ID NO: 240) | *O. sativa* MADS box protein MADS15 (AF058698, AAF19048) | | |

| **INTERACTORS** | | | |
|---|---|---|---|
| OsMADS1 PN19788 (11493806- OS015136 (SEQ ID NO: 230) | *O. sativa* MADS box protein MADS1 (AF204063, AAG35652) | 100-235 | 95-254 4x 74-172 (input trait) |
| OsMADS45 PN20231 (SEQ ID NO: 202) | *O. sativa O. sativa* MADS box protein MADS45 (U31994, AAB50180) | 100-235 | 120-249 (output trait) |
| OsPN29971 (SEQ ID NO: 66) | Novel protein PN29971, fragment, similar to *A. thaliana* centromere protein NP_191066 | 100-235 | 2x 1-108 (input trait) |

### O. sativa MADS box protein MADS45 (OsMADS45) as bait

OsMADS45 (GENBANK® Accession No. AAB50180; Greco et al., 1997) is a 249-amino acid protein that includes a MADS box domain (amino acids 1 to 61), as predicted by amino acid sequence analysis (3.05e⁻⁴¹ prediction value). The analysis also predicted the existence of two coiled coils (amino acids 83 to 117 and amino acids 152 to 176). These coiled coils are likely part of a K-box predicted between amino acids 73 and 176 (3.7e⁻⁴⁵). The bait fragment used in this search encodes amino acids 50 to 198, a sequence that includes both predicted coiled coils and the K-box of OsMADS45.OsMADS45 is highly homologous to the AGL2 and AGL4 MADS box genes, which are thought to play an important role in the development of all floral organs by acting as intermediates between the meristem identity and organ identity genes (Greco et al., 1997; Savidge et al., 1995). In agreement with the expression pattern of AGL2 and AGL4, Northern blot and *in situ* hybridization experiments show that the rice OsMADS45 RNA is highly expressed in the floral meristem, in all the primordia, in mature floral organs, and in developing kernels (Greco et al., 1997), consistent with involvement in fruit development. However, temporal and spatial gene expression patterns only suggest that OsMADS45 and *Arabidopsis* AGL2 and AGL4 play similar roles in flower development (Greco et al., 1997).

A BLAST analysis comparing the nucleotide sequence of OsMADS45 against TMRI's GENECHIP® Rice Genome Array sequence database identified probeset OS014912_f_at (6e⁻⁶⁴ expectation value) and probeset OS000555_f_at (6e⁻⁶⁰) as the closest matches. Analysis of gene expression indicated that these genes are expressed early in seed development.

Proteins that were found to interact with OsMADS45 included Os008339 (GENBANK® Accession No. AJ293816), a 233-amino acid protein that includes a MADS box domain (amino acids 10 to 67, 8.4e⁻²⁹), which suggests that Os008339 is a member of the MADS box protein family. Analysis of the amino acid sequence also identified a K-box (amino acids 80 to 181) and a basic leucine zipper domain (bZIP; amino acids 156 to 186). The BZIP domain is often found in transcription factors and includes a basic DNA-binding region and a leucine zipper, which is associated with dimerization in many gene regulatory proteins (Landschulz et al., 1988; Busch et al., 1990; O'Shea et al., 1989). Thus this protein likely functions as do other MADS box family members, and its association with OsMADS45 represents a newly identified heterodimer presumably involved in transcriptional regulation of genes associated with development in rice. The prey clone of Os008339 retrieved encodes a region that spans most of the K-box in Os008339.The retrieval of this clone is consistent with OsMADS45 and Os008339 interacting through their respective K-boxes, as this domain is thought to include coiled coils used for protein interactions. Os008339 was also found to interact with the bait proteins OsRAP1B and OsMADS6 (see Table 9 and Table 10, respectively).

A BLAST analysis comparing the nucleotide sequence of Os008339 against TMRI's GENECHIP® Rice Genome Array sequence database identified probeset OS011977_i_at (7e⁻⁹¹ expectation value) as the closest match. Gene expression analysis indicated that this gene is not specifically induced by a broad range of plant stresses, herbicides, or applied hormones

OsMADS45 was also found to interact with *O. sativa* MADS box protein OsFDRMADS6 (GENBANK® Accession No. AF139664), a 246-amino acid protein that includes a MADS box domain (amino acids 1 to 61, 6.79e⁻³⁹), a coiled coil located C-terminal to the MADS box domain (amino acids 116 to 182). This predicted coiled coil is likely part of a K-box predicted between amino acids 73 and 174 (8.9e⁻⁴⁷), and its validity is supported by the fact that MADS box proteins bind DNA and modulate transcription as heterodimers. Previously published studies indicated that the FDRMADS6 transcript was present in flower, but not in root or shoot, and that transcripts were found in the spikelet apical meristem at the early stage of flower development and again at the late stage when flower organ primordia began differentiating (Jia et al., 2000). The OsFDRMADS6-OsMADS45 interaction has not been previously reported. OsFDRMADS6 was also found to interact with the bait proteins OsRAP1B (see Table 9) and OsMADS5 (see Table 13).

A BLAST analysis comparing the nucleotide sequence of OsFDRMADS6 against TMRI's GENECHIP® Rice Genome Array sequence database identified probeset OS003005.1_i_at (2e⁻⁸² expectation value) as the closest match. Gene expression analysis indicated this gene is not specifically induced by a broad range of plant stresses, herbicides, or applied hormones.

OsMADS45 also interacted with OsFDRMADS8 (GENBANK® Accession No. AF141965), a 233-amino acid protein with a MADS box domain between amino acids 1 and 60 (9.6e⁻³⁹) and a coiled coil signature (amino acids 122 to 178, prediction significance below threshold), as determined by amino acid sequence analysis. This putative coiled coil region overlaps with a K-box domain (amino acids 73 to 173, 1.3e⁻¹⁰). While no information is available in the literature about OsFDRMADS8, the presence of the MADS box and the K-box strongly suggests that it is a transcription factor of the MADS box family. The association of this protein with OsMADS45 suggests a role for OsFDRMADS8 in transcriptional regulation of genes involved in plant development. The OsFDRMADS8-OsMADS45 interaction has not been previously reported. OsFDRMADS8 was also found to interact with the bait proteins OsRAP1B and OsMADS6 (see Table 9 and Table 10).

OsFDRMADS8 was also constructed as a bait. Its interactions are shown in Table 11 and described later in this Example. A BLAST analysis comparing the nucleotide sequence of OsFDRMADS8 against TMRI's GENECHIP® Rice Genome Array sequence database identified probeset OS015116 _at (2e⁻⁸² expectation value) as the closest match. Analysis of gene expression indicated that this gene is not specifically induced by a broad range of plant stresses, herbicides, or applied hormones.

The bait encoding amino acids 50 to 198 OsMADS45 was also found to interact with OsMADS1 (GENBANK® Accession No. AF204063), a 257-amino acid protein that is a member of the MADS box gene family. OsMADS1 includes a MADS domain (amino acids 1 to 60) and a coiled coil (amino acids 119 to 179), as determined by amino acid sequence analysis. OsMADS1 is a member of the AGL2 subfamily in the AP1/AGL9 family of MADS box genes (Moon et al., 1999). Ectopic expression of the OsMADS1 gene in homologous and heterologous plants results in early flowering, thereby suggesting a role for OsMADS1 in flower induction (Chung et al., 1994). OsMADS1 is expressed at the early stage through the later stages of flower development, with transcripts present in paleas/lemmas and carpels (Moon et al., 1999). The OsMADS1 homolog in the grass *Lolium temulentum* is expressed in the vegetative shoot apical meristem, and its expression increases strongly within 30 hours of long day floral induction, as determined by *in situ* hybridization (Gocal et al., 2001). The OsMADS1-OsMADS45 interaction has not been previously reported.

OsMADS1 was also found to interact with the bait proteins OsRAP1B (see Table 9), OsMADS6 (see Table 10), and OsMADS15 (see Table 14). A BLAST analysis comparing the nucleotide sequence of OsMADS1 against TMRI's GENECHIP® Rice Genome Array sequence database identified probeset OS000262_f_at and OS015136_f_at (5e⁻⁴⁶ and 2e⁻³⁶ expectation values, respectively) as the closest matches. Gene expression analysis indicated that this gene is not specifically induced by a broad range of plant stresses, herbicides, or applied hormones.

OsMADS45 was also found to interact with the MADS box protein OsMADS3. The 236-amino acid OsMADS3 protein (GENBANK® Accession No. L37528), includes a MADS box domain (amino acids 1 to 61) and, based on sequence homology, is structurally and functionally related to the AG gene family, as reported by Kang et al., 1995. RNA blot analysis and *in situ* localization studies showed that the OsMADS3 RNA transcript is preferentially expressed in reproductive organs, especially in stamen and carpel. Transgenic plants engineered to ectopically express the OsMADS3 gene exhibit altered morphology and coloration of the perianth organs, suggesting an important role for OsMADS3 in flower development. The OsMADS3-OsMADS45 interaction has not been previously reported.

OsMADS3 was also constructed as a bait protein. Its interactions are shown in Table 12 and described later in this Example. A BLAST analysis comparing the nucleotide sequence of OsMADS3 against TMRI's GENECHIP® Rice Genome Array sequence database identified probeset OS000554_f_at (e⁻⁴³ expectation value) as the closest match. Gene expression analysis indicated that this gene is not specifically induced by a broad range of plant stresses, herbicides, or applied hormones.

OsMADS45 was also found to interact with the rice MADS box protein OsMADS5. OsMADS5 (GENBANK® Accession No. U78890) is a 225-amino acid protein that includes a MADS box domain (amino acids 1 to 61, 3.17e⁻³⁹), as predicted by amino acid sequence analysis. Thus, OsMADS5 is a member of the MADS box protein family. Amino acid sequence analysis also predicted a coiled coil located C-terminal to the MADS box domain (amino acids 142 to 182), although with prediction significance below threshold. This coiled coil is likely part of a K-box predicted between amino acids 73 and 175 (3.4e⁻⁴⁰). OsMADS5 belongs to the AGL2 subfamily in the AP1/AGL9 family of MADS box genes, whose members are for the most part expressed at the early flowering stage (Moon et al., 1999). OsMADS5 is expressed throughout flower development, with higher expression in the early stages than the later stages and transcripts present in anthers and weakly in carpels, as reported by Kang et al., 1997. Ttransgenic plants ectopically expressing OsMADS5 exhibit the phenotype of weak dwarfism and early flowering, suggesting that this protein is involved in controlling flowering time. The OsMADS5- OsMADS45 interaction has not been previously reported.

OsMADS5 was also found to interact with the bait proteins OsRAP1B and OsMADS6 (see Table 9 and Table 10, respectively). OsMADS5 was also constructed as a bait protein. Its interactions are shown in Table 13 and described later in this Example.

A BLAST analysis comparing the nucleotide sequence of OsMADS5 against TMRI's GENECHIP® Rice Genome Array sequence database identified probeset OS011934 _at (e⁻⁵⁸ expectation value) as the closest match. Analysis of temporal and spatial patterns of gene expression indicated that this gene is specifically expressed in panicle, in agreement with expression data previously reported for the OsMADS5 gene (Kang et al., 1997). Further, gene expression experiments indicated that the OsMADS5 gene is not specifically induced by a broad range of plant stresses, herbicides, or applied hormones.

OsMADS45 was also found to interact with rice MADS box protein OsMADS6. OsMADS6 (GENBANK® Accession No. U78782) is a 250-amino acid protein that includes a MADS box domain (amino acids 1 to 59, 3.3e⁻⁴²), as determined by amino acid sequence analysis. Thus, OsMADS6 is a member of the MADS box protein family. The analysis also predicted a K-box (amino acids 72 to 172, 3.4e⁻⁴⁷). In support of the existence of a K-box, the analysis also predicted a coiled coil (amino acids 118 to 172). Moon et al., 1999 report that OsMADS6, like OsMADS14, belongs to the AP1/AGL9 family of genes which control the specification of meristem and organ identity in developing flowers. Both OsMADS6 and OsMADS14 are expressed from the early through the later stages of flower development, with OsMADS6 transcripts detectable in lodicules and also weakly in sterile lemmas and carpels of mature flowers (Moon et al., 1999). Thus, these genes can regulate a very early stage of flower development, based on the observation that transgenic plants ectopically expressing OsMADS6 and OsMADS14 exhibited extreme early flowering and dwarfism. The OsMADS6- OsMADS45 interaction has not been previously reported.

OsMADS6 was also found to interact with the bait protein OsRAP1B (see Table 9). OsMADS6 was also used as a bait. Its interactors are shown in Table 10 and described later in in this Example. A BLAST analysis comparing the nucleotide sequence of OsMADS6 against TMRI's GENECHIP® Rice Genome Array sequence database identified probeset OS000571_f _at (e⁻⁷ expectation value) as the closest match. The expectation value is too low for this probeset to be a reliable indicator of the gene expression of OsMADS6.

OsMADS45 was also found to interact with rice MADS box protein OsMADS 13). OsMADS13 (GENBANK® Accession No. AF151693) is a 250-amino acid protein that includes a MADS box domain (amino acids 1 to 61). Lopez-Dee et al., 1999 determined that this gene is the ortholog of ZAG2, a maize MADS-box gene expressed mainly in the ovule, and of the ZAG2 paralogous gene ZMM1. The OsMADS13 gene is highly expressed in developing ovules and can play a role in rice ovule and seed development (Lopez-Dee et al., 1999). Ovules are contained in the carpel, structures in the flowers of seed plants such as rice, and they develop into seeds after fertilization. The OsMADS13-OsMADS45 interaction has not been previously reported.

OSMADS13 was also found to interact with the bait protein OSMADS5 (see Table 13). A BLAST analysis comparing the nucleotide sequence of OsMADS13 against TMRI's GENECHIP® Rice Genome Array sequence database identified probeset OS000554_f_at (e⁻⁷⁷ expectation value) as the closest match. Gene expression analysis indicated that this gene is not specifically induced by a broad range of plant stresses, herbicides, or applied hormones.

OsMADS45 was also found to interact with rice MADS box protein OsMADS14. OsMADS14 (GENBANK® Accession No. AF058697) is a 246-amino acid protein that includes a MADS box domain (amino acids 1 to 61). OsMADS14 is homologous to the maize AP1 homolog ZAP1 and os a member of the SQUAMOSA-like (SQUA) subfamily in the AP1/AGL9 family of MADS box genes, which control the specification of meristem and organ identity in developing flowers (Moon et al., 1999). OsMADS14, as well as OsMADS6, is expressed from the early through the later stages of flower development, with OsMADS14 transcripts detectable in sterile lemmas, paleas/lemmas, stamens, and carpels of mature flowers. Thus, these genes can regulate a very early stage of flower development, based on the observation that transgenic plants ectopically expressing OsMADS14 and OsMADS6 exhibit extreme early flowering and dwarfism (Moon et al., 1999). The OsMADS14-OsMADS45 interaction has not been previously reported.

OsMADS14 was also found to interact with Os018989-4003 (hypothetical protein 018989-4003 similar to *Triticum sp.* DP Protein). Using a yeast two-hybrid system, OsMADS14 has also been reported to interact with with OsMADS1 (Lim et al., 1999) and with OsMADS6 (Moon et al., 1999). While the K domain is essential for the interaction between OsMADS14 and OsMADS1, a region preceded by the K domain augments this interaction (Lim et al., 1999). Likewise, a 14-amino acid region located immediately downstream of the K domain enhances the OsMADS14-OsMADS6 interaction, and the two leucine residues within this region play an important role in that enhancement (Moon et al., 1999). A BLAST analysis comparing the nucleotide sequence of OsMADS13 against TMRI's GENECHIP® Rice Genome Array sequence database identified probeset OS003005.1_i_at (e⁻⁸² expectation value) as the closest match. Gene expression analysis indicated that this gene is not specifically induced by a broad range of plant stresses, herbicides, or applied hormones.

OsMADS45 was also found to interact with rice MADS box protein OsMADS 15. OsMADS15 (GENBANK® Accession No. U78782) is a 267-amino acid protein with a MADS box domain between amino acids 1 and 60, as determined by amino acid sequence analysis (5.39e⁻⁴² prediction value). The analysis also predicted a coiled coil signature (amino acids 145 to 184). This putative coiled coil region overlaps with a predicted K-box domain (amino acids 73 to 174, 1.20e⁻⁴⁰). OsMADS15 is homologous to the maize AP1 homolog ZAP1 and is classified as a member of the SQUAMOSA-like (SQUA) subfamily in the AP1/AGL9 family of MADS box genes, which control the specification of meristem and organ identity in developing flowers (Moon et al., 1999). The OsMADS15- OsMADS45 interaction represents a heterodimer that has not been previously reported.

OsMADS15 was also found to interact with the bait protein OsMADS6 (see Table 10). OsMADS15 was also constructed as a bait protein. Its interactions are shown in Table 14 and described later in this Example. A BLAST analysis comparing the nucleotide sequence of OsMADS15 against TMRI's GENECHIP® Rice Genome Array sequence database identified probeset OS015053_f_at (e⁻⁷⁷ expectation value) as the closest match. Gene expression analysis indicated that this gene is not specifically induced by a broad range of plant stresses, herbicides, or applied hormones.

OsMADS45 was also found to interact with rice MADS box protein OsMADS18. OsMADS18 (GENBANK® Accession No. AF091458) is a 249-amino acid protein with a MADS box domain between amino acids 1 and 60 (1.67e⁻³⁸), as determined by amino acid sequence analysis. This amino acid sequence analysis also predicted a coiled coil signature (amino acids 141 to 191). This putative coiled coil region overlaps with a K-box domain (amino acids 73 to 173, 3.80e⁻³²). OsMADS18 is highly homologous to the maize AP1 homolog ZAP1 and belongs to the SQUA subfamily in the AP1/AGL9 family of MADS box genes, which control the specification of meristem and organ identity in developing flowers (Moon et al., 1999). The OsMADS18-OsMADS45 interaction represents a heterodimer that has not been previously reported.

OsMADS18 was also found to interact with OsMADS6 (see Table 10). A BLAST analysis comparing the nucleotide sequence of OsMADS18 against TMRI's GENECHIP® Rice Genome Array sequence database identified probeset OS015196_i _at (e⁻⁵⁸ expectation value) as the closest match. Gene expression analysis indicated that this gene is not specifically induced by a broad range of plant stresses, herbicides, or applied hormones.

OsMADS45 was also found to interact with the novel rice protein OsPN23495. OsPN23495 is a novel 335-amino acid protein. A BLAST analysis indicated that OsPN23495 is similar to expressed protein from *A. thaliana* (GENBANK® Accession No. NM_129661, 42.1% identity, 2e⁻⁰⁵⁴), for which no information is available in the public domain. However, OsPN23495 was also found to interact with two rice hypothetical proteins (Os006111-3329 and Os020134-3170) which are similar to the zinc/DNA-binding ascorbate oxidase promoter binding protein (AOBP) from *Curcurbita maxima*, and which include a Dof domain zinc finger DNA-binding domain (amino acids 103 to 165, 1.9e⁻³⁷ for Os006111-33229; amino acids 101 to 163, 3.8e⁻³⁸ for Os020134-3170). The presence of the Dof domain suggests that these two proteins are transcriptional regulators. Thus, by virtue of its interaction with these two proteins and with OsMADS45, novel protein PN23495 can be a novel transcription factor involved in regulation of genes controlling plant development. The OsPN23495-OsMADS45 interaction is a newly identified interaction.

A BLAST analysis comparing the nucleotide sequence of OsPN23495 against TMRI's GENECHIP® Rice Genome Array sequence database identified probeset 05001986_at (e = 0 expectation value) as the closest match. Gene expression analysis indicated that this gene is not specifically induced by a broad range of plant stresses, herbicides, or applied hormones.

OsMADS45 was also found to interact with AP-1 like MADS box protein OsRAP1B. OsRAP1B (GENBANK® Accession No. AB041020) is a 246-amino acid protein encoded by a member of the MADS box gene family. It includes a MADS box domain between amino acids 1 and 60. OsRAP1B was identified by Kyozuka et al., 2000 as a putative rice ortholog of the *Arabidopsis* APETALA1 (AP1), a class of MADS box genes involved in specification of floral organ identity. The OsRAP1B-OsMADS45 interaction has not been previously reported.

OsRAP1B was also constructed as a bait. Its interactors are listed in Table 9 and described later in this Example. These OsRAP1B interactors include prey clones of OsMADS45. A BLAST analysis comparing the nucleotide sequence of OsRAP1B against TMRI's GENECHIP® Rice Genome Array sequence database identified probeset OS003005.1_l_at (2e⁻⁸² expectation value) as the closest match. Gene expression analysis indicated that this gene is expressed in roots and leaves and more highly expressed in flowers, panicles, and seeds. The gene is not specifically induced by a broad range of plant stresses, herbicides, or applied hormones.

### Two-hybrid system using OsRAP1B as bait

Bait constructs containing the *O. sativa* AP1-like MADS box protein RAP1 B (OsRAP1B) were constructed to search for interacting proteins. This protein is described in earlier in this Example as an interactor for OsMADS45. Several bait fragments were used in the search encompassing amino acids 1-150, 125-235, 1-247, 100-247, 65-200, and 30-180 of OsRAP1B (see Table 9).

A bait encoding amino acids 1-150 of OsRAP1B was found to interact with a fragment of the transcription factor Os008339. This protein is described earlier in this Example as an interactor for the bait protein OsMADS45. The Os008339-OsRAP1B interaction has not been previously reported.

A bait encoding amino acids 125-235 of OsRAP1B was also found to interact with rice MADS box-like protein OsBAA81880. OsBAA81880 (GENBANK® Accession No. AB003322) is a 228-amino acid protein with a MADS box domain between amino acids 1 and 60 (4.59e⁻³⁶), as determined by amino acid sequence analysis. The analysis also detected two coiled-coil signatures (amino acids 83 to 113 and amino acids 140 to 174). These putative coiled coil regions overlap with a K-box domain (amino acids 73 to 173, 3.80e⁻³²). The OsBAA81880 protein is not described in the literature; however, the presence of the MADS box and K-box strongly suggests that it is a transcription factor of the MADS box family, and its interaction with OsRAP1B is likely involved in transcriptional regulation of genes associated with plant development.

OsBAA81880 was also found to interact with OsMADS6 (see Table 10). A BLAST analysis comparing the nucleotide sequence of OsBAA81880 against TMRI's GENECHIP® Rice Genome Array sequence database identified probeset OS011977_i_at and OS011794_i_at (e⁻²⁵ and e⁻¹² expectation values, respectively) as the closest matches. The expectation values are too low for these probesets to be reliable indicators of the gene expression of OsBAA81880.

Baits encoding amino acids 1-247 of OsRAP1B and amino acids 100-247 of OsRAP1B were also found to interact with rice MADS-box protein FDRMADS6. This protein is described in earlier in this Example as an interactor for the bait protein OsMADS45. The OsFDRMADS6-OsRAP1B interaction has not been previously reported.

Baits encoding amino acids 1-247 of OsRAP1B and amino acids 100-247 of OsRAP1B was also found to interact with rice MADS box protein OsFDRMADS8. This protein is described earlier in this Example as an interactor for the OsMADS45 bait protein. The OsFDRMADS8-OsRAP1B interaction represents a heterodimer that has not been previously reported.

Baits encoding amino acids 1-247 of OsRAP1B, amino acids 100-247 of OsRAP1B, amino acids 65-200 of OsRAP1B, and amino acids 125-235 of OsRAP1B was also found to interact with MADS box protein OsMADS1. This protein is described herein as an interactor for the OsMADS45 bait protein. The OsMADS1-OsRAP1B interaction has not been previously reported.

Baits encoding amino acids 30-80 of OsRAP1B, amino acids 1-247 of OsRAP1B, amino acids 125-235 of OsRAP1B were also found to interact with rice MADS box protein OsMADS5. This protein is described herein as an interactor for the OsMADS45 bait protein. The OsMADS5-OsRAP1B interaction has not been previously reported.

A bait encoding amino acids 1-247 of OsRAP1B was also found to interact with rice MADS box protein OsMADS6. This protein is described earlier in this Example as an interactor for the OsMADS45 bait protein. The OsMADS6-OsRAP1B interaction has not been previously reported.

A bait encoding amino acids 1-247 of OsRAP1B was also found to interact with rice MADS box protein OsMADS7. OsMADS7 (GENBANK® Accession No. U78891) is a 259-amino acid protein with a MADS box domain between amino acids 11 and 71 (3.22e⁻⁴⁰), as predicted by analysis of the amino acid sequence. The analysis also predicted two coiled-coil signatures (amino acids 93 to 126 and 162 to 186). These coiled coils do not overlap with the MADS box domain. OsMADS7, as well as OsMADS8, is structurally related to the AGL2 gene family based on sequence homology and is a flower-specific MADS box gene (Kang et al., 1997). Both genes are expressed from the young flower stage through the late stage of flower development, with transcripts detected primarily in carpels and also weakly in anthers (Kang et al., 1997). In support of an important role for OsMADS7 in flower development, specifically, in controlling flowering time, transgenic tobacco plants engineered to express the OsMADS7 gene were observed to exhibit early flowering and dwarfism (Kang et al., 1997). The OsMADS7-OsRAP1B interaction has not been previously reported.

OsMADS7 was also found to interact with OsMADS6 (see Table 10). A BLAST analysis comparing the nucleotide sequence of OsMADS8 against TMRI's GENECHIP® Rice Genome Array sequence database identified probeset OS014912_f_at (e⁻⁶¹ expectation value) as the closest match. Gene expression analysis indicated that this gene is expressed early in seed development and is not specifically induced by a broad range of plant stresses, herbicides, or applied hormones.

Baits encoding amino acids 1-247, 30-180, 100-247, and 125-235 of OsRAP1B were found to interact with rice MADS box protein OsMADS8. OsMADS8 (GENBANK® Accession No. U78892) is a 248-amino acid protein that includes a MADS box domain (amino acids 1 to 61, 3 e⁻⁴⁰), as determined by amino acid sequence analysis. Thus, OsMADS8 is a member of the MADS box protein family. The amino acid sequence analysis also predicted a coiled coil C-terminal to the MADS box domain (amino acids 87 to 117). This coiled coil is likely part of a K-box predicted between amino acids 73 and 176 (8.9e⁻⁴⁴ prediction value). OsMADS8, as well as OsMADS7, is structurally related to the AGL2 gene family, as determined by sequence homology, and is a flower-specific MADS box gene (Kang et al., 1997). Both genes are expressed from the young flower stage through the late stage of flower development, with transcripts detectable primarily in carpels and also weakly in anthers (Kang et al., 1997). In support of an important role for OsMADS7 and OsMADS8 in flower development, specifically, in controlling flowering time, is the observation that transgenic tobacco plants engineered to express these genes exhibit early flowering and dwarfism (Kang et al., 1997). The OsMADS8-OsRAP1B interaction represents a heterodimer that has not been previously reported.

OsMADS8 was also found to interact with the bait proteins OsMADS6 (see Table 10) and OsMADS3 (see Table 12). A BLAST analysis comparing the nucleotide sequence of OsMADS8 against TMRI's GENECHIP® Rice Genome Array sequence database identified probeset OS015209_at (e⁻⁸³ expectation value) as the closest match. Analysis of temporal and spatial patterns of gene expression indicated that this gene is expressed early in seed development. Analysis of gene expression in response to various inducers indicated that it is not specifically induced by a broad range of plant stresses, herbicides, or applied hormones.

A bait encoding amino acids 1-247 of OsRAP1B was found to interact with rice MADS box protein OsMADS17. OsMADS17 (GENBANK® Accession No. AF109153) is a 249-amino acid protein that includes a MADS box domain (amino acids 1 to 61), as determined by amino acid sequence analysis (4.31e⁻⁴¹ prediction value). Thus, OsMADS17 is a member of the MADS box protein family. The amino acid sequence analysis also predicted a coiled coil located C-terminal to the MADS box domain (amino acids 122 to 178). This predicted coiled coil is likely part of a K-box predicted between amino acids 72 and 174 (5.2e⁻⁴⁴). The OsMADS17 gene is homologous to ZAG3, the maize homolog of *Arabidopsis* AG, and belongs to the AGL6 subfamily in the AP1/AGL9 family of MADS box genes (Moon et al., 1999). The OsMADS17-OsRAP1B interaction represents a heterodimer that has not been previously reported. The prey clone of OsMADS17 retrieved in the screen includes the predicted coiled coil and most of the K-box in OsMADS17.

OsMADS17 was also found to interact with the bait protein OsMADS5 (see Table 13). An interaction of OsMADS17 with OsMADS6 has also been reported (Moon et al., 1999). A BLAST analysis comparing the nucleotide sequence of OsMADS8 against TMRI's GENECHIP® Rice Genome Array sequence database identified probeset OS000571_f_at (e⁻⁶⁰ expectation value) as the closest match. Analysis of gene expression indicated that this gene is not specifically induced by a broad range of plant stresses, herbicides, or applied hormones.

Baits encoding amino acids 1-247, 30-180, and 125-235 of OsRAP1B were also found to interact with the rice MADS box protein OsMADS45, as has described earlier in this Example. This interaction confirms the interaction between the two proteins used in the reverse bait/prey roles in the yeast two-hybrid system (see Table 1).

A bait encoding amino acids 1-247 of OsRAP1B was also found to interact with novel protein OsPN22834, a protein sharing similarity with Oshox6. OsPN22834 is a 278-amino acid protein that includes a homeobox domain between amino acids 70 and 131, a transposase 8 domain between amino acids 1 and 93, and a bZIP transcription factor domain between amino acids 129 and 167. Hox genes are well defined as modulators of development and pattern formation in a variety or species and organ systems (Fromental-Ramain et al., 1996; Godwin et al., 1998). These genes code for transcription factors that modulate expression of developmentally regulated genes. While most of the published studies pertaining to Hox proteins utilize mouse models, Hox gene products have also been shown to regulate development in plants (Holk et al., 1996). The OsRAP1B-OsPN22834 interaction represents a previously unreported heterodimer of a MADS box protein with a hox gene product.

### Two-hybrid system using OsMADS6 as bait

*O. sativa* MADS box protein MADS6 was also used as a bait protein to identify interactors. This protein is described earlier in this Example as an interactor for the bait protein OsMADS45. The bait fragment used in this search encodes amino acids 50 to 200, a sequence that includes the predicted coiled coil and the K-box of OsMADS6.

OsMADS6 was found to interact with *O. sativa* OS008339 MADS box transcription factor (Os008339). This protein is described earlier in this Example as an interactor for the bait protein OsMADS45. The Os008339-OsMADS6 interaction represents a newly identified interaction that is likely involved in transcriptional regulation of genes associated with development in rice.

OsMADS6 was also found to interact with the *O. sativa* MADS box-like protein OsBAA81880. This protein is described earlier in this Example as an interactor for the bait protein OsRAP1B. The OsBAA81880-OsRAP1B interaction represents a heterodimer that has not been previously reported.

OsMADS6 was also found to interact with *O. sativa* MADS-box protein OsFDRMADS8. This protein is earlier in this Example as an interactor for the bait protein OsMADS45. The OsFDRMADS8- OsMADS6 interaction has not been previously reported.

OsMADS6 was also found to interact with *O. sativa* MADS box protein OsMADS1. This protein is described earlier in this Example as an interactor for the bait protein OsMADS45. This interaction confirms a previous work by Moon et al., 1999, which described the same interaction using a yeast two-hybrid system.

OsMADS6 was also found to interact with *O. sativa* MADS box protein OsMADS5. This protein is described earlier in this Example as an interactor for the bait protein OsMADS45. This interaction confirms a previous work by Moon et al., 1999, which described the same interaction using a yeast two-hybrid system.

OsMADS6 was also found to interact with *O. sativa* MADS box protein OsMADS7. This protein is described earlier in this Example as an interactor for the bait protein OsRAP1B. This interaction confirms a previous work by Moon et al., 1999, which described the same interaction using a yeast two-hybrid system.

OsMADS6 was also found to interact with *O. sativa* MADS box protein OsMADS8. This protein is described earlier in this Example as an interactor for the bait protein OsRAP1B. This interaction confirms a previous work by Moon et al., 1999, which described the same interaction using a yeast two-hybrid system.

OsMADS6 was also found to interact with *O. sativa* MADS box protein OsMADS15. This protein is described earlier in this Example as an interactor for OsMADS45. Its interaction with OsMADS6 confirms a previous work by Moon et al., 1999, which described the same interaction using the yeast two-hybrid system.

OsMADS6 was also found to interact with *O. sativa* MADS box protein OsMADS18. This protein is described earlier in this Example as an interactor for OsMADS45. Its interaction with OsMADS6 confirms a previous work by Moon et al., 1999, which described MADS18, as well as MADS14, MADS15, and MADS17, as interactors for MADS6 using the yeast two-hybrid system.

OsMADS6 was also found to interact with *O. sativa* MADS box protein OsMADS45. This protein is described earlier in this Example as a bait. The OsMADS45- OsMADS6 interaction confirms the interaction observed using OsMADS45 as bait, and represents a newly identified interaction.

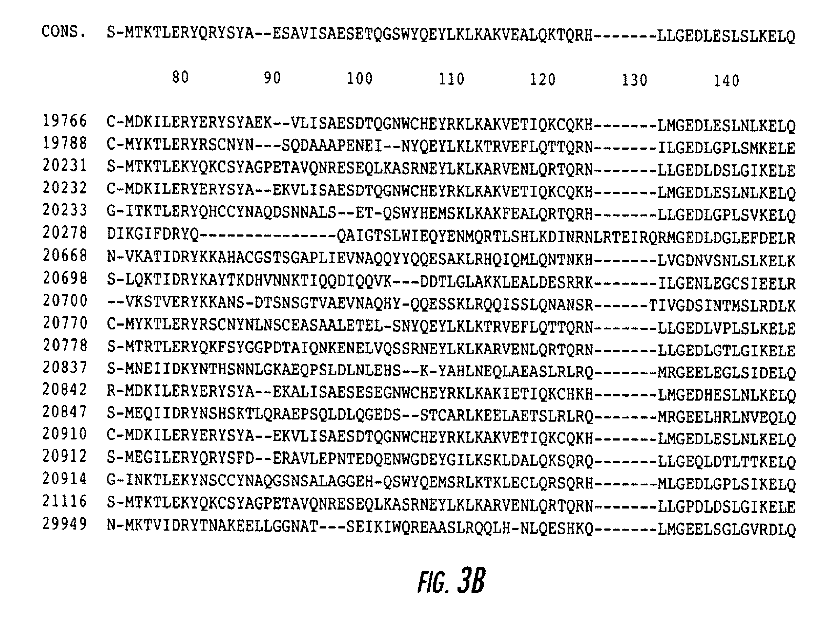

OsMADS6 was also found to interact with novel protein OsPN29949. OsPN29949 is a novel 241-amino acid protein that includes a MADS box domain (amino acids 1-61). The presence of this domain suggests that this protein is a member of the MADS box protein family. Amino acid alignment analysis of the interacting clones (see Figures 3A and 3B) showed that OsPN29949 shares high sequence similarity with OsMADS18, a member of the SQUA subfamily of AP1-like MADS box proteins. OsPN29949 can thus be classified in this group of genes, which are known to be involved in specification of floral organ primordia in snapdragon (reviewed in Moon et al., 1999). The OsPN29949-OsMADS6 interaction represents a newly identified heterodimer that is likely involved in transcriptional regulation of genes associated with development in rice.

Two prey clones encoding amino acids 118-241 and 109-193 of OsPN29949 were retrieved in the screen. These sequences suggest that the domain responsible for the OsPN29949-OsMADS6 interaction resides between amino acids 118 and 193, which includes the K box (amino acids 95-169; see alignment analysis in Figures 3A-3D). There is no match for the OsPN29949 gene on TMRI's GENECHIP® Rice Genome Array.

OsMADS6 was also found to interact with *O. sativa* AP-like MADS box protein OsRAP1B. This protein is described earlier in this Example as an interactor for the bait protein OsMADS45, and was also used as a bait whose interactions are also reported earlier in this Example. The OsRAP1B-OsMADS6 interaction represents a heterodimer that has not been previously reported.

OsMADS6 was also found to interact with *O. sativa* prolamin (OsRP5). Prolamin (GENBANK® Accession Nos. AF156714, AAF73991) is a 156-amino acid protein with a cleavable signal peptide domain (amino acids 1-19), as determined by analysis of the amino acid sequence. Prolamins are seed storage proteins unique to the endosperm of cereals. Seed storage proteins consist of polypeptide chains that are synthesized during seed development and serve as the main source of amino acids for germination and seedling growth. Prolamins accumulate in protein bodies derived from the endoplasmic reticulum (ER). The presence of the cleavable signal peptide domain in OsRP5 is consistent with the structure of prolamins, which possess signal peptides that direct the newly translated polypeptides into the lumen of the ER and are then proteolytically removed. In the ER, prolamins form aggregates and subsequently pinch off to form protein bodies surrounded by an ER-derived membrane (the molecular structure of seed storage proteins and the mechanisms for their delivery into the vacuoles in seeds are discussed in Buchanan et al., 2002). The OsRP5-OsMADS6 interaction represents a previously unreported heterodimer.

In addition to OsMADS6, the prolamin OsRP5 was found to interact with rice hypothetical protein Os006111-3329, which is similar to the zinc/DNA-binding ascorbate oxidase promoter binding protein (AOBP) from *Curcurbita maxima* and which includes a Dof domain zinc finger DNA-binding domain (amino acids 103 to 165, 1.9e⁻³⁷). The presence of the Dof domain suggests that Os006111-3329 is a transcriptional regulator. The interaction of prolamin with this protein and with OsMADS6 can represent steps in the transcriptional regulation of genes controlling seed development.

A BLAST analysis comparing the nucleotide sequence of prolamin against TMRI's GENECHIP® Rice Genome Array sequence database identified probeset OS000235_at (e⁻¹⁵⁵ expectation value) as the closest match. Analysis of gene expression indicated that this gene is not specifically induced by a broad range of plant stresses, herbicides, or applied hormones.

### Two-hybrid system using OsFDRMADS8 as bait

Two-hybrid assays were also performed using the *O. sativa* MADS-box protein FDRMADS8 as bait. This protein is described earlier in this Example as an interactor for the bait protein OsMADS45. The bait clone used in the screen encodes amino acids 60 to 160 of OsFDRMADS8.

OsFDRMADS8 was found to interact with OsMADS45. This protein is described as a bait earlier in this Example. The OsFDRMADS8-OsMADS45 interaction confirms the interaction between the two proteins used in the reverse bait/prey roles in the yeast two-hybrid system.

### Two-hybrid system using OsMADS3 as bait

Two-hybrid assays were also performed using *O. sativa* MADS box protein MADS3 as bait. This protein is described earlier in this Example as an interactor for the bait protein OsMADS45. The bait clone used in the screen encodes amino acids 70 to 170 of OsMADS3.

OsMADS3 was found to interact with MADS box protein OsMADS8. This protein is described earlier in this Example as an interactor for the bait protein OsRAP1B. The OsMAD8-OsMADS3 interaction has not been previously reported.

OsMADS3 was also found to interact with OsMADS45. This protein is described as a bait earlier in this Example. The OsMADS45-OsMADS3 interaction confirms the interaction between the two proteins used in the reverse bait/prey roles in the yeast two-hybrid system.

OsMADS3 was also found to interact with OsPN31165, a novel 301-amino acid protein similar to three proteins of unknown function from *A. thaliana* (the first hit being unknown protein, GENBANK® Accession No. NP_565966, 62% identity; 2e⁻⁰⁸⁷), as determined by BLAST analysis. While the function of OsPN31165 is unknown, its association with OsMADS3 suggests a role for OsPN31165 in plant development, most likely flower development. The OsMADS3-OsPN31165 interaction represents a newly identified heterodimer.

### Two-hybrid assay using OsMADS5 as bait

Two hybrid assays were also performed using OsMADS5 as bait. This protein is described earlier in this Example as an interactor for OsMADS45. The bait clone used in the screen encodes amino acids 50 to 160 of OsMADS5.

OsMADS5 was found to interact with OsFDRMADS6. This protein is described earlier in this Example as an interactor for OsMADS45. The OsFDRMADS6-OsMADS5 interaction represents a heterodimer that has not been previously reported.

OSMADS5 was found to interact with OsMADS13. This protein is described earlier in this Example as an interactor for OsMADS45. The OsMADS13-OsMADS5 interaction has not been previously reported.

OsMADS5 was also found to interact with OsMADS17. This protein is described earlier in this Example as an interactor for OsRAP1B. The OsMADS17-OsMADS5 interaction has not been previously reported.

OsMADS5 was also found to interact with hypothetical protein 000564-1102 (Os000546-1102). Os000564-1102 is a novel 262-amino acid protein similar to the 14-3-3-like homolog GF14-b protein from rice (GENBANK® Accession No. AAB07456.1; 98% identity; 1e⁻¹⁴¹), as determined by BLAST analysis. 14-3-3 proteins include two highly conserved signature patterns: the first is a peptide of 11 amino acids located in the N-terminal section; the second is a 20-amino acid region located in the C-terminal section. Amino acid sequence analysis of Os000564-1102 identified a 14-3-3 signature 1 beginning with amino acid 49 and a 14-3-3 signature 2 beginning with amino acid 221. The 14-3-3 family members interact with, and thereby regulate, proteins that are involved in a variety of signaling pathways including transcriptional regulation. It is likely that Os000564-1102 is a 14-3-3 protein that regulates nuclear events such as transcription by participating in protein-protein interactions. Given the involvement of OsMADS5 in flower development, the interaction between OsMADS5 and Os000564-1102 likely represents a newly identified heterodimer involved in control of transcriptional events associated with plant development, and that Os000564-1102 modulates the MADS box transcription factor function as a member of the 14-3-3 family.

OsMADS6 was also found to interact with rice hypothetical protein BAB56078. This protein is a direct submission to the public domain (GENBANK® Accession No. BAB56078) and is not described in the literature. However, its association with OsMADS5 suggests a role for OsBAB56078 in plant development and this association represents a heterodimer that has not been previously reported.

OsBAB56078 was also found to interact with the rice 14-3-3 protein homolog GF14-b (OsGF14-b), which is up-regulated by stress and the plant hormone abscisic acid (as determined by gene expression analysis; see Example V), and with the transcription factor NAC2 (OsORF01393-P14).

### Two-hybrid assays using OsMADS15 as bait

Two-hybrid assays were also performed using OsMADS15 as bait. This protein is described earlier in this Example as an interactor for OsMADS45. The bait clone used in the screen encodes amino acids 100 to 235 of OsMADS15.

OsMADS15 was found to interact with MADS box protein OsMADS1. This protein is described herein as an interactor for OsMADS45. The OsMADS1-OsMADS15 interaction confirms a previous work by Lim et al., 2000, which describes OsMADS15 as well as OsMADS14 as interactors for OsMADS1 using the yeast two-hybrid system and determined that, while the K domain is essential for the interaction between these proteins, a region preceded by the K domain augments this interaction.

OsMADS15 was also found to interact with OsMADS45. This protein is described herein as a bait protein. The OsMADS45-OsMADS15 interaction confirms the interaction between the two proteins used in the reverse bait/prey roles in the yeast two-hybrid system.

OsMADS15 was also found to interact with OsPN29971, a 108-amino acid protein determined by BLAST analysis to be similar to centromere protein-like from *A. thaliana* (GENBANK® Accession No. 191066.1; 31.1% identity; 9e⁻⁰⁹). The centromere is a region of the chromosome associated with kinetochores, protein-rich structures that are the main sites of interaction between cytoskeletal structures and chromosomes during mitosis and meiosis. Centromere proteins in animals have been implicated in chromosome segregation and cytokinesis events. OsPN29971 can represent a novel centromere-kinetochore-associated protein in plants. Its association with the MADS box protein OsMADS15 represents a newly identified heterodimer that likely regulates transcriptional events related to cell division during plant development.

### Summary

The interacting proteins isolated in the two-hybrid screen using OsMADS45, OsRAP1B, and OsMADS6 as baits form a network comprised mainly of MADS box transcription factors. This indicates that MADS box proteins efficiently interact with each other in yeast, as previously reported (Moon et al., 1999).

Among the interactors found are the previously identified MADS box proteins Os008339, OsFDRMADS6, OsFDRMADS8, OsMADS1, OsMADS3, OsMADS5, OsMADS6, OsMADS7, OsMADS8, OsMADS13, OsMADS14, OsMADS15, OsMADS17, OsMADS18, OsBAA81880, OsMADS45, OsRAP1B and OsMADS6, and the novel protein OsPN29949 (which interacted with OsMADS6). Because MADS box proteins are known to mediate various plant developmental processes as heterodimers, and given the involvement of the bait proteins OsMADS45, OsRAP1B and OsMADS6 in the regulation of flower development, the interactions between the MADS box proteins identified in this Example likely represent a network of heterodimers that regulate transcription of genes associated with plant development in rice. Some of these interactions represent previously unreported heterodimers, as indicated in the description of each interactor hereinabove.

Five additional novel interactors were identified: OsPN23495 is a putative transcriptional regulator that, by association with OsMADS45, is also likely involved in flower development. OsPN22834 is a putative hox gene product. Both MADS box proteins and Hox gene products are well known for their roles in developmental processes, MADS box proteins being linked to flower and fruit development and Hox proteins to embryonic development in plants (Holk et al., 1996). The interaction between RAP1B and OsPN22834 can signify a previously unknown role for one or both of these proteins in the development of the rice plant. Os000564-1102 is a putative 14-3-3 protein that presumably modulates the function of the MADS box transcription factor OsMADS5 with which it interacts. OsPN29971 is a protein whose similarity to a centromere-like protein from *Arabidopsis* (although with low prediction significance) suggests a role in cell division events. The interaction of OsPN29971 with the MADS box protein OsMADS15 is likely involved in regulating transcription of genes during cell division events related to plant development. Finally, OsPN31165 is a protein of unknown function, which by virtue of its interaction with OsMADS3 is likely involved in regulation of plant developmental processes. The association of these novel interactors with the MADS box bait proteins of this Example represent newly identified heterodimers.

Another newly characterized heterodimer reported in this Example is that between OsMADS6 and the seed storage protein prolamin (OsRP5). Expression of storage proteins and timing of their appearance in developing seeds is regulated both transcriptionally and post-transcriptionally. Regulatory sequences have been identified that control their temporal and spatial expression and determine seed and tissue specificity, and more than one regulatory region (promoter) in the storage protein genes is thought to be involved in such regulation by specific DNA-binding proteins (Buchanan et al., 2002). The prolamin OsRP5 was found to interact with OsMADS6 and with another transcriptional regulator (not included in this Example). It is possible that these interactions represent steps in the transcriptional regulation of prolamin expression associated with seed development. Alternatively, the MADS box protein can be sequestered through the interaction with prolamin to be stored with storage proteins that will be used upon seed germination. In either case, this interaction signifies a previously unreported role for OsMADS6 in seed development, in addition to flower development.

It is likely that the coiled coil(s)/K-box identified in the MADS box proteins of this Example facilitate the MADS box protein interactions. Our amino acid sequence alignment analysis of the regions encoded by the interacting clones indicates that all clones share a highly conserved MADS domain, a less conserved K box, and the more variable I region (directly downstream of the MADS domain) and C-terminal domain, in accordance with the modular structure reported in the literature for MADS box proteins (Moon et al., 1999; Lim et al., 2000). The alignments are shown in Figures 3A-3D. This analysis also determined that all interacting fragments include at least the K box, suggesting that this domain is responsible for dimerization, as reported previously. Furthermore, from these alignments a phylogenetic tree was constructed to illustrate the relationships among the interacting proteins (shown in Figure 3E). Based on previous reports (Moon et al., 1999), the tree indicated that OsMADS45, OsMADS7, OsMADS8, OsMADS1 and OsMADS5 are members of the AGL2 subfamily; OsMADS6 and OsMADS17 belong to the AGL6 subfamily; OsFDRMADS6, OsMADS14, RAP1B, OsMADS15, OsMADS18 and novel protein OsPN29949 belong to the SQUA subfamily, all these subfamilies comprised in the AP1/AGL9 family of MADS box genes. The remaining interactors - OsMADS13, OsMADS3, OsFDRMADS8, OsBAA81880, and Os008339 - are classified as others.

MADS box genes isolated from several plant species are known to play important roles in plant development, especially flower development. Knowledge of genes that regulate developmental processes such as flower and fruit development and flowering time has important applications in agriculture, providing new approaches to control of flower and fruit yield. For example, a mutant MADS-box gene, the apple PI homolog (MdPI) of the *Arabidopsis* mutant PI (which causes apetaly) abolishes the normal expression of the MDPI gene, resulting in parthenocarpic fruit (fruit without seed) development in some apple varieties (Yao et al., 2001). Parthenocarpic fruit develops without pollination or fertilization and has a higher commercial value than its seed-bearing counterpart. The identification of the MDPI sequence has led to the proposal of genetic engineering methods to produce parthenocarpic fruit cultivars.

As one of the major human staples, rice has been a target of genetic engineering for higher yields and resistance to diseases, pests, and environmental stresses of various kinds. The proteins encoded by MADS genes regulate transcription of genes associated with developmental processes such as floral organ identity, flowering time, and fruit development. The interactions between rice MADS box transcription factors identified in this Example are relevant to agriculture. Modulation of these interactions can be exploited for the development of genetically engineered plants characterized by a modulated flower development. Because rice is a model for other cereals, knowledge of the genetic mechanisms controlling development in rice will lead to opportunities for enhanced food crops.

The timing of the transition from vegetative growth to flowering, for example, is one of the most important steps in plant development. This step determines the quality and quantity of most crop species by affecting the balance between vegetative and reproductive growth. Therefore, control of flowering time in genetically engineered cereal crops is important in agriculture. One genetic modification that would be economically desirable would be to accelerate the flowering time of a plant. Induction of flowering is often the limiting factor for growing crop plants. One of the most important factors controlling induction of flowering is day length, which varies seasonally as well as geographically. There is a need to develop methods for controlling and inducing flowering in plants, regardless of the locale or the environmental conditions, thereby allowing production of crops, at any given time. Since most crop products (e.g., seeds, grains, fruits), are derived from flowers, such a method for controlling flowering would be economically invaluable. A gene that modulates flowering time in plants was identified and its use proposed for the production of genetically modified plants in which overexpression of this gene results in early flowering in *Arabidopsis,* while loss of function mutations in or antisense directed to the gene cause late flowering (see U.S. Patent Application No. 20010049831). Isolated nucleic acids and methods related to the OsMADS1, OsMADS5, OsMADS6, OsMADS7, and OsMADS8 genes of *Oryza sativa* and the NtMADS3 gene of *Nicotiana tabacum* have also been provided whose expression in transgenic plants causes an altered phenotype, including phenotypes related to the timing of the transition between vegetative and reproductive growth (e.g., diminished apical dominance, early flowering, a partially or completely altered daylength requirement for flowering, greater synchronization of flowering, or a relaxed vernalization requirement; see U.S. Patent No. 5,990,386). Modulation of the protein interactions identified in this Example for OsMADS1, OsMADS5, OsMADS6, OsMADS7, and OsMADS8, for example, could lead to control of flower induction in cereal crops. Additionally, modulation of plant development could be achieved through the identification and application of compounds that can affect the activity of the proteins or the expression of the genes provided in this Example.

In another potential application, the plant-specific K-box domain present in MADS box proteins could be exploited for the development of compounds that increase the quantity or quality of fruit production but do not affect humans or livestock. Additionally, because the K-box domain is the region of the MADS box proteins that confers protein-binding specificity, these domains, either as parts or - whole; can be targets for genetic modification aimed at manipulating traits conferred by specific MADS box protein-protein interactions.

### Example IV

Plant development can also be affected by proteins containing homeobox domains. As reviewed by Gehring, 1992, such homeobox domain containng proteins are DNA-binding transcriptional regulators, many of which are involved in developmental processes. Such proteins have been identified in plants (see e.g., Ruberti et al., 1991; Vollbrecht et al., 1991). Homeobox genes are characterized by the presence within each gene of a well-conserved sequence, the homeobox, which encodes a 61-amino acid DNA-binding domain called the homeodomain. The homeodomain-containing proteins encoded by the homeobox genes are thus capable of binding to specific DNA sequences and act as transcription factors that control the expression of downstream genes to regulate development. In higher plants, homeodomain proteins are mainly implicated in organogenesis or developmental processes (see references below), and also in the pathogenesis-related defense response (Korfhage et al., 1994). The target genes directly regulated by homeodomain-containing proteins are however still largely unidentified (Mannervick, 1999).

Plant homeobox genes (reviewed in Chan et al., 1998) can be subdivided into different families (Hd-Zip, Glabra, Knotted, PHD finger, Bell, Zmbox-PHD) according to sequence conservation within the homeodomain and the presence of additional sequences. Homeobox genes of the plant-specific knotted-like homeobox (KNOX) class contain a conserved domain, the KNOX domain, upstream of the homeodomain. The plant KNOX genes belong to the TALE superclass of homeobox genes, which also comprises genes identified in animals and fungi (Burglin et al., 1997). KNOX genes have been identified in numerous plants, both monocots such as rice and maize, and dicots such as *Arabidopsis* and tomato; they are normally expressed in the meristem and are thought to be primarily involved in shoot and leaf development, particularly in the control of cell fate determination in the shoot meristem (Chan et al., 1998). The first identified plant homeobox gene, the *knotted1* (*kn1*; Vollbrecht et al., 1991) isolated from maize, provided evidence that plant homeobox genes, similar to those of animals, play an important role in regulating developmental processes. Ectopic expression of the maize *kn1* gene (and related dicot genes) often leads to the organization of new meristems in dicot leaves but usually not in monocot leaves (Sinha et al., 1993; Lincoln et al., 1994; Hake et al., 1995; Muller et al., 1995; Haraven et al., 1996; Williams-Carrier et al., 1997). Loss-of-function mutations in the maize *kn1* gene result in defects in shoot meristem maintenance (Kerstetter et al., 1997). *Kn1* belongs to the plant-specific KNOX class of homeobox genes. Other KNOX genes identified in maize include *rough sheath1* (*rs1*) and *liguleless3* (*Lg3*) (reviewed in Chan et al., 1998; Muehlbauer *et al*, 1999), which are thought to be involved in lateral organ development and specifically, in retarding the acquisition of terminal regional identity.

On the basis of sequence homology and expression pattern, KNOX genes are grouped into two classes, I and II (Kerstetter et al., 1997; Chan et al., 1998). Class I genes are mainly expressed in vegetative and inflorescence meristems and are involved in the regulation of shoot apical meristem formation and function and in leaf and flower morphology. The less characterized class II KNOX genes are expressed in most plant organs and tissues and not in meristematic tissues, and they are thought to regulate later stages of development. Further, all class I genes analyzed give rise to similar and distinct phenotypic effects, such as perturbations in the development of leaves leading to morphological defects, when ectopically expressed in transgenic plants. For example, the maize mutant *rough sheath2* (*rs2*) displays ectopic expression of at least three KNOX genes and consequently conditions a range of shoot and leaf phenotypes, including aberrant vascular development, ligular displacements, and dwarfism (Schneeberger et al., 1998). These studies suggest that down-regulation of KNOX gene expression is essential for normal leaf initiation and development. By contrast, no developmental defects have been recorded in plants expressing a class II gene ectopically.

Protein-protein interactions can contribute to the functioning of KNOX proteins, as demonstrated by the ability of two rice KNOX class I proteins to form homo- and heterodimers (Postma-Haarsma et al., 2002). Besides the homeodomain, KNOX proteins contain the conserved ELK and KNOX domains, the latter containing a putative helical structure that suggests a function in protein-protein interaction (Postma-Haarsma et al., 2002). In light of the importance of homeobox genes in controlling plant development, the interaction studies presented here are aimed at characterizing the rice homeobox protein OsHOS59, a member of the class II KNOX genes, which is not described in the literature. The identification of genes encoding proteins that participate in homeobox regulation in rice can allow genetic manipulation of crops to effect agronomically desirable changes in plant growth or development.

This Example provides newly characterized rice proteins interacting with the rice homeobox protein HOS59 (OsHOS59). An automated, high-throughput yeast two-hybrid assay technology was used (provided by Myriad Genetics Inc., Salt Lake City, Utah, United States of America) to search for protein interactions with the bait protein OsHOS59.

### Results

OsHOS59 was found to interact with five proteins annotated in the public domain: a hypothetical protein found similar to GTPase activating protein (OsAAD27557); a putative myosin (OsAAG13633); a putative homeodomain protein (OsAAK00972); putative eukaryotic translation initiation factor 3 large subunit; and the rice probable Myb factor. Seven additional interactors for OsHOS59 are novel rice proteins: a heat shock-like protein (Os000221-3976); a protein similar to the rubber tree latex-abundant protein (OsPN23251); a putative S-adenosyl-L-homocysteine hydrolase (OsPN23829), an enzyme with a role in the control of methylation; a putative PHD-finger protein (OsPN23830); a myosin (OsPN24092) similar to the myosin protein OsAAG13633 described above; and two proteins of unknown function (OsPN23388 and OsPN30858). Additional interactors were identified for some of the prey proteins.

The interacting proteins of the Example are listed in Table 15, followed by detailed information on each protein and a discussion of the significance of the interactions. The nucleotide and amino acid sequences of the proteins of the Example are provided in SEQ ID NOs: 67-80 and 257-268.

Some of the proteins identified represent rice proteins previously uncharacterized. Based on their presumed biological function and on the ability of the prey proteins to specifically interact with the bait protein OsHOS59, the interacting proteins are speculated to be associated with developmental processes in rice.

**Table 15**

| Interacting Proteins Identified for HOS59 (Homeobox Protein HOS59, Fragment) | | | |
|---|---|---|---|
| The names of the clones of the proteins used as baits and found as preys are given. Nucleotide/protein sequence accession numbers for the proteins of the Example (or related proteins) are shown in parentheses under the protein name. The bait and prey coordinates (Coord) are the amino acids encoded by the bait fragment(s) used in the search and by the interacting prey clone(s), respectively. The source is the library from which each prey clone was retrieved. | | | |
| **Gene Name** | **Protein Name** **(GENBANIC® Accession No.)** | **Bait Coord** | **Prey Coord** **(source)** |
| **BAIT PROTEIN** | | | |
| OsHOS59 PN20559 (SEQ ID NO: 258) | *O*. *sativa* Homeobox Protein HOS59, Fragment (BAB55659.1) | | |

| **INTERACTORS** | | | |
|---|---|---|---|
| OsAAD27557* PN22896 (SEQ ID NO: 260) | *O. sativa* Hypothetical Protein, Similar to GTPase Activating Protein (AF111710; AAD27557) | 1-100 | 7-142 (input trait) |
| OsAAG13633# PN25701 (SEQ ID NO: 262) | *O*. *sativa* Putative Myosin (AC078840; AAG13633) | 1-100 | 799-951 (output trait) |
| OsAAK00972 PN23253 (SEQ ID NO: 264) | *O. sativa* Putative Homeodomain Protein OsAAK00972 (AC079736; AAK00972.1) | 1-100 | 236-350 (output trait) |
| OsBAB07943 PN23832 (SEQ ID NO: 266) | *O. sativa* Putative Eukaryotic Translation Initiation Factor 3 Large Subunit (AP002487; BAB07943.1) | 1-100 | 525-767 (output trait) |
| OsMYB PN20689 (SEQ ID NO: 268) | *O. sativa* Probable Myb Factor (T03830) | 1-100 | 36-129 (output trait) |
| Os000221-3976& PN23169 (SEQ ID NO: 68) | Hypothetical Protein 000221-3976, Fragment, Similar to OsHP82 (P33126; e = 0.0) | 1-100 | 2x 123-238 (input trait) |
| OsPN23251 (SEQ ID NO: 70) | Novel Protein PN23251 | 1-206 | 112-291 (input trait) |
| OsPN23388 (SEQ ID NO: 72) | Novel Protein PN23388 | 1-100 | 229-331 (output trait) |
| OsPN23829@ (SEQ ID NO: 74) | Novel Protein PN23829 Putative S- Adenosyl-L-Homocysteine Hydrolase (P32112; e = 0.0) | 1-100 | 3x 2-226 (output trait) |
| | | 1-206 | 3x 1-247 (output trait) |
| OsPN23830 ! (SEQ ID NO: 76) | Novel Protein PN23830, Similar to *A. thaliana* Putative PHD-Finger Protein (NP_566742.1; 2e⁻⁷³) | 1-100 | 4-207 2x 1-169 (output trait) |
| OsPN24092 (SEQ ID NO: 78) | Novel Protein PN24092, Similar to *O. sativa* Putative Myosin | 1-100 | 797-948 (output trait) |
| OsPN30858 (SEQ ID NO: 80) | Novel Protein PN30858 | 1-206 | 230-400 (output trait) |

| | | | |
|---|---|---|---|
| * Additional interactions identified for OsAAD27557 are shown in Table 16 # Additional interactions identified for OsAAG13633 are shown in Table 17 & Additional interactions identified for Os000221-3976 are shown in Table 18 @ Additional interactions identified for OsPN23829 are shown in Table 19 ! Additional interactions identified for OsPN23830 are shown in Table 20 | | | |

**Table 16**

| Interacting Proteins Identified for AAD27557 (Hypothetical Protein Similar to GTPase Activating Protein) | | | |
|---|---|---|---|
| **Gene Name** | **Protein Name** **(GENBANK® Accession No.)** | **Bait Coord** | **Prey Coord** **(source)** |
| **PREY PROTEIN** | | | |
| OsAAD27557 PN22896 (SEQ ID NO: 260) | Hypothetical Protein Similar to GTPase Activating Protein (AF111710; AAD27557) | | |

| **BAIT PROTEIN** | | | |
|---|---|---|---|
| Os003181-3684 PN21036 (SEQ ID NO: 82) | Hypothetical Protein 003181-3684 | 58-140 | 1-149 (output trait) |

**Table 17**

| Interacting Proteins Identified for AAG13633 (Putative Myosin) | | | |
|---|---|---|---|
| **Gene Name** | **Protein Name** **(GENBANK® Accession No.)** | **Bait Coord** | **Prey Coord** **(source)** |
| **PREY PROTEIN** | | | |
| OsAAG13633 PN25701 (SEQ ID NO: 262) | *O. sativa* Putative Myosin (AC078840; AAG13633) | | |

| **BAIT PROTEIN** | | | |
|---|---|---|---|
| Os005750-3115 PN20466 (SEQ ID NO: 270) | *O. sativa* bZIP Transcription Factor (AB051294; BAB72061.1) | 50-150 | 2x 528-789 538-738 612-738 (output trait) |

**Table 18**

| Interacting Proteins Identified for 000221-3976 (Hypothetical Protein 000221-3976, Fragment) | | | |
|---|---|---|---|
| **Gene Name** | **Protein Name** **(GENBANIC® Accession No.)** | **Bait Coord** | **Prey Coord** **(source)** |
| **PREY PROTEIN** | | | |
| Os000221-3976 PN30899 (SEQ ID NO: 24) | Hypothetical Protein 000221-3976, Fragment, Similar to OsHP82 (P33126; e = 0.0) | | |

| **BAIT PROTEIN** | | | |
|---|---|---|---|
| OsCYCOS2 PN20257 (SEQ ID NO: 210) | *Oryza sativa* Cyclin 2 (X82036; CAA57556) | 50-233 | 163-313 (input trait) |

**Table 19**

| Interacting Proteins Identified for PN23829 (Novel Protein PN23829 Putative S-Adenosyl-L-Homocysteine Hydrolase) | | | |
|---|---|---|---|
| **Gene Name** | **Protein Name** **(GENBANK® Accession No.)** | **Bait Coord** | **Prey Coord** **(source)** |
| **PREY PROTEIN** | | | |
| OsPN23829 (SEQ ID NO: 74) | Novel Protein PN23829 Putative S- Adenosyl-L-Homocysteine Hydrolase (P32112; e=0.0) | | |

| **BAIT PROTEIN** | | | |
|---|---|---|---|
| OsTFX1 PN19697 (SEQ ID NO: 272) | *O. sativa* Putative Transcription Factor X1 (AF101045; AAF21887) | 400-629 | -21-216 -4-226 -2-195 (output trait) |
| Os005792-3529 PN20080 (SEQ ID NO: 274) | Hypothetical Protein 005792-3529 Similar to *O. sativa* Receptor Kinase (AAK18840.1; 8e⁻⁰⁷) | 1-55 | 3-220 (output trait) |

**Table 20**

| Interacting Proteins Identified for PN23830 (Similar to *A. thaliana* Putative PHD-Finger Protein) | | | |
|---|---|---|---|
| **Gene Name** | **Protein Name** **GENBANK® Accession No.)** | **Bait Coord** | **Prey Coord** **(source)** |
| **PREY PROTEIN** | | | |
| OsPN23830 (SEQ ID NO: 76) | Novel Protein PN23830, Similar to *A*. *thaliana* Putative PHD-Finger Protein (NP_566742.1; 2e⁻⁷³) | | |

| **BAIT PROTEIN** | | | |
|---|---|---|---|
| Os018049-3655 PN20534 (SEQ ID NO: 176) | Hypothetical Protein 018049-3655, Fragment, *O. sativa* Putative Homeodomain Transcription Factor, 3'-Partial (AC092697; AAL58126.1) | 1-148 | 89-250 (output trait) |

### Two-hybrid assay using OsHOS59 as bait

OsHOS59 is a 205-amino acid protein fragment with a homeobox domain profile (Gehring, 1992; Gehring & Hiromi, 1986; Schofield, 1987), namely at amino acids 122 to 185, as determined by analysis of its amino acid sequence. Proteins within this group are DNA-binding transcriptional regulators that are involved in developmental processes. A BLAST analysis of the amino acid sequence indicated OsHOS59 is the rice KNOX Family Class II Homeodomain Protein (GENBANK® Accession No. BAB55659.1). The analysis indicated that all proteins displaying close homology to OsHOS59 are also homeodomain proteins, particularly from plant species. This strongly suggests that OsHOS59, although not described in the literature, is a rice homeobox protein that most likely functions as do other members of this protein family.

There is not much evidence on the role of class II KNOX genes. However, based on studies with the class 11 gene *KNAT3* from *Arabidopsis,* which was found to be expressed in young leaves, buds and pedicels, at the junction between organs and in maturing tissues, and whose expression is regulated by light, class II KNOX genes are suggested to be involved in later stages of plant development (discussed in Chan et al., 1998).

Two bait fragments, encoding amino acid 1-100 and 1-206, of OsHOS59 were used in the yeast two-hybrid screen.

A BLAST analysis comparing the nucleotide sequence of OsHOS59 against TMRI's GENECHIP® Rice Genome Array sequence database identified probeset OS011682_at and OS002989.1_i_at (e⁻¹⁰⁰ and 7e⁻²⁶ expectation values, respectively) as the closest matches. Analysis of gene expression in rice plants indicated that this gene is down-regulated by environmental cold, and by abscisic acid and jasmonic acid.

OsHOS59 was found to interact with OsAAD27557. OsAAD27557 is annotated as a rice Hypothetical Protein (GENBANK® Accession No. AAD27557). It is a 789-amino acid protein with a leucine-rich repeat between amino acids 214 and 241, as determined by analysis of its amino acid sequence (1.28e⁻⁰³ prediction value). Leucine-rich repeats are thought to be involved in protein-protein interactions (Kobe et al., 1994). A BLAST analysis against the public database indicated that the amino acid sequence of OsAAD27557 is similar to those of Ran GTPase activating protein from the plant *Medicago sativa* subsp. x varia (GENBANK® Accession No. AAF19528.1, 66.4% identity, e = 0.0) and GTPase activating protein 2 from *A. thaliana* (GENBANK® Accession No. NP_197433, 62% identity, e⁻¹⁷⁹). In agreement with these results, a BLAST analysis against Myriad's proprietary database indicated human Ran GTPase activating protein 1 (RANGAP1) as the most similar protein to OsAAD27557 (28% identity, 5e⁻²⁴). GTPase activating proteins interact with GTPases such as Ras thereby enhancing the GTPase activity (Bischoff et al., 1994). Hydrolysis of GTP to GDP is an important step in many intracellular signal transduction pathways that control various cellular processes such as cell growth and development, apoptosis, lipid metabolism, cytoarchitecture, membrane trafficking, and transcriptional regulation (Aznar & Lacal, 2001). Ran GTPases are required for nucleo-cytoplasmic transport, regulation of cell cycle progression, mitotic spindle formation, and postmitotic nuclear assembly (reviewed by Sazer & Dasso, 2000, and Dasso, 2000). Plants Ran proteins are thought to be functionally equivalent to their mammalian and yeast homologs and to be necessary for maintaining a coordinated cell cycle, for protein import into the nucleus and for the onset of mitosis (Ach & Gruissem, 1997; Merkle et al., 1994). Moreover, plant small GTP-binding proteins have been linked to disease resistance (Ono et al., 2001). Thus, the prey protein OsAAD27557 is a rice GTPase activating protein that likely participates in signal transduction involving GTP hydrolysis during events related to cell division as part of either plant development and/or response to pathogen invasion.

OsAAD27557 also interacts with Hypothetical Protein 003181-3684 (Os003181-3684; see Table 16). Os003181-3684 is a hypothetical protein of 176 amino acids that includes a predicted transmembrane domain (amino acids 43 to 59). A BLAST analysis of the amino acid sequence indicated no proteins highly similar to Os003181-3684 in either public or Myriad's proprietary databases. However, the predicted transmembrane domain suggests that this protein can be some type of cell surface receptor or receptor-interacting protein that is important for signal transduction. The OsAAD27557-Os0031813684 interaction can represent a step in a signal transduction pathway involving GTP hydrolysis and transcriptional regulation in developmental processes.

OsHOS59 was also found to interact with *O. sativa* putative myosin (OsAAG13633). A BLAST analysis of the amino acid sequence of OsAAG13633 indicated that this prey protein is the rice putative myosin (GENBANK® Accession No. AAG13633, 100% identity, e = 0.0). Myosins are discussed in Example I. Based on current knowledge of plant myosins, the prey protein OsAAG13633 can be a cytoskeletal component that participates in events relating to cytoplasmic streaming or cell division during plant development.

OsAAG13633 also interacts with *O. sativa* bZIP Transcription Factor (Os005750-3115; see Table 17). Os005750-3115 is a 333-amino acid protein with a predicted basic leucine zipper (bZIP) domain (amino acids 45 to 108, 1.54e⁻⁶; see Hurst, 1995; Ellenberger, 1994). This domain includes a basic DNA-binding region and a leucine zipper used to initiate protein-protein interactions, and it is often found in transcription factors. A BLAST analysis of the amino acid sequence of Os005750-3115 indicated that this protein is the rice bZIP Transcription Factor (GENBANK® Accession No. BAB72061.1, 99.3% identity, e = 0.0).

OsHOS59 was also found to interact with OsAAK00972, a 642-amino acid protein that includes a homeobox domain profile (amino acids 379 to 442 by Prosite, amino acids 406 to 441 by Pfam), as determined by analysis of its amino acid sequence. The analysis also identified a POX domain (a domain associated with HOX domains) between amino acids 188 and 333 (1.36e⁻⁵⁶). The retrieved prey clone encodes amino acids 236 to 350 of OsAAK00972, a region that includes the POX domain of OsAAK00972. Hox genes are clustered sets of homeobox-containing genes that play a central role in animal development (Mann & Affolter, 1998). A BLAST analysis of the amino acid sequence of OsAAK00972 indicated that it is the rice Putative Homeodomain Protein (GENBANK® Accession No. AAK00972.1, 100% identity, e = 0.0). OsAAK00972 is thus a member of the homeobox protein family.

OsHOS59 was also found to interact with OsBAB07943, a protein of 984 amino acids with a predicted transmembrane domain (amino acids 316 to 332). Analysis of its sequence also identified a PINT (Proteasome, Int-6, Nip-1 and TRIP-15) motif (amino acids 441 to 532, 3.91e⁻⁰⁷), which is present in the C-terminal region of several regulatory components of the 26S proteasome and other proteins. The function of this motif is not known. The analysis also predicted three coiled coils (amino acids 91 to 123, 552 to 700, and 794 to 963). The prey clone retrieved encodes amino acids 525 to 767 of OsBAB07943, a region that includes one of the predicted coiled coils within OsBAB07943. The presence of the PINT motif is in agreement with the results of BLAST analysis, which indicated that OsBAB07943 is the rice putative eukaryotic translation initiation factor 3 (eIF3) large subunit (GENBANK® Accession No. BAB07943.1, 100% identity, e = 0.0), eIF3e being homologous to the product of Int-6 (eIF3e; Shalev et al., 2001). The analysis also indicated that OsBAB07943 is similar to eukaryotic translation initiation complexes of other species including *Zea mays* (GENBANK® Accession No. AAD39834, 69% identity, e = 0.0) and *Nicotiana tabacum* (GENBANK® Accession No. Q40554, 66% identity, e = 0.0). Therefore, it is likely that OsBAB07943 truly is a rice translation initiation factor subunit.

The mammalian eukaryotic initiation factor 3 (eIF3) is composed of at least eight subunits, the largest of which has a relative molecular mass of 180 kDa. A comparison of the sequences of the corresponding eIF3 large subunits from several species led to the conclusion that eIF3 large subunit is highly conserved across the animal, plant, and fungal kingdoms (Johnson et al., 1997). In *Z*. *mays*, eukaryotic translation initiation factor 3 large subunit is expressed in the region of the root meristem surrounding the central stele and in the young root, the male inflorescence, and the developing cob and seed (Sabelli et al., 1999). Eukaryotic initiation factor complexes initiate translation of mRNA (reviewed by Hannig et al., 1995), in part by using their helicase activity to unwind the mRNA strand secondary structure in the 5'-untranslated region of mRNA, which facilitates binding of the mRNA to the 40 S ribosomal subunit (Rogers et al., 2001). In addition, eIF3 in humans is in some circumstances regulated by protein-protein interaction (Guo et al., 2000).

OsHOS59 was also found to interact with *O. sativa* Myb factor (OsMYB). A BLAST analysis of the amino acid sequence of OsMYB indicated that this prey protein is the rice Probable Myb Factor (GENBANK® Accession No. T03830, 100% identity, e⁻¹⁶⁸). OsMYB is a protein of 279 amino acids that includes an ATP/GTP-binding site motif A (P-loop, amino acids 45 to 52 (see e.g., Saraste et al., 1990; Koonin, 1993) and two Myb DNA-binding domain repeats (amino acids 17 to 25 for signature 1, and amino acids 89 to 112 for signature 2; see e.g., Grotewold et al., 1991; Oppenheimer et al., 1991). The prey clone retrieved encodes amino acids 36 to 129 of OsMYB, a region that includes the P-loop and the Myb DNA-binding domain signature 2. Myb proteins are nuclear DNA-binding proteins that recognize the sequence pyAAC(G/T)G (Biedenkapp et al., 1988). The presence of two Myb DNA-binding signatures suggests that OsMYB is a member of the two-repeat family of Myb proteins. The number of these repeats determines how the protein binds DNA and, consequently, its function (reviewed by by Jin & Martin, 1999).

OsHOS59 was also found to interact with Os000221-3976, a 480-amino acid protein fragment that includes an Hsp90 domain (amino acids 6 to 480), as determined by analysis of its amino acid sequence (e = 0.0). A BLAST analysis against the public and Myriad's proprietary databases showed that Os000221-3976 shares amino acid sequence similarity with many heat shock proteins, the top hit being the rice heat shock protein 82 (Van Breusegem et al., 1994; GENBANK® Accession No. P33126, 96.4% identity, e = 0.0). Therefore, Os000221-3976 is either a splice variant of heat shock protein 82 or a separate but very similar protein. A comparison of the nucleotide sequences suggests the latter is more likely. The rice HSP82 mRNA is induced specifically upon heat stress (Van Breusegem et al., 1994).

While heat shock proteins (HSPs) have been ascribed a main role in the plant stress response, some of these proteins are designated as HSPs solely based on sequence homology and their functions in plants have not been demonstrated *in vitro.* Indeed, some HSPs are expressed throughout development. HSPs function as molecular chaperones that promote proper protein folding and can have roles not related to the stress response. HSP70 proteins, for instance, are essential for normal cell function. They are ATP-dependent molecular chaperones that can interact with many different proteins, given their role in protein folding, unfolding, assembly, and disassembly. These topics are discussed in Buchanan et al., 2002 at pages 1197-1202. The heat shock protein HSP70 in sea urchin cells has been proposed to have a chaperone role in tubulin folding when localized on centrosomes, and in the assembling and disassembling of the mitotic apparatus when localized on the fibres of spindles and asters (Agueli et al., 2001).

The heat shock protein Os000221-3976 also interacts with rice Cyclin 2 (OsCYCOS2; see Table 18). The 419-amino acid protein OsCYCOS2 (GENBANK® Accession No. CAA57556) is a G2/M type cyclin that contains two cyclin domains spanning amino acids 200 to 284 (2.7e⁻²⁶) and amino acids 297 to 379 (1.29e⁻²²). Type G2/M cyclins regulate the cell cycle progression from G2 to mitosis during plant development. Cyclins are regulatory proteins that activate cyclin-dependent protein kinases (CDKs), which are essential for cell cycle progression in eukaryotes. The binding of cyclins to specific proteins is thought to provide potential substrates to CDKs. Cyclins are thus important regulators that couple control of proliferation to the many environmental and developmental cues that affect plant growth. (The role of cyclin-CDK complexes in regulation of the plant cell cycle is reviewed in John et al., 2001 and Potuschak & Doerner, 2001. Interactions identified for OsCYCOS2 are discussed in Example II above.)

OsHOS59 was also found to interact with OsPN23251, a novel 420-amino acid protein with a possible cleavage site between amino acids 19 and 20, although no N-terminal signal peptide is evident. A BLAST analysis of the OsPN23251 amino acid sequence determined that it is similar to latex-abundant protein from the rubber tree *Hevea brasiliensis* (GENBANK® Accession No. AAD13216.1, 62% identity, e⁻¹⁴¹). Many proteins isolated from latex are defense-related allergens (Kostyal et al., 1998). A BLAST analysis comparing the nucleotide sequence of OsPN23251 against TMRI's GENECHIP® Rice Genome Array sequence database identified probeset Os004430.1_at (e = 0.0 expectation value) as the closest match. Analysis of gene expression indicated that this gene is specifically expressed in root.

OsHOS59 was also found to interact with novel protein OsPN23388. OsPN23388 is a 509-amino acid protein with a predicted BRCA1 C-terminus (BRCT) domain (amino acids 1 to 42, 5.2e⁻⁰⁵), which is known to facilitate protein-protein interactions. This domain was originally identified in the breast/ovarian cancer suppression protein, BRCA1, and is found in a large number of proteins involved in DNA repair, recombination, and cell cycle control (Zhang et al., 1998). These include p53-binding protein (53BP1) and two uncharacterized hypothetical proteins (KIAA0170 and SPAC19G10.7) (Callebaut & Mornon, 1997). A BLAST analysis against the Genpept database indicated that OsPN23388 is similar to two *A. thaliana* proteins of unknown function: hypothetical protein (GENBANK® Accession No. NP_180195, 49.3% identity, e⁻¹¹⁴) and hypothetical protein T15B3.70 (GENBANK® Accession No. T48947, 44% identity, e⁻⁷²).

OsHOS59 was also found to interact with OsPN23829, a protein of 485 amino acids. An analysis of its amino acid sequence identified an S-adenosyl-L-homocystein hydrolase signature 1 (amino acids 85 to 99) and an S-adenosyl-L-homocystein hydrolase signature 2 (amino acids 262 to 278) (see Sganga et al., 1992). In agreement with the presence of these protein signatures, a BLAST analysis against the Genpept database indicated that the amino acid sequence of OsPN23829 is similar to those of S-adenosyl-L-homocysteine hydrolase proteins from several other species including *Triticum aestivum* (top hit, GENBANK® Accession No. P32112, 95.2% identity, e = 0.0), asparagus (GENBANK® Accession No. CAA03454, 90% identity, e = 0.0), and *Catharanthus roseus* (GENBANK® Accession No. S38379, 90% identity, e = 0.0). In agreement with these results, the most similar protein in Myriad's proprietary database is *Triticum aestivum* S-adenosyl-L-homocysteine hydrolase (92% identity, e = 0.0).

S-adenosyl-L-homocysteine hydrolase is a key enzyme in the activated methyl cycle, which involves the production of S-adenosylmethionine (reviewed in Kawalleck et al., 1992), whose fate is important for protein synthesis or DNA modification. This enzyme hydrolyzes S-adenosyl-L-homocysteine into adenosine and L-homocysteine (a reaction that requires NAD as a cofactor) and thus plays a crucial role in normal cellular metabolism. Because S-adenosyl-L-homocysteine is a competitive inhibitor of S-adenosyl-L-methionine-dependent methyl transferase reactions, S-adenosyl-L-homocysteine hydrolase is though to play a key role in the control of methylation via regulation of the intracellular concentration of S-adenosyl-L-homocysteine. Transmethylation reactions are important components of the biosynthetic machinery in most plant cells. The regulation of intracellular methylation reactions mediated by S-adenosyl-L-homocysteine hydrolase has been linked to morphogenesis *in planta.* Deregulation of methylation resulted in morphological changes including a floral homeotic change in transgenic tobacco expressing antisense RNA of the S-adenosyl-L-homocysteine hydrolase gene (Tanaka et al., 1997). In addition, a role for S-adenosyl-L-homocysteine hydrolase in the plant pathogen-induced defense response has been suggested based on the observation that elicitor treatment induces both S-adenosyl-L-homocysteine hydrolase mRNA expression and activity in parsley cultured cells and in intact leaves (Kawalleck et al., 1992). In a contrasting role, S-adenosyl-L-homocysteine hydrolase activity can be involved in mechanisms leading to viral infection, as the effectiveness of antiviral compounds correlates with their ability to inhibit its activity (Robins et al., 1998; Liu et al., 1992; Wolf & Borchardt, 1991; Kitade et al., 1999).

A BLAST analysis comparing the nucleotide sequence of OsPN23829 against TMRI's GENECHIP® Rice Genome Array sequence database identified probeset Os001768.1_at (e = 0.0) expectation value) as the closest match. Analysis of gene expression indicated that this gene is induced by jasmonic acid and by *Magnaporthe grisea*, the fungal pathogen that causes rice blast disease.

OsPN23829 also interacts with rice putative transcription factor X1 (OsTFX1; GENBANK® Accession No. AAF21887.1), and with hypothetical protein 005792-3529 (Os005792-3529; see Table 19). OsTFX1 is an uncharacterized transcription factor. It can form a complex with both OsPN23829 and OsHOS59 to regulate transcriptional events related to cell cycle/development. Os005792-3529 is a hypothetical protein of 54 amino acids in which no well-characterized protein domain was identified. The isolated cDNA sequence starts with the putative ATG initiation codon, leaving the reading frame potentially open in the 5' direction, suggesting that the real protein might be larger than 54 residues. BLAST analysis of the available amino acid sequence indicated that Os005792-3529 is similar to a putative receptor kinase from rice (GENBANK® Accession No. AAK18840.1, 72% identity, 8e⁻⁰⁷). Note, however, that the domain of similarity with the putative receptor kinase AAK18840.1 is only 36-residue long.

OsHOS59 was also found to interact with novel protein PN23830, which is similar to the putative *Arabidopsis* PHD-Finger protein OsPN23830. OsPN23830 is a protein of 253 amino acids. An analysis of its amino acid sequence identified a PHD domain (plant homeo domain, Pascual et al., 2000; Aasland et al., 1995; amino acids 199 to 246, e⁻¹⁰). The presence of the PHD finger domain is in agreement with BLAST analysis which indicated similarity of OsPN23830 to *Arabidopsis* putative PHD-finger protein (GENBANK® Accession No. NP_566742.1, 53.8% identity, 2e⁻⁷³). The PHD finger is a Cys⁴-His-Cys³ zinc finger found primarily in a wide variety of chromatin-associated proteins, including HAT3.1, a plant homeobox gene (Aasland et al., 1995). Although the exact function of the PHD finger is not known, it is thought to facilitate protein-protein interactions (O'Connell et al., 2001). The association OsPN23830 with OsHOS59 suggests a role for OsPN23830 in transcriptional regulation during development.

OsPN23830 also interacts with another homeodomain protein, Hypothetical Protein 018049-3655 (Os018049-3655; see Table 20). A BLAST analysis of the amino acid sequence of Os018049-3655 determined that this protein is the rice Putative Homeodomain Transcription Factor, 3'-Partial (GENBANK® Accession No. AAL58126.1, 100% identity, 5e⁻¹³⁴).

OsHOS59 was also found to interact with novel protein PN24092. A BLAST analysis of the amino acid sequence of OsPN24092 determined that this protein is similar to the same rice putative myosin (GENBANK® Accession No. AAG13633, 84.7% identity, e = 0.0) found to interact with OsHOS59 (see *O. sativa* Putative Myosin; OsAAG13633).

OsHOS59 was also found to interact with novel protein PN30858. A BLAST analysis of the amino acid sequence of OsPN30858 determined that this protein is similar to Expressed Protein from *A. thaliana* (GENBANK® Accession No. NP_566372.1, 63.2% identity, e = 0.0), a protein of unknown function.

### Summary

The KNOX homeodomain protein OsHOS59 interacts with other DNA-binding proteins thought to be involved in transcriptional regulation, including a putative homeodomain protein (OsAAK00972) and a Myb protein (OsMYB). These interactions are consistent with published evidence that KNOX proteins function as homo- and heterodimers. Indeed, the specificity of KNOX proteins can be further enhanced by interactions with other transcription factors (Mann & Affolter, 1998; Postma-Haarsma et al., 2002). Based on the presumed role of OsHOS59 in plant development, we speculate that the OsHOS59-OsAAK00972 and OsHOS59-OsMYB interactions represent protein complexes that regulate transcription of genes involved in developmental processes and, in the case of OsMYB regulation, which include a specific sequence in their promoters. This hypothesis is supported by the observation that both HOX and Myb transcription factors cooperatively function to regulate myeloid cell differentiation in mammals (Nagamara-Inoue et al., 2001, and reviewed by Lenny et al., 1997).

OsHOS59 was also found to interact with a putative Ran GTPase activating protein (OsAAD27557). Given the function of Ran GTPases in nucleo-cytoplasmic transport, regulation of cell cycle progression, mitotic spindle formation, and postmitotic nuclear assembly Sazer & Dasso, 2000 and Dasso, 2000), the OsHOS59-OsAAD27557 interaction is speculated to represent a step in a signal transduction pathway that involves GTP hydrolysis during events related to cell cycle progression or cell division as part either plant development and/or response to pathogen invasion.

Two of the interactors identified in the yeast two-hybrid screen, OsAAG13633 and the novel protein OsPN24092, are putative myosins highly similar to each other (84.7% identity). Note that OsAAG13633 also interacts with another transcription factor (Os005750-3115). Molecular motors, including kinesins, myosins and dyneins, have been well characterized in non-plant organisms and implicated in a variety of cellular functions such as vesicle and organelle transport, cytoskeleton dynamics, morphogenesis, polarized growth, cell movements, spindle formation, chromosome movement, nuclear fusion, and signal transduction. In contrast, the roles of the many kinesins and myosins identified in plants are largely unknown (reviewed in Reddy, 2001). A few studies suggest that myosins in higher plants are involved in the movement of organelles and vesicles during cytoplasmic streaming and in pollen tube growth, and in maturation of the cell plate at cytokinesis (reviewed in Yokota et al., 1999b; Reichelt et al., 1999). The rice myosins identified in this Example are likely involved in dynamic cytoskeletal events, such as cytoplasmic streaming, intracellular cargo movement or cell division, associated with development processes. Their interactions with the transcription factors OsHOS59 and Os005750-3115 can represent steps in transcriptional regulation of such events.

Another interactor, Os000221-3976, is a putative heat shock protein similar to rice HSP82. Heat shock proteins (HSPs) act as molecular chaperones and, while these molecules in plants have been mainly linked to the stress response, some are not related to stress and their functions remain to be defined (Buchanan et al., 2002, at page 1198). Indeed, some HSPs are expressed throughout development. In the context of all the interactions identified for OsHOS59, it is possible that Os000221-3976 acts as a molecular glue to hold together interacting proteins or to promote proper protein folding in events related to plant development that might or might not be associated with stress. An alternative role for this prey protein can be deduced by functional homology with animal heat shock proteins whose chaperone roles in tubulin folding or mitotic structures assembly/disassembly depends on their localization on centrosomes or spindle fibers, respectively (Agueli et al., 2001). The heat shock protein Os000221-3976 can thus act as a chaperone in events related to tubulin folding or mitotic structure assembly/disassembly. These are functions associated with the phase of the cell cycle controlled by OsCYCOS2, a type G2/M cyclin that regulates the cell cycle progression from G2 to mitosis during plant development. The interaction identified in this Example between the heat shock protein Os000221-3976 and OsCYCOS2 substantiates this hypothesis and further supports the involvement of this novel rice heat shock protein in developmental processes. Discovery of the subcellular localization of Os000221-3976 can clarify its function.

Another protein interacting with OsHOS59 with a role in regulation of development is a putative S-adenosyl-L-homocysteine hydrolase (OsPN23829), an enzyme involved in control of methylation reactions. Transmethylation reactions are important components of the biosynthetic machinery in most plant cells. S-adenosyl-L-homocysteine hydrolase participates in the activated methyl cycle that yields methionine, whose fate is important for protein synthesis or DNA modification. In plants, the regulation of intracellular methylation reactions mediated by S-adenosyl-L-homocysteine hydrolase has been linked to morphogenesis through in *planta* studies. Deregulation of methylation results in morphological changes including a floral homeotic change in transgenic tobacco expressing antisense RNA of the S-adenosyl-L-homocysteine hydrolase gene (Tanaka et al., 1997). Our gene expression experiments indicate that OsPN23829 is induced by jasmonic acid which, in addition to having a role in the defense response, inhibits growth processes in many tissues and is active in reproductive development (it is thought to play some role in the formation of flowers, fruit, and seeds; Buchanan et al., 2002, at page 917). These data suggest that OsPN23829 can be involved in development/plant morphogenesis, and its association with the OsHOS59 can regulate transcriptional events related to these processes. In addition, a metabolic link can exist between the activated methyl cycle reactions mediated by S-adenosyl-L-homocysteine hydrolase and the plant pathogen-induced defense response (Kawalleck et al., 1992). While no other published evidence points to this conclusion, our gene expression experiments indicate that the gene encoding OsPN23829 is induced by jasmonic acid, which is also a component of plant defense response pathways, and by the fungal pathogen *M. grisea.* It is thus possible that the rice S-adenosyl-L-homocysteine hydrolase OsPN23829 can also have a role in defense against pathogens.

The remaining novel proteins found to interact with OsHOS59 include a eukaryotic translation initiation factor 3 large subunit (OsBAB07943) with a putative role in initiation of mRNA translation, a protein similar to latex-abundant protein (OsPN23251), and three proteins similar to *Arabidopsis* proteins of unknown function (OsPN23388, OsPN30858, and a putative PHD-finger protein OsPN23830). The association of these prey proteins with OsHOS59 suggests a role in transcriptional regulation of genes involved in development.

Many of the rice proteins found to interact with the KNOX homeodomain protein OsHOS59 have roles in plant cell cycle/development. This observation corroborates the notion that the previously uncharacterized protein OsHOS59 is involved in transcriptional regulation of development genes. Some of these interactors are newly characterized rice proteins, and their interactions with OsHOS59 represent molecular mechanisms for transcriptional regulation of developmental processes in rice that have not been previously described.

The identification of protein-protein interactions in rice has important commercial applications. Modulation of these interactions can allow control of biological processes mediated by these molecules, resulting in the introduction of desirable traits in genetically engineered plants. The proteins identified in the present Example can be exploited for the development of genetically engineered crops that exhibit desirable changes in plant development. In addition, these proteins can allow the identification of compounds that affect plant development.

Plants can regenerate individual plants through the regeneration of adventitious shoots or adventitious embryos from undifferentiated tissues derived from somatic cells, a process regulated by the interaction of plant hormones such as auxins and cytokinins. In addition to responding to the signals produced by plant hormones, homeobox genes are involved in plant morphogenesis. The regeneration ability of plants is exploited for the production of young plants from cultured shoot and for regenerating transformed plants after the introduction of genes into somatic cell tissues or cultured plant cells. Proposed applications for homeobox proteins include the control of plant regeneration, differentiation, and growth, processes. For example, genes capable of promoting regeneration of adventitious roots or adventitious shoots from undifferentiated cells or plant tissues would be useful for agricultural applications. In one such application, an *Arabidopsis* gene has been identified encoding a protein with a homeodomain which is involved in differentiation, specifically, it induces adventitious shoots and branching from cultured tissue (see PCT International Publication No. WO 01/07618, corresponding to European Patent No. 1 116 793). In another application, ectopic expression of a plant homeobox gene encoding a transcription factor involved in the metabolism of gibberellic acid and resulting in a delayed flowering phenotype was proposed for the production of genetically modified grasses that exhibit inhibition of flowering, absence of inflorescence, increased production of tillers, delayed heading, and inhibition of the developmental switch from vegetative to generative growth. These modified phenotypes represent agronomically valuable traits in grasses bred for both forage and amenity purposes (see European Patent Application No. EP0109570 EP).

Applications can also be envisioned for the individual proteins identified in this Example. For example, the rice putative eukaryotic translation initiation factor 3 large subunit (OsBAB07943) could be used to identify compounds that inhibit the binding of this plant initiation factor to the cap structure of its mRNAs. Such compounds could function as herbicides. A similar application has been proposed for a plant eukaryotic initiation factor 4E (eIF4E) (Canadian Patent Application No. CA0001412 CA, published July 6,2001).

### Example V

The example describes the identification and characterization of rice proteins that interact at the thylakoid of chloroplasts and other cellular membranes. Specifically, described in this example are newly characterized rice proteins interacting with the rice 14-3-3 protein homolog GF14-c (OsGF14-c) and with Defender Against Apoptotic Death 1 (OsDAD1).

The 14-3-3 proteins (reviewed in Muslin & Xing, 2000) interact with a variety of regulators of cellular signaling, cell cycle, and apoptosis by binding to their partner proteins. The high potential for specific protein-protein interactions makes these proteins suitable for two-hybrid assays. The 14-3-3 proteins are known to participate in protein complexes within the nucleus and are commonly found in the cytoplasm. Studies using yeast two-hybrid assays have also localized GF14 isoforms to the chloroplast stroma and the stromal side of thylakoid membranes (Sehnke et al., 2000). However, the subcellular localization of GF14-c had not been directly assessed to date. Investigation of the protein interactions involving OsGF14-c can lead to the identification of its location within the cell.

OsDAD1 is encoded by the rice homolog of the highly conserved DAD gene, a suppressor of endogenous programmed cell death, or apoptosis, in animals and plants (Apte et al., 1995; Gallois et al., 1997). In support of this role for DAD, expression of a DAD plant homolog has been shown to be down-regulated during flower petal senescence (an example of programmed cell death) and by the plant hormone ethylene, which is associated with a variety of stress responses and developmental processes (Orzaez & Granell, 1997). While these studies have been conducted with DAD homologs from *Arabidopsis* and pea, the rice DAD1 is not described in the literature. The interaction studies provided below were aimed at further characterizing this protein.

An automated, high-throughput yeast two-hybrid assay technology (as described above) was used to search for rice protein that interacted with the bait proteins OsGF14-c and OsDAD1. The sequences encoding the protein fragments used in the search were then compared by BLAST analysis against databases to determine the sequences of the full-length genes. The proteins found appear to be localized to the thylakoid of chloroplasts, vacuolar membrane, and plasma membrane. The results indicate that OsGF14-c is a membrane component in rice. The subset of proteins interacting with OsGF14-c at the thylakoid form a novel chloroplast protein complex involved in the photosynthetic processes. This interaction study also identifies the rice OsDAD1 as a membrane protein, in agreement with previously characterized DAD homologs from other species. Elucidation of the role of proteins interacting at the thylakoid and other cellular membranes in rice chloroplasts can allow the development of herbicides specifically targeted to disrupting the structure and function of the thylakoid or endomembrane system.

### Results

GF14-c was found to interact with EPSP synthase, an enzyme in the shikimate pathway (OsBAB61062); two enzymes with roles in the Calvin cycle reactions in chloroplasts, a rice chloroplastic aldolase (OsBAA02730) and a the chloroplast enzyme ribulose-1,5-bisphosphate carboxylase/oxygenase (RUBISCO; OsRBCL); the RUBISCO activase precursor (OsRCAA1); and two rice photosystem proteins, putative 33kDa oxygen-evolving protein of photosystem II (OsPN23059) and photosystem II 10 kDa polypeptide (OsAAB46718). Eight additional interactors for GF14-c are novel rice proteins: a photosystem protein (OsPN23061) similar to barley (*Hordeum vulgare*) photosystem I reaction center subunit II, chloroplast precursor; a protein (OsPN22858) similar to *Arabidopsis thaliana* GTP cyclohydrolase II, an enzyme involved in the biosynthesis of vitamin B riboflavin (a cofactor in the shikimate pathway); a protein (OsPN22874) similar to *A. thaliana* phosphatidylinositol-4-phosphate 5 kinase (PI4P5K), an enzyme involved in signaling events associated with water-stress response in plants; two H⁺-ATPases, similar to *A. thaliana* vacuolar ATP synthase subunit C (OsPN22866) and to barley plasma membrane H⁺-ATPase (OsPN23022); a putative dynamin homolog (OsPN30846) that is likely localized to the chloroplast, as are other plant dynamin family members; and two proteins of unknown function (OsPN29982 and OsPN30974).

OsDAD1 was found to interact with three membrane proteins: rice beta-expansin (OsEXPB2), which is localized to the plasma membrane adjacent to the cell wall; a novel putative phosphate cotransporter (OsPN23053); and the H⁺-ATPase-like protein OsPN23022 that also interacts with GF14-c.

The proteins that interacted with OsGF14-c (14-3-3 protein homolog GF14-c) and OsDAD1 are listed in Tables 21 and 22, respectively, followed by detailed information on each protein and a discussion of the significance of the interactions. A diagram of the interactions is provided in Figure 4. The nucleotide and amino acid sequences of the proteins of the Example are provided in SEQ ID NOs: 83-100 and 277-294.

Nine of the proteins identified represent rice proteins previously uncharacterized. Based on their presumed biological function and on the ability of the prey proteins to specifically interact with the bait proteins OsGF14-c and OsDAD1, it was speculated that OsGF14-c is a membrane component. Based on the results described below, OsGF14-c is presumably localized to the thylakoid of rice chloroplasts and to other cellular membranes. The proteins interacting in the thylakoid are part of a novel protein complex and are involved in the photosynthetic processes occurring in the chloroplasts. Knowledge of the role of proteins interacting at the thylakoid in rice could be exploited for the development of herbicides specifically targeted to disrupting the structure and function of the thylakoid membrane. The interactions found in this study also identify OsDAD1 as a likely membrane component in rice, an observation consistent with previous reports on other animal and plant DAD homologs.

**Table 21**

| Interacting Proteins Identified for OsGF14-c (14-3-3 protein homolog GF14-c) | | | |
|---|---|---|---|
| The names of the clones of the proteins used as baits and found as preys are given. Nucleotide/protein sequence accession numbers for the proteins of the Example (or related proteins) are shown in parentheses under the protein name. The bait and prey coordinates (Coord) are the amino acids encoded by the bait fragment(s) used in the search and by the interacting prey clone(s), respectively. The source is the library from which each prey clone was retrieved. | | | |
| **Gene Name** | **Protein Name** **(GENBANK® Accession No.)** | **Bait Coord** | **Prey Coord** **(source)** |
| **BAIT PROTEIN** | | | |
| OsGF14-c PN12464 (SEQ ID NO: 278) | *O. sativa* 14-3-3 Protein Homolog GF14-c (U65957) | 1-257# | |

| **INTERACTORS** | | | |
|---|---|---|---|
| OsBAB61062 PN22844 (SEQ ID NO: 280) | *O. sativa* 3-Phosphoshikimate 1-carboxyvinyltransferase (a.k.a. EPSP Synthase) (AB052962; BAB61062.1) | 1-150 | 463-511 (input trait) |
| OsPN22858 (SEQ ID NO: 84) | Novel Protein 22858, Fragment, similar to *Arabidopsis* GTP Cyclohydrolase II (BAB09512.1; e=0) | 1-150 | 27-154 (input trait) |
| OsPN22874 (SEQ ID NO: 86) | Novel Protein 22874, Fragment, similar to *Arabidopsis* Putative Phosphatidylinositol-4-phosphate 5-kinase (NP_187603.1; 4e⁻¹⁸) | 1-150 | 1-88 (input trait) |
| OsBAA02730 PN22832 (Contig4280.fasta.C ontig1) (SEQ ID NO: 282) | *O. sativa* Fructose-Bisphosphate Aldolase, Chloroplast Precursor (Q40677) | 1-150 | 206-269 (input trait) |
| OsRBCL PN23426 (SEQ ID NO: 284) | *O*. *sativa* Chloroplast Ribulose Bisphosphate Carboxylase, Large Chain (D00207; P12089) | 1-150 | 287-462 (input trait) |
| OsRCAA1 PN19842 (SEQ ID NO: 286) | *O*. *sativa* Ribulose Bisphosphate Carboxylase/Oxygenase Activase, Large Isoform A1 (AB034698, BAA97583) | 1-150 | 68-210 (input trait) |
| OsPN22866 (Contig388.fasta.Co ntig2) (SEQ ID NO: 88) | Novel Protein PN22866, Fragment, Similar to *A. Thaliana* Vacuolar ATP Synthase Subunit C (V-ATPase C subunit) (Vacuolar proton pump C subunit) (Q9SDS7; e⁻¹⁵²) | 1-150 | 95-305 (input trait) |
| OsPN23022*$* (SEQ ID NO: 90) | Novel Protein PN23022, Fragment, similar to *H. Vulgare* Plasma Membrane H⁺-ATPase (CAC50884; e=0.0) | 1-150 | 149-285 (input trait) |
| OsPN23061 (Contig3864.fasta.C ontig1) (SEQ ID NO: 92) | Hypothetical Protein OsContig3864, Similar to *H. vulgare* Photosystem I Reaction Center Subunit II, Chloroplast Precursor (P36213; 6e⁻⁸⁷) | 1-150 | 94-203 (input trait) |
| OsPN23059 (Contig4331.fasta.C ontig1) (SEQ ID NO: 288) | OsContig4331, *O*. *sativa* Putative 33kDa Oxygen-Evolving Protein of Photosystem II (BAB64069) | 1-150 | 193-333 90-169 (input trait) |
| OsAAB46718 PN22840 (FL_R01_003_H20.g .1a.Sp6a TMRI) (SEQ ID NO: 290) | *O*. *sativa* Photosystem II 10 kDa Polypeptide (U86018; T04177) | 1-150 | 82-126 (input trait) |
| OsPN29982 (SEQ ID NO: 94) | Novel Protein PN29982 | 1-150 | 201-300 (input trait) |
| OsPN30846 (SEQ ID NO: 96) | Novel Protein PN30846 | 1-150 | 1-266 (input trait) |
| OsPN30974 (SEQ ID NO: 98) | Novel Protein PN30974 | 1-150 | 38-178 (input trait) |

| | | | |
|---|---|---|---|
| NOTE: Interactions of GF14-c with the maize transcription factor Viviparous-1 (ZmVP1) and with Em binding protein (EmBp) are also reported in the literature (Schultz et al., 1998). # Self-activating clone, i.e., it activates the reporter genes in the two-hybrid system in the absence of a prey protein, and thus it was not used in the search. $ A prey clone of OsPN23022 also interacts with a clone of Defender Against Apoptotic Death 1 (QsDAD1) used as a bait, and the bait OsDAD1 interacts with Beta-Expansin EXPB2 (OsEXPB2) and with Novel Protein 23053, Fragment, Similar to *Arabidopsis* Putative Na+-Dependent Inorganic Phosphate Cotransporter (OsPN23053). These interactions are shown in Table 22 below. | | | |

**Table 22**

| Interacting Proteins Identified for OsDAD1 (Defender Against Apoptotic Death 1) | | | |
|---|---|---|---|
| **Gene Name** | **Protein Name** (**GENBANK® Accession No.)** | **Bait Coord** | **Prey Coord** **(source)** |
| **BAIT PROTEIN** | | | |
| OsDAD1 PN20251 (SEQ ID NO: 292) | *O*. *sativa* Defender Against Apoptotic Death 1 (D89727; BAA24104) | | |

| **INTERACTORS** | | | |
|---|---|---|---|
| OsPN23022 (SEQ ID NO: 90) | Novel Protein PN23022, Fragment, similar to *H. Vulgare* Plasma Membrane H⁺-ATPase (CAC50884; e=0.0) | 30-115 | 37-371 (input trait) |
| OsPN23053 (SEQ ID NO: 100) | Novel Protein 23053, Fragment, Similar to *Arabidopsis* Putative Na+-Dependent Inorganic Phosphate Cotransporter (NP_181341.1; e⁻¹⁰⁵) | 30-115 | 2x 1-180 (input trait) |
| OsEXPB2 PN19902 (SEQ ID NO: 294) | Beta-Expansin EXPB2 (U95968; AAB61710) | 1-115 | 80-207 (input trait) |
| | | 30-115 | 183-261 2x 80-218 (input trait) |

### Two-hybrid system using Os GF14-c as bait

GF14-c (GENBANK® Accession No. U65957) is a 256-amino acid protein that has been reported to interact with site-specific DNA-binding proteins (e.g., basic leucine zipper factor EmBP1) and tissue-specific regulatory factors (i.e., viviparous-1; VP-1; Schultz et al., 1998). It can act to form complexes with EmBP1 and VP-1 to mediate gene expression. The 14-3-3 proteins are found in virtually every eukaryotic organism and tissue and usually consist, in any given organism, of multiple protein isoforms (De Lille et al., 2001). They are thought to act as molecular scaffolds or chaperones and to regulate the cytoplasmic and nuclear localization of proteins with which they interact by regulating their nuclear import/export (Zilliacus et al., 2001; reviewed by Muslin & Xing, 2000. The 14-3-3 proteins bind to a multitude of functionally diverse regulatory proteins involved in cellular signaling pathways, cell cycling, and apoptosis. In plants, enzymes under the control of 14-3-3 proteins include starch synthase, Glu synthase, F1 ATP synthase, ascorbate peroxidase, and affeate o-methyl transferase, plasmamembrane H⁺-ATPase, light- and substrate-regulated metabolic enzymes of the nitrogen and carbon assimilation pathways, and those involved in transcriptional regulation such as the G-box complex and core transcription factors TBP, TFIIB, and EmBP. However, the specific 14-3-3 isoforms required by each of these pathways have not been fully characterized (De Lille et al., 2001). The 14-3-3 proteins have previously been detected as participants in protein complexes within the nucleus (Bihn et al., 1997; Imhof & Wolffe, 1999; Zilliacus et al., 2001), in the cytoplasm, and mitochondria (De Lille et al., 2001). Plant 14-3-3 proteins have also been localized to the chloroplast stroma and the stromal side of thylakoid membranes (Sehnke et al., 2000). However, subcellular localization of GF14-c has not been directly assessed and thus its location within the cell is yet to be precisely defined.

Analysis of the amino acid sequence of GF14-c identified a cAMP-and GMP-dependent phosphorylation site at amino acids 107 to 110, six protein kinase C phosphorylation sites (amino acids 10 to 12, 29 to 31, 56 to 61, 29 to 31, 59 to 61, and 74 to 76), three casein kinase II phosphorylation sites (amino acids 110 to 113, 120 to 123, and 177 to 180), an N-myristoylation site (amino acids 9 to 14), and two amidation sites (amino acids 77 to 80 and 105 to 108). The bait fragment used in this search encodes amino acids 1 to 150 of GF14-c. A BLAST analysis comparing the nucleotide sequence of GF14-c against TMRI's GENECHIP® Rice Genome Array sequence database identified probeset OS009195_at (e⁻⁴⁸expectation value) as the closest match. Gene expression experiments indicated that this gene is not specifically expressed in several different tissue types and is not specifically induced by a broad range of stresses, herbicides, and applied hormones.

The bait protein encoding amino acids 1 to 150 of GF14-c was found to interact with *O*. *sativa* 3-phosphoshikimate 1-carboxyvinyltransferase (also referred to as 5-enolpyruvylshikimate-3-phosphate (EPSP) synthase (EPSPS); OsBAB61062). OsBAB61062 is a 511-amino acid protein that contains an EPSP synthase signature 1 site (amino acids 162 to 176), an EPSP signature 2 site (amino acids 423 to 441), and it is alanine-rich at the N-terminus. A BLAST analysis of the amino acid sequence of OsBAB61062 determined that this protein is the rice 3-phosphoshikimate 1-carboxyvinyltransferase (also commonly referred to as EPSP synthase) (GENBANK® Accession No. BAB61062.1, 83.9% identity, e=0.0). This 511-amino acid enzyme is located in the chloroplasts where it catalyzes an essential step in aromatic amino acid synthesis, referred to as the shikimate pathway. Because EPSP synthase is essential to algae, higher plants, bacteria, and fungi, but not present in mammals, this enzyme is a useful herbicide and antimicrobial target.

A BLAST analysis comparing the nucleotide sequence of EPSP synthase against TMRI's GENECHIP® Rice Genome Array sequence database identified probeset OS020639.1_at (e⁻¹⁵⁶ expectation value) as the closest match. Gene expression experiments indicated that this gene is induced by jasmonic acid, a plant hormone involved in signal transduction events associated with a plant's stress response, and by *M. grisea,* the fungus that causes rice blast disease. The gene is repressed under drought conditions.

The bait protein encoding amino acids 1 to 150 of GF14-c was found to interact with protein 22858, a fragment which is similar to *A. thaliana* GTP cyclohydrolase II (OsPN22858). This prey clone of OsPN22858 is a 460-amino acid protein fragment with a transmembrane region spanning amino acids 182 to 198 and a possible cleavage site between amino acids 24 and 25, although no N-terminal signal peptide is present. A BLAST analysis of OsPN22858 determined that its amino acid sequence most nearly matches that of GTP cyclohydrolase II; 3,4-dihydroxy-2-butanone-4-phoshate synthase from *A. thaliana* (GENBANK® Accession No. BAB09512.1, 74.4% identity, e = 0). GTP cyclohydrolase II catalyzes the first committed reaction in the biosynthesis of the B vitamin riboflavin (Ritz et al., 2001).

A BLAST analysis comparing the nucleotide sequence of Novel Protein 22858 against TMRI's GENECHIP® Rice Genome Array sequence database identified OS015318_s_at (5e⁻¹⁰ expectation value) as the closest match. The expectation value is too low for this probeset to be a reliable indicator of the gene expression of this GTP cyclohydrolase.

The bait protein encoding amino acids 1 to 150 of GF14-c was found to interact with Protein 22874, a fragment that is similar to *A. thaliana* putative phosphatidylinositol-4-phosphate 5-kinase (OsPN22874). A BLAST analysis of OsPN22874 determined that its 89-amino acid sequence most nearly matches that of phosphatidylinositol-4-phosphate 5-kinase (PI4P5K) from *A. thaliana* (GENBANK® Accession No. NP_187603.1, 65.5% identity, 4e⁻¹⁸). PI4P5K is an enzyme that plays a well-defined role in many signaling events in many species, including the endoplasmic reticulum (ER) stress response in plants (Shank et al., 2001). Animal and yeast PI4P5K phosphorylates phosphatidylinositol-4-phosphate to produce phosphatidylinositol-4,5-bisphosphate as a precursor of two second messengers, inositol-1,4,5-triphosphate and diacylglycerol, and as a regulator of many cellular proteins involved in signal transduction and cytoskeletal organization (reviewed in Mikami et al., 1998). Mikami et al. identified a full-length cDNA clone encoding a PI4P5K protein in *A. thaliana* whose mRNA expression is induced by treatment of the plant with drought, salt and abscisic acid, suggesting that this protein is involved in water-stress signal transduction (Mikami et al., 1998). Elge et al. report that *A. thaliana* PI4P5K is expressed predominantly in vascular tissues of leaves, flowers and roots, namely in cells of the lateral meristem, i.e., the procambium (Elge et al., 2001). I

The bait protein encoding amino acids 1 to 150 of GF14-c was also found to interact with *O. sativa* fructose-bisphosphate aldolase, a chloroplast precursor (OsBAA02730). OsBAA02730 (GENBANK® Accession No. Q40677) is a 388-amino acid protein that includes a fructose-bisphosphate aldolase class-I active site (amino acids 44 and 388), as determined by analysis of the amino acid sequence (8.5e⁻²²⁸). A BLAST analysis of the amino acid sequence of OsBAA02730 indicated that this protein is the rice fructose-bisphosphate aldolase, chloroplast precursor (GENBANK® Accession No. Q40677). The gene encoding chloroplastic aldolase was isolated along with that encoding the cytoplasmic form of the enzyme (Tsutsumi et al., 1994). The chloroplastic aldolase is encoded at a single locus, while the cytoplasmic form is distributed between three loci on the genome. Aldolases are present in higher plants as two isoforms: the cytosolic and the chloroplastic types. The cytoplasmic form is highly conserved among plants and appears to be regulated through a Ca²⁺- mediated protein kinase/phosphatase pathway (Nakamura et al., 1996). This enzyme is though to have a role in the fruit ripening process (Schwab et al., 2001). The chloroplastic enzyme is involved in two major sugar phosphate metabolic pathways of green chloroplasts: the C3 photosynthetic carbon reaction cycle (Calvin cycle) and reactions of the starch biosynthetic pathway. In both cases, aldolase catalyzes the formation of fructose 1,6-biphosphate from dihydroxyacetone 3-phosphate and glyceraldehyde 3-phosphate. These topics are reviewed by Michelis et al., 2000, in which is described a 44-kDa heat-induced isoform of the fructose-bisphosphate aldolase in oat chloroplast, confirming its localization to the thylakoid membrane and suggesting that this enzyme is not embedded but rather tends to adhere to the chloroplast membranes. Similar heat-induced thylakoid-associated aldolase homologues were found in other plant species.

A BLAST analysis comparing the nucleotide sequence of the aldolase protein against TMRI's GENECHIP® Rice Genome Array sequence database identified probeset OS006916.1_at (e⁻¹⁵⁶ expectation value) as the closest match. Our gene expression experiments indicate that this gene is down-regulated by jasmonic acid and drought.

In addition, the bait protein encoding amino acids 1 to 150 of GF14-c was found to interact with *O*. *sativa* ribulose bisphosphate carboxylase large chain precursor (RUBISCOLarge Subunit; OsRBCL). A BLAST analysis of the amino acid sequence of OsRBCL determined that this protein is the rice chloroplast ribulose bisphosphate carboxylase, large chain precursor (RUBISCO; GENBANK® Accession No. P12089). RUBISCO is a 477-amino acid protein present in the chloroplast of higher plants, with an active site in position 196-204. The chloroplast RUBISCO is part of the CO₂-fixing multienzyme complexes bound to the thylakoid membrane (Suss et al., 1993) with roles in the Calvin cycle reactions that occur in the stroma of the chloroplast during photosynthesis. The starting and ending compound in the Calvin cycle is the five-carbon sugar ribulose 1,5-bisphosphate (RuBP). As its name indicates, RuBP carboxylase/oxygenase catalyzes two types of reactions that involve RuBP. In the presence of high carbon dioxide and low oxygen concentrations, the carboxylase activity of RUBISCO is favored and the enzyme catalyzes the initial reaction in the Calvin cycle, the carboxylation of RuBP, leading to the formation of 3-phosphoglyceric acid (PGA). However, in the presence of low carbon dioxide and high oxygen concentrations, oxygen competes with carbon dioxide as a substrate for RUBISCO and the enzyme's oxygenase activity also occurs, resulting in condensation of oxygen with RuBP to form 3-phosphoglycerate and phosphoglycolate. RUBISCO is the world's most abundant enzyme, accounting for as much as 40 percent of total soluble protein in leaves (these topics are discussed in Raven et al., 1999).

A BLAST analysis comparing the nucleotide sequence of the RUBISCO protein against TMRI's GENECHIP® Rice Genome Array sequence database identified probeset OS000296_s_at (e = 0 expectation value) as the closest match. Gene expression experiments indicated that this gene is down-regulated by BAP, 2,4-D, BL2, jasmonic acid, gibberellin, and abscisic acid. The gene is up-regulated under osmotic stress conditions.

The bait protein encoding amino acids 1 to 150 of GF14-c was found to interact with *O. sativa* ribulose bisphosphate carboxylase/oxygenase activase, large isoform A1 (OsRCAA1). A BLAST analysis of the amino acid sequence of OsRCAA1 determined that this 466-amino acid protein is the rice RUBISCO activase large isoform precursor (GENBANK® Accession No. BAA97583). It contains two active sites (amino acid 31 to 38 and 156 to 163). RUBISCO activase is an AAA+ (ATPases associated with a variety of cellular activities) protein that facilitates the ATP-dependent removal of sugar phosphates from RUBISCO active sites. This action frees the active site of RUBISCO for spontaneous carbamylation by CO2 and metal binding, prerequisites for activity (reviewed in Salvucci et al., 2001; Salvucci & Ogren, 1996).

The bait protein encoding amino acids 1 to 150 of GF14-c was found to interact with protein PN22866, a fragment similar to *A. thaliana* vacuolar ATP synthase subunit C (V-ATPase C subunit; vacuolar proton pump C subunit; OsPN22866). OsPN22866 is a 408-amino acid protein fragment. Its amino acid sequence most nearly matches that of *A*. *thaliana* Vacuolar ATP synthase subunit C (V-ATPase C subunit; Vacuolar proton pump C subunit; Q9SDS7, 72.7% identity, e⁻¹⁵²), as determined by BLAST analysis. The H⁺-translocating ATPases (H⁺-ATPase, V-ATPase) are multi-subunit enzymes that function as essential proton pumps in eukaryotes. The catalytic site of human V-ATPase consists of a hexamer of three A subunits and three B subunits that bind and hydrolyze ATP and are regulated by accessory subunits C, D and E (van Hille et al., 1993).

ATPases are essential cellular energy converters that transduce the chemical energy of ATP hydrolysis from transmembrane ionic electrochemical potential differences. The plant ATPases are present in chloroplasts, mitochondria and vacuoles. In vacuoles, ATPases regulate the contents and volume of vacuoles, which depends on the coordinated activities of transporters and channels located in the tonoplast (vacuolar membrane). The V-ATPase uses the energy released during cleavage of the phosphate group of cytosolic ATP to pump protons into the vacuolar lumen, thereby creating an electrochemical H⁺-gradient that is the driving force for transport of ions and metabolites. Thus V-ATPase is important as a 'house-keeping' and as a stress response enzyme. Expression of V-ATPase has been shown to be highly regulated depending on metabolic conditions. The V-ATPase consists of several polypeptide subunits that are located in two major domains, a membrane peripheral domain (V₁) and a membrane integral domain (Vₒ). Subunit C is a highly hydrophobic protein containing four membrane-spanning domains. The function of subunit C is unknown, although it is suggested to be directly involved in H⁺ transport and might be involved in stabilization of V₁. The structure, function and regulation of the plant V-ATPase are reviewed in Ratajczak R., 2000.

The bait protein encoding amino acids 1 to 150 of GF14-c was also found to interact with protein PN23022, a fragment similar to *H. Vulgare* plasma membrane H⁺-ATPase (OsPN23022). Protein PN23022 is a 534-amino acid fragment that includes seven transmembrane domains (amino acids 170 to 186, 202 to 218, 226 to 242, 266 to 282, 308 to 324, 337 to 353, and 373 to 389), as predicted by analysis of its amino acid sequence. A BLAST analysis of the amino acid sequence of OsPN23022 determined that this protein is similar to *H. vulgare* plasma membrane H⁺-ATPase (GENBANK® Accession No. CAC50884; 88.2% identity, e = 0 expectation value), an enzyme that translocates protons into intracellular organelles or across the plasma membrane of eukaryotic cells. A BLAST analysis comparing the nucleotide sequence of Novel protein PN23022 against TMRI's GENECHIP® Rice Genome Array sequence database identified OS000972_f_at (e⁻¹¹ expectation value) as the closest match. The expectation value is too low for this probeset to be a reliable indicator of the gene expression of this ATPase. OsPN23022 was also found to interact with Defender Against Apoptotic Death 1 (OsDAD1; see Table 22).

The bait protein encoding amino acids 1 to 150 of GF14-c was found to interact with protein OsContig3864, which is similar to *H. vulgare* photosystem I reaction center subunit II, chloroplast precursor (OsPN23061). Analysis of the OsContig3864 amino acid sequence predicted that it is a 203-amino acid protein containing a possible cleavage site between amino acids 21 and 22, although there appears to be no N-terminal signal peptide. A BLAST analysis determined that the OsContig3864 clone has an amino acid sequence that most nearly matches that of *H. vulgare* photosystem I reaction center subunit II, chloroplast precursor (Photosystem I 20 kDa subunit; PSI-D; GENBANK® Accession No. P36213, 80% identity, 3e⁻⁸⁶). The photosystems (photosystems I and II) are large multi-subunit protein complexes embedded into the photosynthetic thylakoid membrane. They operate in series and catalyze the primary step in oxygenic photosynthesis, the light-induced charge separation process by which light energy from the sun is converted to carbon dioxide and carbohydrates in plants and cyanobacteria. Photosystem I catalyzes the light-induced electron transfer from plastocyanin/cytochrome c₆ on the lumenal side of the membrane (inside the thylakoids) to ferredoxin/flavodoxin at the stromal side by a chain of electron carriers (reviewed in Fromme et al., 2001).

A BLAST analysis comparing the nucleotide sequence of OsContig3864 against TMRI's GENECHIP® Rice Genome Array sequence database identified probeset OS000721_at (e = 0 expectation value) as the closest match. Gene expression experiments indicated that this gene is not specifically expressed in several different plant tissue types and is not specifically induced by a broad range of stresses, herbicides, and applied hormones.

The bait protein encoding amino acids 1 to 150 of GF14-c was also found to interact with OsContig4331, an *O*. *Sativa* putative 33kDa oxygen-evolving protein of photosystem II (OsPN23059). The two prey clones retrieved from the input trait library encode amino acids 193 to 333 and 90 to 169 of OsContig4331. These clones are non-overlapping, suggesting that multiple GF14-c-binding sites exist within OsContig4331. Analysis of the OsContig4331 protein sequence predicted that it codes for a 333-amino acid protein. The analysis also indicated that OsContig 4331 contains a possible cleavage site between amino acids 37 and 38, although no N-terminal signal peptide is evident. A BLAST analysis of the OsContig 4331 amino acid sequence determined that this protein is the rice putative 33kDa oxygen-evolving protein of photosystem II (GENBANK® Accession No. BAB64069, 90.6% identity, e⁻¹⁶⁹). Photosystem II uses photooxidation to convert water to molecular oxygen, thereby releasing electrons into the photosynthetic electron transfer chain.

A BLAST analysis comparing the nucleotide sequence of OsContig4331, rice Photosystem I Reaction Center Subunit II Precursor against TMRI's GENECHIP® Rice Genome Array sequence database identified probeset OS000372_at (e = 0 expectation value) as the closest match. The gene expression experiments disclosed herein indicate that this gene is down-regulated during cold stress.

The bait protein encoding amino acids 1 to 150 of GF14-c was also found to interact with *O*. *Sativa* photosystem II 10 kDa polypeptide (OSAAB46718). OSAAB46718 is a 126-amino acid protein fragment that includes a predicted transmembrane domain (amino acids 102 to 118). A BLAST analysis against the Genpept database revealed that OsAAB46718 is the *Oryza sativa* photosystem II 10kDa polypeptide (GENBANK® Accession No. T04177, 91.2% identity, 2e⁻⁶¹).

The bait protein encoding amino acids 1 to 150 of GF14-c was also found to interact with protein PN29982 (OsPN29982). The 300-amino acid sequence of the protein OsPN29982 most nearly matches that of a putative protein of unknown function from *A. thaliana* (GENBANK® Accession No. NP_196688.1, 47% identity, 3e-054), as determined by BLAST analysis. The second best match was CHICK LIM/homeobox protein Lhx1 (Homeobox protein LIM-1; GENBANK® Accession No. P53411, 28% identity, e = 0.002). Based on the homeoboxdomain, this interaction can be similar to 14-3-3 protein interactions with transcription factors like VP1.

The bait protein encoding amino acids 1 to 150 of GF14-c was also found to interact with protein PN30846 (OsPN30846). A BLAST analysis of protein OsPN30846 determined that its 266-amino acid sequence most nearly matches that of dynamin homolog from the leguminous plant *Astragalus sinicus* (GENBANK® Accession No. AAF19398.1, 70.6% identity, 2e⁻⁹⁹). Since the discovery of the GTP-binding dynamin in rat brain, dynamin-like proteins have been isolated from various organisms and tissues and shown to be involved in diverse and seemingly unrelated biological processes. Many different isoforms of dynamin-like proteins have been identified in plant cells, and these plant homologs can be grouped into several subfamilies, such as G68/ADL1, ADL2 and ADL3, based on their amino acid sequence similarity (reviewed in Kim et al., 2001). The biological roles have been characterized for a few of these plant dynamin-like proteins. The dynamin-like protein ADL1 from *Arabidopsis* has been shown to be localized to and to be involved in biogenesis of the thylakoid membranes of chloroplasts (Park et al., 1998). Another *Arabidopsis* dynamin-like protein, ADL2, is targeted to the plastid, and its recombinant form expressed in *E*. *coli* binds specifically to phosphatidylinositol 4-phosphate through the pleckstrin homology (PH) domain present in ADL2 (Kim et al., 2001). Based on the similarity between the biochemical properties of ADL2 and those of dynamin and other related proteins, ADL2 can be involved in vesicle formation at the chloroplast envelope membrane.

The bait protein encoding amino acids 1 to 150 of GF14-c was also found to interact with protein PN30974 (OsPN30974). A BLAST analysis of the novel protein OsPN30974 determined that its 476-amino acid sequence most nearly matches that of an *Arabidopsis* hypothetical protein of unknown function (GENBANK® Accession No. NP_173623.1, 49% identity, e⁻¹³⁷). The next 13 best hits with an expectation value <e⁻¹⁵ are all *Arabidopsis* or rice proteins of unknown function annotated in the public domain.

### Two-hybrid system using OsDAD1 as bait

A second bait protein, namely *O*. *sativa* Defender Against Apoptotic Death 1 (OsDAD1), was used to identify interactors. OsDAD1 (GENBANK® Accession No. BAA24104) is a 114-amino acid protein that includes three predicted transmembrane domains (amino acids 33 to 49, 59 to 75, and 94 to 110). DAD1 is a suppressor of programmed cell death, or apoptosis, a process in which unwanted cells are eliminated during growth and development. DAD is a highly conserved protein with homologs identified in animals and plants (Apte et al., 1995; Gallois *et al*, 1997). Dysfunction and down-regulation of this gene has been linked to programmed cell death in these organisms (Lindholm et al., 2000). DAD1 is an essential subunit of the oligosaccharyltransferase that is located in the ER membrane (Lindholm et al., 2000). DAD1 expression declines dramatically upon flower anthesis disappearance in senescent petals and is down-regulated by the plant hormone ethylene (Orzaez & Granell, 1997), which is involved in a variety of stress responses and developmental processes including petal senescence (Shibuya et al., 2000), cell elongation, cell fate patterning in the root epidermis, and fruit ripening (Ecker, 1995).

Two clones, encoding amino acids 1-115 and 30-115 of OsDAD1, were used as baits in this Example.

OsDAD1 was found to interact with protein 23053, a fragment which is similar to *Arabidopsis* putative Na⁺-dependent inorganic phosphate cotransporter (OsPN23053). OsPN23053 is a protein fragment; however, its available 379-amino acid sequence contains five predicted transmembrane regions (amino acids 100 to 116, 118 to 134, 226 to 242, 259 to 275, and 324 to 340) and a cleavable signal peptide (amino acids 1 to 46). A BLAST analysis determined that OsPN23053 is similar to an *Arabidopsis* putative Na⁺-dependent inorganic phosphate cotransporter (GENBANK® Accession No. NP_181341.1, 55.4% identity, e⁻¹⁰⁵). In mammals, Na⁺-dependent inorganic phosphate cotransporter is present in neuronal synaptic vesicles and endocrine synaptic-like microvesicles as a vesicular glutamate transporter and is responsible for storage of glutamate, the major excitatory neurotransmitter in the mammalian central nervous system (CNS; Takamori et al., 2000). At least two isoforms of Na⁺-dependent inorganic phosphate cotransporter exist (Takamori et al., 2000; Aihara et al., 2000) and are expressed in pancreas and brain (Hayashi et al., 2001; Fujiyama et al., 2001). OsPN23053 is the first of a family of Na⁺-dependent inorganic phosphate cotransporters to be discovered in rice. Plants utilize glutamate in important biological processes including protein synthesis and glutamate-mediated signaling (Lacombe et al., 2001). The formation of glutamate from glutamine during nitrogen recycling (Singh et al., 1998) and the control of nitrogen assimilatory pathways by light-signaling (Oliveira et al., 2001) in plants suggest a link between glutamate formation and light-signal transduction.

OsDAD1 was found to interact with beta-expansin EXPB2 (OsEXPB2). A BLAST analysis of the amino acid sequence of OsEXPB2 determined that this protein is rice beta-expansin (GENBANK® Accession No. AAB61710, 99.6% identity, e⁻¹⁵⁶). Expansins promote cell wall extension in plants. Shcherban et al. isolated two cDNA clones from cucumber that encode expansins with signal peptides predicted to direct protein secretion to the cell wall (Shcherban et al., 1995). These authors identified at least four distinct expansin cDNAs in rice and at least six in *Arabidopsis* from collections of anonymous cDNAs (i.e. Expressed Sequence Tags; ESTs). They determined that expansins are highly conserved in size and sequence and suggest that this multigene family formed before the evolutionary divergence of monocotyledons and dicotyledons. Their analyses indicate no similarities to known functional domains that might account for the action of expansins on wall extension, though a series of highly conserved tryptophans can mediate expansin binding to cellulose or other glycans.

### Summary

The thylakoid membrane of the chloroplasts contains the photosynthetic pigments, reaction centres, and electron transport chains associated with photosynthesis. Localization of OsGF14-c to this site is consistent with the interactions of OsGF14-c with the photosystem proteins of this Example. The photosystems (photosystems I and II) are large multi-subunit protein complexes embedded in the thylakoid membrane. As part of a larger group of protein-pigment complexes, the photosynthetic reaction centers, they catalyze the light-induced charge separation associated with photosynthesis. Both photosystems use the energy of photons from sunlight to translocate electrons across the thylakoid membrane via a chain of electron carriers. The electron transfer processes are coupled to a build-up of a difference in proton concentration across the thylakoid membrane. The resulting electrochemical membrane potential drives the synthesis of ATP, which is used to reduce CO₂ to carbohydrates in the subsequent dark reactions. OsGF14-c is found to interact with OsContig3864, similar to photosystem I reaction center subunit II, chloroplast precursor, with OsContig4331, the rice putative 33kDa oxygen-evolving protein of photosystem II, and with rice photosystem II 10 kDa polypeptide. The validity of these interactions is supported by results in a report by Sehnke et al., 2000, which reported the use of yeast two-hybrid technology to identify an interaction between a plant 14-3-3 protein and another photosystem I subunit protein, *A. thaliana* photosystem I N-subunit At pPSI-N. The interactions of OsGF14-c with OsPN23061 (OsContig3864), OsPN23059 (OsContig4331), and OsAAB46718 (photosystem II 10 kDa polypeptide) suggest that OsGF14-c has a role in coupling the physical contact between proteins in or on the periphery of thylakoid membranes.

Given the interactions of OsGF14-c and components of the chloroplast photosystem, some of the other proteins found to interact with OsGF14-c in this study are likely to be localized to the chloroplast as well, and they are possibly co-located to the thylakoid membrane as interaction complexes. For example, OsGF14-c interacts with EPSP synthase (OsBAB61062), a shikimate pathway enzyme located in the chloroplast, where aromatic amino acid synthesis initiates. It is interesting to note that an enzyme in the shikimate pathway requires a flavin as a cofactor (Bornemann et al., 1996) and that OsGF14-c also interacts with OsPN22858, a novel protein fragment similar to *A. thaliana* GTP cyclohydrolase II. GTP cyclohydrolase II participates in the biosynthesis of the B vitamin riboflavin, which is a cofactor for enzymes functioning in the shikimate pathway. The interactions of these proteins with OsGF14-c can keep key proteins of the shikimate pathway in close proximity in or at the thylakoid. The interactions of OsGF14-c with chloroplastic aldolase (OsBAA02730), an enzyme shown to be localized to the thylakoid membrane and involved in the sugar phosphate metabolic pathway of chloroplasts, and with the Calvin cycle enzyme RUBISCO (OsRBCL) and RUBISCO activase large isoform precursor (OsRCAA1) further support localization of OsGF14-c and these interactors to the thylakoid membrane. Previous reports have identified a fructose-bisphosphate aldolase isoform at the thylakoid membrane in oat chloroplasts (Michelis et al., 2000).

In addition, a novel interactor identified for OsGF14-c is a putative dynamin homolog (OsPN30846). Plant dynamin-like proteins have been localized to the thylakoid and envelope membranes of chloroplasts (Park et al., 1998; Kim et al., 2001). Thus it is likely that this rice dynamin homolog is a membrane protein that resides in the chloroplast. This and the fact that other interactors identified for OsGF14-c are present in the thylakoid of chloroplasts substantiates the notion that the 14-3-3 protein functions as a component of the thylakoid or envelope membrane of chloroplasts. In further support of this hypothesis, a recombinant *Arabidopsis* dynamin-like protein member of the ADL2 subfamily binds specifically to phosphatidylinositol 4-phosphate. The interactions between dynamins and phosphoinositides documented in the literature (reviewed in Kim et al., 2001) are consistent with the concomitant presence of the dynamin-like protein OsPN30846 and the phosphatidylinositol-4-phosphate 5-kinase OsPN22874 (rice PI4P5K), both interacting with OsGF14-c, at the thylakoid. The interactors described above might be part of a protein complex involved in the photosynthetic processes at the thylakoid membrane.

In addition to components of the chloroplast thylakoid, OsGF14-c was found to interact with proteins similar to a plasma membrane H⁺-ATPase (OsPN23022) and to a vacuolar ATPase (OsPN22866), which suggests that OsGF14-c is also present in plasma and vacuolar membranes. The interactions of OsGF14-c with the ATPases can represent 14-3-3 regulation of the plant turgor pressure. This hypothesis is corroborated by reports of 14-3-3 proteins accomplishing this function via regulation of at least one form of a plasma membrane H+ ATPase (reviewed in DeLille et al., 2001). The interaction of the vacuolar ATPase with OsGF14-c can occur in the vacuolar membrane, but also in membranes of the ER, Golgi bodies, coated vesicles, and provacuoles.

The biological significance of the interaction of OsGF14-c with the novel protein OsPN22874 (rice PI4P5K) can be defined based on functional homology with *A. thaliana* PI4P5K, which is induced under water-stress conditions and is expressed in leaves. Given the interaction of OsGF14-c with components of the thylakoid and vacuolar membranes, the rice PIP5K can be located in the chloroplast but it can also reside at the vacuole, with the vacuolar ATPase. In either case, the rice PIP5K can direct synthesis of molecules involved in kinase signaling events associated with chloroplast protection or vacuole size regulation under abiotic stress.

Two additional interactors, OsPN29982 and OsPN30974, found for OsGF14-c are proteins of unknown function. Nevertheless, because 14-3-3 proteins acts as chaperones, these interactions can represent a process in which the prey proteins achieve proper protein folding, or OsGF14-c can be responsible for proper subcellular localization of OsPN29982 and OsPN30974. Because all other interactors for OsGF14-c appear to be membrane-associated proteins, OsPN29982 and OsPN30974 are likely to be membrane proteins and can reside at the thylakoid or other cellular membrane structures.

In summary, some of the rice proteins found to interact with OsGF14-c appear to be located at the thylakoid membrane where they participate in photosynthetic processes occurring in the chloroplast; these interactions are consistent with previously reported localization of 14-3-3 proteins to the chloroplast stroma and the stromal side of thylakoid membranes (Sehnke et al., 2000). Other interactors identified are associated with the plasma or vacuolar membrane. OsGF14-c is, thus, likely to be a membrane component in rice. Because 14-3-3 proteins participate in many types of signaling pathways and are thought to act as molecular chaperones necessary for the assembly, unfolding or transport of proteins through membranes, it is likely that OsGF14-c functions as a molecular glue or stabilizer to regulate the function of the proteins with which it interacts at the thylakoid or other membrane structures. The identification of OsGF14-c as a membrane component represents a novel observation and the first functional characterization of the GF14-c protein in rice. In particular, the proteins identified in this Example as interacting at the thylakoid membrane of chloroplasts represent a novel rice protein complex.

Three interactors were identified in this study for OsDAD1. One is the putative plasma membrane H⁺-ATPase (OsPN23022) that interacts with OsGF14-c. Evidence exists that both OsDAD1 and H⁺-ATPase are integral membrane proteins (Lindholm et al., 2000; Ratajczak et al., 2000). H⁺-ATPase translocates protons into intracellular organelles or across the plasma membrane of specialized cells, its activity resulting in acidification of intracellular compartments in eukaryotic cells. The acidic interior of lysosomes has been shown to be necessary for apoptosis under some conditions (Kagedal et al., 2001; Bursch, 2001). Thus, the activities of these two enzymes can be necessary for regulation of programmed cell death, and their physical interaction can represent a step in control of this event. Furthermore, 14-3-3 proteins have been implicated in regulation of many cellular processes including apoptosis (van Hemert et al., 2001). It is possible that the interactions of OsPN23022 with GF14-c and with OsDAD1 represent steps in such regulation.

Another novel interactor found for OsDAD1 is the novel rice Na⁺- dependent inorganic phosphate cotransporter. The rice phosphate cotransporter might also be a membrane protein based on functional homology with its mammalian homologs, which are localized to neuronal and endocrine vesicles and have a role in glutamate storage (Takamori et al., 2000). It is likely that glutamate participates in apoptosis regulation in plants as it does in mammals (Bezzi et al., 2001), and that this occurs in rice through the association of the phosphate cotransporter OsPN23053 with OsDAD1.

Finally, OsDAD1 was found to interact with the rice beta-expansin. Expansins are localized to the plasma membrane adjacent to the cell wall, from which they mediate cell wall extension. Since genes regulating cell death are part of the defense response, this interaction can be associated with structural changes in the cell wall in response to cell death.

The interactions here reported represent the first characterization of the DAD1 protein homolog in rice. Notably, the fact that OsDAD1 and its interactors appear to be membrane proteins and that one of them, OsPN23022, interacts with OsGF14-c lend further support to the notion that OsGF14-c is a membrane component.

### Example VI

The rice senescence-associated protein (Os006819-2510) shares 61.4% amino acid sequence similarity with daylily Senescence-Associated Protein 5, a protein encoded by one (DSA5) of six cDNA sequences the levels of which increase during petal senescence. Transcripts of these genes are found predominantly in petals, their expression increase during petal but not leaf senescence, and they are induced by a concentration of abscisic acid (ABA) that causes premature senescence of the petals. Petal senescence is an example of endogenous programmed cell death, or apoptosis, a process in which unwanted cells are eliminated during growth and development. Genes performing a regulatory function in cell death or survival are important to developmental processes. The rice senescence-associated protein Os006819-2510 was chosen as a bait for these interaction studies based on its potential relevance to plant growth and development.

To identify proteins that interacted with the rice senescence-associated protein Os006819-2510, an automated, high-throughput yeast two-hybrid assay technology (provided by Myriad Genetics Inc., Salt Lake City, Utah, United States of America) was employed, as has been described above.

### Results

The rice senescence-associated protein Os006819-2510 was found to interact with eight rice proteins. Five interactors are known, namely, the rice histone deacetylase HD1 (OsAAK01712), an enzyme involved in regulation of core histone acetylation; the calcium-binding protein calreticulin precursor (OsCRTC), which also interacts with the starch biosynthetic enzyme soluble starch synthase (OsSSS) and with a novel protein (OsPN29950) of unknown function; low temperature-induced protein 5 (OsLIP5); the dehydrin RAB 16B, which is induced by water stress; and rice putative myosin (OsPN23878), an actin motor protein which also interacts with a putative calmodulin-kinase that is associated with a network of proteins involved in cell cycle regulation (see Examples I and II). Three interactors for senescence-associated protein are novel proteins including a putative callose synthase (OsPN23226), an enzyme involved in the biosynthesis of the glucan callose; a protein similar to barley coproporphyrinogen III oxidase, chloroplast precursor, an enzyme of the chlorophyll biosynthetic pathway (OsPN23485); and a protein similar to *Arabidopsis* Gamma Hydroxybutyrate Dehydrogenase.

The interacting proteins of this Example are listed in Table 23, followed by detailed information on each protein and a discussion of the significance of the interactions. The nucleotide and amino acid sequences of the proteins of the Example are provided in SEQ ID NOs: 101-106 and 295-306.

Note that several prey proteins identified are, like the bait protein Os006819-2510, membrane-associated molecules (OsCRTC, OsPN23226, OsLIP5). Several appear to be associated with cell cycle processes in rice (OsPN23878, Os003118-3674, OsCRTC, OsSSS, OsPN23226, OsAAK01712), while others are involved in the plant stress response (OsRAB16B, OsLIP5, OsCRTC). Some of the proteins identified represent rice proteins previously uncharacterized. Based on the presumed biological function of the prey proteins and on their ability to specifically interact with the bait protein Os006819-2510, Os006819-2510 is speculated to be involved in cell cycle/mitotic processes and in the plant resistance to stress, and can actually represents a link between these processes in rice.

Proteins that participate in cell cycle regulation in rice can be targets for genetic manipulation or for compounds that modify their level or activity, thereby modulating the plant cell cycle. The identification of genes encoding these proteins can allow genetic manipulation of crops or application of compounds to effect agronomically desirable changes in plant development or growth. Likewise, genes that are involved in conferring plants resistance to stress have important commercial applications, as they could be used to facilitate the generation and yield of crops.

**Table 23**

| Interacting Proteins Identified for Os006819-2510 (Hypothetical Protein 006819-2510, Similar to *Hemerocallis* Senescence-Related Protein 5) | | | |
|---|---|---|---|
| The names of the clones of the proteins used as baits and found as preys are given. Nucleotide/protein sequence accession numbers for the proteins of the Example (or related proteins) are shown in parentheses under the protein name. The bait and prey coordinates (Coord) are the amino acids encoded by the bait fragment(s) used in the search and by the interacting prey clone(s), respectively. The source is the library from which each prey clone was retrieved. | | | |
| **Gene Name** | **Protein Name** **(GENBANK® Accession No.)** | **Bait Coord** | **Prey Coord** **(source)** |
| **BAIT PROTEIN** | | | |
| Os006819-2510 PN20462 (SEQ ID NO: 296) | Hypothetical Protein 006819-2510, Similar to Senescence-Related Protein 5 from *Hemerocallis* Hybrid Cultivar (AAC34855.1; e⁻⁹⁷) | | |

| **INTERACTORS** | | | |
|---|---|---|---|
| OsAAK01712 PN24059 (SEQ ID NO: 298) | *O. sativa* Histone Deacetylase HD1 (AF332875; AAK01712.1) | 1-150 | 90-221 (output trait) |
| OsCRTC* PN20544 (SEQ ID NO: 300) | *O. sativa* Calreticulin Precursor (AB021259; BAA88900) | 1-273 | 283-301 (output trait) |
| OsLIP5 PN22883 (SEQ ID NO: 302) | *Oryza sativa* Low Temperature-Induced Protein 5 (AB011368; BAA24979.1) | 1-150 | 29-60 (input trait) |
| OsPN23878# (SEQ ID NO: 304) | *Oryza sativa* Putative Myosin (AC090120; AAL31066.1) | 1-150 | 685-888 (output trait) |
| OsRAB16B PN20554 (SEQ ID NO: 306) | *O. sativa* DEHYDRIN RAB 16B (P22911) | 1-273 | 147-164 (output trait) |
| OsPN23226 (SEQ ID NO: 102) | Novel Protein PN23226, Callose synthase | 1-273 | 345-432 (output trait) |
| OsPN23485 (SEQ ID NO: 104) | Novel Protein PN23485, Similar to *Hordeum vulgare* Coproporphyrinogen III Oxidase, chloroplast precursor (Q42840; e⁻¹⁶⁹) | 1-273 | 90-243 (output trait) |
| OsPN29037 (SEQ ID NO: 106) | Novel Protein PN29037 | 1-150 | 73-165 (input trait) |

| | | | |
|---|---|---|---|
| * Additional interactions identified for OsCRTC are listed in Table 24 # Additional interactions identified for OsPN23878 are listed in Table 25 | | | |

**Table 24**

| Interacting Proteins Identified for OsCRTC (Calreticulin Precursor) | | | |
|---|---|---|---|
| **Gene Name** | **Protein Name** **(GENBANK® Accession No.)** | **Bait Coord** | **Prey Coord** **(source)** |
| **BAIT PROTEIN** | | | |
| OsCRTC PN20544 (SEQ ID NO: 300) | Calreticulin Precursor (AB021259; BAA88900) | | |

| **INTERACTORS** | | | |
|---|---|---|---|
| OsPN29950 (SEQ ID NO: 108) | Novel Protein PN29950 | 1-150 | 7-103 2x 138-343 50-343 (output trait) |
| OsSSS PN19701 (SEQ ID NO: 308) | Soluble Starch Synthase (AF165890; AAD49850) | 250-425 | 68-270 (input trait) 97-263 (output trait) |

**Table 25**

| Interacting Proteins Identified for OsPN23878 (Putative Myosin) | | | |
|---|---|---|---|
| **Gene Name** | **Protein Name** **(GENBANK® Accession No.)** | **Bait Coord** | **Prey Coord** **(source)** |
| **PREY PROTEIN** | | | |
| OsPN23878 (SEQ ID NO: 304) | *Oryza sativa* Putative Myosin (AC090120; AAL31066.1) | | |

| **BAIT PROTEIN** | | | |
|---|---|---|---|
| Os003118-3674 PN20551 (SEQ ID NO: 110) | Hypothetical Protein 003118-3674 Similar to *Lycopersicon esculentum* Calmodulin | 75-149 | 824-935 (output trait) |

Os006819-2510 is a 276-amino acid protein that includes a cleavable signal peptide (amino acids 1 to 27) and three transmembrane domains (amino acids 48 to 64, 82 to 98, and 233 to 249), as predicted by analysis of its amino acid sequence. The analysis also predicted two endoplasmic reticulum retention motifs, one N-terminal (AFRL) and the other C-terminal (KGGY), and a prokaryotic membrane lipoprotein lipid attachment site beginning with amino acid 57 (Prosite). This site, when functional, is a region of protein processing. Analysis by Pfam also identified a transmembrane superfamily domain, also called a tetraspanin family domain, typically found in a group of eukaryotic cell surface antigens that are evolutionarily related and include transmembrane domains.

A BLAST analysis against the Genpept database indicated that Os006819-2510 is similar to Senescence-Associated Protein 5 from *Hemerocallis* hybrid cultivar (daylily; GENBANK® Accession, No. AAC34855.1; 61.4% identity; e⁻⁹⁷). In agreement with this result, the protein with the amino acid sequence most similar (63% identity) to that of Os006819-2510 in Myriad's proprietary database is Hypothetical Protein 005991-3479, Similar to *Hemerocallis* Senescence-Associated Protein 5 (Os005991-3479). In an effort to identify the components of the genetic program that leads daylily petals to senescence and cell death ca. 24 hours after the flower opens, the cDNA encoding senescence-associated protein 5 in petals was isolated as one of six cDNAs (designated DSA3, 4, 5, 6, 12 and 15) whose levels increase during petal senescence (Panavas et al., 1999). However, no sequence homology was identified in the public database for the DSA5 gene product, which remains as yet unidentified. The levels of DSA mRNAs in leaves was determined to be less than 4% of the maximum detected in petals, with no differences between younger and older leaves, and the DSA genes (except DSA12) are expressed at low levels in daylily roots and (except DSA4) induced by a concentration of abscisic acid that causes premature senescence of the petals.

Two bait fragments, encoding amino acid 1-273 and 1-150, of Os006819-2510 were used in the yeast two-hybrid screen.

A bait fragment encoding amino acids 1-150 of Os006819-2510 was found to interact with *O. sativa* histone deacetylase HD1 (OsAAK01712). A BLAST analysis of the amino acid sequence of OsAAK01712 indicated that this prey protein is the rice Histone Deacetylase HD1 (GENBANK® Accession No. AAK01712.1, 100% identity, e = 0.0). Histone deacetylase (HD) enzymes have been isolated from plants, fungi and animals (reviewed by Lechner et al., 1996). The enzymatic activity of histone deacetylase and that of histone acetyltransferase maintain the enzymatic equilibrium of reversible core histone acetylation. Core histones are a group of highly conserved nuclear proteins in eukaryotic cells; they represent the main component of chromatin, the DNA-protein complex in which chromosomal DNA is organized. Besides their role in chromatin structural organization, core histones participate in gene regulation, their regulatory function being ascribed to their ability to undergo reversible posttranslational modifications such as acetylation, phosphorylation, glycosylation, ADP-ribosylation, and ubiquitination. Histone deacetylase exists as multiple enzyme forms, and this multiplicity reflects the complex regulation of core histone acetylation. Four nuclear HDs have been identified and characterized from germinating maize embryos (HD1-A, HD1-BI, HD1-BII, and HD2), based on their expression during germination, molecular weight, physiochemical properties and inhibition by various compounds. Based on these data, Lechner et al., 1996, suggest that HD enzymes have a role in establishing and maintaining histone-protein interactions, and that acetylation can modulate the binding of proteins with anionic domains to certain chromatin areas.

Os006819-2510 was found to interact with *O. sativa* Calreticulin Precursor (OsCRTC). A BLAST analysis of the amino acid sequence of the prey clone OsCRTC indicated that this protein is the rice Calreticulin Precursor (GENBANK® Accession No. BAA88900/SwissProt No. Q9SLY8, 100% identity, e = 0.0). OsCRTC is a 424-amino acid protein with a cleavable signal peptide (amino acids 1 to 29), a calreticulin family repeat motif (amino acids 218 to 230), and an endoplasmic reticulum targeting sequence (amino acids 421 to 424), as predicted by analysis of the OsCRTC amino acid sequence (see Munro & Pelham, 1987; Pelham, 1990). In agreement with its designation as a calreticulin precursor, the analysis identified a calreticulin family signature calreticulin family signature (amino acids 31 to 343, 1.3e⁻¹⁶⁶) (see Michalak et al., 1992; Bergeron et al., 1994; Watanabe et al., 1994). The analysis also predicted a transmembrane domain (amino acids 7 to 29) and a coiled coil (amino acids 360 to 389). The cDNA encoding the rice calreticulin OsCRTC was first identified by Li & Komatsu, 2000 who found this gene to be involved in the regeneration of rice cultured suspension cells. These authors report that the rice calreticulin protein is highly conserved, showing high homology (70-93%) to other plant calreticulins, but only 50-53% homology to mammalian calreticulins. Calreticulin (CRT) is an endoplasmic reticulum (ER) calcium-binding protein thought to be involved in many functions in eukaryotic cells, including Ca²⁺ signaling, regulation of intracellular Ca²⁺ storage and store-operated Ca²⁺ fluxes through the plasma membrane, modulation of endoplasmic reticulum Ca²⁺-ATPase function, chaperone activity to promote protein folding, control of cell adhesion, gene expression, and apoptosis (reviewed by Michalak et al., 1998 and by Persson et al., 2001). In plants, CRT has been localized to the endoplasmic reticulum, Golgi, plasmodesmata, and plasma membrane (Hassan et al., 1995; Borisjuk et al., 1998; Baluska et al., 2001), and it has been shown to affect cellular calcium homeostasis, as reported by Persson et al., supra. This study shows that induction of calreticulin expression in transgenic tobacco and *Arabidopsis* plants enhances the ATP-dependent Ca²⁺ accumulation of the endoplasmic reticulum, and that this CRT-mediated alteration of the ER Ca²⁺ pool regulates ER-derived Ca²⁺ signals. These results demonstrate that CRT plays a key role as a regulator of calcium storage in the endoplasmic ER, and that the ER, in addition to the vacuole, is an important Ca²⁺ store in plant cells. A role for the *Arabidopsis* calreticulin homolog in anther maturation or dehiscence has also been proposed (Nelson et al., 1997) based on localization of this protein in anthers which are degenerating at the time of maximum CRT expression. Furthermore, the tobacco homolog of mammalian CRTC participates in protein-protein interactions in a stress- and ATP-dependent fashion (Denecke et al., 1995). This notion supports the use of the yeast two-hybrid technology to identify proteins that interact with OsCRTC.

OsCRTC was also used as bait and found to interact with rice Soluble Starch Synthase (OsSSS; see Table 24) and Novel Protein PN29950 (OsPN29950). OsSSS is the rice homolog of soluble starch synthase (SSS), one of the three enzymes involved in starch biosynthesis in plants. Starch is the major component of yield in the world's main crop plants and one of the most important products synthesized by plants that is used in industrial processes. It consists of two kinds of glucose polymers: highly branched amylopectin and relatively unbranched amylose. Starch synthase contributes to the synthesis of amylopectin. The enzyme utilizes the glucosyl donor ADP-glucose (ADPGlc) to add glucosyl units to the nonreducing end of a glucan chain through α(1 → 4) linkages, thus elongating the linear chains (reviewed by Cao et al., 2000; Kossman & Lloyd, 2000). Distinct classes of isoforms of starch synthase were defined on the basis of similarity in amino acid sequence, molecular mass, and antigenic properties. Plant organs vary greatly in the classes they possess and in the relative contribution of the classes to soluble starch synthase activity (Smith et al., 1997, cited in Cao et al., 2000). OsPN29950 is a protein of unknown function determined by BLAST analysis to be similar to putative protein from *Arabidopsis thaliana* (GENBANK® Accession No. NP_199037.1, 32% identity, 2e⁻²⁹).

Os006819-2510 was found to interact with low temperature-induced protein 5 (OsLIP5). OsLIP5 is a 276-amino acid protein with a cleavable signal peptide (amino acids 1 to 27) and three putative transmembrane regions (amino acids 48 to 64, 82 to 98, and 233 to 249). A BLAST analysis of the amino acid sequence of this prey clone determined that it is the rice LIP5 protein (GENBANK® Accession No. BAA24979.1, 100% identity, 8e⁰⁵²). The rice LIP5 protein is a direct submission to the public database and is not described in the literature. In yeast, LIP5 is involved in lipoic acid metabolism (Sulo & Martin, 1993). The BLAST analysis shows that the rice LIP5-like protein OsLIP5 is also similar to rice WSI724 (GENBANK® Accession No. T07613, 98% identity, 3e⁻⁰⁵¹), a protein encoded by one of nine cDNAs induced by short-term water stress and thought to be responsible for acquired resistance to chilling in a chilling-sensitive variety of rice (Takahashi et al., 1994). Among the proteins encoded by these cDNAs, which were found to be differentially expressed following water stress, expression of the WSI724 protein remained relatively fixed. A BLAST analysis comparing the nucleotide sequence of OsLIP5 against TMRI's GENECHIP® Rice Genome Array sequence database identified probeset OS000070_r_at (e = 4e⁻⁷⁵) as the closest match. Gene expression experiments indicated that this gene is down-regulated by the herbicide BL2.

Os006819-2510 was also found to interact with *Oryza sativa* putative myosin (OsPN23878). A BLAST analysis of the amino acid sequence of OsPN23878 indicated that this prey protein is the rice putative myosin (GENBANK® Accession No. AAL31066.1, 99% identity, e = 0.0). OsPN23878 is also similar to Myosin VIII, ZMM3 - maize (fragment) from Z. *mays* (GENBANK® Accession No. A59311, 89% identity, e = 0.0). Myosins are discussed in Example I. Based on current knowledge of plant myosins, the myosin VIII prey protein OsPN23878 can be a cytoskeletal component that participates in events relating to cytokinesis.

The prey protein OsPN23878 also interacts with hypothetical protein 003118-3674, which is similar to *Lycopersicon esculentum* Calmodulin (Os003118-3674; see Table 25). Os003118-3674 is a 148-amino acid protein with two EF-hand calcium-binding domains (amino acids 22 to 34 and 93 to 105). In agreement with the observation that Os003118-3674 includes EF-hand calcium-binding domains, a BLAST analysis of the Genpept database indicated that this protein shares 72% identity with *A. thaliana* putative calmodulin (GENBANK® Accession No. NP_1764705, e⁻⁵⁷), although the top hit in this search is *A. thaliana* putative serine/threonine kinase (GENBANK® Accession No. NP_172695.1, 76% identity, 7e⁻⁶⁰). Therefore, the possibility that this calmodulin-like protein possesses kinase activity is worth consideration.

A BLAST analysis comparing the nucleotide sequence of OsPN23878 against TMRI's GENECHIP® Rice Genome Array sequence database identified probeset OS002190_I_at (e⁻¹⁶⁵) as the closest match. The gene expression experiments disclosed herein indicated that this gene is not specifically induced under a range of given conditions.

Additionally, Os006819-2510 was found to interact with OsRAB16B (OsRAB16B), a 164-amino acid protein that has a possible cleavage site between amino acids 51 and 52, although it does not appear to have a cleavable signal peptide. Analysis of its amino acid sequence predicted (2.6e⁻⁸¹) this protein to be a member of a group of plant proteins called dehydrins, which are induced in plants by water stress (see Close et al., 1989; Robertson & Chandler, 1992; Dure et al., 1989). Dehydrins include the basic, glycine-rich RAB (responsive to abscisic acid) proteins. In agreement with this notion, the analysis indicated that OsRAB16B is a basic, glycine-rich protein. A BLAST analysis against the public database revealed that OsRAB16B is the rice DEHYDRIN RAB 16B (GENBANK® Accession No. P22911, 100% identity, 4e⁻⁹⁵). The cDNA encoding this protein was isolated by (Yamaguchi-Shinozaki et al., 1990) as one of four rice RAB genes that are differentially expressed in rice tissues. In agreement with the notion that OsRAB16B is a rice RAB protein, a BLAST analysis against Myriad's proprietary database indicated that OsRAB16B shares 57% identity with OsRAB25. While expression data for OsRAB16B are not available, the rice RAB16B promoter contains two abscisic acid (ABA)-responsive elements required for ABA induction (Ono et al., 1996). Among other rice RAB proteins, the RAB16A gene has been linked to salt stress (Saijo et al., 2001), and the activity of the RAB16A promoter is also induced by ABA and by osmotic stresses in various tissues of vegetative and floral organs (Ono et al., 1996). Another rice RAB protein, RAB21, is induced in rice embryos, leaves, roots and callus-derived suspension cells treated with NaCl and/or ABA (Mundy & Chua, 1988). Based on these data, it is likely that the OsRAB16B prey protein has a role in the stress response.

Os006819-2510 was found to interact with protein PN23226 (OsPN23226). A BLAST analysis against the public database indicated that OsPN23226 is similar to putative glucan synthase (GENBANK® Accession No. NP_563743.1, 78% identity, e = 0.0) and to callose synthase 1 catalytic subunit (GENBANK® Accession No. NP_563743.1, 78% identity, e = 0.0) from *A. thaliana.* Callose synthase (CalS) from higher plants is a multisubunit membrane-associated enzyme involved in callose synthesis (reviewed in Hong et al., 2001). Callose is a linear 1,3-β-glucan with some 1,6- branches and differs from cellulose, the major component of the plant cell wall. Callose is synthesized on the forming cell plate and several other locations in the plant, and its deposition at the cell plate precedes the synthesis of cellulose. Callose synthesis can also be induced by wounding, pathogen infection, and physiological stress. The activity of callose synthase is highly regulated during plant development and can be affected by various biotic and abiotic factors. CalS, like cellulose synthase, is a large transmembrane protein. Its structure includes a large hydrophilic loop that is relatively conserved among the CalS isoforms, a less conserved, long N- terminal segment, and a short C-terminal segment, all located on the cytoplasmic side. The central loop is thought to act as a receptacle to hold other proteins that are essential for CalS catalytic activity (see below); the N-terminal segment can contain subdomains for interaction with proteins that regulate 1,3-β-glucan synthase activity.

The cDNA encoding the callose synthase (CalS1) catalytic subunit from *Arabidopsis* was identified by Hong et al., 2001, who demonstrated that higher plants encode multiple forms of CalS enzymes and that the *Arabidopsis* CalS1 is a cell plate-specific isoform. In addition, these authors used yeast two-hybrid and *in vitro* experiments to show that CalS1 interacts with two other cell plate-specific proteins, phragmoplastin and a UDP- glucose transferase, and suggest that it can form a large complex with these and other proteins to facilitate callose deposition on the cell plate. Moreover, the plasma membrane CalS is strictly Ca²⁺-dependent, and Ca²⁺ plays a key role in cell plate formation and can activate the cell plate-specific CalS1. The prey protein OsPN23226 is likely a rice callose synthase homolog that can function similarly to the *Arabidopsis* CalS1 catalytic subunit.

In addition to the cell plate, callose is synthesized in a variety of specialized tissues and in response to mechanical and physiological stresses. Multiple CalS isozymes are thought to be required in higher plants to catalyze callose synthesis in different locations and in response to different physiological and developmental signals (Hong et al., 2001).

Os006819-2510 was also found to interact with protein PN23485, which is similar to *Hordeum vulgare* coproporphyrinogen III oxidase, chloroplast precursor (OsPN23485). A BLAST analysis of the amino acid sequence of OsPN23485 determined that this protein is similar to barley (*H*. *vulgare*) Coproporphyrinogen III Oxidase, Chloroplast Precursor (coprogen oxidase; GENBANK® Accession No. Q42840, 89.3% identity, e⁻¹⁶⁹). Coproporphyrinogen III oxidase (CPO) catalyzes a step in the pathway from 5-amino-levulinate to protoporphyrin IX, a common reaction in the biosynthesis of heme in animals and chlorophyll in photosynthetic organisms. The N-terminal sequences of plant CPOs are characteristic of plastid transit peptides. CPO is exclusively located in the stroma of plastids, and *in vitro* transcribed and translated CPO is imported into the stroma of pea plastids and truncated by a stromal endopeptidase (reviewed by Ishikawa et al., 2001). Plant cDNA sequences encoding CPO were obtained from soybean, tobacco and barley (Kruse et al., 1995). They found that the plant coprogen oxidase mRNA was expressed to different extents in various tissues, with maximum amounts in developing cells and drastically decreased amounts in completely differentiated cells, suggesting differing requirements for tetrapyrroles in different organs. Based on these results, these authors propose that enzymes involved in tetrapyrrole (porphyrin) synthesis are regulated developmentally rather than by light, and that regulation of these enzymes guarantees a constant flux of metabolic intermediates and help avoid photodynamic damage by accumulating porphyrins. Inhibition of the pathway for chlorophyll synthesis causes lesion formation such as that found in the pale green and lesion-formation phenotype of lin2 plants. Ishikawa et al., *supra* found that a deficiency of coproporphyrinogen III oxidase causes lesion formation in these *Arabidopsis* mutants. Furthermore, based on the observation that transgenic tobacco plants with reduced CPO activity accumulate photosensitizing tetrapyrrole intermediates and exhibit antioxidative responses and necrotic leaf lesions, these authors suggest that CPO inhibition causes lesion formation leading to induction of a set of defense responses that resemble the HR observed after pathogen attack. These lesions are the equivalent of diseases known as porphyrias in humans. If accumulated, coproporphyrin(ogen), as a photosensitizer, induces damage through generation of reactive oxidative species, which play a key role in the initiation of cell death and lesion formation both in the HR and in certain lesion mimic mutants. They suggest that in lin2 mutants, the generation of an oxidative burst triggered by coproporphyrin accumulation leads to cell death.

Os006819-2510 was found to interact with protein PN29037 (OsPN29037). A BLAST analysis of the amino acid sequence of OsPN29037 indicated that this prey protein is similar to Gamma Hydroxybutyrate Dehydrogenase from *A. thaliana* (GENBANK® Accession No. AAK94781.1, 80.7%, identity, e⁻¹²⁷). This enzyme oxidizes gamma-hydroxybutyrate. As a minor brain metabolite directly or indirectly involved in scavenging oxygen-derived free radicals in animals, gamma-hydroxybutyrate demonstrates similarities with melatonin (Cash, 1996).

### Summary

Thus, the senescence-associated protein Os006819-2510 interacts with several proteins that have possible roles in cell cycle processes. One of these is OsPN23878, a protein annotated in the public domain as the rice putative myosin. Myosins are cytoskeletal proteins that function as molecular motors in ATP-dependent interactions with actin filaments in various cellular events. Based on the similarity of the prey protein to a class VIII myosin and on the reported role of plant myosin VIII in maturation of the cell plate and in organization of the actin cytoskeleton at cytokinesis, we speculate that the myosin OsPN23878 is a cytoskeletal component that participates in events occurring at cytokinesis in rice. The association of the myosin OsPN23878 with senescence-associated protein can be a step in cell-cycle-dependent events involving cytoskeleton organization and senescence. Specific expression of the gene encoding OsPN23878 in panicle (our gene expression experiments) is consistent with an interaction between this protein and Os006819-2510, and with a role for the latter in flower senescence, as suggested for the gene encoding the daylily homolog of this protein (Panavas et al., 1999). Localization of senescence-associated protein to the ER suggests that some of the events in which OsPN23878 functions could be associated with plasmodesmata function.

Note that the myosin protein OsPN23878 also interacts with a novel calmodulin-kinase-like protein Os003118-3674 (see Table 25), and that the latter interacts with a myosin heavy chain (OsAAK98715) found to interact with rice cyclin OsCYCOS2 and presumed to be involved in cytoskeleton organization during mitotic events (see Example II). The interactions of myosins with a calcium-binding calmodulin-like protein are consistent with published evidence of regulation of myosin function by calcium (Yokota et al., 1999a; reviewed in Reddy, 2001). The possibility that Os003118-3674 possesses kinase activity raises the probability that these interactions propagate a cell-cycle-related signaling event. The calmodulin-like protein Os003118-3674 thus provides a link between the senescence-associated protein and interacting partners of this Example and the cell cycle network.

Another interactor with a possible role in cell cycle regulation is the rice histone deacetylase OsAAK01712. This enzyme includes a transmembrane domain and is involved in regulation of core histones acetylation. The acetylation/deacetylation of histones, the main protein component of chromatin, is connected to replication during the cell cycle in plants, as is in other eukaryotes (Jasencakova et al., 2001). Thus, the Os006819-2510-OsAAK01712 interaction likely participates in mitotic events involving chromatin organization.

Another novel interactor found for senescence-associated protein is OsPN23485, similar to coproporphyrinogen III oxidase, chloroplast precursor, an enzyme of the pathway leading to the biosynthesis of chlorophyll in plants. The observation that the lesion formation in the lin2 mutant *Arabidopsis* plants is the result of loss-of-function of CPO (Ishikawa et al., 2001) links the gene encoding CPO to regulation of cell death pathways. Moreover, plant CPO enzymes are regulated developmentally and by light (reviewed by Ishikawa et al., 2001). Based on these reports, the interaction of rice CPO (OsPN23485) with senescence-associated protein can participate in regulation of programmed cell death in a development-dependent manner in rice.

The senescence-associated protein Os006819-2510, which is presumed to be a transmembrane protein based on analysis of its amino acid sequence, interacts with the rice calreticulin OsCRTC which, like other plant calreticulins, is likely an ER transmembrane protein. The presence of two endoplasmic reticulum retention motifs in Os006819-2510 and of an endoplasmic reticulum targeting sequence in OsCRTC suggests that both proteins are localized in the ER. This notion is in agreement with the possibility of an interaction between Os006819-2510 and OsCRTC *in planta*. Os006819-2510 can participate in events controlled by OsCRTC within the endoplasmic reticulum. This interaction is consistent with the suggested role of plant CRT in anther maturation and dehiscence, which was proposed by Nelson et al., 1997 based on the observation that maximum expression of the *Arabidopsis* CRT in the anthers coincides with anther degeneration. Moreover, Denecke et al., 1995 reported detection of another plant CRT homolog in the nuclear envelope, in the ER, and in mitotic cells in association with the spindle apparatus and the phragmoplast. Given the interaction of senescence-associated protein with proteins having roles in mitosis, it is possible that the rice CRT of this Example functions in mitotic events. However, Nelson et al., 1997, indicate possible additional roles for plant CRT in developmental processes, including a chaperone function that can be reconciled with CRT localization in the developing endosperm, a site characterized by high protein synthesis rates, and in secreting nectaries, which are associated with heavy traffic of secretory proteins through the ER. Note that OsCRTC also interacts with the rice soluble starch synthase homolog OsSSS. Soluble starch synthase enzymes have been isolated from plant endosperm cells (Cao et al., 2000). These data suggest that the rice CRT homolog of this Example can also be found in this tissue, where it is conceivable that it interacts with the soluble starch synthase OsSSS in a chaperone role to promote proper folding of this protein during protein synthesis.

To further corroborate the notion that the rice senescence-associated protein Os006819-2510 is a membrane-associated protein, a novel interactor identified for this protein is a putative callose synthase catalytic subunit (OsPN23226), another transmembrane enzyme involved in glucan synthesis. Plasma membrane proteins participate in a variety of interactions with the cell wall, including synthesis and assembly of cell wall polymers (Buchanan et al., 2002, at page 13). The prey protein OsPN23226 likely functions as its *Arabidopsis* homolog, a plasma membrane enzyme that utilizes UDP-glucose as substrate to synthesize callose for deposition in the cell wall. The interactions of senescence-associated protein with the rice putative callose synthase OsPN23226 and with the calreticulin OsCRTC, and the interaction between OsCRTC and the soluble starch synthase OsSSS all involve membrane-associated proteins. While there is no evidence that such interactions occur at the same time, they can be associated with the traffic that sorts, distributes and targets membrane proteins and other molecules between compartments of the endomembrane system (Buchanan et al., 2002, at page 14) during the different stages of the cell cycle/development and in response to different physiological and developmental signals. Moreover, the interactions identified in this Example link the senescence-associated bait protein to glucan synthesis, a process that is vital to the plant normal growth. For example, the formation of a functional callose synthase 1 catalytic subunit (CalS1) complex is vital to cell plate formation. Functional characterization of the various components of the CalS1 complex and CalS-associated proteins has been proposed as a means to reveal how the activity of this enzyme is regulated during cell plate formation and to clarify callose synthesis and deposition in plants (Hong et al., 2001). The interaction identified here between senescence-associated protein and the novel putative callose synthase catalytic subunit (OsPN23226) provides new insight into this process in rice.

Other interactors identified for senescence-associated protein link this protein to the plant stress response. OsRAB16B is a member of the RAB family of proteins known to be induced by water stress and treatment with the plant hormone abscisic acid. ABA levels increase during seed development in many plant species, stimulating production of seed storage proteins and preventing premature germination; ABA is also induced by water stress and is thought to regulate stomatal transpiration (Raven et al., 1999, at page 684). Based on functional homology with other RAB proteins and on the presence of the ABA-responsive elements in the OsRAB16B promoter, we presume that OsRAB16B has a role in the response to abiotic stress in rice and that its function can be regulated by Ca²⁺. Another interactor correlated with stress is low temperature-induced protein 5 (OsLIP5), which in yeast is involved in lipoic acid metabolism. Lipoic acid in animals has been shown to help minimize the effects of systemic stress (Kelly, 1999) and to provide animal cells with significant protection against the cytotoxic effects of repin, a sesquiterpene lactone isolated from Russian knapweed (Robles et al., 1997). The high similarity (98%) of the rice LIP5-like protein to rice WSI724, a protein encoded by a gene induced by water stress and linked to resistance to chilling in rice, points to similar roles for the OsLIP5 prey protein. Gene expression experiments indicate that the gene encoding OsLIP5 is down-regulated upon treatment with the herbicide BL2. This finding suggests a role for OsLIP5 in the response to abiotic stress. While the specific function of the interactions between Os006819-2510 and the prey proteins OsRAB16B and OsLIP5 is not obvious, these interactions can participate in biological processes related to flower senescence and response to water stress and chilling.

In addition, the rice calreticulin OsCRTC discussed above can also have a role in the stress response. This hypothesis is based on functional homology with the tobacco CRT protein studied by Denecke et al., 1995 and found to participate in protein-protein interactions in a stress-dependent fashion.

In summary, among the interactors identified for the rice senescence-associated protein Os006819-2510 are several membrane-associated proteins, which supports the notion that the rice Os006819-2510 is a transmembrane protein. Among the interactors identified are proteins involved in cell cycle processes/mitosis and proteins with functions in the plant stress response. Some are newly characterized rice proteins. The interactions identified for rice senescence-associated protein with proteins involved in cell cycle/development and in resistance to stress suggests an overlapping of roles for the bait protein. Indeed, Os006819-2510 can constitute a link between stress tolerance and processes for cell division in rice.

### Example VII

OsSGT1 is a 367-amino acid protein that includes a tetratricopeptide repeat domain, two variable regions, the CS motif present in metazoan CHORD and SGT1 proteins, and the SGS motif. In yeast, *Sgt1* is required for cell-cycle signaling. In yeast, SGT1 associates with the kinetochore complex and the SCF-type E3 ubiquitin ligase by interacting with SKP1. COP9 signalosome interacts with SCF E3 ubiquitin ligases. By its interaction with SCF complexes, SGT1 exerts its essential activity in degrading of SIC1 and CLN1. Thus, one possible role of SGT1 could be to target proteins for degradation by the 26S proteasome via specific SCF complexes or the SGT1 complex can participate in the modification of protein activity or can have a dual role for activation and degradation of the target via ubiquitylation. *A. thaliana* has two SGT1 homologs. At nonpermissive temperatures AtSGT1a and AtSGT1b can complement G1 and G2 arrest in temperature sensitive *sgt1* yeast mutants. However, SGT1b interacts with RAR1 which is required for RPP5 regulated disease resistance to downy mildew. In this scenario, target proteins involved in disease resistance can be targeted for protein degradation by the SGT1 pathway. Barley encodes a SGT1 homolog that also interacts with barley RAR1, which is implicated in disease resistance in barley to downy mildew. (Austin et al., 2002; Azevedo et al., 2002). A BLAST analysis comparing the nucleotide sequence of OsSGT1 against TMRI's GENECHIP® Rice Genome Array sequence database identified probeset OS016424.1 (98%) as the closest match. Gene expression experiments indicated that this gene is up-regulated by the blast infection.

The rice SGT1 protein shares 74 and 75% amino acid sequence similarity with two *Arabidopsis thaliana* SGT1 homologs and 45% amino acid sequence similarity with *Saccharomyces cerevisiae* SGT1. In yeast, SGT1 is required for cell-cycle progression at the G1/S-phase and G2/M-phase transitions. In *A. thaliana,* SGT1 b interacts with Rar1 and mediates disease resistance. Thus, in plants, SGT1 likely controls processes that are fundamental to disease resistance and development. The rice OsSGT1 protein was chosen as a bait for these interaction studies based on its potential relevance to disease resistance and development. One bait fragment encoding amino acid 200-368 of OsSGT1 was used in the yeast two-hybrid screen, as described above.

### Results

The OsSGT1 was found to interact with ten rice proteins. Three interactors have been previously described, namely OsSGT1, a Ras GTPase (GENBANK® Accession No. P40392), and elicitor responsive protein (GENBANK® Accession No. T50649). The remaining seven interactors are novel proteins with identifiable protein domains, or are similar to other proteins. These are an L-aspartase-like protein, an RNA binding domain protein, an auxin induced-like protein, an archain delta COP-like protein, a fibrillin-like protein, a HSP70-like protein, and a proline rich protein. The elicitor responsive protein was also used as a bait and interacted with 12 novel proteins with identifiable protein domains, with similarity to known proteins or that are unidentifiable by sequence similarity. These were an NAD(P) binding domain protein, a gamma adaptin-like protein, a pectinesterase-like protein, a receptor like kinase protein kinase like protein, a pyruvate orthophosphate dikinase like protein, an Isp-4 like protein, a xanthine dehydrogenase like protein, a ubiquitin specific protease like protein and 4 unknown proteins.

The interacting proteins of this Example are listed in Table 26, followed by detailed information on each protein and a discussion of the significance of the interactions. The nucleotide and amino acid sequences of the proteins of the Example are provided in SEQ ID NOs: 111-150 and 309-316. Based on the biological function of SGT1, it is possible that the interacting proteins are also involved in cell cycle/mitotic processes and/or in the plant resistance to stress. Likewise, the interactors with the elicitor responsive protein can also be involved in plant resistance to stress. Proteins that participate in cell cycle regulation in rice can be targets for genetic manipulation or for compounds that modify their level or activity, thereby modulating the plant cell cycle. The identification of genes encoding these proteins can allow genetic manipulation of crops or application of compounds to effect agronomically desirable changes in plant development or growth. Likewise, genes that are involved in conferring plants resistance to stress have important commercial applications, as they could be used to facilitate the generation and yield of stress-resistant crops.

**Table 26**

| Interacting Proteins Identified for Os006819-2510 (Hypothetical Protein 006819-2510, Similar to *Hemerocallis* Senescence-Related Protein 5) | | | |
|---|---|---|---|
| The names of the clones of the proteins used as baits and found as preys are given. Nucleotide/protein sequence accession numbers for the proteins of the Example (or related proteins) are shown in parentheses under the protein name. The bait and prey coordinates (Coord) are the amino acids encoded by the bait fragment(s) used in the search and by the interacting prey clone(s), respectively. The source is the library from which each prey clone was retrieved. | | | |
| **Gene Name** | **Protein Name** **(GENBANK® Accession No.)** | **Bait Coord** | **Prey Coord** **(source)** |
| **BAIT PROTEIN** | | | |
| PN20285 (SEQ ID NO: 310) | OsSGT1 (gi\|6581058) | | |

| **INTERACTORS** | | | |
|---|---|---|---|
| PN24060 (SEQ ID NO: 112) | L-aspartase-like protein-like | 200-368 | 176-315 (output trait) |
| PN20696* (OsERP) (SEQ ID NO: 312) | Elicitor responsive protein (gi\|11358958) | 200-368 | 54-144 (input trait) |
| PN23914 (SEQ ID NO: 114) | RNA binding domain protein | 200-368 | 1-263 x 3 (output trait) |
| PN23221# (SEQ ID NO: 116) | Proline rich protein | 200-368 | 182-366 x 2 (output trait) 207-344 (input trait) 134-254 (output trait) |
| PN20285 (SEQ ID NO: 310) | OsSGT1 (gi\|6581058) | 200-368 | 9-227 (output trait) |
| PN24061 (SEQ ID NO: 118) | Auxin induced protein-like | 200-368 | 34-236 (output trait) |
| PN24063 (SEQ ID NO: 314) | RAS GTPase (gi\|730510) | 200-368 | 63-202 (output trait) |
| PN23949 (SEQ ID NO: 120) | HSP70-like | 200-368 | 244-418 (outpu trait) |
| PN29042 (SEQ ID NO: 122) | Fibrillin-like | | |
| PN28982 (SEQ ID NO: 124) | archain delta COP-like | | |

| | | | |
|---|---|---|---|
| * Additional interactions identified for elicitor responsive protein are shown in Table 27 # Additional interactions identified for PN23221 are shown in Table 28 | | | |

**Table 27**

| Interacting Proteins Identified for OsERP (Elicitor Responsive Protein) | | | |
|---|---|---|---|
| **Gene Name** | **Protein Name** **(GENBANK® Accession No.)** | **Bait Coord** | **Prey Coord** **(source)** |
| **BAIT PROTEIN** | | | |
| PN20696 (OsERP) (SEQ ID NO: 312) | Elicitor responsive protein (gi\|11358958) | | |

| **INTERACTORS** | | | |
|---|---|---|---|
| PN29984 (SEQ ID NO: 126) | Novel Protein PN29984 | 50-145 | 1-38 5-41 (input trait) |
| PN30844 (SEQ ID NO: 128) | Novel protein PN30844 | 50-145 | 1-64 (input trait) |
| PN30868 (SEQ ID NO: 130) | NAD(P) binding domain protein | 50-145 | 167-336 (input trait) |
| PN24292 (SEQ ID NO: 132) | Gamma adaptin-like | 23-120 | 737-918 (output) |
| PN29983 (SEQ ID NO: 134) | Novel protein PN29983 | 50-145 | 1-131 (input trait) |
| PN30845 (SEQ ID NO: 136) | Pectinesterase-like | 50-145 | 1-64 (input trait) |
| PN31085 (SEQ ID NO: 138) | Receptor-like protein kinase-like | 23-120 | 378-553 (output trait) |
| PN20674 (SEQ ID NO: 140) | Pyruvate orthophosphate dikinase-like | 50-145 | 64-263 71-298 (input trait) |
| PN30870 (SEQ ID NO: 142) | Isp-4 like | 50-145 | 1-446 (input trait) |
| PN29997 (SEQ ID NO: 144) | Xanthine dehydrogenase-like | 23-120 | 737/918 (output trait) |
| PN30843 (SEQ ID NO: 146) | Ubiquitin specific protease-like | 50-145 | 164-221 (input trait) |
| PN30857 (SEQ ID NO: 148) | Novel protein PN30857 | 50-145 | 1-148 (input trait) |

**Table 28**

| Interacting Proteins Identified for PN23221 (Proline Rich Protein) | | | |
|---|---|---|---|
| **Gene Name** | **Protein Name** **(GENBANK® Accession No.)** | **Bait Coord** | **Prey Coord (source)** |
| **PREY PROTEIN** | | | |
| PN23221 (SEQ ID NO: 116) | Proline rich protein | | |

| **BAIT PROTEIN** | | | |
|---|---|---|---|
| PN20621 (SEQ ID NO: 316) | Shaggy kinase (gi\|13677093) | 120-435 | 175-311 (output trait) |
| PN20115 (SEQ ID NO: 150) | Ring zinc finger protein | 5-140 | 84-302 191-324 (output trait) |

### Yeast Two-Hyrid using OsSGT1 as Bait

The bait fragment encoding amino acid 200-368 of OsSGT1 was found to interact with L-aspartase-like protein PN24060. A BLAST analysis of the amino acid sequence of PN24060 indicated that this prey protein has 36.5% similarity to *A. thaliana* L-aspartase (nucleotide sequence available at GENBANK® Accession No. NM_101325). The enzyme L-aspartate ammonia-lyase (aspartase) catalyzes the reversible deamination of the amino acid L-aspartic acid, using a carbanion mechanism to produce fumaric acid and ammonium ion. While the catalytic activity of this enzyme has been known for nearly 100 years, a number of recent studies have revealed some interesting and unexpected new properties of this reasonably well-characterized enzyme. The non-linear kinetics that are seen under certain conditions have been shown to be caused by the presence of a separate regulatory site. The substrate, aspartic acid, can also play the role of an activator, binding at this site along with a required divalent metal ion. So it is possible that PN24060 catalyses a reaction that pertains to protein modification and the modification can be important for disease resistance or cell cycling.

The bait fragment encoding amino acid 200-368 of OsSGT1 was also found to interact with elicitor responsive protein, PN20696. A BLAST analysis of the amino acid sequence of the prey clone PN20696 indicated that this protein is the rice elicitor responsive protein (GENBANK® Accession no. T50649; OsERP). OsERP is a 144-amino acid protein that, according to GENBANK®, is expressed by rice culture cells in the presence of the rice blast fungal elicitor. Thus, OsERP can have a role in disease responses in rice.

OsERP was also used as bait and found to interact with 12 other proteins (see Table 27). These prey are described in this Example below.

An *A. thaliana* homologue to OsERP was identified by BLAST. At1g63220 shares 75% amino acid similarity with OsERP. To see if *Arabidopsis* homologues of OsERP have roles in disease resistance, *Arabidopsis thaliana* with T-DNA insertions in *At1g63220* (line SAIL_320_D02) was identified from a random insertion seed library. DNA regions surrounding the insertions were sequenced and revealed that the T-DNAs were located within exon 5 of *At1g63220*. Plants were backcrossed and plants homozygous for the T-DNA insertion were identified by PCR. Homozygous mutants and wild type plants were challenged with *Pseudomonas syringae* pv. *maculicola* ES4326 and plants were assayed for amount of P. *syringae* bacteria accumulation 3 days post inoculation (Glazebrook et al., 1996) These experiments were repeated twice on at least six plants. Data are reported as means and standard deviations of the log of colony forming units per leaf cm². By three days after inoculation, the mutant plants accumulated more than 10 times as much bacteria as wild type plants (wt = 3.94 log cfu/leaf disk std. 0.57, *at1g63220* = 5.34 std. 0.63). Hence, At1g63220 contributes to disease resistance in *A. thaliana.* It is possible that the At1g63220 mutation inhibits defense responses that are dependent upon SGT1 interactions.

In addition, the bait fragment encoding amino acid 200-368 of OsSGT1 was found to interact with RNA-binding domain protein, PN23914. PN23914 is a 164-amino acid protein. A BLAST analysis of the amino acid sequence of this prey shows it has 35.9% sequence identity to tFZR1 from *Oncorhynchus mykiss* (GENBANK® Accession No. BAA25269). TFZR1 is an orphan nuclear receptor family member, tFZR1, which has a FTZ-F1 box. The amino acid sequences of the zinc finger domain and the FTZ-F1 box has 92.8% and 100% identity, respectively, with those of zebrafish FTZ-F1. On the other hand, the overall homology between tFZR1 and zebrafish FTZ-F1 is low (33.0%). The results indicate that tFZR1 is a new member of fushitarazu factor 1 (FTZ-F1) subfamily. It is possible that PN23914 shares functionality through the zing finger domain.

In addition, bait fragment encoding amino acid 200-368 of OsSGT1 was found to interact with proline rich protein, PN23221. A BLAST analysis of the amino acid sequence of PN23221 indicated that this prey protein is 40.3% similar to a rice repetitive proline rich protein (GENBANK® Accession No. AAL73214). Proline rich proteins can mediate interaction among proteins (Zhao et al., 2001). Note that proline rich protein PN23221 also interacts with shaggy kinase PN20621 and ring zinc finger protein-like PN20115 (see Table 28). Thus, the proline rich protein PN23221 can serve to bring these proteins together with OsSGT1.

The bait fragment encoding amino acid 200-368 of OsSGT1 was also found to interact with OsSGT1. In other words, OsSGT1 interacts with itself. Although the bait for OsSGT1 included amino acids 200-368, the prey included amino acids 9-227. Although OsSGT1 can be a self-regulator through aggregation, these bait and prey domains can reflect natural protein folding of a single native OsSGT1 protein.

Additionally, the bait fragment encoding amino acid 200-368 of OsSGT1 was found to interact with an auxin-induced protein like protein, PN24061. A BLAST analysis against the public database indicated that PN24061 is 63.5% similar to a rice putative IAA1 protein (GENBANK® Accession No. CAC80823). Indole acetic acid (IAA) is a plant growth hormone and is classified as an auxin. IAA is associated with a variety of physiological processes, including apical dominance, tropisms, shoot elongation, induction of cambial cell division and root initiation. Thus, genes that are induced by IAA likely produce proteins that are responding developmental changes. This associated goes hand in hand with regulation of cell division by interaction with SGT1.

The bait fragment encoding amino acid 200-368 of OsSGT1 was also found to interact with Ras GTPase, PN24063. A BLAST analysis of the amino acid sequence of PN24063 determined that this protein is ras-related GTP binding protein possessing GTPase activity (GENBANK® Accession No. P40392). This protein has four conserved regions involved in GTP binding and hydrolysis which are characteristic in the ras and ras-related small GTP-binding protein genes. In addition, two consecutive cysteine residues near the carboxyl-terminal end required for membrane anchoring are also present. This protein synthesized in *Escherichia coli* possessed GTPase activity (i.e., hydrolysis of GTP to GDP; Kidou et al., 1993). Ras GTPases are likely involved in signaling processes for development. ORFX from tomato that is expressed early in floral development, controls carpel cell number, and has a sequence suggesting structural similarity to the human oncogene c-H-ras p21 (fw2.2: a quantitative trait locus key to the evolution of tomato fruit size; Frary et al., 2000). The Rho family of GTPases are also involved in control of cell morphology, and are also thought to mediate signals from cell membrane receptors (Winge et al., 1997).

An *A. thaliana* homologue to PN24063 was identified by BLAST. At1g02130 shares 90% amino acid similarity with PN24063. To see if *Arabidopsis* homologues of PN24063 have roles in disease resistance *Arabidopsis thaliana* with T-DNA insertions in *At1g02130* (line SAIL_680_D03) was identified from a random insertion seed library. DNA regions surrounding the insertions were sequenced and revealed that the T-DNAs were located within the promoter of *At1g02130*. Plants were backcrossed and plants homozygous for the T-DNA insertion were identified by PCR. Homozygous mutants and wild type plants were challenged with *Pseudomonas syringae* pv. *maculicola* ES4326 and plants were assayed for amount of P. *syringae* bacteria accumulation 3 days post inoculation (Glazebrook et al., 1996). These experiments were repeated twice on at least six plants. Data are reported as means and standard deviations of the log of colony forming units per leaf cm². By three days after inoculation, the mutant plants accumulated more than 10 times as much bacteria as wild type plants (wt = 3.93 log cfu/leaf disk std. 0.57, *at1g02130* = 5.22 std. 0.9). Hence, At1g02130 contributes to disease resistance in *A. thaliana.* It is possible that the At1g02130 mutation inhibits defense responses that are dependent upon SGT1 interactions.

The bait fragment encoding amino acid 200-368 of OsSGT1 was found to interact with Archain delta COP, PN28982. A BLAST analysis of the amino acid sequence of PN28982 indicated that this prey protein is 92% similar to rice archain delta COP (GENBANK® Accession No. P49661). Cytosolic coat proteins that bind reversibly to membranes have a central function in membrane transport within the secretory pathway. One well-studied example is COPI or coatomer, a heptameric protein complex that is recruited to membranes by the GTP-binding protein Arf1. Assembly into an electron-dense coat then helps in budding off membrane to be transported between the endoplasmic reticulum (ER) and Golgi apparatus. Activated Arf1 brings coatomer to membranes. However, once associated with membranes, Arf1 and coatomer have different residence times: coatomer remains on membranes after Arf1-GTP has been hydrolysed and dissociated. Rapid membrane binding and dissociation of coatomer and Arf1 occur stochastically, even without vesicle budding. This continuous activity of coatomer and Arf1 generates kinetically stable membrane domains that are connected to the formation of COPI-containing transport intermediates. This role for Arf1/coatomer might provide a model for investigating the behaviour of other coat protein systems within cells. (Presley et al., 2002). It is possible that this delta COP interacts with the OsSGT1 and a Ras GTPase to coordinate membrane transport for proteolytically processed proteins.

An *A. thaliana* homologue to PN28982 was identified by BLAST. At5g05010 shares 77% amino acid similarity with PN28982. To see if *Arabidopsis* homologues of PN28982 have roles in disease resistance *Arabidopsis thaliana* with T-DNA insertions in *At5g05010* (line SAIL_84_C10) was identified from a random insertion seed library. DNA regions surrounding the insertions were sequenced and revealed that the T-DNAs were located within the promoter of *At5g05010*. Plants were backcrossed and plants homozygous for the T-DNA insertion were identified by PCR. Homozygous mutants and wild type plants were challenged with *Pseudomonas syringae* pv. *maculicola* ES4326 and plants were assayed for amount of *P. syringae* bacteria accumulation 3 days post inoculation (Glazebrook et al., 1996). These experiments were repeated twice on at least six plants. Data are reported as means and standard deviations of the log of colony forming units per leaf cm². By three days after inoculation, the mutant plants accumulated more than 10 times as much bacteria as wild type plants (wt = 3.93 log cfu/leaf disk std. 0.57, *at5g05010*= 5.24 std. 0.52). Hence, At5g05010 contributes to disease resistance in *A. thaliana.* It is possible that the At5g05010 mutation inhibits defense responses that are dependent upon SGT1 interactions.

The bait fragment encoding amino acid 200-368 of OsSGT1 was found to interact with fibrillin-like protein, PN29042. A BLAST analysis of the amino acid sequence of OsPN29037 indicated that this prey protein is 75% similar to the potato fibrillin homolog CDSP34 precursor from chloroplasts (GENBANK® Accession No. T07825). Plastid lipid-associated proteins, also termed fibrillin/CDSP34 proteins, are known to accumulate in fibrillar-type chromoplasts such as those of ripening pepper fruit, and in leaf chloroplasts from Solanaceae plants under abiotic stress conditions. Further, substantially increased levels of fibrillin/ CDSP34 proteins are shown in various dicotyledonous and monocotyledonous plants in response to water deficit. (Langenkamper et al., 2001) In water-stressed tomato plants, similar increases in the CDSP 34-related transcript amount were noticed in wild-type and ABA-deficient flacca mutant, but protein accumulation was observed only in wild-type, suggesting a posttranscriptional role of ABA in CDSP 34 synthesis regulation. Substantial increases in CDSP 34 transcript and protein abundances were also observed in potato plants subjected to high illumination. The CDSP 34 protein is proposed to play a structural role in stabilizing stromal lamellae thylakoids upon osmotic or oxidative stress. (Gillet et al., 1998).

A BLAST analysis comparing the nucleotide sequence of PN29042 against TMRI's GENECHIP® Rice Genome Array sequence database identified probeset OS011738 (100%) as the closest match. Gene expression experiments indicated that this gene is up-regulated by ABA treatment.

An *A. thaliana* homologue to PN29042 was identified by BLAST. At4g22240 shares 79% amino acid similarity with PN29042. To see if *Arabidopsis* homologues of PN29042 have roles in disease resistance *Arabidopsis thaliana* with T-DNA insertions in *At4g22240* (line SAIL_691_B11) was identified from a random insertion seed library. DNA regions surrounding the insertions were sequenced and revealed that the T-DNAs were located within exon 1 of *At4g22240*. Plants were backcrossed and plants homozygous for the T-DNA insertion were identified by PCR. Homozygous mutants and wild type plants were challenged with *Pseudomonas syringae* pv. *maculicola* ES4326 and plants were assayed for amount of *P. syringae* bacteria accumulation 3 days post inoculation (Glazebrook et al., 1996). These experiments were repeated twice on at least six plants. Data are reported as means and standard deviations of the log of colony forming units per leaf cm². By three days after inoculation, the mutant plants accumulated more than 10 times as much bacteria as wild type plants (wt = 3.93 log cfu/leaf disk std. 0.57, *at4g22240*= 5.21 std. 0.43). Hence, At4g22240 contributes to disease resistance in *A. thaliana.* It is possible that the At4g22240 mutation inhibits defense responses that are dependent upon SGT1 interactions.

Additionally, the bait fragment encoding amino acid 200-368 of OsSGT1 was found to interact with HSP70-like protein, PN23949. A BLAST analysis of the amino acid sequence of OsPN3949 indicated that this prey protein is 71% similar to the cucumber 70K heat shock protein found in chloroplasts (GENBANK® Accession No. T10248). Heat shock proteins (reviewed in Bierkens et al., 2000) are stress proteins that function as intracellular chaperones to facilitate protein folding/unfolding and assembly/disassembly. They are selectively expressed in plant cells in response to a range of stimuli, including heat and a variety of chemicals. As regulators, HSP proteins are thus part of the plant protective stress response. A BLAST analysis comparing the nucleotide sequence of PN23949 against TMRI's GENECHIP® Rice Genome Array sequence database identified probeset OS015016 (97%) as the closest match. Gene expression experiments indicated that this gene is down-regulated by herbicide and JA treatment.

### Yeast Two-Hybrid Using OsERP (PN20696) as Bait

Next, one of the proteins found to interact with OsSGT1, namely the elicitor responsive protein PN20696 (GENBANK® Accession No. T50649; OsERP), was used as a bait. As shown in Table 27, the rice elicitor responsive protein PN20696 (GENBANK® Accession No. T50649; OsERP) was found to interact with a receptor-like protein kinase like protein, PN31085. A BLAST analysis of the amino acid sequence of OsPN31085 indicated that this prey protein is 48% similar to a rice receptor like protein kinase (GENBANK® Accession No. T04124). The receptor protein kinases include a large group of proteins and most contain a cytoplasmic protein kinase catalytic domain, a transmembrane region, and and/or an extracellular domain consisting of leucine-rich repeats, which are thought to interact with other macromolecules. Cell to cell communication is likely mediated by receptor kinases which have important roles in plant morphogenesis.

OsERP was also found to interact with pyruvate orthophosphate dikinase, PN20674. A BLAST analysis of the amino acid sequence of PN20674 indicates that this prey protein is 96% similar to rice pyruvate orthophosphate dikinase (GENBANK® Accession No. T02979). Pyruvate orthophosphate dikinase (PPDK) is known for its role in C4 photosynthesis but has no established function in C3 plants. Abscisic acid, PEG and submergence were found to markedly induce a protein of about 97 kDa, identified by microsequencing as PPDK, in rice roots (C3). One rice PPDK is ABA-induced protein from roots. Western blot analysis showed a PPDK induction in roots of rice seedlings during gradual drying, cold, high salt and mannitol treatment, indicating a water deficit response. PPDK was also induced in the roots and sheath of submerged rice seedlings, and in etiolated rice seedlings exposed to an oxygen-free N2 atmosphere, which indicated a low-oxygen stress response. None of the stress treatments induced PPDK protein accumulation in the lamina of green rice seedlings. Ppdk transcripts were found to accumulate in roots of submerged seedlings, concomitant with the induction of alcohol dehydrogenase 1. Low-oxygen stress triggered an increase in PPDK activity in roots and etiolated rice seedlings, accompanied by increases in phosphoenolpyruvate carboxylase and malate dehydrogenase activities. The results indicate that cytosolic PPDK is involved in a metabolic response to water deficit and low-oxygen stress in rice, an anoxia-tolerant species (Moons et al., 1998).

Additionally, OsERP was found to interact with gamma adaptin, PN24292. A BLAST analysis of the amino acid sequence of PN24292 indicated that this prey protein is 97% similar to the *Arabidopsis* gamma adaptin (GENBANK® Accession No. BAA78745). Eukaryotic vesicular transport requires the recognition of membranes through specific protein complexes. The heterotetrameric adaptor protein complexes 1, 2, and 3 (AP1/2/3) are composed of two large, one small, and one medium adaptin subunit. Large subunits of AP1/2/3 are homologous and two subunits of the heptameric coatomer I (COPI) complex belong to this gene family. In addition, all small subunits and the aminoterminal domain of the medium subunits of the heterotetramers are homologous to each other; this also holds for two corresponding subunits of the COPI complex. AP1/2/3 and a substructure (heterotetrameric, F-COPI subcomplex) of the heptameric COPI have a common ancestral complex (called pre-F-COPI). Since all large and all small/medium subunits share sequence similarity, the ancestor of this complex is inferred to have been a heterodimer composed of one large and one small subunit. (Schledzewski et al., 1999). An archain delta COP interacts with OsSGT1 which interacts with the Gamma adaptin bait ERP.

OsERP was also found to interact with xanthine dehydrogenase, PN29997. A BLAST analysis of the amino acid sequence of PN29997 indicated that this prey protein is 66% similar to the *Arabidopsis* xanthine dehydrogenase (GENBANK® Accession No. NP_195215). Xanthine dehydrogenase is the enzyme responsible for xanthine degradation. Xanthine dehydrogenase is involved in purine catabolism and stress reactions. A BLAST analysis comparing the nucleotide sequence of PN29997 against TMRI's GENECHIP® Rice Genome Array sequence database identified probeset OS013724 (100%) as the closest match. Gene expression experiments indicated that this gene is expressed in seeds.

OsERP was also found to interact with ubiquitin specific protease, PN30843. A BLAST analysis of the amino acid sequence of PN30843 indicated that this prey protein is 40% similar to an *Arabidopsis* ubiquitin specific protease (GENBANK® Accession No. AAG42761). The ubiquitin/26S proteasome pathway is a major route for selectively degrading cytoplasmic and nuclear proteins in eukaryotes. In this pathway, chains of ubiquitins become attached to short-lived proteins, signaling recognition and breakdown of the modified protein by the 26S proteasome. During or following target degradation, the attached multi-ubiquitin chains are released and subsequently disassembled by ubiquitin-specific proteases (UBPs) to regenerate free ubiquitin monomers for re-use. T-DNA insertion mutations in an *Arabidopsis* ubiquitin protease cause an embryonic lethal phenotype, with the homozygous embryos arresting at the globular stage. The arrested seeds have substantially increased levels of multi-ubiquitin chains, indicative of a defect in ubiquitin recycling. Thus, there is essential role for the ubiquitin/26S proteasome pathway in general and for AtUBP14 in particular during early plant development (Doelling et al., 2001). SGT1 also interacts with components of the ubiquitin/26S proteasome pathway and the ERP that interacts with this ubiquitin specific protease interacts with OsSGT. This protease can be have roles in disease resistance as well as development.

OsERP was also found to interact with pectinesterase, PN30845. A BLAST analysis of the amino acid sequence of PN30845 indicated that this prey protein is 71 % similar to a rice pectinesterase (GENBANK® Accession No. BAB64824). Pectinesterases catalyse the esterification of cell wall polygalacturonans. In dicot plants, these ubiquitous cell wall enzymes are involved in important developmental processes including cellular adhesion and stem elongation. A BLAST analysis comparing the nucleotide sequence of PN30845 against TMRI's GENECHIP® Rice Genome Array sequence database identified probeset OS007057 (99%) as the closest match. Gene expression experiments indicated that this gene is up-regulated as a result of JA treatment, high saline growth conditions and herbicide treatment.

OsERP was also found to interact with several proteins, namely PN30870, PN29984, PN30844, PN29983, PN30868 and PN30857. A BLAST analysis of the amino acid sequence of PN30870, PN29984, PN30844, PN29983, PN30868 and PN30857 indicates that these prey proteins have no sufficient homology to any other characterized proteins. However, based on association with the rice elicitor responsive protein PN20696, these proteins can have roles in disease resistance or cell cycling.

A BLAST analysis comparing the nucleotide sequence of PN30857 against TMRI's GENECHIP® Rice Genome Array sequence database identified probeset OS008661.1 (99%) as the closest match. Gene expression experiments indicated that this gene is up-regulated as a result of blast infection.

An *A. thaliana* homologue to PN29983 was identified by BLAST. At2g36950 shares 52% amino acid similarity with PN29983. To see if *Arabidopsis* homologues of PN29983 have roles in disease resistance, *Arabidopsis thaliana* with T-DNA insertions in *At2g36950* (line SAIL_779_E11) was identified from a random insertion seed library. DNA regions surrounding the insertions were sequenced and revealed that the T-DNAs were located within exon 3 of *At2g36950*. Plants were backcrossed and plants homozygous for the T-DNA insertion were identified by PCR. Homozygous mutants and wild type plants were challenged with *Pseudomonas syringae* pv. *maculicola* ES4326 and plants were assayed for amount of *P. syringae* bacteria accumulation 3 days post inoculation (Glazebrook et al., 1996). These experiments were repeated twice on at least six plants. Data are reported as means and standard deviations of the log of colony forming units per leaf cm². By three days after inoculation, the mutant plants accumulated more than 10 times as much bacteria as wild type plants (wt = 3.94 log cfu/leaf disk std. 0.57, *at2g36950* = 5.95 std. 0.72). Hence, At2g36950 contributes to disease resistance in *A. thaliana.* It is possible that the At2g36950 mutation inhibits defense responses that are dependent upon ERP/SGT1 interactions.

It should be noted that the all of the following bait proteins, namely OsSGT, ring zinc finger, PN20115, and shaggy kinase, PN20621, identified proline rich protein, PN23221, as their prey. OsSGT and PN23221 have been described earlier in this Example.

A BLAST analysis of the amino acid sequence of ring zinc finger PN20115 indicated that this bait protein is 65% similar to *A. thaliana* ring zinc finger protein At1g63170. The RING domain is a conserved zinc finger motif, which serves as a protein-protein interaction interface. This protein can interact with other proteins to control developmental or stress tolerance processes. A BLAST analysis comparing the nucleotide sequence of PN20115 against TMRI's GENECHIP® Rice Genome Array sequence database identified probeset OS015830 (90%) as the closest match. Gene expression experiments indicated that this gene is up-regulated as a result of conditions of drought.

A BLAST analysis of the amino acid sequence of shaggy kinase PN20621 indicated that this bait protein is the rice shaggy kinase (gi|13677093). GSK3/SHAGGY is a highly conserved serine/threonine kinase implicated in many signaling pathways in eukaryotes. Many GSK3/SHAGGY-like kinases have been identified in plants. The *Arabidopsis* BRASSINOSTEROID-INSENSITIVE 2 (*BIN*2) gene encodes a GSK3/SHAGGY-like kinase. Gain-of-function mutations within its coding sequence or its overexpression inhibit brassinosteroid (BR) signaling, resulting in plants that resemble BR-deficient and BR-response mutants. In contrast, reduced BIN2 expression via cosuppression partially rescues a weak BR-signaling mutation. Thus, BIN2 acts as a negative regulator to control steroid signaling in plants (Li & Nam, 2002).

### Summary

As one of the major human staples, rice has been a target of genetic engineering for higher yields and resistance to diseases, pests, and environmental stresses of various kinds. The proteins identified in the present Example have presumed roles in cell cycle processes and/or the stress response. Knowledge of the proteins and molecular interactions associated with cell cycle processes and stress response in rice could lead to important applications in agriculture. Modulation of these interactions can be exploited to effect changes in plant development or growth that would result in increased crop yield and tolerance to environmental stress conditions.

Plant disease response often mimics certain normal developmental processes. For example, plants responses to fungal gibberellic acid and fusicoccin toxin are similar to responses to plant-produced gibberellin and auxin, respectively (Hedden & Kamiya, 1977; Baunsgaard et al., 1998). The same can be said for abiotic stress responses and certain stages of plant development. Leaf cells undergoing dehydration stress express some of the same genes that embryonic cells express during development or seed desiccation (Medina et al., 2001). Since systematic regulation of gene expression drives developmental processes and stress responses (Chen et al., 2002) it is likely that there is a broader overlapping set of genes and their cognate proteins involved in such responses. This Example describes one such overlapping set of genes.

The results described in this Example are useful for predicting gene function in rice or other plants. For example, rice has a homolog (OsSGT1; GENBANK® Accession No. AAF18438) to the barley SGT1 and *A. thaliana* SGT1b proteins that participate in pathogen defense through interactions with resistance gene and ubiquitinylation protein degradation pathways. *OsSGT1* is inducible by blast infection and likely participates in pathogen defense. OsSGT1 interacted with several undefined and known proteins, including one whose transcript is induced upon treatment with a rice blast fungal elicitor (GENBANK® Accession No. AF090698). The elicitor-responsive protein (OsERP) interacted with other undefined proteins and an ubiquitin protease-related protein, which implicates OsERP in SGT1 mediated protein degradation. These rice proteins, as well as other plant homologs, are suspected to have associated roles in disease resistance.

*A. thaliana* proteins homologous to OsERP (PN20696), Ras GTPase (PN24063), Archain delta COP-like (28982), fibrillin-like (PN29042) and to one of the undefined proteins that interacted with OsERP (PN29983) have also been identified. *A.thaliana* homozygous for insertion mutations in the cognate genes were challenged with *Pseudomonas syringae.* By three days after inoculation, the mutant plants accumulated more than 10 times as many bacteria as wild type plants. Hence, these *Arabidopsis* homologs contribute to disease resistance in *A. thaliana.* It is possible that these mutations inhibit defense responses that are dependent upon SGT1 interactions. Based upon homology and the interaction map, the rice homologs from which are associated the *Arabidopsis* genes can also involved in disease resistance and other processes utilizing SGT1 as a factor. These results demonstrate that the combined datasets can be used to predict gene functions that can be verified using phenotypes of mutants.

### Example VIII

This Example describes the identification and characterization of rice proteins that interact at the cell wall in response to biotic stress. As has been described above, an automated, high-throughput yeast two-hybrid assay technology was used to identify proteins interacting with rice chitinase, class III, and with cellulose synthase catalytic subunit. The sequences encoding the protein fragments used in the search were then compared by BLAST analysis against proprietary and public databases to determine the sequences of the full-length genes. The proteins found appear to be localized or targeted to the cell wall and to participate in the plant pathogen-induced defense response. The identification and characterization of proteins participating in pathways and biochemical reactions associated with defense against pathogens in rice can allow the development of genetically modified crops with enhanced or reduced disease resistance.

Chitinases are glycohydrolases that degrade chitin, a structural component of insects and plant pathogens such as nematodes, fungi, and bacteria. These enzymes are involved in multiple biological functions that include defense against chitin-containing pathogens, with class III chitinases having a substrate specificity for bacterial cell walls (Brunner et al., 1998). Chitinase was chosen as a bait for these interaction studies based on its relevance to TMRI's plant health programs. The high potential for specific enzyme-substrate interactions makes these proteins suitable for two-hybrid assays. The identification of rice genes encoding proteins involved in the plant response to pathogens are important to agriculture, as their discovery can allow genetic manipulation of crops to obtain plants with enhanced or reduced disease resistance.

The second bait used in this Example, namely cellulose synthase catalytic subunit, is part of a membrane-bound enzyme complex involved in the synthesis of cellulose, an essential component of the cell wall of higher plants whose production is central to morphogenesis and many other biological processes in plants (reviewed in Perrin, 2001).

This example provides newly characterized rice proteins interacting with a rice chitinase, class III (OsCHIB1), and with rice cellulose synthase catalytic subunit, RSW1-like (OsCS). An automated, high-throughput yeast two-hybrid assay technology (provided by Myriad Genetics Inc., Salt Lake City, Utah, United States of America) was used to search for protein interactions with the chitinase and cellulose synthase bait proteins.

### Results

Chitinase, class III, was found to interact with rice catalase A, an antioxidant enzyme that is part of the plant's detoxification mechanism against molecules induced in response to environmental stresses. A second interactor, cellulose synthase catalytic subunit, is an enzyme involved in cellulose biosynthesis and is the second bait protein of this Example. The search also identified four novel rice proteins interacting with chitinase: a protein similar to plant ABC transporter proteins, which play an important role in defense responses by eliminating toxins from tissues; a peptidase similar to *Arabidopsis thaliana* glutamyl aminopeptidase, whose proteolitic activity can be associated with activation of signaling molecules during the response of the plant to pathogens; a protein similar to a putative ATPase from *A. thaliana,* and one unknown protein, similar to a putative protein from *A. thaliana.*

The cellulose synthase catalytic subunit bait clone was found to interact with itself and with twelve proteins. These include three known rice proteins: the DNAJ homologue, a type of molecule known to participate in the plant protective stress response as a regulator of heat shock proteins, and two proteins that function as membrane-spanning pumps: the product of the salT gene, which is induced by salt and stress, and the channel protein aquaporin. Nine interactors are novel proteins: a DNA-damage inducible-like protein with a putative role in the plant defense mechanism against nucleic acid damage; a putative BAG protein which presumably participates in the plant stress response by regulating heat shock proteins; a protein similar to the riboflavin precursor 6,7-dimethyl-8-ribityllumazine synthase precursor from *A. thaliana* and possibly involved in biosynthesis of riboflavin during oxidative stress; a protein similar to soybean calcium-dependent protein kinase and one similar to *A. thaliana* putative zinc finger protein, with likely roles as mediators of molecular signaling or transcription following damage to the cell wall; and four proteins of unknown function.

The interacting proteins of the Example are listed in Table 29 and Table 30 below, followed by detailed information on each protein and a discussion of the significance of the interactions. A diagram of the interactions is provided in Figure 5. The nucleotide and amino acid sequences of the proteins of the Example are provided in SEQ ID NOs: 151-176 and 317-328.

Some of the proteins identified represent rice proteins previously uncharacterized. These proteins appear to participate in the plant defense mechanism against pathogens. Based on their presumed biological function and on their ability to specifically interact with the chitinase and cellulose synthase bait proteins, the interacting proteins can be localized or targeted to the cell wall, where they are involved in biochemical reactions and gene induction associated with local or systemic defense against pathogens.

**Table 29**

| Interacting Proteins Identified for OsCHIB1 (Chitinase, Class III) | | | |
|---|---|---|---|
| The names of the clones of the proteins used as baits and found as preys are given. Nucleotide/protein sequence accession numbers for the proteins of the Example (or related proteins) are shown in parentheses under the protein name. The bait and prey coordinates (Coord) are the amino acids encoded by the bait fragment(s) used in the search and by the interacting prey clone(s), respectively. The source is the library from which each prey clone was retrieved. | | | |
| **Gene Name** | **Protein Name** **(GENBANK® Accession No.)** | **Bait Coord** | **Prey Coord** **(Source)** |
| **BAIT PROTEIN** | | | |
| OsCHIB1 PN19651 (SEQ ID NO: 318) | *O. sativa* Chitinase, Class III (AF296279; AAG02504) | | |

| **INTERACTORS** | | | |
|---|---|---|---|
| OsCATA PN20899 (SEQ ID NO: 320) | *O. sativa* Catalase A Isozyme (D29966; BAA06232) | 10-200 | 332-433 (input trait) |
| OsCS* PN19707 (SEQ ID NO: 322) | *O. sativa* Cellulose Synthase Catalytic Subunit, RSW1-Like (AF030052; AAC39333) | 10-200 | 411-489 (input trait) |
| OsPN22823 (SEQ ID NO: 152) | Novel Protein PN22823, Similar to ABC Transporter Proteins (T02187, AB043999.1, NP_171753; e = 0) | 10-200 | 25-106 (input trait) |
| OsPN22154 (SEQ ID NO: 154) | Novel Protein PN22154, Similar to *A. thaliana* Glutamyl Aminopeptidase (AL035525; e = 0) | 10-200 | 390-562 (input trait) |
| OsPN29041 (SEQ ID NO: 156) | Novel Protein PN29041, Fragment, Similar to *A. thaliana* Putative ATPase (AAG52137; e⁻¹⁷) | 10-200 | 2x 5-108 (input trait) |
| OsPN22020 (FL_R01_P005_C09.g.1a. Sp6a) (SEQ ID NO: 158) | Novel Protein PN22020, Fragment, Similar to *A. thaliana* Putative Protein (NP_197783; 3e^{- 34}) | 10-200 | 3x 76-170 128-170 (input trait) |

| | | | |
|---|---|---|---|
| * The cellulose synthase catalytic subunit was also used as a bait; its interactions are shown in Table 30. | | | |

**Table 30**

| Interacting Proteins Identified for OsCS (Cellulose Synthase Catalytic Subunit, RSW1-Like) | | | |
|---|---|---|---|
| **Gene Name** | **Protein Name** **(GENBANK® Accession No.)** | **Bait Coord** | **Prey Coord** **(Source)** |
| **BAIT PROTEIN** | | | |
| OsCS PN19707 (SEQ ID NO: 322) | *O. sativa* Cellulose Synthase Catalytic Subunit, RSW1-Like (AF030052; AAC39333) | | |

| **INTERACTORS** | | | |
|---|---|---|---|
| OsCS PN19707 (SEQ ID NO: 322) | *O. sativa* Cellulose Synthase Catalytic Subunit, RSW1-Like (AF030052; AAC39333) | 316-583 | 316-582 (input trait) |
| OsAAB53810 PN29086 (SEQ ID NO: 324) | *O*. *sativa* salT Gene Product (AF001395; AAB53810.1) | 316-583 | 6-145 (output trait) |
| OsPIP2A PN29098 (SEQ ID NO: 326) | *O. sativa* Aquaporin (AF062393) | 316-583 | 123-290 (output trait) |
| OsPN22825 (SEQ ID NO: 160) | Novel Protein PN22825, Fragment | 316-583 | 5-129 (input trait) |
| OsPN29076 (SEQ ID NO: 162) | Novel Protein PN29076, Fragment | 316-583 | 1-187 43-388 122-304 (output trait) |
| OsPN29077 (SEQ ID NO: 164) | Novel Protein PN29077, Fragment, Similar to *A. thaliana* DNA-Damage Inducible Protein DDI1-Like (BAB02792; 5e⁻⁹⁴) | 316-583 | 4x 1-242 (output trait) |
| OsPN29084 (SEQ ID NO: 166) | Novel Protein PN29084, Fragment, Similar to Soybean (*Glycine max*) Calcium-Dependent Protein Kinase (A43713, 2e⁻⁷⁹) | 316-583 | 3x 1-253 (output trait) |
| OsPN29113 (SEQ ID NO: 328) | *O. sativa* DNAJ Homologue (BAB70509.1) | 316-583 | 1-92 (output trait) |
| OsPN29115 (SEQ ID NO: 168) | Novel Protein PN29115, Fragment, Similar to *A. thaliana* 6,7-Dimethyl-8-Ribityllumazine Synthase Precursor (AAK93590, 6e⁻³⁷) | 316-583 | 1-188 (output trait) |
| OsPN29116 (SEQ ID NO: 170) | Novel Protein PN29116, Fragment | 316-583 | 1-169 (output trait) |
| OsPN29117 (FL_R01_P078_N1 1.fasta.contig1)* (SEQ ID NO: 172) | Novel Protein PN29117 | 316-583 | -7-151 (output trait) |
| OsPN29118 (SEQ ID NO: 174) | Novel Protein PN29118, Fragment | 316-583 | 1-136 (output trait) |
| OsPN29119 (FL_R01_P084_P 01.g.1a.Sp6a) (SEQ ID NO: 176) | Novel Protein PN29119, Fragment | 316-583 | -53 to 155 (output trait) |

| | | | |
|---|---|---|---|
| * OsPN29117 also interacts with heat shock protein hsp70 (OsHSP70, PN20775): three prey clones of OsPN29117 (one encoding amino acids 11-160, two encoding amino acids 29-160) from the output trait library interacted with a clone (amino acids 138-360) of OsHSP70 used as bait. | | | |

### Yeast Two-Hybrid Using OsCHIB1 (Chitinase, Class III) as Bait

The rice class III chitinase (GENBANK® Accession No. AF296279) is a 286-amino acid protein. Chitinases are glycohydrolases that degrade chitin. Chitin is a structural component of insects, nematodes, fungi, and bacteria. Chitinases are one of the several kinds of pathogenesis-related (PR) proteins induced in higher plants in response to infection by pathogens (reviewed in Stintzi et al., 1993). While chitinases perform multiple biological functions, the class III chitinases' substrate specificity for bacterial cell walls suggests a main role for these enzymes as defense proteins (Brunner et al., 1998). The enzyme directly attacks the pathogen by degrading the fungal or bacterial cell wall.

The bait fragment used in this search encodes amino acids 10 to 200 of OsCHIB1 (Chitinase, Class III). This region of the protein includes the active site of the enzyme (amino acids 127 to 135). There is no match for the gene encoding OsCHIB1 on TMRI's GENECHIP® Rice Genome Array.

OsCHIB1 (Chitinase, Class III) was found to interact with OsCATA. PN20899 (*O. sativa* Catalase A Isozyme; D29966; BAA06232). Catalase A (GENBANK® Accession No. D29966) is the product of the rice *CatA* gene, which was identified by Higo & Higo, 1996 as the homologue of the Cat-3 gene from Indian corn (*Zea mays*;GENBANK® Accession No. L05934). Both rice *CatA* and Z. *mays Cat-3* genes belong to the monocot-specific group, one of three groups into which plant catalase genes have been classified based on their molecular evolution from a common ancestor (Guan & Scandalios, 1996). Rice catalase A contains 491 amino acids with two catalytic sites in position H65 and N138, and a heme binding-site in position Y348. The heme group is a cofactor for catalases' enzymatic activity. Higo & Higo, 1996, showed that the *CatA* gene is expressed at high levels in seeds during early development and also in young seedlings, and that this gene is induced by the herbicide paraquat, but not or only slightly by abscisic acid (ABA), wounding, salicylic acid, and hydrogen peroxide.

Catalases are stress-induced enzymes found in almost all aerobic organisms. They are part of the enzymatic detoxification mechanism against active oxygen species (AOS) in plant cells. AOS are induced in response to environmental stress and act as signaling molecules to activate multiple defense responses through induction of PR genes and of other signaling molecules (e.g., salicylic acid, SA), leading to increased stress tolerance (Lamb & Dixon, 1997). AOS, however, can also damage proteins, membrane lipids, DNA and other cellular components of the plant. The balance between these two diverging effects depends on the tight control of cellular levels of AOS, which is achieved through a diverse battery of oxidant scavengers. Among these antioxidant molecules, catalases protect plant cells from the toxic effects of the AOS precursor hydrogen peroxide generated in the oxidative burst by converting it to dioxygen and water (reviewed in Dat et al., 2001).

OsCHIB1 (Chitinase, Class III) was found to interact with *O. Sativa* Cellulose Synthase Catalytic Subunit, RSW1-Like (OsCS; PN19707). The prey clone found in our search, retrieved from the input trait library, encodes amino acids 411 to 489 of rice cellulose synthase catalytic subunit. This region of the 583-amino acid protein is C-terminal to the transmembrane domains and is predicted by amino acid sequence analysis to be on the cytoplasmic side of the plasma membrane.

Cellulose synthase is a membrane-bound enzyme complex comprising multiple isoforms. Cellulose synthase catalytic subunit (GENBANK® Accession No. AF030052) is involved in the synthesis of cellulose, a polysaccharide that is an essential component of the cell wall of higher plants. Cellulose imparts mechanical properties to plants which determine plant growth and cell shape, and its production impacts many aspects of plant biology. Most plants synthesize cellulose at the plasma membrane through the activity of cellulose synthase. As part of a structure called the rosette, the enzyme extends nascent cellulose chains by adding a sugar nucleotide precursor, and these chains then assemble into microfibrils that align in the same direction on the surface of the plasma membrane. This process seems to depend on a precise organization and orientation of the rosette (Perrin, 2001). A mutation in the *A. thaliana rsw1* gene that causes cellulose disassembly results in altered root morphogenesis (Baskin et al., 1992), indicating that proper cellulose synthesis is critical to plant development and morphology. Arioli et al., 1998 showed that the *rsw1* gene in *A. thaliana* encodes a catalytic subunit of cellulose synthase. However, genetic and biochemical evidence now supports the concept that a family of genes encode the catalytic subunit of cellulose synthase in higher plants, with various members showing tissue-specific expression or being differentially expressed in response to various conditions. These topics are reviewed in Perrin, 2001. These authors indicate that the presence of many genes for the cellulose synthase catalytic subunit in plants suggests that multiple isoforms of cellulose synthase can be needed in the same cell for the formation of functional multimeric complexes, most likely dimers. In addition, many other polypeptides have been detected within the rosette whose identities have not been determined. Interaction studies aimed at identifying the proteins interacting with synthase can help elucidate the organization of the cellulose synthase rosette machinery and address some of the questions that still remain about the biosynthesis of cellulose. There is no match for the gene encoding OsCS on TMRI's GENECHIP® Rice Genome Array.

Cellulose synthase catalytic subunit was also used as a bait protein. Its interactors are shown in Table 30 and discussed in later in this Example.

OsCHIB1 (Chitinase, Class III) was found to interact with Protein PN22823, which is similar to ABC Transporter Proteins (OsPN22823). Protein PN22823 is a 1239-amino acid protein that includes ten predicted transmembrane domains (amino acids 45 to 61, 154 to 170, 174 to 190, 253 to 269, 295 to 311, 671 to 687, 715 to 731, 794 to 810, 818 to 834, and 933 to 949) and two ATP/GTP-binding site motifs A (P-loops) (amino acids 383 to 390 and 1031 to 1038). A BLAST analysis against the Genpept database indicated that PN22823 shares 55% identity with Japanese goldthread *(Coptis japonica)* CjMDR1 (GENBANK® Accession No. AB043999.1; e = 0.0). CjMDR1is a multidrug resistance gene expressed in the rhizome, where alkaloids are highly accumulated compared to other organs (Yazaki et al., 2001). Other proteins highly similar to PN22823 include *A. thaliana* putative ABC transporter (GENBANK® Accession No. T02187; e = 0) and putative P-glycoprotein (GENBANK® Accession No. NP_171753; e = 0). These types of proteins contain ATP-binding cassettes (ABC) and belong to a family that includes P-glycoprotein (P-gp) and multidrug resistance-associated protein (MRP) 2 (reviewed by Fardel et al., 2001). ABC proteins are membrane-spanning proteins that transport a wide variety of compounds across biological membranes, including phospholipids, ions, peptides, steroids, polysaccharides, amino acids, organic anions, drugs and other xenobiotics.

In mammals, ABC transporters participate in the biliary elimination of exogenous compounds and xenobiotics, and their expression can be up-regulated by these toxins. The large number of ABC transporter protein family members identified in *A. thaliana* (129 according to Sanchez-Fernandez et al., 2001), suggests an important role for these proteins in plants. In agreement with this notion, ABC transporters were among the immediate early genes found to be up-regulated in a tropical *japonica* rice cultivar (*Oryza sativa cv. Drew*) in response to jasmonic acid, benzothiadiazole, and/or blast infection (Xiong et al., 2001 b). This suggests that ABC proteins play a role in defense against toxins in plants as they do in mammals. Most of the ABC transporters characterized in plants to date have been localized in the vacuolar membrane and are considered to be involved in the intracellular sequestration of cytotoxins (reviewed in Leslie et al., 2001). Furthermore, plant ABC transporters appear to have a role equivalent to that of the mammalian ABC transporter in multidrug resistance, as shown in a study in which an ABC transporter protein was up-regulated in a *Nicotiana plumbaginifolia* cell culture following treatment with a close analog of the antifungal diterpene sclareol (Jasinski et al., 2001). MRP homologues isolated from *A. thaliana* (AtMRPs) are implicated in providing herbicide resistance to plants (Rea et al., 1998). There is also evidence that ABC transporter proteins act as hormone transporters as they do in mammals. Specifically, a mutation in one of the ABC transporters in A. *thaliana,* AtMRP5, results in decreased root growth and increased lateral root formation possibly due to the inability of the mutant AtMRP5 to act as an auxin conjugate transporter (Gaedeke et al., 2001).

A BLAST analysis comparing the nucleotide sequence of Novel Protein PN22823 against TMRI's GENECHIP® Rice Genome Array sequence database identified probeset OS_ORF012127_at (e⁻¹⁴⁵ expectation value) as the closest match. Gene expression experiments indicated that this gene is induced by the fungal pathogen *M. grisea.*

OsCHIB1 (Chitinase, Class III) was found to interact with protein PN22154, which is similar to *A. thaliana* Glutamyl Aminopeptidase (OsPN22154). OsPN22154 is a 173-amino acid protein fragment that is 65% identical to a protein from *A. thaliana* (GENBANK® Accession No. AL035525) described as a homologue of mouse aminopeptidase (GENBANK® Accession No. U35646). The cDNA sequence of the *A. thaliana* aminopeptidase-like protein and the rice genome sequence (as a template) were used to generate a rice DNA sequence coding for a protein of 874 amino acids, which is 54.7 % identical to the *A. thaliana* aminopeptidase-like protein. Indeed, domain analysis of the novel rice protein detected a peptidase M1 domain (amino acids 17 to 402), and a zinc-binding domain (amino acids 311 to 320), suggesting that this protein is a metallo-aminopeptidase. It is unclear whether this protein is encoded by an orthologue or an analogue of the *A. thaliana* aminopeptidase-like gene. A BLAST analysis comparing the nucleotide sequence of Novel Protein PN22154 against TMRI's GENECHIP® Rice Genome Array sequence database identified probeset OS_004263_at (4e⁻⁸³ expectation value) as the closest match. Gene expression experiments indicated that this gene is expressed in panicle.

OsCHIB1 (Chitinase, Class III) was found to interact with protein PN29041 (OsPN29041). A BLAST analysis indicated that this protein fragment is similar to putative ATPase from *A. thaliana* (GENBANK® Accession No. AAG52137; e⁻¹⁷). ATPases can be localized to the plasma membrane which is adjacent to the cell wall. There is no match for this gene on TMRI's GENECHIP® Rice Genome Array, and thus no gene expression data that would allow prediction of its function during stress or infection. It is possible that this protein can have no role in pathogen invasion. However, it is part of the chitinase multiprotein complex identified in this Example through the yeast two-hybrid interactions, which we suggest exists at the cell wall interface. One hypothesis is that the ATPase-like protein can reside in the plasma membrane and participate in cell wall synthesis. Further interaction data can help elucidate the biological significance of its participation in the chitinase multiprotein complex.

OsCHIB1 (Chitinase, Class III) was found to interact with protein PN22020 (OsPN22020). Protein PN22020 is a 175-amino acid protein fragment that shares 55% identity with *A. thaliana* putative protein (GENBANK® Accession No. NP_197783; 3e⁻³⁴). Analysis of the amino acid sequence identified a C2 domain (amino acids 5 to 90, e = 0.037), as found in protein kinase C isozymes, which suggests that PN22020 can participate in signaling pathways similar to those modulated by protein kinase C. Perhaps its interaction with chitin represents a signaling event that occurs in response to pathogen or toxin exposure. However, this domain has been detected in other kinases and nonkinase proteins (Ponting & Parker, 1996). Identification of the full amino acid sequence of novel protein PN22020 can make it possible to determine the class of C2 domain-containing proteins to which it belongs.

A BLAST analysis comparing the nucleotide sequence of Novel Protein PN22020 against TMRI's GENECHIP® Rice Genome Array sequence database identified probeset OS008182_r_at (e⁻¹⁰² expectation value) as the closest match. Gene expression experiments indicated that this gene is constitutively expressed in leaves, stems, roots, seeds, panicle and pollen.

### Yeast Two-Hybrid Using OsCS as Bait

A second bait, namely *O. sativa* Cellulose Synthase Catalytic Subunit, RSW1-Like (OsCS; PN19707; GENBANK® Accession No. AF030052), was also used. This protein is described earlier in this Example because it was found to interact with the bait protein *O. sativa* Chitinase, Class III (OsCHIB1; PN19651). The bait fragment used in the search encodes amino acids 316 to 583 of OsCS.

OsCS was found to interact with *O. sativa* Cellulose Synthase Catalytic Subunit, RSW1-like (OsCS). In other words, OsCS was found to interact with itself. The prey clone was retrieved from the input trait library, and encoded almost the same amino acids as the bait clone (the prey clone encoded amino acids 316 to 582). The self-interaction supports the concept of cellulose synthase acting as a dimer, as has been suggested (see Perrin, 2001)).

OsCS was also found to interact with *O. sativa* salT Gene Product (OsAAB53810). A BLAST analysis of the 145-amino acid protein OsAAB53810 amino acid sequence indicated that this protein is the rice salT Gene Product (GENBANK® Accession No. AAB53810.1; 100% identity; 3e⁸⁰). This protein is encoded by a cDNA clone, salT, which was isolated from rice roots subjected to salinity stress, as reported by Claes et al., 1990. These authors showed that the salT mRNA is specifically expressed in sheaths and roots from mature plants and seedlings in response to salt stress and drought. Expression data reported previously by Garcia et al., 1998 indicate that expression of salT in each region of the plant is dependent on the metabolic activity of the cells as well as on whether or not they are responding to stress. These authors also found that the salT gene is induced by gibberellic acid and abscisic acid and suggest that induction by these growth regulators occurs through independent and possibly antagonistic pathways. Analysis of the OsAAB53810 protein sequence predicted a jacalin-like lectin domain (amino acids 14 to 145, 2.3e⁻³²). Jacalin interacts with carbohydrates in a highly specific manner (Sankaranarayanan et al., 1996).

OsCS was also found to interact with Aquaporin (OsPIP2a). Aquaporin (GENBANK® Accession No. AF062393) is a 290-amino acid protein that includes six predicted transmembrane domains (amino acids 48 to 64, 83 to 99, 131 to 147, 175 to 191, 207 to 223, and 254 to 270) and a Major Intrinsic Protein (MIP) family signature (amino acids 34 to 271), as determined by amino acid sequence analysis. The prey clone retrieved from the output trait library encodes amino acids 123 to 290 of OsPIP2a, a region that includes the four most C-terminal predicted transmembrane domains and part of the MIP family signature. Aquaporin is thought to be a plasma membrane intrinsic protein (Malz & Sauter, 1999). Such proteins facilitate movement of small molecules, often times functioning as water channels. This is why OsPIP2a is also called aquaporin. Malz and Sauter identified OsPIP2a along with OsPIP1a and report that these two proteins possess several hallmark motifs and homologies that justify their assignment to their respective PIP subfamilies. They report that OsPIP2a and OsPIP1a display similar, but not identical, expression patterns in rice, both being expressed at higher levels in seedlings than in adult plants, and that expression in the primary root is regulated by light. Furthermore, their study indicates that gibberellic acid also regulates the expression of these OsPIP transcripts in internodes of deepwater rice plants induced to grow rapidly by submergence, although expression did not correlate with growth. In *A*. *thaliana,* different PIP proteins are expressed in response to different agonists and conditions, e.g., salt stress induced tonoplast intrinsic protein (SITIP), as reported by Pih et al., 1999. These authors suggest that PIP proteins can be responsible for osmoregulation in plants under high osmotic stress such as a high salt condition.

OsCS was also found to interact with protein PN22825 (OsPN22825). OsPN22825 is a 229-amino acid protein fragment for which the complete sequence is not known. A BLAST analysis against the public and Myriad's proprietary databases indicated that OsPN22825 is similar to two unknown proteins from *A. thaliana* (GENBANK® Accession No. NP_188565, 67% identity, 3e⁻⁸²; and GENBANK® Accession No. AB025624, 37% identity, 3e⁸²). There is no match for the gene encoding OsPN22825 on TMRI's GENECHIP® Rice Genome Array, and thus no gene expression data that would allow prediction of its function during stress or infection.

OsCS was also found to interact with protein PN29076 (OsPN29076). OsPN29076 is a 389-amino acid protein fragment for which the complete sequence is not known. Analysis of the available amino acid sequence identified a cytochrome c family heme-binding site (amino acids 142 to 147). A BLAST analysis revealed no proteins with high similarity to OsPN29076, the best hit being an *A. thaliana* unknown protein (GENBANK® Accession No. AAF24616, 34% identity, 3e⁻⁴⁶). Three prey clones encoding amino acids 1 to 187, 42 to 389, and 121 to 304 of OsPN29076 were retrieved from the output trait library. The clones share an overlapping region which spans amino acids 121 to 187 of OsPN29076 and which includes the cytochrome c family heme-binding site. There is no match for the gene encoding OsPN29076 on TMRI's GENECHIP® Rice Genome Array, and thus no gene expression data that would allow prediction of its function during stress or infection. The lack of information about OsPN29076 makes it difficult to determine its function. Identification of the complete amino acid sequence for OsPN29076 can contribute to clarifying the function of this protein and the biological significance of the OsCS-OsPN29076 interaction.

OsCS was also found to interact with protein PN29077, which is similar to *A. thaliana* DNA-Damage Inducible Protein DDI1-Like (OsPN29077). OsPN29077 is 243-amino acid protein fragment for which the complete sequence is not known. A BLAST analysis indicated that OsPN29077 shares 73% identity with *A. thaliana* DNA-damage inducible protein DDI1-like (GENBANK® Accession No. BAB02792; 5e⁻⁹⁴). DDI1 is thought to be a cell-cycle checkpoint protein in yeast and its expression is induced by a variety of DNA-damaging agents. Such proteins arrest cells at certain stages and regulate the transcriptional response to DNA damage (Zhu & Xiao, 1998). DDI1 has been reported to interact with ubiquitin (Bertolaet et al., 2001), an observation that supports the use of the yeast two-hybrid approach to study such proteins.

A BLAST analysis comparing the nucleotide sequence of OsPN29077 against TMRI's GENECHIP® Rice Genome Array sequence database identified probeset OS016688.1_at (e⁻⁸³ expectation value) as the closest match. Gene expression experiments indicated that this gene is not specifically expressed in several different tissue types and is not specifically induced by a broad range of plant stresses, herbicides, and applied hormones.

OsCS was also found to interact with protein PN29084, which is similar to *G. max* calcium-dependent protein kinase (OsPN29084). OsPN29084 is a 284-amino acid protein fragment for which the complete sequence is not known. Analysis of the available amino acid sequence identified four EF-hand calcium-binding domains (amino acids 110 to 122, 146 to 158, 182 to 194, and 216 to 228). In agreement with the presence of these domains, a BLAST analysis indicated that OsPN29084 is highly similar to many calcium-dependent protein kinases including soybean (*G. max*) calcium-dependent protein kinase (GENBANK® Accession No. A43713, 81% identity, 2e⁻⁷⁹). This soybean protein also includes four EF-hand calcium-binding domains and requires calcium but not calmodulin or phospholipids for activity (Harper et al., 1991). Calcium can function as a second messenger through stimulation of such calcium-dependent protein kinases.

A BLAST analysis comparing the nucleotide sequence of OsPN29084 against TMRI's GENECHIP® Rice Genome Array sequence database identified probeset OS004083.1_at (e⁻⁸³ expectation value) as the closest match. Gene expression experiments indicated that this gene is not specifically expressed in several different tissue types and is not specifically induced by a broad range of plant stresses, herbicides, and applied hormones.

OsCS was also found to interact with *O. sativa* DNAJ homologue (OsPN29113). OsPN29113 is a 92-amino acid protein whose sequence includes an ATP/GTP-binding site motif A (P-loop, amino acids 43 to 50). A BLAST analysis of the available amino acid sequence indicated that OsPN29113 is the rice DNAJ homologue (GENBANK® Accession No. BAB70509.1; 100% identity; 5e⁻³⁹). In eukaryotic cells, DnaJ-like proteins regulate the chaperone (protein folding) function of Hsp70 heat-shock proteins through direct interaction of different Hsp70 and DnaJ-like protein pairs (Cyr et al., 1994). Heat shock proteins (reviewed in Bierkens et al., 2000) are stress proteins that function as intracellular chaperones to facilitate protein folding/unfolding and assembly/disassembly. They are selectively expressed in plant cells in response to a range of stimuli, including heat and a variety of chemicals. As regulators of heat shock proteins, DnaJ-like proteins are thus part of the plant protective stress response.

A BLAST analysis comparing the nucleotide sequence of OsPN29113 against TMRI's GENECHIP® Rice Genome Array sequence database identified probeset OS002926_at (e⁻¹²⁴ expectation value) as the closest match. Gene expression experiments indicated that this gene is not specifically expressed in several different tissue types and is not specifically induced by a broad range of plant stresses, herbicides, and applied hormones.

OsCS was also found to interact with protein PN29115, which is similar to *A. thaliana* 6,7-dimethyl-8-ribityllumazine synthase precursor (OsPN29115). OsPN29115 is a 188-amino acid protein fragment for which the complete sequence is not known. The available sequence includes an ATP/GTP-binding site motif A (P-loop, amino acids 94 to 101) and a 6,7-dimethyl-8-ribityllumazine synthase family signature (amino acids 42 to 186), as determined by analysis of the available amino acid sequence. The presence of the latter domain is in agreement with the results of a BLAST analysis indicating that OsPN29115 shares 50% identity with *A. thaliana* putative 6,7-dimethyl-8-ribityllumazine synthase precursor (GENBANK® Accession No. AAK93590, 6e⁻³⁷). The cofactor riboflavin is synthesized from the precursor 6,7-dimethyl-8-ribityllumazine (Nielsen et al., 1986). Flavins are involved in numerous biological processes (reviewed by Massey, 2000). For example, they participate in electron transfer reactions and thereby contribute to oxidative stress through their ability to produce superoxide, but at the same time flavins participate in the reduction of hydroperoxides, the products of oxygen-derived radical reactions. Flavins also contribute to soil detoxification and are linked to light-induced DNA repair in plants. The chemical versatility of flavoproteins is controlled by specific interactions with the proteins with which they are bound.

A BLAST analysis comparing the nucleotide sequence of OsPN29115 against TMRI's GENECHIP® Rice Genome Array sequence database identified probeset OS015577_at (e⁻⁴¹ expectation value) as the closest match. Gene expression experiments indicated that this gene is not specifically expressed in several different tissue types and is not specifically induced by a broad range of plant stresses, herbicides, and applied hormones.

OsCS was also found to interact with protein PN29116 (OsPN29116). OsPN29116 is a 170-amino acid protein fragment for which the complete sequence is not known. Analysis of the available amino acid sequence identified a WD40 domain (amino acids 82 to 118), which is reported to participate in protein-protein interactions (Ajuh et al., 2001). A BLAST analysis indicated that OsPN29116 shares identity with two unknown proteins from *A. thaliana* (GENBANK® Accession No. T45879, 67% identity, e⁻⁶⁴; and GENBANK® Accession No. NP_181253, 69% identity, e⁻⁵⁸). The lack of information about OsPN29116 makes it difficult to determine its function. Identification of the complete amino acid sequence for OsPN29116 can clarify the function of this protein and the biological relevance of the OsCSC-OsPN29116 interaction.

A BLAST analysis comparing the nucleotide sequence of OsPN29116 against TMRI's GENECHIP® Rice Genome Array sequence database identified probeset OS016500_r_t (e⁻¹² expectation value) as the closest match. The expectation value is too low for this probeset to be a reliable indicator of the gene expression of OsPN29116.

OsCS was also found to interact with protein PN29117 (OsPN29117). OsPN29117 is a 237-amino acid protein that includes a ubiquitin domain (amino acids 12 to 84). Analysis of the amino acid sequence identified a BAG domain (amino acids 106 to 187, 2.1e⁻¹¹), which is known to bind and regulate Hsp70/Hsc70 molecular chaperones (Briknarova et al., 2001). The BAG family of cochaperones functionally regulates signal-transducing proteins and transcription factors important for cell stress responses, apoptosis, proliferation, cell migration and hormone action (Briknarova et al., 2001; Antoku et al., 2001). A BLAST analysis indicated that OsPN29117 shares identity with an *A. thaliana* unknown protein (GENBANK® Accession No. AAC14405, 44% identity, 4e⁻⁵²). In agreement with the notion that OsPN29117 is a member of the BAG family of proteins, it was also found to interact with hsp70 (OsHSP70; see note * under Table 30). Heat shock proteins (discussed above) are stress proteins which function as ATP-dependent intracellular chaperones and which are selectively expressed in plant cells in response to a range of stimuli, including heat and a variety of chemicals. As a regulator of heat shock proteins, the BAG protein OsPN29117 can thus be part of the plant protective stress response.

The prey clone retrieved in the search encodes amino acids 1 to 151 of OsPN29117, a region that includes the ubiquitin domain. Note that the prey clone includes a small portion (-7 to 0) of the 5' untranslated region, and thus its coordinates are shown in Table 2 as amino acids -7 to 151. A BLAST analysis comparing the nucleotide sequence of OsPN29117 against TMRI's GENECHIP® Rice Genome Array sequence database identified probeset OS017803_at (e⁻⁷³ expectation value) as the closest match. Gene expression experiments indicated that this gene is not specifically expressed in several different tissue types and is not specifically induced by a broad range of plant stresses, herbicides, and applied hormones.

OsCS was also found to interact with protein PN29118 (OsPN29118). OsPN29118 is a 136-amino acid protein fragment for which the complete sequence is not known. A BLAST analysis indicated that OsPN29118 has only weak similarity to proteins in the public domain and in Myriad's proprietary database, the best hit being an *A. thaliana* putative zinc finger protein SHI-like (GENBANK® Accession No. NP_201436, 42% identity, 5e¹⁵). The protein with the next highest identity is an *A. thaliana* hypothetical protein (GENBANK® Accession No. T04595, 38% identity, 9e⁻¹⁵). Discovery of the complete amino acid sequence for OsPN29118 can contribute to clarifying the function of this protein and the biological relevance of the OsCSC-OsPN29118 interaction.

A BLAST analysis comparing the nucleotide sequence of OsPN29118 against TMRI's GENECHIP® Rice Genome Array sequence database identified probeset OS004996.1_at (e-³⁸ expectation value) as the closest match. Gene expression experiments indicated that this gene is not specifically expressed in several different tissue types and is not specifically induced by a broad range of plant stresses, herbicides, and applied hormones.

OsCS was also found to interact with protein PN29119 (OsPN29119). OsPN29119 is a 327-amino acid protein fragment for which the complete sequence is not known. A BLAST analysis indicated that OsPN29119 shares 38% identity with an *A. thaliana* unknown protein, T17H3.9 (GENBANK® Accession No. AAD45997, 7e-⁵⁴). Discovery of the complete amino acid sequence for OsPN29119 can contribute to clarifying the function of this protein and the biological relevance of the OsCSC-OsPN29119 interaction. One prey clone encoding amino acids 1 to 155 of OsPN29119 was retrieved from the output trait library. This prey clone includes a portion of the 5' untranslated region and thus its coordinates are shown in Table 2 as amino acids -53 to 155. A BLAST analysis comparing the nucleotide sequence of OsPN29119 against TMRI's GENECHIP® Rice Genome Array sequence database identified probeset OS014829.1_at (e⁻¹³¹ expectation value) as the closest match. Gene expression experiments indicated that this gene is not specifically expressed in several different tissue types and is not specifically induced by a broad range of plant stresses, herbicides, and applied hormones.

### Summary

### Proteins that Interact with OsCHIB1 (Chitinases, Class III)

The yeast two-hybrid assay designed to search for proteins interacting with the chitinase bait proteins led to the isolation of proteins that appear to be associated with the plant defense response to pathogens. Resistance to disease occurs on several levels that include local and nonspecific systemic responses. The hypersensitive response (HR) in plants is a mechanism of local resistance to pathogenic microbes characterized by a rapid and localized tissue collapse and cell death at the infection site, resulting in immobilization of the intruding pathogen. This process is triggered by pathogen elicitors and orchestrated by an oxidative burst, which occurs rapidly after the attack (Lamb & Dixon, 1997). The accumulation of active oxygen species (AOS) is a central theme during plant responses to both biotic and abiotic stresses. AOS are generated at the onset of the HR and might be instrumental in killing host tissue during the initial stages of infection. AOS also act as signaling molecules that induce expression of PR genes and production of other signaling molecules which participate in the signal cascade that leads to PR gene induction. The triggering of defense genes can extend to the uninfected tissues and the whole plant, leading to local resistance (LR) and systemic acquired resistance (SAR; reviewed in Martinez et al., 2000). As a result of SAR, other portions of the plant are provided with long-lasting protection against the same and unrelated pathogens.

Hydrogen peroxide from the oxidative burst plays an important role in the localized HR not only by driving the cross-linking of cell wall structural proteins, but also by triggering cell death in challenged cells and as a diffusible signal for the induction in adjacent cells of genes encoding cellular protectants such as glutathione S-transferase and glutathione peroxidase, and for the production of salicylic acid (SA). SA is thought to act as a signaling molecule in LR and SAR through generation of SA radicals, a likely by-product of the interaction of SA with catalases and peroxidases, as reported by Martinez et al., 2000. These authors showed that recognition of a bacterial pathogen by cotton triggers the oxidative burst that precedes the production of SA in cells undergoing the HR, and that hydrogen peroxide is required for local and systemic accumulation of SA, thus acting as the initiating signal for LR and SAR. The involvement of catalase in SA-mediated induction of SAR in plants was previously demonstrated by Chen et al., 1993 who showed that binding of catalase to SA results in inhibition of catalase activity, and that consequent accumulation of hydrogen peroxide induces expression of defense-related genes associated with SAR.

In this study, chitinase was found to interact with catalase A. Given the established role of chitinase as a defense protein, this interaction is consistent with the presence of the stress-induced catalase during pathogen attack and suggests that both enzymes can be located at the cell wall, where they participate in PR gene induction. The significance of the chitinase-catalase interaction as part of the defense response against microbes finds further support in the observation that fungal catalase has a role in protecting necrotrophic fungi from the deleterious effects of AOS during colonization of a host expressing the HR (Mayer et al., 2001). These organisms were shown to secrete catalase, among other enzymes, to remove or inactivate AOS from the host.

In addition, the cell wall can play a role in defense against bacterial and fungal pathogens by receiving information from the surface of the pathogen from molecules called elicitors, and by transmitting this information to the plasma membrane of plant cells, resulting in gene-activated processes that lead to resistance. One type of biochemical reaction induced by elicitors and associated with the hypersensitive response is the synthesis and accumulation of phytoalexins, antimicrobial compounds produced in the plant after fungal or bacterial infection (reviewed in Hammerschmidt, 1999). One of the proteins found to interact with chitinase is an ABC transporter. ABC transporters are known to sequester cytotoxins, metabolites and other molecules from plant tissues. It is thus likely that the ABC transporter found to interact with chitinase resides at the cell wall, where it participates in the transport of toxins. Though the function of phytoalexins in the plant defense response has not been thoroughly elucidated (Hammerschmidt, R., 1999), it is tempting to speculate that the ABC transporter can be involved in the elimination of these toxins from the plant cells during the plant pathogen-induced defense response. Furthermore, gene expression experiments indicated that the gene encoding the ABC transporter protein is induced by the fungal pathogen *M. grisea.* These results are consistent with the putative role of this protein in the defense response induced by pathogenic fungi and bacteria in rice.

Chitinase was also found to interact with novel protein PN22154 similar to *A. thaliana* glutamyl aminopeptidase. While the specific function of this prey protein has not been determined, it is well known that proteolytic activity is a common component of plant defense mechanisms against pathogens. These mechanisms include both chitinases and proteases. Peptidase activity has been associated with regulation of signaling. Carboxypeptidases, for instance, hydrolytically remove the pyroglutamyl group from peptide hormones, thereby activating these signaling molecules. A carboxypeptidase regulates Brassinosteroid-insensitive 1 (BRI1) signaling in *A. thaliana* by proteolytic processing of a protein (Li et al., 2001). Based on its ability to interact with chitinase and on the well-established role of the latter in PR defense, chitinase and novel protein PN22154 can interact as components of a complex with chitinolytic and proteolytic activities targeted against plant invaders, and that the rice glutamyl aminopeptidase-like protein can have a role in activating signaling molecules at the cell wall that are involved in the plant defense response.

A fourth interactor found for chitinase is cellulose synthase catalytic subunit. This enzyme acts as a complex at the plasma membrane where it participates in cell wall synthesis, and its regulation can allow the plant to respond with morphological changes to physical insult produced by pathogen attack. This interaction can be significant to maintaining the balance of the metabolism of cell wall components during the defense response. It is possible that either chitinase resides at the cell wall where it interacts with cellulose synthase immediately following pathogen attack, or chitinase is targeted to this site and interacts with synthase after PR gene induction.

Aside from novel proteins PN22020 and PN29041, the rice proteins found to interact with chitinase appear to be localized at or recruited to the cell wall where they participate in the plant defense response to pathogen attack. Two of the interactors, an ABC transporter and a glutamyl aminopeptidase-like protein, are newly characterized proteins in rice.

As a whole, all of these proteins can interact as a multicomponent complex at the cell wall interface in the plant cell, and all can have roles in controlling AOS levels, inducing PR genes, and synthesizing and maintaining the integrity of the cell wall to protect the plant against the effects of pathogen invasion.

### Proteins that interact with Cellulose Synthase Catalytic Subunit (OsCS)

The interactions involving OsCS expand the stress-response protein network identified for the chitinase bait protein. OsCS interacts with several proteins that appear to participate in the plant response to pathogen-induced stress at the cell wall. Published evidence links some of these proteins to the plant response to various stresses. These include aquaporin (OsPIP2a) and salt-stress induced protein (OsAAB53810), two molecules that, although they can not have a direct role in disease resistance, can function as membrane-spanning pumps in the protein complex at the cell wall to regulate turgor pressure or transmit solutes. Moreover, the presence of the jacalin-like lectin domain in OsAAB53810 is of particular interest in the context of its interaction with an enzyme that synthesizes carbohydrate chains. Given the carbohydrate-binding property of jacalin (Sankaranarayanan et al., 1996), OsAAB53810 can specifically bind nascent cellulose chains as they are produced by OsCS, thus playing an active role in OsCS-dependent events relating to cell wall metabolism. The fact that OsAAB53810 is induced by salt and stress supports a role for this protein in such physiological events.

Another interactor, the rice DNAJ homologue OsPN29113, likely participates in the plant protective stress response by regulating the chaperone function of heat shock proteins, which are induced by various forms of stress. It is possible that the interaction of the DNAJ protein with cellulose synthase is part of the plant response to chemicals produced by pathogens or generated in cells undergoing the HR, and that such response is associated with injury to the cell wall that has occurred in response to the stress.

Among the novel proteins found to interact with OsCS, OsPN29077 is similar to *A. thaliana* DNA-damage inducible protein DDI1-like. Based on the expression of yeast DDI1 in response to DNA damage and on sequence homology, we speculate that OsPN29077 performs the same function as DDI1 and that the OsCS-OsPN29077 interaction is associated with the plant defense mechanism against DNA damage. Likewise, we attribute the BAG-like protein OsPN29117 a putative role in the plant protective stress response as a regulator of heat shock proteins. In agreement with this role, OsPN29117 also interacts with hsp70, which gene expression experiments indicate is expressed constitutively and is down-regulated by jasmonic acid, a component of plant defense response pathways. Since OsPN29077 and OsPN29117 interact with the cellulose synthase catalytic subunit, and the latter interacts with the pathogen-induced defense protein chitinase, these interactors can be a part of the same complex at the cell wall where they participate in the response to pathogen attack.

The novel protein OsPN29115 is similar to the riboflavin precursor 6,7-dimethyl-8-ribityllumazine synthase precursor from *A. thaliana.* Among the roles reported for riboflavin is its association with the redox reactions occurring as a result of oxidative stress (Massey, 2000). Based on this evidence and on sequence homology for the identified interactor, the OsCS-OsPN29115 interaction can link the plant response to stress and toxins produced by pathogens with structural changes requiring OsCS activity.

Additional novel proteins interacting with OsCS include a protein similar to soybean calcium-dependent protein kinase (OsPN29084) and a protein similar to *A. thaliana* putative zinc finger protein (OsPN29118). The similarities of these interactors to protein kinases and zinc finger proteins suggest that they function as mediators of molecular signaling and transcription, respectively. Their interactions with OsCS can represent signaling or transcriptional events occurring after disruption following damage to the cell wall by pathogens, and these prey proteins can move from the cell wall to other parts of the cell to mediate such events. The OsCS-OsPN29084 interaction likely represents a step in the transduction of an extracellular signal that results in a physiological response, while the OsCS-OsPN29118 interaction can be associated with transcriptional regulation also in response to an extracellular signal. This signal can be in the form of an insult to the plant produced by pathogen attack.

For the remaining proteins found to interact with OsCS-OsPN22825, OsPN29076, OsPN29116, and OsPN29119--based on their association with cellulose synthase and chitinase, these prey proteins can also be important factors for pathogen defense, cell wall integrity, or for holding together protein complexes.

Thus, the results presented in this Example show that proteins interacting with the cellulose synthase catalytic subunit are also part of the chitinase multiprotein complex localized at the cell wall interface.

### Example IX

Janssens & Goris teach that type 2A serine/threonine protein phosphatases (PP2A) are important regulators of signal transduction, which they affect by dephosphorylation of other proteins (Janssens & Goris, 2001). Members of the protein phosphatase 2A (PP2A) family of serine/threonine phosphatases contain a well-conserved catalytic subunit, the activity of which is highly regulated (Janssens and Goris, 2001). There are multiple PP2A isoforms in plants and other organisms, and they appear to be differentially expressed in various tissues and at different stages of development (Arino et al., 1993). Harris et al. cites a number of reports describing the association of PP2A subunits with a variety of cellular proteins in addition to regulatory subunits, suggesting that PP2As function as regulators of various signaling pathways associated with protein synthesis, cell cycle and apoptosis (Harris et al., 1999). PP2A enzymes have been implicated as mediators of a number of plant growth and developmental processes.

In addition, PP2A enzymes play a role in pathogen invasion. In animals, a variety of viral proteins target specific PP2A enzymes to deregulate chosen cellular pathways in the host and promote viral progeny (Sontag, 2001; Garcia et al., 2000). PP2A enzymes interact with many cellular and viral proteins, and these protein-protein interactions are critical to modulation of PP2A signaling (Sontag, 2001). The proteins interacting with PP2A (e.g., PP2A) can, for example, target PP2A to different subcellular compartments, or affect PP2A enzyme activity. Moreover, PP2A enzymes play a role in plants in their response to viral infection (Dunigan & Madlener, 1995). Indeed, serine/threonine protein phosphatase is required for tobacco mosaic virus-mediated programmed cell death (Dunigan & Madlener, 1995).

OsPP2A-2 (GENBANK® Accession No. AF134552) is a 308-amino acid subunit of a family of protein phosphatases that contains a serine/threonine protein phosphatase signature (amino acids 112 to 117).

As described above, a yeast two-hybrid approach was taken to dissect PP2A-mediated signaling events. The bait fragments used in this search and found to have interactors encode amino acids 1 to 308 and 150-308 of OsPP2A-2.

The second bait used in this Example, OsCAA90866, is a protein encoded by a complete cDNA sequence that is only known to be inducible by chilling in rice. OsCAA90866 was chosen as a bait for these interaction studies based on its relevance to abiotic stress. Investigation into the interactions involving OsCAA90866 will provide insight into the function of this poorly defined protein. The identification of rice genes involved in modulating the response of the plant to an environmental challenge, thus conferring it a selective advantage, would facilitate the generation and yield of crops resistant to abiotic stress.

### Results

OsPP2A-2 was found to interact with rice putative proline-rich protein, which is possibly a transcriptional regulator, and with the seed storage protein glutelin. The search also identified five novel rice proteins interacting with OsPP2A-2: a putative PP2A regulatory subunit protein also similar to rice chilling-inducible protein CAA90866 (the second bait protein of this Example); an enzyme similar to phosphoribosylanthranilate transferase that is likely involved in the plant response to pathogen infection; a disulfide isomerase, with a putative role in protein folding; a voltage-dependent ion channel protein; and a DnaJ-like protein with a putative role in the pathogen-induced defense response.

The second bait protein of this Example, chilling-inducible protein CAA90866 was found to interact with itself and with six proteins. One of these is the same putative PP2A regulatory subunit protein (similar to the bait protein itself) found to interact with the bait OsPP2A-2 of described in this Example. This interaction links the two networks of proteins identified in this Example (i.e., links proteins associated with biotic and abiotic stress to phosphatases). The other interactors identified in this search include a 14-3-3-like protein that is induced under various abiotic stress conditions; a pyrrolidone carboxyl peptidase-like protein with a putative role in activating signaling peptides involved in the plant's response to cold stress; a novel protein containing an inositol phosphate domain likely involved in regulation of signaling events associated with cold tolerance; a novel rice homolog of wheat initiation factor (iso)4f p82 subunit with a putative role in RNA decay pathways associated with stress conditions; and a novel protein similar to plants 2-dehydro-3-deoxyphosphooctonate aldolase.

The interacting proteins of the Example are listed in Table 31 and Table 32 below, followed by detailed information on each protein and a discussion of the significance of the interactions. A diagram of the interactions is provided in Figure 6. The nucleotide and amino acid sequences of the proteins of the Example are provided in SEQ ID NOs: 177-192 and 329-340.

Some of the proteins identified represent rice proteins previously uncharacterized. Based on their presumed biological function and on their ability to specifically interact with the bait proteins OsPP2A-2 or OsCAA90866, it appears that the proteins interacting with OsPP2A-2 represent a network involved in the rice defense response to biotic stress, and those interacting with OsCAA90866 are associated with the abiotic stress response. Importantly, the interactions identified suggest that phosphatases play a role in the regulation of both biotic and abiotic stress response in rice.

**Table 31**

| Interacting Proteins Identified for OsPP2A-2 (Serine/Threonine Protein Phosphatase PP2A-2) | | | |
|---|---|---|---|
| The names of the clones of the proteins used as baits and found as preys are given. Nucleotide/protein sequence accession numbers for the proteins of the Example (or related proteins) are shown in parentheses under the protein name. The bait and prey coordinates (Coord) are the amino acids encoded by the bait fragment(s) used in the search and by the interacting prey clone(s), respectively. The source is the library from which each prey clone was retrieved. | | | |
| **Gene Name** | **Protein Name** **(GENBANK® Accession No.)** | **Bait Coord** | **Prey Coord** **(Source)** |
| **BAIT PROTEIN** | | | |
| OsPP2A-2 PN20254 (AF134552- OS002763) (SEQ ID NO: 330) | *O. sativa* Serine/Threonine Protein Phosphatase PP2A-2, Catalytic Subunit (AF134552, AAD22116) | | |

| **INTERACTORS** | | | |
|---|---|---|---|
| OsAAK63900 PN23266 (SEQ ID NO: 332) | *O. sativa* Putative Proline-Rich Protein AAK63900 (AC084884) | 1-308 | 122-224 (input trait) |
| OsORF020300-2233.2 PN21639 (2233(2)-OS-ORF020300 novel (SEQ ID NO: 178) | Hypothetical Protein ORF020300-2233.2, Putative PP2A Regulatory Subunit, Similar to OsCAA90866 (AAD39930; 5e⁻⁹²) (CAA90866; 5e⁻⁵³) | 1-308 | 93-387 118-388 (input trait) |
| OsPN23268 PN23268 novel (SEQ ID NO: 180) | Novel Protein 23268, Similar to Phosphoribosylanthranilate Transferase, Chloroplast Precursor, Fragment (AAB02913.1; 5e⁻⁹⁵) | 1-308 | 2x 12-200 (input trait) |
| OsCAA33838 PN24775 (SEQ ID NO: 334) | *O. sativa* Glutelin CAA33838 (X15833) | 150-308 | 5-155 (output trait) |
| OsPN26645 (Contig3412.fasta.Contig1 novel) (SEQ ID NO: 182) | Novel Protein PN26645, Putative Protein Disulfide Isomerase-Related Protein Precursor (BAB09470.1; e⁻²⁸) | 1-308 | 24-164 (input trait) |
| OsPN24162 (Contig3453.fasta.Contig1 novel (SEQ ID NO: 184) | Novel Protein PN24162, Porin-like, Voltage-Dependent Anion Channel Protein (NP_201551; 3e⁻⁸⁶) | 150-308 | 28-164 (output trait) |
| Os011994-D16 PN20618 (FL_R01_P028_D160S01 1994 novel (SEQ ID NO: 186) | Hypothetical Protein 011994-D16, Similar to *Z. mays* DnaJ protein (T01643; e = 0) | 150-308 | 99-368 (output trait) |

**Table 32**

| Interacting Proteins Identified for OsCAA90866 (*O*. *sativa* Chilling-Inducible Protein CAA90866) | | | |
|---|---|---|---|
| The names of the clones of the proteins used as baits and found as preys are given. Nucleotide/protein sequence accession numbers for the proteins of the Example (or related proteins) are shown in parentheses under the protein name. The bait and prey coordinates (Coord) are the amino acids encoded by the bait fragment(s) used in the search and by the interacting prey clone(s), respectively. The source is the library from which each prey clone was retrieved. | | | |
| **Gene Name** | **Protein Name** **(GENBANK® Accession No.)** | **Bait Coord** | **Prey Coord** **(Source)** |
| **BAIT PROTEIN** | | | |
| OsCAA90866 PN20311 (984756_OS015052) (SEQ ID NO: 336) | *O. sativa* Chilling-Inducible Protein CAA90866 (Z54153, CAA90866) | | |

| **INTERACTORS** | | | |
|---|---|---|---|
| OsCAA90866 PN20311 (SEQ ID NO: 336) | *O. sativa* Chilling-Inducible Protein CAA90866 (Z54153, CAA90866) | 100-250 | 1-126 (output trait) |
| Os008938-3209 PN20215 (3209-OS208938) (SEQ ID NO: 338) | *O. sativa* Putative 14-3-3 Protein (AAK38492) | 100-250 | 4x 53-259 (input trait) |
| OsAAG46136 PN23186 (SEQ ID NO: 340) | *O. sativa* Putative Pyrrolidone Carboxyl Peptidase (AAG46136) | 100-250 | 2x 92-222 (input trait) |
| OsORF020300-2233.2 PN21639 (SEQ ID NO: 178) | Hypothetical Protein ORF020300-2233.2, Putative PP2A Regulatory Subunit, Similar to OsCAA90866 (AAD39930; 5e⁻⁹²) (CAA90866, 5e⁻⁵³) | 100-250 | 3x 1-206 3x 1-190 (output trait) |
| OsPN23045 (SEQ ID NO: 188) | Novel Protein PN23045 | 100-250 | 2x 240-287 (input trait) |
| OsPN23225 (SEQ ID NO: 190) | Novel Protein PN23225, Similar to *Tritticum aestivum* Initiation Factor (iso)4f p82 Subunit (AAA74724; e=0) | 100-250 | 639-792 (input trait) |
| OsPN29883 (SEQ ID NO: 192) | Novel Protein PN29883, Fragment | 100-250 | 58-175 (output trait) |

### Two Hybrid Using OsPP2A as a Bait

The bait fragment encoding amino acids 1 to 308 of *O. sativa* Serine/Threonine Protein Phosphatase PP2A-2, Catalytic Subunit (OsPP2A-2) was found to interact with *O. sativa* (rice) putative proline-rich protein, which is possibly a transcriptional regulator. The bait fragment (i.e., aa 1-308 of OsPP2A-2) includes the serine/threonine protein phosphatase signature of OsPP2A-2. One prey clone encoding amino acids 122 to 224 of OsAAK63900 was retrieved from the input trait library. Somewhat surprisingly, this prey clone does not code for the HLH domain of OsAAK63900.

*O. sativa* Putative Proline-Rich Protein AAK63900 (OsAAK63900) (GENBANK® Accession No. AC084884) is a 224-amino acid protein that includes a putative transmembrane spanning region (amino acids 7 to 23). It also contains a gntR family signature (amino acids 10 to 34) common to a group of DNA-binding transcriptional regulation proteins in bacteria (see Buck & Guest, 1989; Haydon & Guest, 1991; Reizer et al., 1991). This signature includes a helix-loop-helix (HLH) protein dimerization domain (amino acids 5 to 20) that is often found in transcription factors (see Murre et al., 1989; Garrel & Campuzano, 1991, Kato & Dang, 1992; Krause et al., 1990; Riechmann et al., 1994). However, no DNA-binding motif is detectable.

Note that analysis of the amino acid sequence of OsAAK63900 also detected an Ole e I family signature (amino acids 30 to 162) including six conserved cysteines that are involved in disulfide bonds. This signature is a conserved region found in a group of plant pollen proteins of unknown function which tend to be secreted and consist of about 145 amino acids (and thus are shorter than OsAAK63900). The first of the Ole e I family of proteins to be discovered was Ole e I (IUIS nomenclature), a constitutive protein in the olive tree *Olea europaea* pollen and a major allergen (Villalba et al., 1993).

The bait fragment encoding amino acids 1 to 308 of OsPP2A-2 (which includes the serine/threonine protein phosphatase signature of OsPP2A-2) was also found to interact with *O. sativa* OsORF020300-223.2, a novel 418-amino acid protein which has a putative PP2A regulatory subunit, similar to OsCAA90866. Two prey clones encoding amino acids 93 to 387 and 118 to 388 of ORF020300-233 were retrieved from the input trait library, which indicates that OsORF020300-2233.2 interacts with OsPP2A-2 through a region within amino acids 118 to 387. OsORF020300-2233.2 includes a possible cleavage site between amino acids 50 and 51, although it appears to have no N-terminal signal peptide. OsORF020300-2233.2 is similar to *A. thaliana* PP2A regulatory subunit (GENBANK® Accession No. AAD39930.1; 44.5% amino acid sequence identity; 5e⁻⁹¹ expectation value). OsORF020300-2233.2 is also similar to rice chilling-inducible protein CAA90866 (GENBANK® Accession No. CAA90866, 68% sequence identity; 9e⁻⁴⁸ expectation value), a protein related to chilling tolerance in rice, with which OsORF020300-2233.2 also interacts. CAA90866 was also used as a bait protein, and the interactions identified for it are discussed later in this Example.

A BLAST analysis comparing the nucleotide sequence of OsORF020300-2233.2 against TMRI's GENECHIP® Rice Genome Array sequence database identified probeset OS015607_at (e⁻¹³⁵ expectation value) as the closest match. Gene expression experiments indicated that this gene is induced by the fungal pathogen *M. grisea*.

The bait fragment encoding amino acids 1 to 308 of OsPP2A-2 (which includes the serine/threonine protein phosphatase signature of OsPP2A-2) was also found to interact with a novel protein (PN23268), an enzyme similar to phosphoribosylanthranilate transferase that is likely involved in the plant response to pathogen infection. The novel protein, which was named OsPN23268, is similar to anthranilate phosphoribosyltransferase, a chloroplast precursor. Two prey clones encoding amino acids 12 to 200 of novel protein OsPN23268 were retrieved from the input trait library.

OsPN23268 is a novel 320-amino acid protein with a possible cleavage site between amino acids 43 and 44, although there does not appear to be an N-terminal peptide sequence. Analysis of the Os23268 protein sequence detected two domains originally defined in *E. coli* thymidine phosphorylase (Walter et al., 1990): the glycosyl transferase family, helical bundle domain (amino acids 1 to 61) and a glycosyl transferase family, a/b domain (amino acids 66 to 303). The latter contains a beta-sheet that is splayed open to accommodate a putative phosphate-binding site (Walter et al., 1990). Two prey clones of OsPN23268 retrieved from the input trait library and found to interact with OsPP2A-2 included sequence encoding amino acids 12 to 200 of novel protein OsPN23268. This sequence of OsPN23268 includes the glycosyl transferase family helical bundle domain and part of the a/b domain.

The glycosyl transferase family includes thymidine phosphorylase and anthranilate phosphoribosyltransferase enzymes. In mammalian cells, thymidine phosphorylase is identical to the angiogenic factor, platelet-derived endothelial cell growth factor (Morita et al., 2001; Browns & Bicknell, 1998), and it also controls the effectiveness of the chemotherapeutic drug capecitabine by converting it to its active form (Ackland & Peters, 1999). As its name indicates, novel protein 23268 is similar to *A. thaliana* phosphoribosylanthranilate transferase (GENBANK® Accession No. AAB02913.1; 56.6% identity; 5e⁻⁹⁵), an enzyme with a role in the tryptophan biosynthetic pathway which is also found in bacteria (Edwards et al., 1988). In *A. thaliana*, this tryptophan biosynthetic enzyme is synthesized as a higher-molecular-weight precursor and then imported into chloroplasts to be processed into its mature form (Zhao & Last, 1995). The *A. thaliana* anthranilate phosphoribosyltransferase is also similar to DESCA11 (GENBANK® Accession No. BI534445; e⁻¹⁷), one of the genes identified in *Chenopodium amaranticolor* (a plant with broad-spectrum virus resistance) which are induced during the hypersensitive response (HR) response of the plant subsequent to infection with tobacco mosaic virus and tobacco rattle tobravirus (Goff, 2001).

A BLAST analysis comparing the nucleotide sequence of OsPN23268 against TMRI's GENECHIP® Rice Genome Array sequence database identified probeset OS015603_s_at (3e⁻⁴¹ expectation value) as the closest match. The gene expression experiments disclosed herein indicate that this gene is induced by the fungal pathogen *M. grisea.*

The bait fragment of OsPP2A-2 containing amino acids 150 to 308 was also found to interact with the seed storage protein glutelin CAA33838 (OsCAA33838). Glutelin CAA33838 is the major seed storage protein in rice. Its cDNA sequence was identified by Wen et al., 1989, and the accumulation of the protein in rice endosperm occurs between five and seven days after flowering (Udaka et al., 2000). One prey clone encoding amino acids 5 to 155 of OsCAA33838 was retrieved from the output trait library.

OsCAA33838 (GENBANK® Accession No. X15833) is a 499-amino acid protein that includes a cleavable signal peptide (amino acids 1 to 24), as determined by analysis of the amino acid sequence. The analysis identified an 11S plant seed storage protein domain (amino acids 1 to 469; 1e⁻²⁴³). The 11S plant seed storage proteins tend to be glycosylated proteins that form hexameric structures. They are composed of two peptides linked by disulfide bonds and are also members of the cupin superfamily of proteins by virtue of their two beta-barrel domains. The analysis also detected this domain but localized it to a narrower region (amino acids 302 to 324). In addition, a 7S seed storage protein, C-terminal domain (amino acids 319 to 478; 602e⁻⁰⁴), was identified which is also found in members of the cumin superfamily. In agreement with the evidence that OsCAA33838 is a glycosylated protein, an N-glycosylation site (amino acids 491 to 494) was identified.

A BLAST analysis comparing the nucleotide sequence of OsCAA33838 against TMRI's GENECHIP® Rice Genome Array sequence database identified probeset OS000688.1_ at (e = 0 expectation value) as the closest match. Our gene expression experiments indicate that this gene is not specifically expressed in several different tissue types and is not specifically induced by a broad range of plant stresses, herbicides, or applied hormones.

The bait fragment of OsPP2A-2 was also found to interact with novel protein PN26645, a putative protein disulfide isomerase-related protein precursor (also called OsPN26645). The bait fragment used in this search encodes amino acids 1 to 308 of OsPP2A-2, which includes the serine/threonine protein phosphatase signature of OsPP2A-2. One prey clone encoding amino acids 24 to 164 of OsPN26645 was retrieved from the input trait library. OsPN26645 is a 311-amino acid protein that includes a cleavable signal peptide (amino acids 1 to 17) and a predicted transmembrane domain (amino acids 210 to 226), as determined by analysis of the amino acid sequence. A BLAST analysis against the Genpept database revealed that OsPN26645 is similar to an *A. thaliana* protein (GENBANK® Accession No. BAB09470.1; 32.8% identity; e⁻²⁸) that is similar to the rat protein disulfide isomerase-related protein precursor (GENBANK® Accession No. AAD46003, 46% identity, 1e⁻⁶³). As its name indicates, disulfide isomerase catalyzes the formation of disulfide bonds. This enzyme can therefore be important for proper protein folding. In mammals, disulfide isomerase in the lumen of the endoplasmic reticulum creates disulfide bonds in secretory and cell-surface proteins, and microsomes deficient in this enzyme are unable to conduct cotranslational formation of disulphide bonds (Bulledi & Freedman, 1988). Although the activity of this enzyme is not as well characterized in plants, it is likely that it serves in a similar capacity.

A BLAST analysis comparing the nucleotide sequence of OsPN26645 against TMRI's GENECHIP® Rice Genome Array sequence database identified probeset OS002485.1_at (e⁻¹⁰⁵ expectation value) as the closest match. Gene expression experiments indicated that this gene is not specifically expressed in several different tissue types and is not specifically induced by a broad range of plant stresses, herbicides, or applied hormones.

The bait fragment of OsPP2A-2 was also found to interact with novel protein PN24162 (OsPN24162), a porin-like, voltage-dependent anion channel protein. The bait fragment used in this search encodes amino acids 150 to 308 of OsPP2A-2. One prey clone encoding amino acids 28 to 164 of OsPN24162 was retrieved from the output trait library. BLAST analysis of the OsPN24162 amino acid sequence indicated that this protein is most similar to a porin-like protein from *A. thaliana* (GENBANK® Accession No. NP_201551; 53% amino acid sequence identity; 3e⁻⁸⁶). OsPN24162 is also similar to a rice mitochondrial voltage-dependent anion channel (GENBANK® Accession #Y18104; 44% identity; 2e⁻⁶¹), a 274-amino acid protein encoded by a cDNA found to belong to a small multigene family in the rice genome (Roosens et al., 2000). Expression of this gene was found to be regulated in function of the plantlets maturation and organs, and not responsive to osmotic stress (Roosens et al., 2000). Mitochondrial voltage-dependent ion channels are also called mitochondrial porins by analogy with the proteins forming pores in the outer membrane of Gram-negative bacteria.

A BLAST analysis comparing the nucleotide sequence of OsPN24162 against TMRI's GENECHIP® Rice Genome Array sequence database identified probeset OS007036.1_at (e⁻⁶⁵ expectation value) as the closest match. Our gene expression experiments indicate that this gene is not specifically expressed in several different tissue types and is not specifically induced by a broad range of plant stresses, herbicides, or applied hormones.

The bait fragment of OsPP2A-2 was also found to interact with search a DnaJ-like protein with a putative role in the pathogen-induced defense response. The bait fragment used in this search encodes amino acids 150 to 308 of OsPP2A-2. One prey clone encoding amino acids 99 to 368 of Os011994-D16 was retrieved from the output trait library. This new protein was named 011994-D16 or, because it was identified from O. sativa, Os011994-D16.

BLAST analysis of the Os011994-D16 amino acid sequence indicated that this protein is similar to maize (*Zea mays*) DnaJ protein homolog ZMDJ1 (GENBANK® Accession No. T01643; 84% identity; e = 0). In eukaryotic cells, DnaJ-like proteins regulate the chaperone (protein folding) function of Hsp70 heat-shock proteins through direct interaction of different Hsp70 and DnaJ-like protein pairs (Cyr et al., 1994). Heat shock proteins (reviewed in Bierkens et al., 2000) are stress proteins which function as intracellular chaperones to facilitate protein folding and assembly and which are selectively expressed in plant cells in response to a range of stimuli, including heat and a variety of chemicals. As regulators of heat shock proteins, DnaJ-like proteins are thus part of the plant protective stress response.

A BLAST analysis comparing the nucleotide sequence of Os011994-D16 against TMRI's GENECHIP® Rice Genome Array sequence database identified probeset OS009139.1_at (e = 0 expectation value) as the closest match. Gene expression experiments indicated that expression of this gene is repressed by the plant hormone jasmonic acid.

### Yeast Two-Hybrid Using O. sativa Chilling-Inducible Protein CAA90866 (OsCAA90866) as Bait

The bait protein, namely *O. sativa* chilling-inducible protein CAA90866 (OsCAA90866), is a 379-amino acid protein encoded by a complete cDNA sequence related to chilling tolerance in rice. BLAST analysis indicated that OsCAA90866 is similar to the same PP2A regulatory subunit from *A. thaliana* (GENBANK® Accession No. AAD39930; 35% amino acid sequence identity; e⁻⁵⁷ expectation value) that was found similar to OsORF020300-223, interactor for the bait protein PP2A-2 (see Example III). A BLAST analysis comparing the nucleotide sequence of the chilling-inducible protein against TMRI's GENECHIP® Rice Genome Array sequence database identified probeset OS015052_at (4e⁻⁷⁸ expectation value) as the closest match. Gene expression experiments indicated that this gene is induced by cold stress.

As described in Table 32, a bait clone encoding amino acids 100 to 250 of *O. sativa* Chilling-Inducible Protein CAA90866 (OsCAA90866) was found to interact with a prey clone encoding amino acids 1 to 126 of the same protein retrieved from the output trait library.

In addition, the bait clone encoding amino acids 100 to 250 of *O. sativa* Chilling-Inducible Protein CAA90866 (OsCAA90866) was found to interact with Os008938-3209. Four prey clones encoding amino acids 53-259 of Os008938-3209 were retrieved from the input trait library. Os008938-3209 is a 260-amino acid protein that includes a 14-3-3 protein signature 1 (amino acids 48-60) and a 14-3-3 protein signature 2 (amino acids 220 to 260), which suggests that Os008938-3209 is a member of the 14-3-3 family. BLAST analysis indicated that the amino acid sequence of Os008938-3209 shares 100% identity with that of rice putative 14-3-3 protein (GENBANK® Accession No. AAK38492, 8e⁻¹⁴⁵). The 14-3-3 proteins interact with regulators of cellular signaling, cell cycle regulation, and apoptosis. They are thought to act as molecular scaffolds or chaperones and to regulate the cytoplasmic and nuclear localization of proteins with which they interact by regulating their nuclear import/export (Zilliacus et al., 2001; reviewed by Muslin & Xing, 2000). Since 14-3-3 proteins participate in protein complexes within the nucleus (Imhof & Wolffe, 1999; Zilliacus et al., 2001), cytoplasm (De Lille et al., 2001), mitochondria (De Lille et al., 2001) and chloroplast (Sehnke et al., 2000), additional information would be necessary to determine where Os008938-3209 resides within the cell. Cellular localization of this prey protein could lead to a better interpretation of the significance of its interaction with chilling-inducible protein CAA90866.

A BLAST analysis comparing the nucleotide sequence of the Os008938-3209 protein against TMRI's GENECHIP® Rice Genome Array sequence database identified probeset OS008938_s_at (e⁻⁶¹ expectation value) as the closest match. Gene expression experiments indicated that this gene is induced by salicylic acid, ABA, BAP, BL2, and 2,4-D, during cold stress, and under drought conditions.

In addition, the bait clone encoding amino acids 100 to 250 of *O. sativa* Chilling-Inducible Protein CAA90866 (OsCAA90866) was found to interact with OsAAG46136, a pyrrolidone carboxyl peptidase from *O. sativa.* Two prey clones encoding amino acids 92-222 of OsAAG46136 were retrieved from the input trait library. These clones include the pyroglutamyl peptidase I motif of OsAAG46136.

OsAAG46136 is a 222-amino acid protein that contains a pyroglutamyl peptidase I motif (amino acids 11 to 221). This motif is found in the N-terminal regions of peptide hormones (including thyrotropin-releasing hormone and luteinizing hormone releasing hormone), and it confers protease resistance to the protein (Odagaki et al., 1999). BLAST analysis indicated that the amino acid sequence of OsAAG46136 shares 100% identity with that of rice putative pyrrolidone carboxyl peptidase (GENBANK® Accession No. AAG46136; 4e⁻¹²⁶). OsAAG46136 is also similar to two unknown proteins from *A. thaliana* (GENBANK® Accession Nos. NP_176063, 8e⁻⁰⁸⁰ and AAK25976.1, e⁻⁰⁷⁶, both not described in the literature. The similarity of OsAAG46136 to pyrrolidone carboxyl peptidase gives some suggestion as to the function of this poorly defined rice protein. Pyrrolidone carboxyl peptidase (Pcps) is an enzyme that removes an N-terminal pyroglutamyl group from some proteins. It is present in many species (reviewed by Awade et al., 1994) and is a valuable tool for bacterial diagnosis (most of the literature describing this protein addresses bacterial homologs). The active site of the *Pseudomonas fluorescens* Pcps has been characterized and the nature of this site (Cys-144 and His-166 are necessary for activity) suggests that it can represent a new class of thiol aminopeptidases (Le Saux et al., 1996). Peptidases in this protein family are necessary for processing and activation of important bioactive peptides including amyloid precursor protein (APP), strongly implicated in Alzheimer's disease (Lefterov et al., 2000). Furthermore, this enzyme deaminates and thus inactivates the glycopeptide anticancer agent bleomycin (Schwartz et al., 1999).

A BLAST analysis comparing the nucleotide sequence of OsAAG46136 against TMRI's GENECHIP® Rice Genome Array sequence database identified probeset OS013894_s_ at (e⁻⁸ expectation value) as the closest match. The expectation value is too low for this probeset to be a reliable indicator of the gene expression of OsAAG46136.

The bait clone encoding amino acids 100 to 250 of *O. sativa* Chilling-Inducible Protein CAA90866 (OsCAA90866) was also found to interact with protein ORF020300-2233.2 (OsORF020300-223), having a putative PP2A regulatory subunit and being similar to OsCAA90866 (see description in Example III). Three prey clones encoding amino acids 1 to 206 and three prey clones encoding amino acids 1-190 of OsORF020300-2233.2 were retrieved from the output trait library.

Additionally, the bait clone encoding amino acids 100 to 250 of *O*. *sativa* Chilling-Inducible Protein CAA90866 (OsCAA90866) was found to interact with protein PN23045 (OsPN23045). Two prey clones encoding amino acids 240 to 287 of OsPN23045 were retrieved from the input trait library.

OsPN23045 is a 287-amino acid protein that includes an inositol P domain (amino acids 233 to 272). This domain was identified in bovine inositol polyphosphate 1-phosphatase protein, which is involved in signal transduction (see York et al., 1994). Mikami et al. showed that phosphatidylinositol-4-phosphate 5-kinase (AtPIP5K11) is induced by water stress and abscisic acid (ABA) in *A. thaliana,* suggesting a link between phosphoinositide signaling cascades with water-stress responses in plants (Mikami et al., 1998). Xiong et al. reported that FRY1, a mutant gene in *A. thaliana* encoding an inositol polyphosphate 1-phosphatase, is a negative regulator of ABA and stress signaling in this plant (Xiong et al., 2001 a), providing evidence that phosphoinositols mediate ABA and stress signal transduction in plants.

A BLAST analysis comparing the nucleotide sequence of OsPN23045 against TMRI's GENECHIP® Rice Genome Array sequence database identified probeset OS006742.1_ at (e = 0 expectation value) as the closest match. Gene expression experiments indicated that this gene is specifically expressed in leaf and stem.

The bait clone encoding amino acids 100 to 250 of *O. sativa* Chilling-Inducible Protein CAA90866 (OsCAA90866) was also found to interact with protein PN23225, which is a novel 792-amino acid protein similar to *T. aestivum* initiation factor (iso)4f p82 subunit (p82) (GENBANK® Accession No. AAA74724; 69.6% amino acid sequence identity; e = 0). One prey clone encoding amino acids 639 to 792 of OsPN23225 was retrieved from the input trait library. The wheat protein contains possible motifs for ATP binding, metal binding, and phosphorylation (Allen et al., 1992). OsPN23225 contains an MIF4G domain (amino acids 207 to 434) named after Middle domain of eukaryotic initiation factor 4G (eIF4G), and an MA3 domain (amino acids 627 to 739) also found in eIF proteins (Ponting, 2000). These domains are found in molecules that participate in mRNA decay pathways. Although the function of the bait chilling-inducible protein CAA90866 is not well defined, it appears to be a nuclear protein and its interaction with the eIF-like protein OsPN23225 supports the notion that CAA90866 participates in the rice transcriptional machinery. The identification of the OsPN23225 prey protein likely represents the discovery of a novel rice eIF.

A BLAST analysis comparing the nucleotide sequence of OsPN23225 against TMRI's GENECHIP® Rice Genome Array sequence database identified probeset OS003249_ at (e⁻¹⁷ expectation value) as the closest match. The expectation value is too low for this probeset to be a reliable indicator of the gene expression of OsPN23225.

The bait clone encoding amino acids 100 to 250 of *O. sativa* Chilling-Inducible Protein CAA90866 (OsCAA90866) was also found to interact with OsPN29883, a 340-amino acid fragment that is similar to *A. thaliana* putative 2-dehydro-3-deoxyphosphooctonate aldolase (GENBANK® Accession No. NP_178068; 3e⁻¹⁴² expectation value) and pea (*Pisum sativum*) 2-dehydro-3-deoxyphosphooctonate aldolase (Kdo8P synthase; GENBANK® Accession No. 050044; 3e⁻¹⁴² expectation value). One prey clone encoding amino acids 58 to 175 of OsPN29883 was retrieved from the output trait library. Kdo8P synthase in pea catalyzes the biosynthesis of Kdo-8-P, a component of lipopolysaccharide of plant cell walls, with high structural and functional similarities to enterobacterial Kdo8P synthase (Brabetz et al., 2000).

### Summary

The interactors identified for the OsPP2A-2 bait protein (i.e., proteins that bind to OsPP2A-2) comprise a network that is speculated to be associated with the plant defense response to pathogens. Among the five novel rice proteins identified as interactors for OsPP2A-2, Os23268 is similar to the *A. thaliana* tryptophan biosynthetic enzyme anthranilate phosphoribosyltransferase. This enzyme is encoded by a gene that is similar to the DESCA11 gene involved in resistance to virus infection (Goff, 2001). While the role of tryptophan in disease resistance is unknown, tryptophan is used in the biosynthesis of indol-3-acetic acid, a plant hormone and signaling molecule. Tryptophan can thus have a role in modulation of gene expression in plants. Moreover, the glycosyl transferase function in Os23268 can be associated with disease resistance signaling pathways or with phytoalexin cellular distribution. Phytoalexins are low-molecular-weight antimicrobial compounds that accumulate in plants as a result of infection or stress, and the rapidity of their accumulation is associated with resistance in plants to diseases caused by fungi and bacteria. Taken altogether, these data suggest that anthranilate phosphoribosyltransferases plays a role in the plant response to pathogen infection. Moreover, gene expression experiments confirmed that this gene is induced by the fungal pathogen *M. grisea.* Thus, the anthranilate phosphoribosyltransferase-like novel protein Os23268 is believed to be involved in the signaling and regulation pathways that mediate the response of rice to biotic stress.

Novel protein Os011994-D16, similar to DnaJ protein, is another interactor for OsPP2A-2 with a likely role in the pathogen-induced defense response. DnaJ-like proteins are known to be regulators of heat shock proteins and are thus part of the plant protective stress response. Gene expression experiments support this notion, indicating that the gene encoding the DnaJ-like protein of this Example is repressed by jasmonic acid, a component of signaling networks that provide the specificity of plant pathogen-induced defense responses (reviewed in Nurnberger & Scheel, 2001);

OsPP2A-2 was also found to interact with the novel protein OsORF020300-223, which is similar to *A. thaliana* PP2A regulatory subunit and to rice chilling inducible protein CAA90866 (OsCAA90866; the second bait protein of this Example). The similarity of OsORF020300-2233.2 to PP2A regulatory subunit validates its interaction with the PP2A-2 catalytic subunit, this interaction being consistent with the subunit composition of PP2A enzymes (Awotunde et al., 2000). The OsORF020300-223-OsPP2A-2 interaction suggests that OsORF020300-2233.2 participates in signaling events that involve OsPP2A-2 enzymatic activity, and the similarity of OsORF020300-2233.2 to rice chilling-inducible protein OsCAA90866 suggests that cold tolerance can involve one of these signaling events.

OsPP2A-2 was also found to interact with rice putative proline-rich protein OsAAK63900. Though it has no known DNA-binding motif, there are indications that OsAAK63900 can play a role as a transcriptional regulator. It has an HLH domain common to transcription factors, although this domain mediates protein dimerization only. It also has a gntR family signature common to bacterial DNA-binding transcriptional regulators, although the function of this domain is not known. The existence of the Ole e I suggests that OsPP2-2 can dephosphorylate OsAAK69300, thus regulating its function as a pollen protein, although the lack of data on the Ole e I signature function makes this possibility more difficult to argue. Evidence also exists that PP2A proteins regulate the DNA-binding activity of transcription factors in plants (Vazquez-Tello et al., 1998) and mammalian cells (Wadzinski et al., 1993). Therefore, it is most likely that the OsPP2A-2-OsAAK63900 interaction occurs in the nucleus and that it plays a role in regulating transcriptional events in rice.

Other proteins found to interact with OsPP2A-2 include a disulfide isomerase with a putative role in protein folding (novel protein OsPN26645), a voltage-dependent ion channel protein (novel protein OsPN24162) and the seed storage protein glutelin (OsCAA33838). The biological significance of these interactions is unclear. Analysis of the amino acid sequence of glutelin identified several protein kinase C and casein kinase II phosphorylation sites. It is possible that the phosphorylation state of glutelin determines its function or stability, and its interaction with OsPP2A-2 can occur during dephosphorylation of glutelin. Alternatively, this interaction can result in localization of OsPP2A-2 and thereby affect events downstream of OsPP2A-2-dependent dephosphorylation. Given the presence of a disulfide bond between the two peptide chains of typical plant seed storage proteins, it is interesting that OsPP2A-2 also interacts with a putative protein disulfide isomerase (OsPN26645). Perhaps OsPP2A-2 interacts with other enzymes to create a co-translational modification complex. Additional yeast-two-hybrid data can clarify the purpose of these interactions. However, given the association of PP2A with other proteins involved in biotic stress responses, the aforementioned associations could also be involved in biotic stress responses.

The chilling-inducible protein CAA90866 was found to interact with itself and with six proteins. These proteins are speculated to interact as components of a network of proteins relevant to the rice response to cold stress. This hypothesis finds support in gene expression experiments, which confirmed that the gene encoding the chilling-inducible protein is induced by cold. One of the interactors is the putative 14-3-3 protein Os008938-3209. The relationship to chilling tolerance of the bait protein OsCAA90866 suggests that its interaction with Os008938-3209 can be associated with cold tolerance. Gene expression experiments showed that this protein is induced under a broad range of stress conditions. Its activation probably allows its interaction with a number of stress proteins. Given the function of 14-3-3 proteins as molecular chaperones, Os008938-3209 can act as a molecular glue for these interactions to preserve protein complex stability in membranes, or it can coordinate interactions involving transcription factors associated with stress genes. Subcellular localization of Os008938-3209 can further clarify the significance of its interaction with OsCAA90866.

Another interactor for OsCAA90866 is a pyrrolidone carboxyl peptidase-like protein (OsAAG46136). The putative pyrrolidone carboxyl peptidase function of OsAAG46136 suggests that it participates in processing and/or activation of substrate proteins, and these proteins can be important to the plant response to chilling. Peptidase activity has been associated with regulation of signaling. Carboxypeptidases, for instance, hydrolytically remove the pyroglutamyl group from peptide hormones, thereby activating these signaling molecules. A carboxypeptidase regulates Brassinosteroid-insensitive 1 (BRI1) signaling in *A*. *thaliana* by proteolytic processing of a protein (Li et al., 2001). Based on its ability to interact with chilling-inducible protein and on the role of the latter in chilling tolerance, it is speculated that the carboxypeptidase-like protein OsAAG46136 can have a role in activating signaling molecules/hormonal peptides that are involved in the plant response to cold stress.

The interactions of OsCAA90866 with OsPN23045, a protein with a putative inositol phosphate function, and with OsPN23225, a rice homolog of wheat initiation factor (iso)4f p82 subunit, provide further insight into the function of the bait protein. Phosphoinositols are known to mediate ABA and stress signal transduction in plants (Mikami et al., 1998; Xiong et al., 2001 a). The putative inositol phosphatase protein OsPN23045 can function in a similar way and its interaction with the chilling-inducible protein can be associated with regulation of cell signaling events that relate to cold tolerance. The prey protein OsPN23225 likely represents a novel rice eIF. The eIF proteins have a role in RNA processing pathways (Ponting, 2000) and stress is typically associated with an abundance of RNA transcripts. Based on this information and on the relationship that CAA90866 has to chilling tolerance, the OsCA90866- PN23225 interaction is speculated to control translational events related to cold stress.

Finally, OsCAA90866 interacts with and is similar to the same putative PP2A regulatory subunit protein OsORF020300-2233.2 found to interact with the bait protein OsPP2A-2. This interaction provides a link between the two networks of this Example and suggests the involvement of OsPP2A-2 in both biotic and abiotic stress response pathways. Based on the observed interactions and on sequence similarities among the proteins involved in these interactions, OsPP2A-2 appears to regulate both biotic and abiotic stress response pathways. Thus, the two pathways, though independent, are speculated to be linked through protein phosphatases, and that these enzymes likely mediate the plant's stress response by dephosphorylation of the proteins participating in these pathways. In this scenario, it is possible that the self-interaction observed for OsCAA90866 participates in the creation of multicomponent phosphatase complexes. Furthermore, the interaction of OsCA90866 with the aldolase-like protein OsPN29883 suggests that the aldolase needs to be dephosphorylated for activation/inactivation, and that this novel protein can have roles during stress responses based upon the other interactions and the gene expression patterns of the chilling-inducible protein.

Moreover, OsORF020300-2233.2 the *A. thaliana* regulatory A subunit of protein phosphatase 2A (PP2A-A) has been implicated in the regulation of auxin transport in *A. thaliana* (Garbers et al., 1996). The phytohormone auxin controls processes such as cell elongation, root hair development and root branching. Since OsORF020300-2233.2 is also similar to and interacts with chilling-inducible protein CAA90866, it is possible that the latter can be involved in auxin transport.

### References

The references listed below as well as all references cited in the specification are incorporated herein by reference to the extent that they supplement, explain, provide a background for or teach methodology, techniques and/or compositions employed herein.
Aasland et al. (1995) Trends Biochem. Sci. 20: 56-59.
Abdel-Ghany et al. (2000) DNA Cell Biol. 19: 567-578.
Abler et al. (1993) Plant Mol. Biol. 22: 1031-1038.
Ach & Gruissem (1997) Proc. Natl. Acad. Sci. USA 91: 5863-5867.
Ackland & Peters, (1999) Drug Resist. Update 2: 205-214.
Agrawal S (ed.) (1993) Methods in Molecular Biology, volume 20, Humana Press, Totowa, New Jersey, United States of America.
Agueli et al. (2001) Biochem. J. 360: 413-419.
Aihara et al. (2000) J. Neurochem. 74: 2622-2625.
Ajuh et al. (2001) J. Biol. Chem. 276: 42370-42381.
Allen et al. (1992) J. Biol. Chem. 267: 23232-23236.
Allison et al. (1986) Virology 154:9-20.
Altschul et al. (1990) J. Mol. Biol. 215:403.
Altschul et al. (1997) Nucl. Acids Res. 25: 3389.
An et al. (1985) EMBO J. 4: 277.
Antoku et al. (2001) Biochem. Biophys. Res. Commun. 286: 1003-1010.
Aoyama & Chua (1997) Plant J 11:605-612.
Apte et al. (1995) FEBS Lett. 363: 304-306.
Arino et al. (1993) Plant Mol. Biol. 21: 475-485.
Arioli et al. (1998) Science 279: 717-720.
Auch & Reth (1990) Nucl. Acids Res. 18: 6743.
Austin et al. (2002) Science 295: 2077-2080.
Ausubel et al. (1988) Current Protocols in Molecular Biology, John Wiley & Sons, New York, New York, United States of America.
Awade et al. (1994) Proteins 20: 34-51.
Awotunde et al. (2000) Biochim Biophys Acta 1480: 65-76.
Azevedo et al. (2002) Science 295: 2073-2076.
Aznar & Lacal (2001) Prog. Nucleic Acid Res. Mol. Biol. 67: 193-234.
Baluska et al. (2001) Plant Physiol. 126: 39-46.
Bartel & Fields (eds.), (1997) The Yeast Two-Hybrid System, Oxford Press.
Bartlett et al. (1982) in Methods in Chloroplast Molecular Biology, (Edelman et al., eds.) Elsevier Biomedical Press, New York, New York, United States of America, pp. 1081-1091.
Baskin et al. (1992) Aust. J. Plant Physiol. 19: 427-437.
Batzer et al. (1991) Nucleic Acid Res. 19:5081.
Baunsgaard et al. (1998) Plant J. 13: 661.
Beerli et al. (1998) Proc Natl Acad Sci U S A 95:14628-14633.
Bergeron et al. (1994) Trends Biochem. Sci. 19: 124-128.
Bertolaet et al. (2001) Nat. Struct. Biol. 8: 417-422.
Bevan (1984) Nucl. Acids Res. 12:8711.
Bevan et al. (1983) Nature 304:184-187.
Bezzi et al. (2001) Nat. Neurosci. 4: 702-710.
Biedenkapp et al. (1988) Nature 335: 835-837.
Bierkens et al. (2000) Toxicology 153: 61-72.
Bihn et al. (1997) Plant J. 12: 1439-1445.
Binet et al. (1991) Plant Mol Biol 17:395-407.
Bischoff et al. (1994) Proc. Natl. Acad. Sci. USA 91: 2587-2591.
Bisikirska et al. (1997) Z. Naturforsch 52: 180-186.
Blochinger & Diggelmann (1984) Mol Cell Biol 4:2929-2931.
Bolhuis et al. (1998) J. Biol. Chem. 273: 21217-21224.
Borisjuk et al. (1998) Planta 206: 504-14.
Bornemann et al. (1996) Biochemistry 35: 9907-9916.
Bourouis & Jarry (1983) EMBO J 2:1099-1104.
Brabetz et al. (2000) Planta 212: 136-143.
Bradwell et al. (1994) Genes Dev. 8: 1664-1677.
Braun (2001) Plant Physiol. 125:1611-1619.
Briknarova et al. (2001) Nat. Struct. Biol. 8: 349-352.
Browns & Bicknell (1998) Biochem. J. 334: 1-8.
Bruggemann et al. (1996) Plant J 10:755-760.
Brummelkamp et al. (2002) Science 296: 550-3.
Brunner et al. (1998) Plant J. 14: 225-34.
Buchanan et al. (eds.), (2002) Biochemistry and Molecular Biology of Plants, John Wiley & Sons, New York, New York, United States of America.
Buck & Guest (1989) Biochem. J. 260: 737-747.
Bulledi & Freedman (1988) Nature 335: 649-651.
Burglin et al. (1997) Nucl. Acids Res. 25: 4173-4180.
Bursch (2001) Cell Death Diber. 8: 569-81.
Busch et al. (1990) Trends Genet. 6: 36-40.
Byrne et al. (1987) Plant Cell Tissue Org Culture 8: 3.
Caddick et al., (1998) Nat Biotechnol 16:177-180.
Callebaut & Mornon (1997) FEBS Lett. 400: 25-30.
Callis et al. (1987) Genes Dev. 1: 1183.
Callis et al., (1990) J Biol Chem 265:12486-12493.
Cao et al. (2000) Arch Biochem Biophys. 373: 135-46.
Casas et al., (1993) Proc Natl Acad Sci U S A 90:11212-6.
Cash (1996) Med Hypotheses 47: 455-459.
Chan et al. (1998) Biochim. Biophys. Acta 1442: 1-19.
Chee et al. (1989) Plant Physiol. 91:1212.
Chen et al. (1993) Science 262: 1883-1886.
Chen et al. (2002) Plant Cell 14: 559-574.
Chibbar et al., (1993) Plant Cell Rep 12:506-509.
Choi et al. (1995) Mol Gen, Genet. 246:266.
Christensen et al., (1989) Plant Mol Biol 12:619-632.
Christou et al. (1988) Plant Physiol. 87:671.
Christou et al. (1989) Proc. Natl. Acad. Sci. USA 86:7500.
Christou et al., (1991) Bio/Technology 9: 957-962.
Chuang & Meyerowitz, (2000) Proc Natl Acad Sci U S A 97:4985-90.
Chung et al. (1994) Plant Mol. Biol. 26: 657-665.
Claes et al. (1990) Plant Cell 2: 19-27.
Close et al. (1989) Plant Mol. Biol. 13: 95-108.
Comai et al., (1988) J Biol Chem 263:15104-15109.
Conceicao et al. (1994) Plant 5:493.
Corpet et al. (1988) Nucl. Acids Res. 16:10881.
Creighton, (1984) Proteins, WH Freeman & Co., New York, New York, United States of America.
Crossway et al. (1986) Bio/Techniques 4:320.
Cruz et al. (1998) P. R. Health Sci. J. 17: 323-326.
Cyr et al. (1994) Trends Biochem. Sci. 19: 176-181.
Dasgupta et al. (1993) Gene 133: 301.
Dasso (2000) Cell 104: 321-324.
Dat et al. (2001) Redox Rep. 6: 37-42.
Datta et al. (1990) Bio/Technology 8: 736.
Davies et al. (1996) EMBO J. 15:4330-4343.
De Block et al. (1989) Plant Physiol. 91:694.
de Framond, (1991) FEBS Lett 290:103-6.
Della-Cioppa et al. (1987) Plant Physiol. 84:965.
De Lille et al. (2001) Plant Physiol. 126: 35-38.
Denecke et al. (1995) Plant Cell 7: 391-406.
Doelling et al. (2001) Plant J. 27: 393-405.
Dong et al., (1996) Mol Breeding 2:267-276.
Doonan et al. (1997) Curr. Opin. Cell. Biol. 9: 824-830.
Dunigan & Madlener (1995) Virology 207: 460-466.
Dure et al. (1989) Plant Mol. Biol. 12: 475-486.
Dwyer et al. (1996) Biochim Biophys Acta 1289: 231-237.
Ecker (1995) Science 268: 667-675.
Ecker & Davis, (1986) Proc Natl Acad Sci USA 83:5372-5376.
Edwards et al. (1988) J. Mol. Biol. 203: 523-524.
Elbashir et al., (2001) EMBO J 20:6877-88.
Elge et al. (2001) Plant J. 26: 561-571.
Ellenberger (1994) Curr. Opin. Struct. Biol. 4: 12-21.
Ellis et al. (1987) EMBO J., 6:3203.
Elroy-Stein et al. (1989) Proc. Natl. Acad. Sci. USA. 86:6126.
EP 0 120 516
EP 0 138341
EP 0 292 435
EP 0 295 959
EP 0 301 749
EP 0 332 104
EP 0 332 581
EP 0 342 926
EP 0 392 225
EP 1 116 793
Everett et al. (1987) Bio/Technology 5:1201.
Fardel et al. (2001) Toxicology 167: 37-46.
Feys et al. (2001) EMBO J. 20: 5400-5411.
Fire et al. (1998) Nature 391: 806-811.
Firek et al., (1993) Plant Mol Biol 22:129-142.
Frary et al. (2000) Science 289: 85-88.
Freier et al., (1986) Proc Natl Acad Sci USA 83:9373-9377.
Fromental-Ramain et al. (1996) Development 122: 461-472.
Fromm et al. (1986) Nature 319: 791.
Fromm et al. (1990) Bio/Technology 8: 833.
Fromme et al. (2001) Biochim. Biophys Acta 1507: 5-31.
Fujiyama et al. (2001) J. Comp. Neurol. 435: 379-387.
Gaedeke et al. (2001) EMBO J. 20: 1875-1887.
Gallie et al. (1987) Nucl. Acids Res. 15:3257.
Gallie et al. (1989) Plant Cell 1:301.
Gallois et al. (1997) Plant J. 11: 1325-1331.
Garbers et al. (1996) EMBO J. 15: 2115-2124.
Garcia et al. (1998) Planta 207: 172-80.
Garcia et al. (2000) Microbes Infect. 2: 401-407.
Garrel & Campuzano (1991) BioEssays 13: 493-498.
Gehring (1992) Trends Biochem. Sci. 17: 277-280.
Gehring & Hiromi (1986) Ann. Rev. Genet. 20: 147-173.
Gillet et al. (1998) Plant J. 16: 257-262.
Glazebrook et al. (1996) Genetics 143: 973-982.
Godwin et al. (1998) Proc. Natl. Acad. Sci. USA 95: 13042-13047.
Goeddel, (1990) Methods in Enzymology, Volume 185, Academic Press, San Diego, California, United States of America.
Goff (2001) Plant J. 26: 339-349.
Goff et al. (2002) Science 296: 92-100.
Gocal et al. (2001) Plant Physiol. 125: 1788-1801.
Gordon Kamm et al. (1990) Plant Cell 2:603.
Greco et al. (1997) Mol. Gen. Genet. 253: 615-623.
Green, (2000) Trends Biochem Sci 25:59-63.
Green et al., (1986) Ann Rev Biochem 55:569-597.
Gritz et al. (1983) Gene 25:179.
Grotewold et al. (1991) Proc. Natl. Acad. Sci. USA 88: 4587-4591.
Groves, et al. (1999) Curr. Opin. Struct. Biol. 9: 383-389.
Gruber et al. (1993) Vectors for Plant Transformation, in Methods in Plant Molecular Biology, Glich et al. eds, pp. 89-119, CRC Press.
Guan & Scandalios (1996) J. Mol. Evol. 42: 570-579.
Guo et al. (2000) EMBO J. 19: 6891-6899.
Gyuris et al. (1993) Cell 1993, 75: 791-803.
Hake et al. (1995) Philos Trans. R Soc. Lond. B Biol. Sci. 350: 45-51.
Hammerschmidt (1999) Ann. Rev. Phytopathol. 37: 285-306.
Hannig et al. (1995) Bioessays 17: 915-919.
Haraven et al. (1996) Cell 84: 735-44.
Harlow & Lane, (1988) Antibodies: A Laboratory Manual, Cold Spring Harbor Laboratory Press, Cold Spring Harbor, New York, United States of America.
Harper et al. (1991) Science 252: 951-954.
Harris et al. (1999) Plant Physiol. 121: 609-617.
Hartwig (1995) Protein Profile 2: 703-800.
Hassan et al. (1995) Biochem. Biophys. Res. Commun. 211: 54-49.
Hatzfeld (1999) Int. Rev. Cytol. 186: 179-224.
Hayashi et al. (2001) J. Biol. Chem. 276: 43400-43406.
Haydon & Guest (1991) FEMS Microbiol. Lett. 79: 291-296.
Hedden & Kamiya (1977) Annual Rev. Plant Physiol. Plant Mol. Biol. 48: 431.
Henikoff & Henikoff (1989) Proc. Natl. Acad. Sci. USA, 89:10915.
Hiei et al. (1994) Plant J. 6:271.
Hiei et al., (1997) Plant Mol Biol 35:205-18.
Higgins et al. (1988) Gene 73:237.
Higgins et al. (1989) CABIOS 5:151.
Higo & Higo (1996) Plant Mol. Biol. 30: 505-521.
Hinchee et al. (1988) Bio/Technology 6:915.
Hoekema (1985) in The Binary Plant Vector System, Offset-drukkerij Kanters B.V.
Holk et al. (1996) Plant Mol. Biol. 31: 1153-1161.
Hong et al. (2001) Plant Cell 13: 755-768.
Huang et al. (1992) CABIOS 8:155.
Huang et al. (1996) Plant Mol. Biol. 33:125.
Huber et al. (1997) Cell 90: 871-882.
Hudspeth & Grula, (1989) Plant Molec Biol 12:579-589.
Hurst (1995) Protein Prof. 2: 105-168.
Imhof & Wolffe (1999) Biochemistry 38: 13085-13093.
Ishikawa et al. (2001) Plant J. 27: 89-99.
Janssens & Goris (2001) Biochem J. 353: 417-439.
Jasencakova et al. (2001) Chromosoma 110: 83-92.
Jasinski et al. (2001) Plant Cell 13: 1095-107.
Jia et al. (2000) Plant Sci. 155:115-122.
Jin & Martin (1999) Plant Mol. Biol. 4: 577-585.
Jobling & Gehrke (1987) Nature 325:622.
John et al. (2001) Protoplasma 216: 119-142.
Johnson et al. (1997) J. Biol. Chem. 272: 7106-7113.
Jorgensen et al., (1996) Plant Mol Biol 31:957-973.
Josefsson et al. (1987) J. Biol. Chem. 262:12196.
Kagedal et al. (2001) Biochem J. 359: 335-343.
Kaitna et al. (2000) Curr. Biol. 10:1172-1181.
Kang et al. (1995) Plant Mol. Biol. 29:1-10.
Kang et al. (1997) Mol. Cells 7: 45-51.
Kao et al. (2000) Biochem. Biophys. Res. Commun. 267: 201-207.
Karlin & Altschul (1990) Proc. Natl. Acad. Sci. USA 87:2264.
Karlin & Altschul (1993) Proc. Natl. Acad. Sci. USA 90:5873.
Kato & Dang (1992) FASEB J. 6: 3065-3072.
Kawalleck et al. (1992) Proc. Natl. Acad. Sci. USA 89:4713-7.
Keegan et al., (1986) Science 231:699-704.
Kehres et al. (1998) Microb. Comp. Genomics 3: 151-169.
Kelly (1999) Altern. Med. Rev. 4: 249-265.
Kempin et al., (1997) Nature 389:802-803.
Kerstetter et al. (1997) Development 124: 3045-3054.
Kidou et al. (1993) FEBS Lett. 332: 282-286.
Kim et al. (2001) Plant Physiol. 127: 1243-1255.
Kitade et al. (1999) Nucl. Acids Symp. Ser. 42: 25-26.
Klein et al. (1988) Bio/Technology 6:559.
Kline et al. (1987) Nature 327:70.
Knauf et al. (1983) Analysis of Host Range Expression by Agrobacterium, in Molecular Genetics of the Bacteria-Plant Interaction, Puhler, (ed.), Springer-Verlag.
Kobe et al. (1994) Trends Biochem. Sci. 19: 415-421.
Kong & Steinbiss (1998) Arch Virol 143:1791-1799.
Koonin (1993) J. Mol. Biol. 229: 1165-1174.
Korfhage et al. (1994) Plant Cell 6: 695-708.
Kossman & Lloyd (2000) Crit. Rev. Biochem. Mol. Biol. 35: 141-196.
Kostyal et al. (1998) Clin. Exp. Immunol. 112: 355-362.
Kosugi & Ohashi (2002) Plant Physiol. 128: 833-843.
Koziel et al. (1993) Biotechnology 11:194.
Krause et al. (1990) Cell 63: 907-919.
Kruse et al. (1995) Planta 196: 796-803.
Kyozuka et al. (2000) Plant Cell Physiol. 41:710-718.
Kyte & Doolittle, (1982) J Mol Biol 157:105-132.
Lacombe et al. (2001) Science 292: 1486-1487.
Lamb & Dixon (1997) Ann. Rev. Plant Biol. 48: 251.
Landolt (1986) Biosystematic Investigation on the Family of Duckweeds: The family of Lemnaceae - A Monograph Study, Geobatanischen Institut ETH, Stiftung Rubel, Zurich.
Landschulz et al. (1988) Science 240: 1759-1764.
Langenkamper et al. (2001) J. Exp. Bot. 52: 1545-1554.
Laursen et al. (1994) Plant Mol. Biol. 24:51.
Lebel et al., (1998) Plant J 16:223-233.
Lechner et al. (1996) Biochim Biophys Acta 1296: 181-188.
Lee et al. (1994) Plant Mol. Biol. 26:1981.
Lefterov et al. (2000) FASEB J. 14: 1837-1847.
Lenny et al. (1997) Mol. Biol. Rep. 24: 157-168.
Le Saux et al. (1996) J. Bacteriol. 178: 3308-3313.
Leslie et al. (2001) Toxicology 167: 3-23.
Li & Komatsu (2000) Eur. J. Biochem. 267: 737-745.
Li & Nam (2002) Science 295: 1299-1301.
Li et al. (2001)Proc. Natl. Acad. Sci. USA 98: 5916-5921.
Lim et al. (1999) Plant Physiol. 120: 1193-1204.
Lim et al. (2000) Plant Mol. Biol. 44: 513-527.
Lincoln et al. (1994) Plant Cell 6: 1859-1876.
Lindholm et al. (2000) Mech. Dev. 93: 169-173.
Lindsey et al. (1993) Transgen. Res. 2: 3347.
Liu et al. (1992) Antiviral Res. 19: 247-265.
Liu et al., (1999) Genome Res 9:859-867.
Logemann et al., (1989) Plant Cell 1:151-158.
Lommel et al. (1991) Virology 181:382.
Lopez-Dee et al. (1999) Dev. Genet. 25: 237-244.
Lorz et al. (1985) Mol. Gen. Genet. 199: 178.
Ma et al. (1988) Nature 334: 631.
Macejak & Sarnow, (1991) Nature 353:90-94.
Magyar et al. (2000) FEBS Lett. 486: 79-87.
Maki et al. (1993) Methods in Plant Molecular Biology, Glich et al. eds, pp. 67-88, CRC Press.
Malz & Sauter (1999) Plant Mol. Biol. 40: 985-995.
Manjunath et al. (1997) Plant Mol. Biol. 33:97.
Mann & Affolter (1998) Curr. Opin. Genet. Dev. 8: 423-429.
Mannervick (1999) Bioessays 21: 267-270.
Martinez et al. (1989) J. Mol. Biol. 208:551.
Martinez et al. (2000) Plant Physiol. 122: 757-766.
Massey (2000) Biochem. Soc. Trans. 28: 283-296.
Mayer et al. (2001) Phytochemistry 58: 33-41.
Mayo, (1987) The Theory of Plant Breeding, Second Edition, Clarendon Press, New York, New York, United States of America.
McBride et al. (1994) Proc. Natl. Acad. Sci. USA 91: 7301.
McBride et al. (1990) Plant Mol. Biol. 14: 266.
McCabe et al. (1988) Bio/Technology 6:923.
McEIroy et al., (1990) Plant Cell 2:163-71.
McEwen, et al. (2001) Mol. Biol. Cell. 12: 2776-89.
McLeod (1986) Bioessays 6: 208-212.
Medina et al. (2001) Plant Physiol. 125: 1655.
Merkle et al. (1994) Plant J. 6: 555-565.
Messing & Vierra (1982) Gene 19:259.
Miao & Lam, (1995) Plant J 7:359-365.
Michalak et al. (1992) Biochem. J. 285: 681-692.
Michalak et al. (1998) Biochem. Cell. Biol. 76: 779-785.
Michelis et al. (2000) Plant Mol. Biol. 44: 487-498.
Mikami et al. (1998) Plant J. 15: 563-568.
Moon et al. (1999) Plant Physiol. 120: 1193-1204.
Moons et al. (1998) Plant J. 15: 89-98.
Morita et al. (2001) Curr. Pharm. Biotechnol. 2: 257-267.
Muehlbauer et al, (1999) Plant Physiol. 119: 651-62.
Mukumoto et al., (1993) Plant Mol Biol 23: 995-1003.
Muller et al. (1995) Nature 374: 727-730.
Mundy & Chua (1988) EMBO J. 7: 2279-2286.
Munro & Pelham (1987) Cell 48: 899-907.
Munster et al. (2001) Gene 262:1-130.
Murre et al. (1989) Cell 56: 777-783.
Muslin & Xing (2000) Cell Signal 12: 703-709.
Myers & Miller (1988) CABIOS 4:11.
Nagamara-Inoue et al. (2001) Int. Rev. Immunol. 20: 83-105.
Nakamura et al. (1996) Plant Mol. Biol. 30: 381-385.
Needleman & Wunsch (1970) J. Mol. Biol. 48:443.
Negrotto et al., (2000) Plant Cell Reports 19:798-803.
Nelson et al. (1997) Plant Physiol. 114: 29-37.
Nepveu (2001) Gene 270: 1-15.
Ng & Yanofsky (2001) Nat. Rev. Genet. 2: 186-195.
Nielsen et al. (1986) J. Biol. Chem. 261: 3661-3669.
Norris et al., (1993) Plant Mol Biol 21:895-906.
Nurnberger & Scheel (2001) Trends Plant Sci. 6: 372-379.
O'Connell et al. (2001) J. Biol. Chem. 276: 43065-43073.
Odagaki et al. (1999) Structure Fold Des. 7: 399-411.
Ohtsuka et al., (1985) J. Biol. Chem. 260:2605-2608.
Oliveira et al. (2001) Braz. J. Med. Biol. Res. 34: 567-575.
Ono et al. (1996) Plant Physiol. 112: 483-491.
Ono et al. (2001) Proc. Natl. Acad. Sci. USA 98: 759-764.
Oppenheimer et al. (1991) Cell 67: 483-493.
Orzaez & Granell (1997) FEBS Lett. 404: 275-278.
O'Shea et al. (1989) Science 243: 538-542.
Pacciotti et al. (1985) Bio/Technology 3:241.
Panavas et al. (1999) Plant Mol. Biol. 40: 237-248.
Park et al. (1985) J. Plant Biol., 38:365.
Park et al. (1998) EMBO J. 17: 859-867.
Pascual et al. (2000) J. Mol. Biol. 304: 723-729.
Paszkowski et al. (1984) EMBO J. 3:2717.
PCT International Publication No. WO 93/05163
PCT International Publication No. WO 93/07278
PCT International Publication WO 93/21335
PCT International Publication WO 94/00977
PCT International Publication No. WO 94/20627
PCT International Publication No. WO 95/16783
PCT International Publication WO 95/19431
PCT International Publication WO 96/06166
PCT International Publication WO 98/54311
PCT International Publication WO 99/32619
PCT International Publication WO 99/53050
PCT International Publication WO 99/61631
PCT International Publication No. WO 00/07210
PCT International Publication No. WO 00/760067
PCT International Publication No. WO 01/07618
Pearson & Lipman (1988) Proc. Natl. Acad. Sci. USA 85:2444.
Pearson et al. (1994) Meth. Mol. Biol. 24:307.
Peifer et al. (1990) Cell 63:1167-76.
Peifer et al. (1994) Cell 76:789-791.
Pelham (1990) Trends Biochem. Sci. 15: 483-486.
Perrin (2001) Curr. Biol. 11: R213-R216.
Persson et al. (2001) Plant Physiol. 126: 1092-1104.
Phillips et al. (1988) in Corn and Corn Improvement, 3rd ed, Sprague et al. eds., Amer. Soc of Agronomy.
Picard et al., (1988) Cell 54: 1073-1080.
Pih et al. (1999) Mol. Cells 9: 84-90.
Ponting (2000) Trends Biochem. Sci. 25: 423-426.
Ponting & Parker (1996) Protein Sci. 5: 162-166.
Postma-Haarsma et al. (2002) Plant Mol Biol 48: 423-41.
Potrykus (1985) Trends Biotech. 7:269.
Potuschak & Doerner (2001) Curr. Opin. Plant Biol. 4: 501-506.
Powell et al., (1989) Proc. Natl. Acad. Sci. USA 86:6949-6952.
Presley et al. (2002) Nature 417: 187-193.
Purugganan et al. (1995) Genetics 140: 345-356.
Ratajczak (2000) Biochim Biophys Acta 1465: 17-36.
Raven et al. (1999) Biology of Plants, Freeman/Worth.
Rea et al. (1998) Annu. Rev. Plant Physiol. Plant Mol. Biol. 49: 727-760.
Reddy (2001) Int. Rev. Cytol. 204: 97-178.
Redei & Koncz, (1992) in Methods in Arabidopsis Research (Koncz C, Chua N-H & Schell J, eds.) World Scientific Press, River Edge, New Jersey, United States of America, pp. 16-82.
Reed et al., (2001) In Vitro Cell Dev Biol-Plant 37:127-132.
Reichelt et al. (1999) Plant J. 19: 555-567.
Reiser et al. (1995) Cell 83:735.
Reizer et al. (1991) Mol. Microbiol. 5: 1081-1089.
Riechmann et al. (1994) Nucl. Acids Res. 22: 749-755.
Riechmann & Meyerowitz (1997) Biol. Chem. 378: 1079-1101.
Riggs et al. (1986) Proc. Natl. Acad. Sci. USA 83:5602.
Ritz et al. (2001) J. Biol. Chem. 276: 22273-22277.
Robertson & Chandler (1992) Plant Mol. Biol. 19: 1031-1044.
Robins et al. (1998) J. Med. Chem. 41: 3857-3864.
Robles et al. (1997) J. Neurosci. Res. 47: 90-97.
Rogers et al., (1985) Proc. Natl. Acad. Sci. USA 82:6512-6516.
Rogers et al. (2001) J. Biol. Chem. 276: 30914-30922.
Rohrmeier & Lehle, (1993) Plant Mol Biol 22:783-792.
Roosens et al. (2000) Biochim. Biophys. Acta 1463: 470-476.
Rossolini et al., (1994) Mol Cell Probes 8:91-98*.*
Roth et al., (1991) Plant Cell 3:317-325.
Rothstein et al. (1987) Gene 53:153.
Ruberti et al. (1991) EMBO J. 10: 1787-1791.
Sabelli et al. (1999) Mol. Gen. Genet. 261: 820-830.
Saijo et al. (2001) Plant Cell Physiol. 42: 1228-1233.
Salvucci & Ogren (1996) Phosynthesis Res. 47: (1) 1-11.
Salvucci et al. (2001) Plant Physiol. 127: 1053-1064.
Sanchez-Fernandez et al. (2001) J. Biol. Chem. 276: 30231-30244. Sanderfoot et al. (1999) Plant Physiol. 121: 929-938.
Sanford et al. (1987) Particulate Sci. Tech. 5:27.
Sankaranarayanan et al. (1996) Nat. Struct. Biol. 3: 596-603.
Saraste et al. (1990) Trends Biochem. Sci. 15: 430-434.
Sato et al. (1997) J. Biol. Chem. 272:24530-5.
Sauter et al. (1995) Plant J. 7: 623-632.
Savidge et al. (1995) Plant Cell 7: 721-33.
Sazer & Dasso (2000) J. Cell Sci. 113: 1111-1118.
Scharfmann et al., (1991) Proc Natl Acad Sci U S A 88:4626-4630.
Schledzewski et al. (1999) J. Mol. Evol. 48: 770-778.
Schmidhauser & Helinski (1985) J. Bacteriol. 164:446.
Schneeberger et al. (1998) Development 125: 2857-2865.
Schocher et al., (1986) Bio/Technology 4:1093-1096.
Schofield (1987) Trends Neurosci. 10: 3-6.
Schultz et al. (1998) Plant Cell 10: 837-47.
Schwab et al. (2001) Phytochemistry 56: 407-415.
Schwartz et al. (1999) Proc. Natl. Acad. Sci. USA 96: 4680-4685.
Sehnke et al. (2000) Plant Physiol. 122: 235-242.
Sganga et al. (1992) Proc. Natl. Acad. Sci. USA 89: 6328-6332.
Shalev et al. (2001) J. Biol. Chem. 276: 34948-34957.
Shank et al. (2001) Plant Physiol. 126: 267-277.
Shcherban et al. (1995) Proc. Natl. Acad. Sci. USA 92: 9245-9249.
Sheridan et al. (1996) Genetics 142:1009.
Shibuya et al. (2000) J. Exp. Bot. 51: 2067-2073.
Shimamoto et al. (1989) Nature 338:274.
Shinshi et al., (1990) Plant Mol Biol 14:357-368.
Silverstone et al., (1998) Plant Cell 10:155-169.
Singh, (1986) Breeding for Resistance to Diseases and Insect Pests, Springer-Verlag, New York, New York, United States of America.
Singh et al. (1998) J. Plant. Physiol. 153: 316-323.
Sinha et al. (1993) Genes Dev. 7: 787-795.
Sjodahl et al. (1995) Planta 197:264.
Skuzeski et al. (1990) Plant Mol. Biol., 15: 65.
Smilie (1979) Trends Biochem. Sci. 4: 151-155.
Smith et al., (2000) Nature 407:319-320.
Smith & Waterman (1981) Adv. Appl. Math. 2: 482.
Smith et al. (1997) Ann. Rev. Plant Biol. 48: 67.
Solocombe et al. (1994) Plant Physiol. 104:1167.
Sontag (2001) Cell Signal 13: 7-16.
Spencer et al. (1990) Theor. Appl. Genet. 79:625.
Staub et al. (1992) Plant Cell 4:39.
Staub et al. (1993) EMBO J. 12:601.
Stintzi et al. (1993) Biochimie. 75: 687-706.
Sukhapinda et al. (1987) Plant Mol. Biol. 8:209.
Sulo & Martin (1993) J. Biol. Chem. 268: 17634-17639.
Sung et al. (2001) Mol. Cells 11: 352-359.
Suss et al. (1993) Proc. Natl. Acad. Sci. USA 90: 5514-5518.
Svab et al. (1990) Proc. Natl. Acad. Sci. USA 87:8526.
Takahashi et al. (1994) Plant Mol. Biol. 26: 339-352.
Takamori et al. (2000) Nature 407: 189-194.
Tanaka et al. (1997) Plant Mol. Biol. 35: 981-986.
Tapon & Hall (1997) Curr. Opin. Cell. Biol. 9: 86-92.
Taylor et al., (1993) Plant Cell Rep 12:491-495.
Theissen et al. (2000) Plant Mol. Biol. 42: 115-149.
Thompson et al. (1987) EMBO J. 6:2519.
Tomes et al. (1995) Plant Cell, Tissue and Organ Culture: Fundamental Methods, Springer-Verlag.
Triezenberg et al., (1988) Genes Dev 2:718-729.
Trimarchi & Lees (2002) Nat. Rev. Mol. Cell. Biol. 3: 11-20.
Tsutsumi et al. (1994) Gene 141: 215-220.
Turner et al., (1987) Cold Spring Harb Symp Quant Biol LII:123-133.
Udaka et al. (2000) J. Nutr. Sci. Vitaminol. (Tokyo) 46: 84-90.
Uknes et al., (1992) Plant Cell 4:645-656.
Umeda et al. (1999) Mol. Gen. Genet. 262: 230-238.
Unger et al., (1989) Plant Mol Biol 13:411-418.
Urao et al. (1996) Plant Mol. Biol. 32:571.
U.S. Patent Application No. 20010049831
U.S. Patent No. 4,554,101
U.S. Patent No. 4,940,935
U.S. Patent No. 4,945,050
U.S. Patent No. 4,987,071
U.S. Patent No. 5,036,006
U.S. Patent No. 5,100,792
U.S. Patent No. 5,188,642
U.S. Patent No. 5,270,163
U.S. Patent No. 5,350,689
U.S. Patent No. 5,451,513
U.S. Patent No. 5,466,785
U.S. Patent No. 5,491,288
U.S. Patent No. 5,501,967
U.S. Patent No. 5,523,311
U.S. Patent No. 5,545,817
U.S. Patent No. 5,545,818
U.S. Patent No. 5,591,616
U.S. Patent No. 5,614,395
U.S. Patent No. 5,639,949
U.S. Patent No. 5,767,378
U.S. Patent No. 5,929,226
U.S. Patent No. 5,990,386
U.S. Patent No. 5,994,629
U.S. Patent No. 6,087,175
U.S. Patent No. 6,369,298
Van Breusegem et al. (1994) Planta 193: 57-66.
Van den Broeck et al., (1985) Nature 313:358-363.
van der Krol et al., (1991) In Antisense nucleic acids and proteins (Joseph M & van der Krol A, eds.) Marcel Dekker Inc, New York, New York, United States of America, pp. 125-141.
van Hemert et al. (2001) Bioessays 23: 936-946.
van Hille et al. (1993) Biochem Biophys Res. Commun. 197: 15-21.
Vasil et al. (1989) Mol. Microbiol. 3:371.
Vasil et al., (1992) Bio/Technology 10:667-674.
Vasil et al. (1993) Biotechnology 11:1553.
Vazquez-Tello et al. (1998) Mol. Gen. Genet. 257: 157-166.
Villalba et al. (1993) Eur. J. Biochem. 216: 863-869.
Vollbrecht et al. (1991) Nature 350: 241-243.
Vos et al. (2000) Plant Cell 12: 979-990.
Wadzinski et al. (1993) Mol. Cell Biol. 13: 2822-2834.
Walter et al. (1990) J. Biol. Chem. 265: 14016-22.
Warner et al., (1993) Plant J 3:191-201.
Wasmann et al., (1986) Mol Gen Genet 205:446-453.
Watanabe et al. (1994) J. Biol. Chem. 269: 7744-7749.
Waterhouse et al., (1998) Proc Natl Acad Sci U S A 95:13959-13964.
Weeks et al. (1993) Plant Physiol. 102: 1077.
Weissinger et al. (1988) Ann. Rev. Genet. 22: 421.
Welsh, (1981) Fundamentals of Plant Genetics and Breeding, John Wiley & Sons, New York, New York, United States of America.
Wen et al. (1989) Nucl. Acids Res. 17: 9490.
White et al. (1990) Nucl. Acids Res. 18:1062.
Williams et al., (1993) J Clin Invest 92:503-508.
Williams-Carrier et al. (1997) Development 124: 3737-3745.
Winge et al. (1997) Plant Mol. Biol. 35: 483-495.
Wolf & Borchardt (1991) J. Med. Chem. 34: 1521-1530.
Wood, (1983) Crop Breeding, American Society of Agronomy, Madison, Wisconsin, United States of America.
Wricke & Weber, (1986) Quantitative Genetics and Selection Plant Breeding, Walter de Gruyter and Co., Berlin, Germany.
Wu et al. (1995) Mol. Cell. Biol. 15: 2536-2546.
Xia et al. (1996) Plant J. 10: 761-769.
Xiong et al. (2001 a) Genes Dev. 15: 1971-1984.
Xiong et al. (2001 b) Mol. Plant Microbe Interact. 14: 685-692.
Xu et al., (1993) Plant Mol Biol 22:573-588.
Yamaguchi-Shinozaki et al. (1990) Plant Mol. Biol. 14: 29-39.
Yao et al. (2001) Proc. Natl. Acad. Sci. USA 98: 1306-1311.
Yazaki et al. (2001) J. Exp. Bot. 52: 877-9.
Yokota et al. (1999a) Plant Physiol. 119: 231-240.
Yokota et al. (1999b) Plant Physiol. 121: 525-534.
York et al. (1994) Biochemistry 33: 13164-13171.
Yucel (2000) J. Cell. Biol. 150: 1-11.
Zhang et al. (1998) EMBO J. 17: 6404-6411.
Zhao & Last, (1995) J. Biol. Chem. 270: 6081-6087.
Zhao et al., (2000) Plant Mol Biol 44:789-98.
Zhao et al. (2001) EMBO J. 20: 2315-2325.
Zhong et al. (1996) Mol. Gen. Genet. 251:196.
Zhu & Xiao (1998) Nucl. Acids Res. 26: 5402-5408.
Zhu et al., (1999) Proc Natl Acad Sci U S A 96:8768-8773.
Zhu et al. (2001) Plant Physiol. Biochem. 39: 221-242.
Zilliacus et al. (2001) Mol. Endocrinol. 15: 501-511.
Zollman, et al. (1994) Proc. Natl. Acad. Sci. USA 91: 10717-21.

Those skilled in the art will recognize, or be able to ascertain, using no more than routine experimentation, numerous equivalents to the specific embodiments described specifically herein. Such equivalents are intended to be encompassed in the scope of the following claims.

Preferred embodiments of the present invention are described below and are referred to as embodiments E1 to E58:
E1. An isolated nucleic acid molecule encoding a cell proliferation-related polypeptide, wherein the polypeptide binds in a yeast two hybrid assay to a fragment of a protein selected from the group consisting of OsE2F1 (SEQ ID NO: 194), Os018989-4003 (SEQ ID NO: 2), OsE2F2 (SEQ ID NO: 10), OsS49462 (SEQ ID NO: 206), OsCYCOS2 (SEQ ID NO: 210), OsMADS45 (SEQ ID NO: 202), OsRAP1B (SEQ ID NO: 244), OsMADS6 (SEQ ID NO: 236), OsFDRMADS8 (SEQ ID NO: 228), OsMADS3 (SEQ ID NO: 232), OsMADS5 (SEQ ID NO: 234), OsMADS15 (SEQ ID NO: 240), OsHOS59 (SEQ ID NO: 258), OsGF14-c (SEQ ID NO: 278), OsDAD1 (SEQ ID NO: 292), Os006819-2510 (SEQ ID NO: 296), OsCRTC (SEQ ID NO: 300), OsSGT1 (SEQ ID NO: 310), OsERP (SEQ ID NO: 312), OsCHIB1 (SEQ ID NO: 318), OsCS (SEQ ID NO: 322), OsPP2A-2 (SEQ ID NO: 330), and OsCAA90866 (SEQ ID NO: 336).
E2. The isolated nucleic acid molecule of E1, wherein the isolated nucleic acid molecule is derived from rice (*Oryza sativa*).
E 3. The isolated nucleic acid molecule of E 1, wherein the isolated nucleic acid molecule comprises a nucleic acid sequence selected from the group consisting of odd numbered SEQ ID NOs:1-191.
E 4. The isolated nucleic acid molecule of E3, wherein the isolated nucleic acid molecule comprises a nucleic acid sequence of one of odd numbered SEQ ID NOs: 1-7 and the protein comprises an amino acid sequence of SEQ ID NO: 194.
E 5. The isolated nucleic acid molecule of E 3, wherein the isolated nucleic acid molecule comprises a nucleic acid sequence of one of SEQ ID NOs: 9 and 11 and the protein comprises an amino acid sequence of SEQ ID NO: 2.
E 6. The isolated nucleic acid molecule of E3, wherein the isolated nucleic acid molecule comprises a nucleic acid sequence of one of SEQ ID NOs: 1 and 13 and the protein comprises an amino acid sequence of SEQ ID NO: 10.
E 7. The isolated nucleic acid molecule of E 3, wherein the isolated nucleic acid molecule comprises a nucleic acid sequence of one of odd numbered SEQ ID NOs: 15-21 and the protein comprises an amino acid sequence of SEQ ID NO: 206.
E 8. The isolated nucleic acid molecule of E 3, wherein the isolated nucleic acid molecule comprises a nucleic acid sequence of one of odd numbered SEQ ID NOs: 15, 17, 23-53 and the protein comprises an amino acid sequence of SEQ ID NO: 210.
E 9. The isolated nucleic acid molecule of E 3, wherein the isolated nucleic acid molecule comprises a nucleic acid sequence of SEQ ID NO: 55 and the protein comprises an amino acid sequence of SEQ ID NO: 202.
E 10. The isolated nucleic acid molecule of E 3, wherein the isolated nucleic acid molecule comprises a nucleic acid sequence of SEQ ID NO: 57 and the protein comprises an amino acid sequence of SEQ ID NO: 244.
E 11. The isolated nucleic acid molecule of E 3, wherein the isolated nucleic acid molecule comprises a nucleic acid sequence of SEQ ID NO: 59 and the protein comprises an amino acid sequence of SEQ ID NO: 236.
E 12. The isolated nucleic acid molecule of E 3, wherein the isolated nucleic acid molecule comprises a nucleic acid sequence of SEQ ID NO: 61 and the protein comprises an amino acid sequence of SEQ ID NO: 232.
E 13. The isolated nucleic acid molecule of E 3, wherein the isolated nucleic acid molecule comprises a nucleic acid sequence of SEQ ID NO: 63 and the protein comprises an amino acid sequence of SEQ ID NO: 234.
E 14. The isolated nucleic acid molecule of E 3, wherein the isolated nucleic acid molecule comprises a nucleic acid sequence of SEQ ID NO: 65 and the protein comprises an amino acid sequence of SEQ ID NO: 240.
E 15. The isolated nucleic acid molecule of E 3, wherein the isolated nucleic acid molecule comprises a nucleic acid sequence of one of odd numbered SEQ ID NOs: 67-79 and the protein comprises an amino acid sequence of SEQ ID NO: 258.
E 16. The isolated nucleic acid molecule of E 3, wherein the isolated nucleic acid molecule comprises a nucleic acid sequence of SEQ ID NO: 81 and the protein comprises an amino acid sequence of SEQ ID NO: 260.
E 17. The isolated nucleic acid molecule of E 3, wherein the isolated nucleic acid molecule comprises a nucleic acid sequence of one of odd numbered SEQ ID NOs: 83-97 and the protein comprises an amino acid sequence of SEQ ID NO: 278.
E 18. The isolated nucleic acid molecule of E 3, wherein the isolated nucleic acid molecule comprises a nucleic acid sequence of one of SEQ ID NOs: 89 and 99 and the protein comprises an amino acid sequence of SEQ ID NO: 286.
E 19. The isolated nucleic acid molecule of E 3, wherein the isolated nucleic acid molecule comprises a nucleic acid sequence of one of odd numbered SEQ ID NOs: 101-105 and the protein comprises an amino acid sequence of SEQ ID NO: 296.
E 20. The isolated nucleic acid molecule of E 3, wherein the isolated nucleic acid molecule comprises a nucleic acid sequence of SEQ ID NO: 107 and the protein comprises an amino acid sequence of SEQ ID NO: 300.
E 21. The isolated nucleic acid molecule of E 3, wherein the isolated nucleic acid molecule comprises a nucleic acid sequence of SEQ ID NO: 109 and the protein comprises an amino acid sequence of SEQ ID NO: 304.
E 22. The isolated nucleic acid molecule of E 3, wherein the isolated nucleic acid molecule comprises a nucleic acid sequence of one of odd numbered SEQ ID NOs: 111-123 and the protein comprises an amino acid sequence of SEQ ID NO: 310.
E 23. The isolated nucleic acid molecule of E 3, wherein the isolated nucleic acid molecule comprises a nucleic acid sequence of one of odd numbered SEQ ID NOs: 125-147 and the protein comprises an amino acid sequence of SEQ ID NO: 312.
E 24. The isolated nucleic acid molecule of E 3, wherein the isolated nucleic acid molecule comprises a nucleic acid sequence of one of odd numbered SEQ ID NOs: 151-157 and the protein comprises an amino acid sequence of SEQ ID NO: 318.
E 25. The isolated nucleic acid molecule of E 3, wherein the isolated nucleic acid molecule comprises a nucleic acid sequence of one of odd numbered SEQ ID NOs: 159-175 and the protein comprises an amino acid sequence of SEQ ID NO: 322.
E 26. The isolated nucleic acid molecule of E 3, wherein the isolated nucleic acid molecule comprises a nucleic acid sequence of one of odd numbered SEQ ID NOs: 177-185 and the protein comprises an amino acid sequence of SEQ ID NO: 330.
E 27. The isolated nucleic acid molecule of E 3, wherein the isolated nucleic acid molecule comprises a nucleic acid sequence of one of odd numbered SEQ ID NOs: 177, 187-191 and the protein comprises an amino acid sequence of SEQ ID NO: 336.
E 28.An isolated nucleic acid molecule encoding a cell proliferation-related polypeptide, wherein the nucleic acid molecule is selected from the group consisting of:
   (a) a nucleic acid molecule encoding a polypeptide comprising an amino acid sequence of one of even numbered SEQ ID NOs: 2-192;
   (b) a nucleic acid molecule comprising a nucleic acid sequence of one of odd numbered SEQ ID NOs:1-191;
   (c) a nucleic acid molecule that has a nucleic acid sequence at least 90% identical to the nucleic acid sequence of the nucleic acid molecule of (a) or (b) ;
   (d) a nucleic acid molecule that hybridizes to (a) or (b) under conditions of hybridization selected from the group consisting of:
      (i) 7% sodium dodecyl sulfate (SDS), 0.5 M NaPO₄, 1 mM ethylenediamine tetraacetic acid (EDTA) at 50°C with a final wash in 2X standard saline citrate (SSC), 0.1% SDS at 50°C;
      (ii) 7% SDS, 0.5 M NaPO₄, 1 mM EDTA at 50°C with a final wash in 1X SSC, 0.1% SDS at 50°C;
      (iii) 7% SDS, 0.5 M NaPO₄, 1 mM EDTA at 50°C with a final wash in 0.5X SSC, 0.1% SDS at 50°C;
      (iv) 7% sodium dodecyl sulfate (SDS), 0.5 M NaPO₄, 1 mM EDTA at 50°C with a final wash in 0.1X SSC, 0.1% SDS at 50°C; and
      (v) 7% sodium dodecyl sulfate (SDS), 0.5 M NaPO₄, 1 mM EDTA at 50°C with a final wash in 0.1X SSC, 0.1% SDS at 65°C;
   (e) a nucleic acid molecule comprising a nucleic acid sequence fully complementary to (a); and
   (f) a nucleic acid molecule comprising a nucleic acid sequence that is the full reverse complement of (a).
E 29.An isolated cell proliferation-related polypeptide encoded by the isolated nucleic acid molecule of E 28, or a functional fragment, domain, or feature thereof.
E 30. A method for producing a polypeptide of E 29, comprising the steps of:
   (a) growing cells comprising an expression cassette under suitable growth conditions, the expression cassette comprising a nucleic acid molecule of E 28; and
   (b) isolating the polypeptide from the cells.
E 31. A transgenic plant cell comprising an isolated nucleic acid molecule of E 1.
E 32. The transgenic plant of E 31, wherein the plant is selected from the group consisting of corn (Zea mays), *Brassica* sp., alfalfa (Medicago sativa), rice (Oryza sativa ssp.), rye (Secale cereale), sorghum (Sorghum bicolor, Sorghum vulgare), pearl millet (Pennisetum glaucum), proso millet (Panicum miliaceum), foxtail millet (Setaria italica), finger millet (Eleusine coracana), sunflower (Helianthus annuus), safflower (Carthamus tinctorius), wheat (Triticum aestivum), soybean (Glycine max), tobacco (Nicotiana tabacum), potato (Solanum tuberosum), peanut (Arachis hypogaea), cotton, sweet potato (Ipomoea batatus), cassava (Manihot esculenta), coffee (Cofea spp.), coconut (Cocos nucifera), pineapple (Ananas comosus), citrus trees (Citrus spp.), cocoa (Theobroma cacao), tea (Camellia sinensis), banana (Musa spp.), avocado (Persea ultilane), fig (Ficus casica), guava (Psidium guajava), mango (Mangifera indica), olive (Olea europaea), papaya (Carica papaya), cashew (Anacardium occidentale), macadamia (Macadamia integrifolia), almond (Prunus amygdalus), sugar beets (Beta vulgaris), sugarcane (Saccharum spp.), oats, duckweed (Lemna), barley, a vegetable, an ornamental, and a conifer.
E 33. The transgenic plant of E 32, wherein the plant is rice (Oryza sativa ssp.)
E 34. The transgenic plant of E 32, wherein the duckweed is selected from the group consisting of genus *Lemna*, genus *Spirodela*, genus *Woffia*, and genus *Wofiella*.
E 35. The transgenic plant of E 32, wherein the vegetable is selected from the group consisting of tomatoes, lettuce, guar, locust bean, fenugreek, soybean, garden beans, cowpea, mungbean, lima bean, fava bean, lentils, chickpea, green bean, lima bean, pea, and members of the genus *Cucumis*.
E 36. The transgenic plant of E 32, wherein the ornamental is selected from the group consisting of impatiens, Begonia, Pelargonium, Viola, Cyclamen, Verbena, Vinca, Tagetes, Primula, Saint Paulia, Agertum, Amaranthus, Antihirrhinum, Aquilegia, Cineraria, Clover, Cosmo, Cowpea, Dahlia, Datura, Delphinium, Gerbera, Gladiolus, Gloxinia, Hippeastrum, Mesembryanthemum, Salpiglossos, and Zinnia, azalea, hydrangea, hibiscus, rose, tulip, daffodil, petunia, carnation, poinsettia, and chrysanthemum.
E 37. The transgenic plant of E 32, wherein the conifer is selected from the group consisting of loblolly pine, slash pine, ponderosa pine, lodgepole pine, Monterey pine, Douglas-fir, Western hemlock, Sitka spruce, redwood, silver fir, balsam fir, Western red cedar, and Alaska yellow-cedar.
E 38. The transgenic plant of E 31, wherein the transgenic plant is a plant selected from the group consisting of Acacia, aneth, artichoke, arugula, blackberry, canola, cilantro, clementines, escarole, eucalyptus, fennel, grapefruit, honey dew, jicama, kiwifruit, lemon, lime, mushroom, nut, okra, orange, parsley, persimmon, plantain, pomegranate, poplar, radiata pine, radicchio, Southern pine, sweetgum, tangerine, triticale, vine, yams, apple, pear, quince, cherry, apricot, melon, hemp, buckwheat, grape, raspberry, chenopodium, blueberry, nectarine, peach, plum, strawberry, watermelon, eggplant, pepper, cauliflower, *Brassica*, broccoli, cabbage, ultilan sprouts, onion, carrot, leek, beet, broad bean, celery, radish, pumpkin, endive, gourd, garlic, snapbean, spinach, squash, turnip, ultilane, and zucchini.
E 39. An isolated cell proliferation-related polypeptide, wherein the polypeptide binds in a yeast two hybrid assay to a fragment of a protein selected from the group consisting of OsE2F1 (SEQ ID NO: 194), Os018989-4003 (SEQ ID NO: 2), OsE2F2 (SEQ ID NO: 10), OsS49462 (SEQ ID NO: 206), OsCYCOS2 (SEQ ID NO: 210), OsMADS45 (SEQ ID NO: 202), OsRAP1B (SEQ ID NO: 244), OsMADS6 (SEQ ID NO: 236), OsFDRMADS8 (SEQ ID NO: 228), OsMADS3 (SEQ ID NO: 232), OsMADS5 (SEQ ID NO: 234), OsMADS15 (SEQ ID NO: 240), OsHOS59 (SEQ ID NO: 258), OsGF14-c (SEQ ID NO: 278), OsDAD1 (SEQ ID NO: 292), Os006819-2510 (SEQ ID NO: 296), OsCRTC (SEQ ID NO: 300), OsSGT1 (SEQ ID NO: 310), OsERP (SEQ ID NO: 312), OsCHIB1 (SEQ ID NO: 318), OsCS (SEQ ID NO: 322), OsPP2A-2 (SEQ ID NO: 330), and OsCAA90866 (SEQ ID NO: 336).
E 40. The isolated cell proliferation-related polypeptide of E 39, wherein the isolated proliferation-related polypeptide is selected from the group consisting of:
   (a) a polypeptide comprising an amino acid sequence of even numbered SEQ ID NOs: 2-192; and
   (b) a polypeptide comprising an amino acid sequence at least 80% similar to the polypeptide of (a) using the GCG Wisconsin Package SEQWEB® application of GAP with the default GAP analysis parameters.
E 41. The isolated cell proliferation-related polypeptide of E 40, wherein the polypeptide comprises an amino acid sequence of one of even numbered SEQ ID NOs: 2-192.
E 42. An expression cassette comprising a nucleic acid molecule encoding a cell proliferation-related polypeptide of E 1.
E 43. The expression cassette of E 42, wherein the nucleic acid molecule encoding a cell proliferation-related polypeptide comprises a nucleic acid sequence selected from odd numbered SEQ ID NQs:1-191.
E 44. The expression cassette of E 42, wherein the expression cassette further comprises a regulatory element operatively linked to the nucleic acid molecule.
E 45. The expression cassette of E 44, wherein the regulatory element comprises a promoter.
E 46. The expression cassette of E 45, wherein the promoter is a plant promoter.
E 47. The expression cassette of E 45, wherein the promoter is a constitutive promoter.
E 48. The expression cassette of E 45, wherein the promoter is a tissue-specific or a cell type-specific promoter.
E 49. The expression cassette of E 48, wherein the tissue-specific or cell type-specific promoter directs expression of the expression cassette in a location selected from the group consisting of epidermis, root, vascular tissue, meristem, cambium, cortex, pith, leaf, flower, seed, and combinations thereof.
E 50.A transgenic plant cell comprising the expression cassette of E 42.
E 51. The transgenic plant cell of E 50, wherein the isolated nucleic acid molecule comprises a nucleic acid sequence of one of odd numbered SEQ ID NOs:1-191.
E 52. A transgenic plant comprising the expression cassette of E 42.
E 53. Transgenic seeds or progeny of the trangenic plant of E 52.
E 54. A method for modulating proliferation of a plant cell comprising introducing into the plant cell an expression cassette comprising an isolated nucleic acid molecule encoding a cell proliferation-related polypeptide, wherein the polypeptide binds in a yeast two hybrid assay to a fragment of a protein selected from the group consisting of OsE2F1 (SEQ ID NO: 194), OsO18989-4003 (SEQ ID NO: 2), OsE2F2 (SEQ ID NO: 10), OsS49462 (SEQ ID NO: 206), OsCYCOS2 (SEQ ID NO: 210), OsMADS45 (SEQ ID NO: 202), OsRAP1B (SEQ ID NO: 244), OsMADS6 (SEQ ID NO: 236), OsFDRMADS8 (SEQ ID NO: 228), OsMADS3 (SEQ ID NO: 232), OsMADS5 (SEQ ID NO: 234), OsMADS15 (SEQ ID NO: 240), OsHOS59 (SEQ ID NO: 258), OsGF14-c (SEQ ID NO: 278), OsDAD1 (SEQ ID NO: 292), Os006819-2510 (SEQ ID NO: 296), OsCRTC (SEQ ID NO: 300), OsSGT1 (SEQ ID NO: 310), OsERP (SEQ ID NO: 312), OsCHIB1 (SEQ ID NO: 318), OsCS (SEQ ID NO: 322), OsPP2A-2 (SEQ ID NO: 330), and OsCAA90866 (SEQ ID NO: 336).
E 55. The method of E 54, wherein expression of the polypeptide in the cell results in an enhancement of a rate or extent of proliferation of the cell.
E 56. The method of E 54, wherein expression of the polypeptide in the cell results in a decrease in a rate or extent of proliferation of the cell.
E 57. The method of E 54, wherein the isolated nucleic acid molecule comprises a nucleic acid sequence selected from one of odd numbered SEQ ID NOs: 1-339.
E 58. The method of E 57, wherein the isolated nucleic acid molecule comprises a nucleic acid sequence selected from one of odd numbered SEQ ID NOs:1-191.

## Claims

1. An isolated nucleic acid molecule encoding a cell proliferation-related polypeptide, wherein the polypeptide binds in a yeast two hybrid assay to a fragment of the protein OsCAA90866 (SEQ ID NO: 336).

2. The isolated nucleic acid molecule of claim 1, wherein the isolated nucleic acid molecule is derived from rice (*Oryza sativa*).

3. The isolated nucleic acid molecule of claim 1, wherein the isolated nucleic acid molecule comprises a nucleic acid sequence selected from the group consisting of SEQ ID NOs: 191, 177, 187, 189, 335, 337, and 339.

4. An isolated nucleic acid molecule encoding a cell proliferation-related polypeptide, wherein the nucleic acid molecule is selected from the group consisting of:
(a) a nucleic acid molecule encoding a polypeptide comprising an amino acid sequence of one of SEQ ID NOs: 192, 178, 188, 190, 336, 338, and 340;
(b) a nucleic acid molecule comprising a nucleic acid sequence of one of SEQ ID NOs: 191, 177, 187, 189, 335, 337, and 339;
(c) a nucleic acid molecule that has a nucleic acid sequence at least 90% identical to the nucleic acid sequence of the nucleic acid molecule of (a) or (b);
(d) a nucleic acid molecule that hybridizes to (a) or (b) under conditions of hybridization selected from the group consisting of:
(i) 7% sodium dodecyl sulfate (SDS), 0.5 M NaP0₄, 1 mM ethylenediamine tetraacetic acid (EDTA) at 50°C with a final wash in 2X standard saline citrate (SSC), 0.1% SDS at 50°C;
(ii) 7% SDS, 0.5 M NaP0₄, 1 mM EDTA at 50°C with a final wash in 1X SSC, 0.1 % SDS at 50°C;
(iii) 7% SDS, 0.5 M NaP0₄, 1 mM EDTA at 50°C with a final wash in 0.5X SSC, 0.1% SDS at 50°C;
(iv) 7% sodium dodecyl sulfate (SDS), 0.5 M NaP0₄, 1 mM EDTA at 50°C with a final wash in 0.1X SSC, 0. 1% SDS at 50°C ; and (v) 7% sodium dodecyl sulfate (SDS), 0.5 M NaP0₄, 1 mM EDTA at 50°C with a final wash in 0.1X SSC, 0.1% SDS at 65°C;
(e) a nucleic acid molecule comprising a nucleic acid sequence fully complementary to (a); and
(f) a nucleic acid molecule comprising a nucleic acid sequence that is the full reverse complement of (a).

5. An isolated cell proliferation-related polypeptide encoded by the isolated nucleic acid molecule of claim 4, or a functional fragment thereof.

6. An isolated cell proliferation-related polypeptide, wherein the polypeptide binds in a yeast two hybrid assay to a fragment of the protein OsCAA90866 (SEQ ID NO: 336).

7. The isolated cell proliferation-related polypeptide of claim 6, wherein the isolated proliferation-related polypeptide is selected from the group consisting of:
(a) a polypeptide comprising an amino acid sequence of SEQ ID NOs: 192, 178, 188, 190, 336, 338, and 340; and
(b) a polypeptide comprising an amino acid sequence at least 80% similar to the polypeptide of (a).

8. The isolated cell proliferation-related polypeptide of claim 7, wherein the polypeptide comprises an amino acid sequence of one of SEQ ID NOs: 192, 178, 188, 190, 336, 338, and 340.

9. An expression cassette comprising an isolated nucleic acid molecule encoding a cell proliferation-related polypeptide of claim 1.

10. The expression cassette of claim 9, comprising a regulatory element further comprising a promoter, wherein the promoter is a tissue-specific or cell type-specific promoter.

11. A transgenic plant cell comprising the expression cassette of claim 9.

12. The transgenic plant cell of claim 11, wherein the plant is selected from the group consisting of corn (Zea mays), Brassica sp., alfalfa (Medicago sativa), rice (Oryza sativa ssp.), rye (Secale cereale), sorghum (Sorghum bicolor, Sorghum vulgare), pearl millet (Pennisetum glaucum), proso millet (Panicum miliaceum), foxtail millet (Setaria italic), finger millet (Eleusine coracana), sunflower (Helianthus annuus), safflower (Carthamus tinctorius), wheat (Triticum aestivum), soybean (Glycine max), tobacco (Nicotiana tabacum), potato (Solanum tuberosum), peanut (Arachis hypogaea), cotton, sweet potato (Ipomoea batatus), cassava (Manihot esculenta), coffee (Cofea spp.), coconut (Cocos nucifera), pineapple (Ananas comosus), citrus trees (Citrus spp.), cocoa (Theobroma cacao), tea (Camellia sinensis), banana (Musa spp.), avocado (Persea ultilane), fig (Ficus casica), guava (Psidium guajava), mango (Mangifera indica), olive (Olea europaea), papaya (Carica papaya), cashew (Anacardium occidentale), macadamia (Macadamia integrifolia), almond (Prunus amygdalus), sugar beets (Beta vulgaris), sugarcane (Saccharum spp.), oats, duckweed (Lemna), barley, a vegetable, an ornamental, and a conifer.

13. The transgenic plant cell of claim 12, wherein the plant is rice (Oryza sativa ssp.).

14. The transgenic plant cell of claim 12, wherein the duckweed is selected from the group consisting of genus Lemna, genus Spirodela, genus Woffia, and genus Wofiella.

15. The transgenic plant cell of claim 12, wherein the vegetable is selected from the group consisting of tomatoes, lettuce, guar, locust bean, fenugreek, soybean, garden beans, cowpea, mungbean, lima bean, fava bean, lentils, chickpea, green bean, lima bean, pea, and members of the genus Cucumis.

16. The transgenic plant cell of claim 12, wherein the ornamental is selected from the group consisting of impatiens, Begonia, Pelargonium, Viola, Cyclamen, Verbena, Vinca, Tagetes, Primula, Saint Paulia, Agertum, Amaranthus, Antihirrhinum, Aquilegia, Cineraria, Clover, Cosmo, Cowpea, Dahlia, Datura, Delphinium, Gerbera, Gladiolus, Gloxinia, Hippeastrum, Mesembryanthemum, Salpiglossos, and Zinnia, azalea, hydrangea, hibiscus, rose, tulip, daffodil, petunia, carnation, poinsettia, and chrysanthemum.

17. The transgenic plant cell of claim 12, wherein the conifer is selected from the group consisting of loblolly pine, slash pine, ponderosa pine, lodgepole pine, Monterey pine, Douglas-fir, Western hemlock, Sitka spruce, redwood, silver fir, balsam fir, Western red cedar, and Alaska yellow-cedar.

18. The transgenic plant cell of claim 12, wherein the transgenic plant is a plant selected from the group consisting of Acacia, aneth, artichoke, arugula, blackberry, canola, cilantro, clementines, escarole, eucalyptus, fennel, grapefruit, honey dew, jicama, kiwifruit, lemon, lime, mushroom, nut, okra, orange, parsley, persimmon, plantain, pomegranate, poplar, radiata pine, radicchio, Southern pine, sweetgum, tangerine, triticale, vine, yams, apple, pear, quince, cherry, apricot, melon, hemp, buckwheat, grape, raspberry, chenopodium, blueberry, nectarine, peach, plum, strawberry, watermelon, eggplant, pepper, cauliflower, Brassica, broccoli, cabbage, ultilan sprouts, onion, carrot, leek, beet, broad bean, celery, radish, pumpkin, endive, gourd, garlic, snapbean, spinach, squash, turnip, ultilane, and zucchini.

19. The transgenic plant cell of claim 11, wherein the isolated nucleic acid molecule comprises a nucleic acid sequence of one of SEQ ID NOs: 191, 177, 187, 189, 335, 337, and 339.

20. A method for modulating the response of the plant cell to an environmental challenge comprising introducing into the plant cell an expression cassette comprising an isolated nucleic acid molecule encoding a cell proliferation-related polypeptide, wherein the polypeptide binds in a yeast two hybrid assay to a fragment of the protein OsCAA90866 (SEQ ID NO: 336).
